(19) Europäisches Patentamt
European Patent Office
Office européen des brevets

(11) EP 0 949 238 A1

(12) # EUROPEAN PATENT APPLICATION
published in accordance with Art. 158(3) EPC

(43) Date of publication:
**13.10.1999 Bulletin 1999/41**

(21) Application number: **97950426.3**

(22) Date of filing: **26.12.1997**

(51) Int. Cl.$^6$: **C07C 62/38**, C07C 69/716, C07C 235/06, C07C 235/08, C07C 235/10, C07C 309/17, C07C 311/23, C07C 317/18, C07C 323/11, C07D 319/12, C07D 317/58, C07D 317/54, C07D 295/20, C07D 257/04, C07D 263/56, C07D 277/62, C07D 277/68, C07D 207/16, C07F 9/38, A61K 31/335, A61K 31/495, A61K 31/19, A61K 31/215, A61K 31/275, A61K 31/18

(86) International application number:
**PCT/JP97/04879**

(87) International publication number:
**WO 98/29380 (09.07.1998 Gazette 1998/27)**

(84) Designated Contracting States:
**AT BE CH DE DK ES FI FR GB GR IE IT LI LU MC NL PT SE**

(30) Priority: **27.12.1996 JP 35067396**

(71) Applicant:
**DAIICHI PHARMACEUTICAL CO., LTD.**
**Chuo-ku, Tokyo 103 (JP)**

(72) Inventors:
- **USUI, Hiroyuki;**
  **Daiichi Pharm. Co., Ltd.**
  **Edogawa-ku Tokyo 134 (JP)**
- **KAGECHIKA, Katsuji**
  **Daiichi Pharm. Co., Ltd.,**
  **Edogawa-ku;Tokyo 134 (JP)**
- **NAGASHIMA, Hajime**
  **Daiichi Pharm. Co., Ltd.,**
  **Edogawa-ku;Tokyo 134 (JP)**

(74) Representative:
**Hartz, Nikolai F., Dr. et al**
**Wächtershäuser & Hartz,**
**Patentanwälte,**
**Tal 29**
**80331 München (DE)**

(54) **SUBSTITUTED PROPIONYL DERIVATIVES**

(57) The present invention relates to a compound represented by the following formula (1):

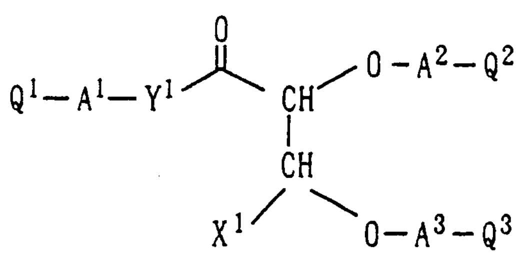

(1)

[wherein, $X^1$ represents a carboxyl group which may be esterified or the like group;

EP 0 949 238 A1

$Y^1$ represents a single bond, -O- or -N($R^1$)-;
at least one of $A^1$, $A^2$ and $A^3$ is a group represented by the following formula (2):

$$-R^2\text{-}a^1\text{-}R^3\text{-}a^2 \rightarrow \quad (2)$$

{wherein, $R^2$ represents a divalent $C_{2\text{-}12}$ hydrocarbon group, $R^3$ represents a single bond or a divalent $C_{1\text{-}12}$ hydrocarbon group, $a^1$ and $a^2$ individually represent a single bond, -S-, -SO-, -$SO_2$-, -$SO_2$NH-, -O-, -N($R^4$)-, -CON($R^5$)-, -C(=O)- or - Si($R^6$)($R^7$)- and → means bonding with $Q^1$, $Q^2$ or $Q^3$}, the remaining one or two of $A^1$, $A^2$ and $A^3$ are the same or different and each independently represents a group represented by the following formula (3):

$$-R^8\text{-}a^3\text{-}R^9\text{-}a^4 \rightarrow \quad (3)$$

{wherein, $R^8$ and $R^9$ individually represent a single bond or a divalent $C_{1\text{-}12}$ hydrocarbon group, $a^3$ and $a^4$ individually represent a single bond, -S-, -SO-, -$SO_2$-, -$SO_2$NH-, -O-, - N($R^{10}$)-, -CON($R^{11}$)-, -C(=O)- or -SI($R^{12}$)($R^{13}$)- and → means bonding with $Q^1$, $Q^2$ or $Q^3$},;
at least one of $Q^1$, $Q^2$ and $Q^3$ represents a cyclic hydrocarbon group or heterocyclic group and the remaining one or two of $Q^1$, $Q^2$ and $Q^3$ individually represent a hydrogen atom, a carboxyl group which may be esterified, a hydrocarbon group or a heterocyclic group] or salt thereof; and a pharmaceutical comprising the same as an effective ingredient. The compound exhibits strong squalene synthetase inhibitory action and is therefore useful as a pharmaceutical for the treatment • prevention of hypercholesterolemia, hyperlipemia or arteriosclerosis.

## Description

### Technical Field

The present invention relates to substituted propionyl derivatives, more specifically, substituted propionyl derivatives which usefully serve as pharmaceuticals for the treatment • prevention of hypercholesterolemia, hyperlipemia or arteriosclerosis based on their squalene synthetase inhibitory action.

### Background Art

Westernization of dietary habit and an increase in the aged population in recent years lead to a high incidence of arteriosclerosis in general and, as a result, increase heart and cerebral diseases in particular. Such vascular diseases are now anticipated to raise serious social problems in the coming aged society. Hypercholesterolemia is widely accepted as a major risk factor for the development of arteriosclerosis, and it is known that cholesterol lowering agents are effective for the treatment of arteriosclerosis.

At present, hydroxymethylglutaryl coenzyme A (HMG-CoA) reductase inhibitors such as lovastatin (U.S. Patent No. 4231938), pravastatin (U.S. Patent No. 4346227) and simvastatin (U.S. Patent No. 4444784) are mainly used as a cholesterol lowering agent.

HMG-CoA reductase is a rate-limiting enzyme for cholesterol biosynthesis, and is located relatively upstream of the metabolic pathway.

Farnesyl pyrophosphate is a key metabolic intermediate produced at relatively late stage in the pathway, then several steps convert farnesyl pyrophosphate into cholesterol. Examples of the metabolite converted from Farnesyl pyrophosphate include non-sterol isoprenoids such as ubiquinone, dolichol and heme A. The non-sterol isoprenoid products have important biological functions; for example, ubiquinone plays a pivotal role as the mitochondrial respiratory chain element and dolichol is prerequisite for a long chain alcohol taking part in the synthesis of glycoprotein. Inhibition of HMG-CoA reductase can block, in principle, not only cholesterol synthesis, but also synthesis of these essential elements and, therefore, could cause side effects. The HMG-CoA reductase inhibitor is therefore not always the best cholesterol lowering agent.

In order to control the biosynthesis of cholesterol without impairing the biosynthesis of essential elements for the body such as ubiquinone and dolichol, it is desired to inhibit enzymes existing downstream, as compared with farnesyl pyrophosphate, in the biosynthesis pathway of cholesterol. It is particularly desired to inhibit squalene synthetase which controls the first committed step en route to choresterol and is responsible for the synthesis of sterols. The compounds presently known as squalene synthetase inhibitors include naturally occurring ones such as zaragozic acids (Tetrahedron, 48 (47) 10221-10226 (1992)), and squalestatins (The Journal of Antibiotics, 45 (5) 639-647 (1992)), and synthetic ones such as 4,1-benzoxazepin derivatives (European Patent No. 567026), substituted amidic acid derivatives (European Patent No. 611749) and α-phosphonosulfonic acid derivatives (Journal of Medicinal Chemistry, 39, 661-664 (1996)). However, none of these compounds has established their sufficient effects for inhibitory activity of cholesterol biosynthesis and/or lowering cholesterol in blood when administered orally. In addition, selectiveity of their action has not yet been elucidated so that their use as pharmaceuticals are open to question.

### Disclosure of the Invention

With a view to providing a remedy • preventive for hypercholesterolemia, hyperlipemia or arteriosclerosis due to inhibition of squalene synthetase, thereby exhibiting cholesterol synthesis inhibitory action or blood cholesterol level lowering action, the present inventors have proceeded with an extensive investigation. As a result, it has been found that the object can be attained by the compound represented by the below-described formula (1), leading to the completion of the present invention.

The present invention therefore provides a substituted propionyl derivative represented by the following formula (1):

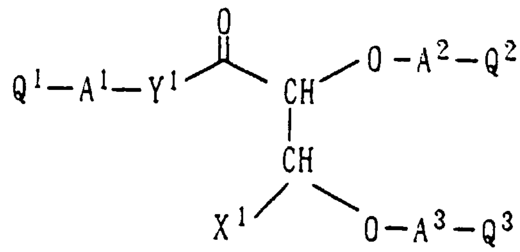

(1)

[wherein, $X^1$ represents a carboxyl, tetrazol-5-yl, phosphonic acid or sulfonic acid group which may be esterified;

$Y^1$ represents a single bond, -O- or $-N(R^1)-$ {wherein $R^1$ represents a hydrogen atom, a hydroxyl group or a substituted or unsubstituted hydrocarbon group};
at least one of $A^1$, $A^2$ and $A^3$ represents a group represented by the following formula (2):

$$-R^2-a^1-R^3-a^2 \rightarrow \quad (2)$$

{wherein, $R^2$ represents a substituted or unsubstituted, divalent hydrocarbon group having 2 to 12 carbon atoms, $R^3$ represents a single bond or a substituted or unsubstituted, divalent hydrocarbon groups having 1 to 12 carbon atoms, $a^1$ and $a^2$ individually represent a single bond, -S-, -SO-, - $SO_2$-, $-SO_2NH-$, -O-, $-N(R^4)-$ {wherein $R^4$ represents a hydrogen atom, a hydroxyl group or a substituted or unsubstituted hydrocarbon group}, $-CON(R^5)-$ {wherein $R^5$ represents a a hydrogen atom, a hydroxyl group or a substituted or unsubstituted hydrocarbon group}, -C(=O)- or $-Si(R^6)(R^7)-${wherein $R^6$ and $R^7$ individually represent a substituted or unsubstituted hydrocarbon group}, and → means bonding with $Q^1$, $Q^2$ or $Q^3$}, the remaining one or two of $A^1$, $A^2$ and $A^3$ are the same or different and each independently represents a group represented by the following formula (3):

$$-R^8-a^3-R^9-a^4 \rightarrow \quad (3)$$

{wherein, $R^8$ and $R^9$ individually represent a single bond or a substituted or unsubstituted, divalent hydrocarbon group having 1 to 12 carbon atoms, $a^3$ and $a^4$ individually represent a single bond, -S-, -SO-, $-SO_2-$, $-SO_2NH-$, -O-, $-N(R^{10})-${wherein $R^{10}$ represents a hydrogen atom, a hydroxyl group or a substituted or unsubstituted hydrocarbon group}, - $CON(R^{11})$- {wherein $R^{11}$ represents a hydrogen atom, a hydroxyl group or a substituted or unsubstituted hydrocarbon group], -C(=O)- or $-Si(R^{12})(R^{13})-$ {wherein $R^{12}$ and $R^{13}$ individually represent a substituted or unsubstituted hydrocarbon group}, and → means bonding with $Q^1$, $Q^2$ or $Q^3$},
at least one of $Q^1$, $Q^2$ and $Q^3$ represents a substituted or unsubstituted cyclic hydrocarbon group or a substituted or unsubstituted heterocyclic group and the remaining one or two of $Q^1$, $Q^2$ and $Q^3$ individually represent a hydrogen atom, a carboxyl group which may be esterified, a substituted or unsubstituted hydrocarbon group or a substituted or unsubstituted heterocyclic group], or salt thereof.

The present invention also provides a pharmaceutical which comprises as an effective ingredient a substituted propionyl derivative represented by the above-described formula (1) or salt thereof.
The present invention also provides a pharmaceutical composition which comprises a substituted propionyl derivative represented by the above-described formula (1) or salt thereof, and a pharmaceutically acceptable carrier.
The present invention also provides the use of a substituted propionyl derivative represented by the above-described formula (1) or salt thereof as a pharmaceutical.
The present invention also provides the treating method of hypercholesterolemia, hyperlipemia or arteriosclerosis, which comprises administering a substituted propionyl derivative represented by the above-described formula (1) or salt thereof.

**Best Modes for Carrying out the Invention**

In the formula (1) which represents the substituted propionyl derivative of the present invention, examples of the carboxyl group which is represented by $X^1$, $Q^1$, $Q^2$ or $Q^3$ and may be esterified include, in addition to a carboxyl group, alkoxycarbonyl, alkenyloxycarbonyl, alkanoyloxyalkoxycarbonyl, alkoxycarbonyloxyalkoxycarbonyl and carbamoyloxyalkoxycarbonyl groups.
Examples of the alkoxycarbonyl group include $C_{1-7}$ alkoxycarbonyl groups such as methoxycarbonyl, ethoxycarbonyl, n-propoxycarbonyl, isopropoxycarbonyl, n-butoxycarbonyl, sec-butoxycarbonyl and tert-butoxycarbonyl. Examples of the alkenyloxycarbonyl group include $C_{2-7}$ alkenyloxycarbonyl groups such as allyloxycarbonyl and 2-butenyloxycarbonyl. Examples of the alkanoyloxyalkoxycarbonyl group include ($C_{2-7}$ alkanoyloxy)-($C_{1-7}$ alkoxycarbonyl) groups such as acetoxymethoxycarbonyl, 1-acetoxyethoxycarbonyl, pivaloyloxymethoxycarbonyl and 1-pivaloyloxyethoxycarbonyl. Examples of the alkoxycarbonyloxyalkoxycarbonyl group include ($C_{1-7}$ alkoxycarbonyloxy)-($C_{1-7}$ alkoxycarbonyl) groups such as 1-(ethoxycarbonyloxy)ethoxycarbonyl and 1-(cyclohexylcarbonyloxy)ethoxycarbonyl. Examples of the carbamoyloxyalkoxycarbonyl group include carbamoyloxy($C_{1-7}$ alkoxycarbonyl) groups such as carbamoyloxymethoxycarbonyl. In addition to them, (5-methyl-2-oxo-1,3-dioxol-4-yl)methoxycarbonyl group can also be mentioned as one example.

Examples of $X^1$ include, in addition to the carboxyl group which may be esterified, tetrazol-5-yl group, phosphonic acid group ($-PO_3H_2$) and sulfonic acid group ($-SO_3H$). Among them, the carboxyl group which may be esterified ($-COOR^{14}$: wherein $R^{14}$ is a hydrogen atom or an ester residue of the above-exemplified esterified carboxyl group) is preferred. Particularly preferred as $X^1$ are carboxyl and alkoxycarbonyl groups.

Examples of the substituted or unsubstituted hydrocarbon group which is represented by $R^1$, $R^4$, $R^5$, $R^6$, $R^7$, $R^{10}$, $R^{11}$, $R^{12}$, $R^{13}$, $Q^1$, $Q^2$ or $Q^3$ include substituted or unsubstituted, saturated or unsaturated hydrocarbon groups, more specifically, substituted or unsubstituted alkyl, alkenyl, alkynyl, cyclic alkyl, cyclic alkenyl, aryl, aralkyl and arylalkenyl groups.

As the alkyl group, linear or branched $C_{1-7}$ alkyl groups are preferred. Examples include methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1-ethylpropyl, n-hexyl, iso-hexyl, 1,1-dimethylbutyl, 2,2-dimethylbutyl, 3,3-dimethylbutyl, 1-ethylbutyl, 2-ethylbutyl, n-hexyl and n-heptyl groups, of which methyl, ethyl, n-propyl, isopropyl, n-butyl, isobutyl and neopentyl groups are particularly preferred.

As the alkenyl group, linear or branched $C_{2-7}$ alkenyl groups are preferred. Examples include vinyl, allyl, isopropenyl, 1-propenyl, 2-methyl-1-propenyl, 2-methyl-2-propenyl, 1-butenyl, 2-butenyl, 3-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and 2-ethyl-1-butenyl groups, of which allyl, isopropenyl and 3-methyl-2-butenyl groups are particularly preferred.

As the alkynyl group, linear or branched $C_{2-7}$ alkynyl groups are preferred. Examples include ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl groups, of which ethynyl, 1-propynyl and 2-propynyl groups are particularly preferred.

As the cyclic alkyl group, cyclic $C_{3-7}$ alkyl groups are preferred, of which cyclopropyl, cyclobutyl and cyclopentyl groups are particularly preferred.

As the cyclic alkenyl group, cyclic $C_{3-7}$ alkenyl groups are preferred, of which 2-cyclopenten-1-yl, 3-cyclopenten-1-yl, 2-cyclohexen-1-yl and 3-cyclohexen-1-yl groups are particularly preferred.

Examples of the aryl group include monocyclic or fused polycyclic $C_{6-18}$ aryl groups such as phenyl, naphthyl, anthryl and phenanthryl groups, of which phenyl, 1-naphthyl and 2-naphthyl groups are particularly preferred.

Examples of the aralkyl and arylalkenyl groups include monocyclic or condensed polycyclic $C_{7-18}$ aryl groups having $C_{1-7}$, alkyl and $C_{1-7}$ alkenyl groups bonded thereto, respectively. Specific examples include benzyl, phenethyl, 3-phenylpropyl, 4-phenylbutyl, 5-phenylpentyl, 6-phenylhexyl, 7-phenylheptyl, 1-naphthylmethyl, 2-naphthylmethyl, 2-(1-naphthyl)ethyl, 2-(2-naphthyl)ethyl, 3-(1-naphthyl)propyl, 3-(2-naphthyl)propyl, 4-(1-naphthyl)butyl, 4-(2-naphthyl)butyl, 5-(1-naphthyl)pentyl, 5-(2-naphthyl)pentyl, 6-(1-naphthyl)hexyl, 6-(2-naphthyl)hexyl, 7-(1-naphthyl)heptyl, 7-(2-naphthyl)heptyl, 3-phenylallyl, 3-(1-naphthyl)allyl and 3-(2-naphthyl)allyl groups, of which aralkyl groups are preferred, with benzyl, phenethyl, 3-phenylpropyl, 2-naphthylmethyl, 2-(2-naphthyl)ethyl and 3-(2-naphthyl)propyl groups being particularly preferred.

Examples of the substituent which the above-exemplified hydrocarbon group may have include hydroxyl group, nitro group, cyano group, halogen atoms, amino group, alkylamino groups, substituted or unsubstituted carbamoyl groups, alkanoyl groups, alkyl groups, alkoxyl groups, carboxyl groups which may be esterified, aryl groups, aryloxy groups and aroyl groups and 1 to 5 substituents are optionally selected from them. Here, examples of the halogen atom include fluorine, chlorine, bromine and iodine atoms. Examples of the alkylamino group include mono- and di-alkylamino groups, more specifically, mono- and di-$C_{1-7}$ alkylamino groups such as methylamino, ethylamino, propylamino, isopropylamino, n-butylamino, dimethylamino, diethylamino, ethylamino groups. Examples of the carbamoyl group which may have a substituent include carbamoyl groups which may be substituted with a $C_{1-7}$ alkyl and/or hydroxyl group, such as carbamoyl, N-methylcarbamoyl, N-ethylcarbamoyl, N-propylcarbamoyl, N,N-dimethylcarbamoyl, N-hydroxycarbamoyl, N-hydroxy-N-methylcarbamoyl and N-hydroxy-N-ethylcarbamoyl groups. Examples of the alkanoyl group include $C_{1-7}$ alkanoyl groups such as formyl, acetyl, propionyl, butyryl, isobutyryl, valeryl, isovaleryl, pivaloyl and hexanoyl. Examples of the alkyl group include $C_{1-7}$ alkyl groups such as methyl, ethyl, n-propyl, isopropyl and tert-butyl. Examples of the alkoxyl group include $C_{1-7}$ alkoxyl groups such as methoxyl, ethoxyl, propoxyl, isopropoxyl and tert-butoxyl. Examples of the carboxyl group which may be esterified include, in addition to a carboxyl group,

$C_{1-7}$ alkoxycarbonyl groups such as methoxycarbonyl, ethoxycarbonyl, propoxycarbonyl, isopropoxycarbonyl and tert-butoxycarbonyl. Examples of the aryl group include phenyl and naphthyl. Examples of the aryloxy group include phenoxy and naphthoxy, while those of the aroyl group include benzoyl, toluoyl and naphthoyl.

Examples of the substituted or unsubstituted, divalent hydrocarbon group having 2 to 12 carbon atoms include linear or branched alkylene, linear or branched alkenylene, linear or branched alkynylene and arylene groups, of which those having 2 to 10 carbon atoms are preferred, with those having 2 to 8 carbon atoms being particularly preferred and those having 3 to 8 carbon atoms being still more preferred. Examples of the linear or branched alkylene group include ethylene, trimethylene, propylene, tetramethylene, butylene, pentamethylene, hexamethylene, heptamethylene, octamethylene, nonamethylene, decamethylene, undecamethylene and dodecamethylene groups. Examples of the linear or branched alkenylene group include vinylene, propenylene, 1-butenylene, 2-butenylene and 1-pentenylene groups. Examples of the linear or branched alkynylene group include propynylene, 1-butynylene, 2-butynylene, 1-pentynylene, 2-pentynylene, 1-hexynylene, 1-heptynylene and 1-octynylene groups. Examples of the arylene group include phenylene. As the substituent for these divalent $C_{2-12}$ hydrocarbon groups, substituents similar to those for the above-exemplified hydrocarbon group represented by $R^1$, $R^4$, $R^5$, $R^6$, $R^7$, $R^{10}$, $R^{11}$, $R^{12}$ or $R^{13}$ can be mentioned as examples.

Examples of the substituted or unsubstituted, divalent hydrocarbon group having 1 to 12 carbon atoms, which hydrocarbon group is represented by $R^3$, $R^8$ or $R^9$, include linear or branched alkylene groups, linear or branched alkenylene groups, linear or branched alkynylene groups and arylene groups, of which those having 1 to 10 carbon atoms are preferred, with those having 1 to 8 carbon atoms being particularly preferred and those having 3 to 8 carbon atoms being more preferred. Examples of the linear or branched alkylene group include methylene, ethylene, trimethylene, propylene, tetramethylene, butylene, pentamethylene, hexamethylene, heptamethylene, octamethylene, nonamethylene, decamethylene, undecamethylene and dodecamethylene. Exampies of the linear or branched alkenylene group include vinylene, propenylene, 1-butenylene, 2-butenylene and 1-pentenylene. Examples of the linear or branched alkylene groups include propynylene, 1-butynylene, 2-butynylene, 1-pentynylene, 2-pentynylene, 1-hexynylene, 1-heptynylene and 1-octynylene groups. Examples of the arylene group include phenylene. As the substituent for these divalent $C_{1-12}$ hydrocarbon groups, substituents similar to those for the above-exemplified hydrocarbon group represented by $R^1$, $R^4$, $R^5$, $R^6$, $R^7$, $R^{10}$, $R^{11}$, $R^{12}$ or $R^{13}$ can be mentioned as examples.

Examples of the substituted or unsubstituted, cyclic hydrocarbon group represented by $Q^1$, $Q^2$ or $Q^3$ include saturated or unsaturated, monocyclic or condensed polycyclic hydrocarbon groups having 6 to 18 carbon atoms, such as phenyl, naphthyl, anthryl, phenanthryl, indanyl and indenyl. If these groups have plural structural isomers, such isomers are also embraced by the present invention. Among them, phenyl, 1-naphthyl, 2-naphthyl, 5-indanyl and the like are preferred in the present invention. As the substituent for these aryl groups, substituents similar to those for the above-exemplified hydrocarbon group represented by $R^1$, $R^4$, $R^5$, $R^6$, $R^7$, $R^{10}$, $R^{11}$, $R^{12}$ or $R^{13}$ can be mentioned as examples.

Examples of the substituted or unsubstituted heterocyclic group represented by $Q^1$, $Q^2$ or $Q^3$ include aromatic heterocyclic groups and nonaromatic heterocyclic groups. Examples of the aromatic heterocyclic groups include aromatic monocyclic heterocyclic groups and aromatic fused heterocyclic groups. Specific examples of the aromatic monocyclic heterocyclic groups include furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl and triazinyl, of which furyl, thienyl and tetrazolyl groups are preferred.

Examples of the aromatic fused heterocyclic groups include benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, 1,2-isobenzothiazolyl, 1H-benzotriazolyl, imidazopyridyl, quinolyl, isoquinolyl, cinnolyl, quinazolinyl, quinoxalinyl, phthalazinyl, naphthyridinyl, purinyl, pteridinyl, carbazolyl, α-carbolinyl, β-carbolinyl, γ-carbolinyl, acridinyl, phenoxazinyl, phenothiazinyl, phenazinyl, phenoxazinyl, thianthrenyl, phenanthridinyl, phenathrolinyl and indolizinyl, of which benzofuranyl, benzo[b]thienyl, benzoxazolyl and benzothiazolyl groups are preferred.

The term "nonaromatic heterocyclic group" as used herein means a saturated or unsaturated heterocyclic group having at least two hydrogen atoms added to the above-exemplified aromatic heterocyclic group. Examples include tetrahydrofuryl, tetrahydrothienyl, pyrrolidyl, tetrahydropyranyl, piperidinyl, morpholinyl, piperazinyl, 2,3-dihydrobenzofuranyl, indolinyl, 4,5,6,7-tetrahydroindolinyl, 4,5,6,7-tetrahydroindazolyl, 3,4-dihydro-2H-benzopyranyl, 2H-benzopyranyl, 1,2-dihydroquinolyl, 1,2,3,4-tetrahydroquinilyl, 1,2,3,4-tetrahydroisoquinilyl, 1,2-dihydroisoquinolyl, 1,3-benzodioxolyl and 1,4-benzodioxanyl groups, of which 2,3-dihydrobenzofuranyl, 3,4-dihydro-2H-benzopyranyl, 2H-benzopyranyl, 1,2-dihydroquinolyl and 1,3-benzodioxolyl groups are preferred.

Examples of the substituent for these heterocyclic groups include hydroxyl group, halogen atoms, alkyl groups, alkoxyl groups and phenyl group and 1 to 5 substituents are optionally selected from them. Here, the halogen atom, alkyl groups and alkoxyl groups are similar to those exemplified above.

As described above, at least one of $A^1$, $A^2$ and $A^3$ is a group represented by the formula (2) and the remaining one or two of $A^1$, $A^2$ and $A^3$ are the same or different and are represented by the formula (3). Among them, it is preferred

that one or two of $A^1$, $A^2$ and $A^3$ are represented by the following formula (2a):

$$-R^2-a^1\rightarrow \quad (2a)$$

{wherein $R^2$, $a^1$ and $\rightarrow$ have the same meanings as described above} and the remaining ones of $A^1$, $A^2$ and $A^3$ are the same or different and are represented by the following formula (3a):

$$-R^8-a^3-R^9-a^4\rightarrow \quad (3a)$$

{wherein $R^8$, $R^9$, $a^3$, $a^4$ and $\rightarrow$ have the same meanings as described above}. Incidentally, the formula (2a) means the formula (2) wherein $R^3$ and $a^2$ both represent a single bond.

Among the compounds of the formula (1), preferred are compounds of the formula (1) wherein $X^1$ represents a carboxyl group ($-COOR^{14}$) which may be esterified, $A^1$ represents $-R^2-a^1\rightarrow$, $A^2$ represents $-R^{8a}-a^{3a}-R^{9a}-a^{4a}\rightarrow$ and $A^3$ represents $-R^{8b}-a^{3b}-R^{9b}-a^{4b}\rightarrow$, that is, the compounds represented by the following formula (1A):

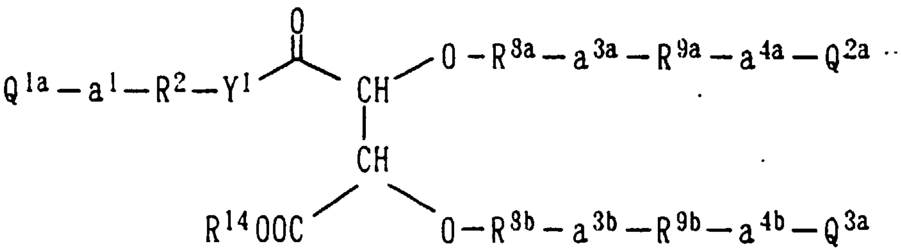

(1A)

[wherein $R^{14}$ represents a hydrogen atom or an ester residue,

$R^{8a}$ and $R^{8b}$ are the same or different and individually has the same meaning as described in $R^8$,
$a^{3a}$ and $a^{3b}$ are the same or different and individually has the same meaning as described in $a^3$,
$R^{9a}$ and $R^{9b}$ are the same or different and individually has the same meaning as described in $R^9$,
$a^{4a}$ and $a^{4b}$ are the same or different and individually has the same meaning as described in $a^4$,
$Q^{1a}$ represents a substituted or unsubstituted cyclic hydrocarbon group or a substituted or unsubstituted heterocyclic group,
either one of $Q^{2a}$ and $Q^{3a}$ represents a hydrogen atom or a carboxyl group which may be esterified and the other one represents a substituted or unsubstituted cyclic hydrocarbon group or a substituted or unsubstituted heterocyclic group, and
$Y^1$, $a^1$ and $R^2$ have the same meanings as described above].

The compound of the formula (1) wherein $X^1$ represents a carboxyl group ($-COOR^{15}$) which may be esterified, $A^1$ represents a single bond (in the case where in the formula (3), $R^8$, $a^3$, $R^9$ and $a^4$ represent a single bond), $Q^1$ represents a hydrogen atom or a substituted or unsubstituted hydrocarbon group ($R^{16}$) and $A^2$ represents $R^2-a^1\rightarrow$, that is, the compound represented by the following formula (1B):

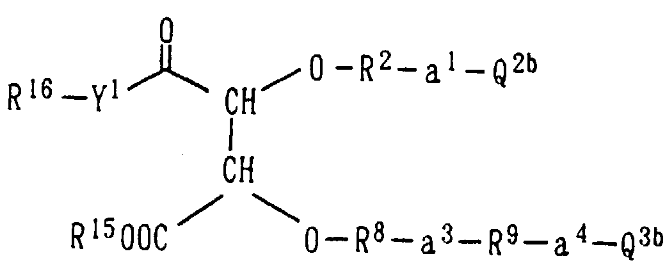

(1B)

[wherein $R^{15}$ represents a hydrogen atom or an ester residue, $R^{16}$ represents a hydrogen atom or a substituted or unsubstituted hydrocarbon group,

$Q^{2b}$ and $Q^{3b}$ are the same or different and individually represent a substituted or unsubstituted cyclic hydrocarbon group or a substituted or unsubstituted heterocyclic group, and

$Y^1$, $R^2$, $R^8$, $R^9$, $a^1$, $a^3$ and $a^4$ have the same meanings as described above].

Between the compounds of the formulas (1A) and (1B), the compounds of the formula (1A) are preferred.

It is more preferred that in the formula (1A), $Q^{1a}$ and $Q^{2a}$ are the same or different and individually represent a substituted or unsubstituted cyclic hydrocarbon group or a substituted or unsubstituted heterocyclic group, while $Q^{3a}$ represents a hydrogen atom or a carboxyl group which may be esterified.

It is preferred that in the formula (1A), the group $Y^1$ represents a single bond or $-(NR^1)-$ [wherein, $R^1$ has the same meaning as described above]. More preferred as $Y^1$ is $-(NR^1)-$, of which $-(NR^1)-$ wherein $R^1$ represents a hydrogen atom or an alkyl group is still more preferred.

It is preferred that in the formula (1A), the group - $R^{8a}$-$a^{3a}$-$R^{9a}$- represents a substituted or unsubstituted divalent hydrocarbon group having 1 to 12 carbon atoms, more preferably a divalent hydrocarbon group having 3 to 8 carbon atoms and particularly preferably an alkylene group having 3 to 8 carbon atoms.

It is preferred that in the formula (1A), the group - $R^{8b}$-$a^{3b}$-$R^{9a}$-$a^{4b}$→ represents a single bond or a substituted or unsubstituted divalent hydrocarbon group having 1 to 12 carbon atoms, more preferably a single bond or a divalent hydrocarbon group having 1 to 3 carbon atoms (more preferably an alkylene group having 1 to 3 carbon atoms) and particularly preferably a single bond or a methylene group.

It is preferred that in the formula (1A), the group $a^1$ represents a single bond or $-(NR^4)-$ (wherein $R^4$ has the same meaning as described above) and as $-(NR^4)-$, -NH- is more preferred.

It is preferred that in the formula (1A), the group $a^{4a}$ represents a single bond or $-(NR^{10})-$ [wherein $R^{10}$ has the same meaning as described above) and as $-(NR^{10})-$, -NH- is more preferred.

In short, more preferred are compounds of the formula (1A) wherein $R^{14}$ represents a hydrogen atom or an ester residue,

$Q^{1a}$ and $Q^{2a}$ are the same or different and individually represent a substituted or unsubstituted cyclic hydrocarbon group or a substituted or unsubstituted heterocyclic group,
$Q^{3a}$ represents a hydrogen atom or a carboxyl group which may be esterified,
$a^1$ represents a single bond or $-(NR^4)-$ [wherein $R^4$ has the same meaning as described above],
$R^2$ represents a substituted or unsubstituted divalent hydrocarbon group having 2 to 12 carbon atoms,
$Y^1$ represents $-(NR^1)-$ [wherein $R^1$ has the same meaning as described above],
the group -$R^{8a}$-$a^{3a}$-$R^{9a}$- represents a substituted or unsubstituted, divalent hydrocarbon group having 1 to 12 carbon atoms,
$a^{4a}$ represents a single bond or $-(NR^{10})-$ [wherein $R^{10}$ has the same meaning as described above],
$R^{8b}$ has the same meaning as $R^8$,
$a^{3b}$ has the same meaning as $a^3$,
$R^{9b}$ has the same meaning as $R^9$ and
$a^{4b}$ has the same meaning as $a^4$.

Still more preferred are compounds of the formula (1A) wherein $R^{14}$ represents a hydrogen atom or an ester residue,

$Q^{1a}$ and $Q^{2a}$ are the same or different and individually represent a substituted or unsubstituted cyclic hydrocarbon group or a substituted or unsubstituted heterocyclic group,
$Q^{3a}$ represents a hydrogen atom or a carboxyl group which may be esterified,
$a^1$ represents a single bond or $-(NR^4)-$ [wherein $R^4$ has the same meaning as described above],
$R^2$ represents a substituted or unsubstituted divalent hydrocarbon group having 2 to 12 carbon atoms,
$Y^1$ represents $-(NR^1)-$ [wherein $R^1$ has the same meaning as described above],
the group -$R^{8a}$-$a^{3a}$-$R^{9a}$- represents a substituted or unsubstituted, divalent hydrocarbon group having 1 to 12 carbon atoms,
$a^{4a}$ represents a single bond or $-(NR^{10})-$ [wherein $R^{10}$ has the same meaning as described above], and
-$R^{8b}$-$a^{3b}$-$R^{9b}$-$a^{4b}$ represents a single bond or a substituted or unsubstituted, divalent hydrocarbon group having 1 to 12 carbon atoms.

Examples of the salt of the compound represented by the formula (1) include pharmaceutically acceptable salts, for example, metal salts such as sodium salt, potassium salt, calcium salt and magnesium salt, ammonium salts and organic amine salts such as trimethylamine salt, triethylamine salt, dicyclohexylamine salt, ethanolamine salt, diethanolamine salt, triethanolamine salt and tert-butylamine salt.

The compound of the present invention represented by the formula (1) may have stereoisomers such as optical isomers, diastereomers or geometrical isomers. These stereoisomers and mixtures thereof are all embraced by the

present invention.

The compound of the formula (1) or salt thereof may exist as a hydrate or various solvates. They are also embraced by the present invention.

Among the invention compounds (1), following compounds and stereoisomers thereof can be mentioned as specific preferred examples.

2-Carboxymethoxy-3-[N-[5-(2-naphthyl)pentyl]carbamoyl]-3-[5-(2-naphthyl)pentyloxy]propionic acid
2-Ethoxycarbonylmethoxy-3-[N-[5-(2-naphthyl)pentyl]carbamoyl]-3-[5-(2-naphthyl)pentyloxy]propionic acid
2-Pivaloyloxymethoxycarbonylmethoxy-3-[N-[5-(2-naphthyl)pentyl]carbamoyl]-3-[5-(2-naphthyl)pentyloxy]propionic acid
3-Ethoxycarbonylmethoxy-3-[N-[5-(2-naphthyl)pentyl]carbamoyl]-2-[5-(2-naphthyl)pentyloxy]propionic acid
Ethyl 2-carboxymethoxy-3-[N-[5-(2-naphthyl)pentyl]carbamoyl]-3-[5-(2-naphthyl)pentyloxy]propionate
Ethyl 3-carboxymethoxy-3-[N-[5-(2-naphthyl)pentyl]carbamoyl]-2-[5-(2-naphthyl)pentyloxy]propionate
Ethyl 2-ethoxycarbonylmethoxy-3-[N-[5-(2-naphthyl)pentyl]carbamoyl]-3-[5-(2-naphthyl)pentyloxy]propionate
Pivaloyloxymethyl 2-ethoxycarbonylmethoxy-3-[N-[5-(2-naphthyl)pentyl]carbamoyl]-3-[5-(2-naphthyl)pentyloxy]propionate
2-Carboxymethoxy-3-[N-[5-(2-naphthyl)pentyl]carbamoyl]-3-[4-(2-naphthoxy)butoxy]propionic acid
2-Carboxymethoxy-3-[N-[4-(2-naphthoxy)butyl]carbamoyl]-3-[5-(2-naphthyl)pentyloxy]propionic acid
2-Carboxymethoxy-3-[N-[4-(2-naphthylamino)butyl]carbamoyl]-3-[5-(2-naphthyl)pentyloxy]propionic acid
2-(2-Hydroxyethoxy)-3-[N-[5-(2-naphthyl)pentyl]carbamoyl]-3-[5-(2-naphthyl)pentyloxy]propionic acid
3-(2-Hydroxyethoxy)-3-[N-[5-(2-naphthyl)pentyl]carbamoyl]-2-[5-(2-naphthyl)pentyloxy]propionic acid
2-Hydroxy-3-[N-[5-(2-naphthyl)pentyl]carbamoyl]-3-[5-(2-naphthyl)pentyloxy)propionic acid
Methyl 2-hydroxy-3-[N-[5-(2-naphthyl)pentyl]carbamoyl]-3-[5-(2-naphthyl)pentyloxy]propionate
Pivaloyloxymethyl 2-hydroxy-3-[N-[5-(2-naphthyl)pentyl]carbamoyl]-3-[5-(2- naphthyl)pentyloxy]propionate
2-Carboxymethoxy-3-[N-methyl-N-[5-(2-naphthyl)pentyl]carbamoyl]-3-[5-(2-naphthyl)pentyloxy]propionic acid
2-Ethoxycarbonylmethoxy-3-[N-methyl-N-[5-(2-naphthyl)pentyl]carbamoyl]-3-[5-(2-naphthyl)pentyloxy]propionic acid
Ethyl 2-ethoxycarbonylmethoxy-3-[N-methyl-N-[5-(2-naphthyl)pentyl]carbamoyl]-3-[5-(2-naphthyl)pentyloxy]propionate
Pivaloyloxymethyl 2-ethoxycarbonylmethoxy-3-[N-methyl-N-[5-(2-naphthyl)pentyl]carbamoyl]-3-[5-(2-naphthyl)pentyloxy]propionate
2-Hydroxy-3-[N-methyl-N-[5-(2-naphthyl)pentyl]carbamoyl]-3-[5-(2-naphthyl)pentyloxy]propionic acid
2-Methoxy-3-[N-methyl-N-[5-(2-naphthyl)pentyl]carbamoyl]-3-[5-(2-naphthyl)pentyloxy]propionic acid
2-Ethoxy-3-[N-[5-(2-naphthyl)pentyl]carbamoyl]-3-[4-(2-naphthoxy)butoxy]propionic acid
2-(2-Oxopropyloxy)-3-[N-[5-(2-naphthyl)pentyl]carbamoyl]-3-[5-(2-naphthoxy)butoxy]propionic acid
2-Carboxymethoxy-3-[N-[3-(2-naphthyl)propyl]carbamoyl]-3-[5-(2-naphthyl)pentyloxy]propionic acid
2-Carboxymethoxy-3-[N-(3,4-dichlorobenzyl)carbamoyl]-3-[5-(2-naphthyl)pentyloxy]propionic acid
2-Carboxymethoxy-3-(N-neopentylcarbamoyl)-3-[5-(2-naphthyl)pentyloxy]propionic acid
2-Carboxymethoxy-3-[N-hydroxy-N-[5-(2-naphthyl)pentyl]carbamoyl]-3-[5-(2-naphthyl)pentyloxy]propionic acid
2-Carboxymethoxy-10-(2-naphthyl)-3-[5-(2-naphthyl)pentyloxy]-4-oxodecanoic acid
2-Carboxymethoxy-3-[N-(7-phenylheptyl)carbamoyl]-3-[5-(2-naphthyl)pentyloxy]propionic acid
2-Carboxymethoxy-3-[5-(2-naphthyl)pentyloxy]-3-[N-[3-(3-phenoxyphenyl)propyl]carbamoyl]propionic acid
2-Carboxymethoxy-3-[5-(2-naphthyl)pentyloxy]-3-[N-[5-(4-phenoxyphenyl)pentyl]carbamoyl]propionic acid
2-Carboxymethoxy-3-[N-[5-(2-naphthyl)pentyl]carbamoyl]-3-[7-(2-naphthyl)heptyloxy]propionic acid
Ethyl 2-carboxymethoxy-3-[N-[5-(2-naphthyl)pentyl]carbamoyl]-3-[7-(2-naphthyl)heptyloxy]propionate
2-(2-Hydroxyethoxy)-3-[N-methyl-N-[5-(2-naphthyl)pentyl]carbamoyl]-3-[5-(2-naphthyl)pentyloxy]propionic acid
2-(2-Oxopropyloxy)-3-[N-[5-(2-naphthyl)pentyl]carbamoyl]-3-[4-(2-naphthoxy)butoxy]propionic acid
2-Carboxymethoxy-3-[N-propyl-N-[5-(2-naphthyl)pentyl]carbamoyl]-3-[5-(2-naphthyl)pentyloxy]propionic acid
2-Carboxymethoxy-3-[N-isobutyl-N-[5-(2-naphthyl)pentyl]carbamoyl]-3-[5-(2-naphthyl)pentyloxy]propionic acid
2-Carboxymethoxy-3-[N-benzyl-N-[5-(2-naphthyl)pentyl]carbamoyl]-3-[5-(2-naphthyl)pentyloxy]propionic acid
2-[N-[5-(2-naphthyl)pentyl]carbamoyl]methoxy-3-[5-(2-naphthyl)pentyloxy]succinic acid
2-Carboxymethoxy-3-[N-[5-(2-naphthyl)pentyl]carbamoyl]-3-(5-phenylpentyloxy)propionic acid
2-Carboxymethoxy-3-[N-[5-(2-naphthyl)pentyl]carbamoyl]-3-(7-phenylheptyloxy)propionic acid
2-Carboxymethoxy-3-[N-[5-(2-chlorophenyl)pentyloxy]-3-[N-[5-(2-naphthyl)pentyl]carbamoyl]propionic acid
2-Carboxymethoxy-3-[5-(3-chlorophenyl)pentyloxy]-3-[N-[5-(2-naphthyl)pentyl]carbamoyl]propionic acid
2-Carboxymethoxy-3-[5-(4-chlorophenyl)pentyloxy]-3-[N-[5-(2-naphthyl)pentyl]carbamoyl]propionic acid
3-[5-(4-chlorophenyl)pentyloxy]-2-ethoxycarbonylmethoxy-3-[N-[5-(2-naphthyl)pentyl]carbamoyl]propionic acid
2-Carboxymethoxy-3-[5-(2,4-dichlorophenyl)pentyloxy]-3-[N-[5-(2-naphthyl)pentyl]carbamoyl]propionic acid

2-Carboxymethoxy-3-[5-(3,4-dichlorophenyl)pentyloxy]-3-[N-[5-(2-naphthyl)pentyl]carbamoyl]propionic acid
2-Carboxymethoxy-3-[N-[5-(2-naphthyl)pentyl]carbamoyl]-3-[5-(4-methylphenyl)pentyloxy]propionic acid
2-Carboxymethoxy-3-[5-(3,4-dimethylphenyl)pentyloxy]-3-[N-[5-(2-naphthyl)pentyl]carbamoyl]propionic acid
3-[5-(3,4-Dimethylphenyl)pentyloxy]-2-ethoxycarbonylmethoxy-3-[N-[5-(2-naphthyl)pentyl]carbamoyl]propionic acid
2-Carboxymethoxy-3-[5-(4-methoxyphenyl)pentyloxy]-3-[N-[5-(2-naphthyl)pentyl]carbamoyl]propionic acid
2-Carboxymethoxy-3-[N-[5-(2-naphthyl)pentyl]carbamoyl]-3-[5-(4-trifluoromethylphenyl)pentyloxy]propionic acid
2-Carboxymethoxy-3-[5-(4-tert-butylphenyl)pentyloxy]-3-[N-[5-(2-naphthyl)pentyl]carbamoyl]propionic acid
2-Carboxymethoxy-3-[5-(4-fluorophenyl)pentyloxy]-3-[N-[5-(2-naphthyl)pentyl]carbamoyl]propionic acid
2-Ethoxycarbonylmethoxy-3-[5-(4-fluorophenyl)pentyloxy]-3-[N-[5-(2-naphthyl)pentyl]carbamoyl]propionic acid
2-Carboxymethoxy-3-[5-(4-cyanophenyl)pentyloxy]-3-[N-[5-(2-naphthyl)pentyl]carbamoyl]propionic acid
2-Carboxymethoxy-3-[5-(4-carboxyphenyl)pentyloxy]-3-[N-[5-(2-naphthyl)pentyl]carbamoyl]propionic acid
2-Carboxymethoxy-3-[5-(4-aminophenyl)pentyloxy]-3-[N-[5-(2-naphthyl)pentyl]carbamoyl]propionic acid
2-Carboxymethoxy-3-[N-[5-(2-naphthyl)pentyl]carbamoyl]-3-[5-(4-nitrophenyl)pentyloxy]propionic acid
2-Carboxymethoxy-3-[5-(3-chloro-4-methylphenyl)pentyloxy]-3-[N-[5-(2-naphthyl)pentyl]carbamoyl]propionic acid
3-[5-(3-Chloro-4-methylphenyl)pentyloxy]-2-ethoxycarbonylmethoxy-3-[N-[5-(2-naphthyl)pentyl]carbamoyl]propionic acid
2-Carboxymethoxy-3-[5-(4-chloro-3-methylphenyl)pentyloxy]-3-[N-[5-(2-naphthyl)pentyl]carbamoyl]propionic acid
2-Carboxymethoxy-3-[5-(3-fluoro-4-methylphenyl)pentyloxy]-3-[N-[5-(2-naphthyl)pentyl]carbamoyl]propionic acid
2-Carboxymethoxy-3-[5-(4-fluoro-3-methylphenyl)pentyloxy]-3-[N-[5-(2-naphthyl)pentyl]carbamoyl]propionic acid
2-Carboxymethoxy-3-[5-(4-chloro-3-fluorophenyl)pentyloxy]-3-[N-[5-(2-naphthyl)pentyl]carbamoyl]propionic acid
2-Carboxymethoxy-3-[(Z)-5-(4-methylphenyl)-4-pentenyloxy]-3-[N-[5-(2-naphthyl)pentyl]carbamoyl]propionic acid
2-Carboxymethoxy-3-[(E)-5-(4-methylphenyl)-4-pentenyloxy]-3-[N-[5-(2-naphthyl)pentyl]carbamoyl]propionic acid
2-Carboxymethoxy-3-[5-(4-ethylphenyl)pentyloxyl-3-[N-[5-(2-naphthyl)pentyl]carbamoyl]propionic acid
2-Carboxymethoxy-3-[N-[5-(2-naphthyl)pentyl]carbamoyl]-3-[5-[4-(2-propyl)phenyl]pentyloxy]propionic acid
2-Carboxymethoxy-3-[5-[4-(2-methyl-1-propyl)phenyl]pentyloxy]-3-[N-5-(2-naphthyl)pentyl]carbamoyl]propionic acid
2-Carboxymethoxy-3-[5-[4-(3-methyl-1-butyl)phenyl]pentyloxy]-3-[N-5-(2-naphthyl)pentyl]carbamoyl]propionic acid
2-Carboxymethoxy-3-[(Z)-5-(3,4-dimethylphenyl)-4-pentenyloxy]-3-[N-5-(2-naphthyl)pentyl]carbamoyl]propionic acid
2-Carboxymethoxy-3-[(E)-5-(3,4-dimethylphenyl)-4-pentenyloxy]-3-[N-[5-(2-naphthyl)pentyl]carbamoyl]propionic acid
2-Carboxymethoxy-3-[6-(3,4-dimethylphenyl)hexyloxy]-3-[N-[5-(2-naphthyl)pentyl]carbamoyl]propionic acid
2-Carboxymethoxy-3-[(Z)-6-(3,4-dimethylphenyl)-5-hexenyl]-3-[N-[5-(2-naphthyl)pentyl]carbamoyl]propionic acid
2-Carboxymethoxy-3-[(E)-6-(3,4-dimethylphenyl)-5-hexenyloxy]-3-[N-[5-(2-naphthyl)pentyl]carbamoyl]propionic acid
2-Carboxymethoxy-3-[7-(3,4-dimethylphenyl)heptyloxy]-3-[N-[5-(2-naphthyl)pentyl]carbamoyl]propionic acid
2-Carboxymethoxy-3-[(Z)-7-(3,4-dimethylphenyl)-6-heptenyloxy]-3-[N-[5-(2-naphthyl)pentyl]carbamoyl]propionic acid
2-Carboxymethoxy-3-[(E)-7-(3,4-dimethylphenyl)-6-heptenyloxy]-3-[N-[5-(2-naphthyl)pentyl]carbamoyl]propionic acid
2-Carboxymethoxy-3-[N-[5-(4-chlorophenyl)pentyl]carbamoyl]-3-[5-(3,4-dimethylphenyl)pentyloxy]propionic acid
2-Carboxymethoxy-3-[5-(3,4-dimethylphenyl)pentyloxy]-3-[N-[5-(4-methylphenyl)pentyl]carbamoyl]propionic acid
2-Carboxymethoxy-3-[5-(3,4-dimethylphenyl)pentyloxy]-3-[N-[5-(4-fluorophenyl)pentyl]carbamoyl]propionic acid
2-Carboxymethoxy-3-[N-[5-(3,4-dimethylphenyl)pentyl]carbamoyl]-3-[5-(3,4-dimethylphenyl)pentyloxy]propionic acid
2-Carboxymethoxy-3-[N-[5-(3,4-dichlorophenyl)pentyl]carbamoyl]-3-[5-(3,4-dimethylphenyl)pentyloxy]propionic acid
2-Carboxymethoxy-3-[N-[5-(3-chloro-4-methylphenyl)pentyl]carbamoyl]-3-[5-(3,4-dimethylphenyl)pentyloxy]propionic acid
2-Carboxymethoxy-3-[N-[6-(4-chlorophenyl)hexyl]carbamoyl]-3-[5-(3,4-dimethylphenyl)pentyloxy]propionic acid
2-Carboxymethoxy-3-[5-(3,4-dimethylphenyl)pentyloxy]-3-[N-[6-(4-methylphenyl)hexyl]carbamoyl]propionic acid
2-Carboxymethoxy-3-[5-(3,4-dimethylphenyl)pentyloxy]-3-[N-[6-(4-fluorophenyl)hexyl]carbamoyl]propionic acid
2-Carboxymethoxy-3-[N-[6-(3,4-dimethylphenyl)hexyl]carbamoyl]-3-[5-(3,4-dimethylphenyl)pentyloxy]propionic acid
2-Carboxymethoxy-3-[N-[6-(3,4-dichlorophenyl)hexyl]carbamoyl]-3-[5-(3,4-dimethylphenyl)pentyloxy]propionic acid
2-Carboxymethoxy-3-[N-(6-(3-chloro-4-methylphenyl)hexyl]carbamoyl]-3-[5-(3,4-dimethylphenyl)pentyloxy]propionic acid

2-Carboxymethoxy-3-[N-[7-(4-chlorophenyl)heptyl]carbamoyl]-3-[5-(3,4-dimethylphenyl)pentyloxy]propionic acid
2-Carboxymethoxy-3-[5-(3,4-dimethyiphenyl)pentyloxy]-3-[N-[7-(4-methylphenyl)heptyl]carbamoyl]propionic acid
2-Carboxymethoxy-3-[5-(3,4-dimethylphenyl)pentyloxy]-3-[N-[7-(4-fluorophenyl)heptyl]carbamoyl]propionic acid
2-Carboxymethoxy-3-[N-[7-(3,4-dimethylphenyl)heptyl]carbamoyl]-3-[5-(3,4-dimethylphenyl)pentyloxy]propionic acid
2-Carboxymethoxy-3-[N-[7-(3,4-dichlorophenyl)heptyl]carbamoyl]-3-[5-(3,4-dimethylphenyl)pentyloxy]propionic acid
2-Carboxymethoxy-3-[N-[7-(3-chloro-4-methylphenyl)heptyl]carbamoyl]-3-[5-(3,4-dimethylphenyl)pentyloxy]propionic acid
2-Carboxymethoxy-3-[4-(3,4-dimethylphenoxy)butoxy]-3-[N-[5-(2-naphthyl)pentyl]carbamoyl]propionic acid
2-Carboxymethoxy-3-[N-[5-(4-chlorophenyl)pentyl]carbamoyl]-3-[5-(2-naphthyl)pentyloxy]propionic acid.
2-Carboxymethoxy-3-[N-[5-(4-methylphenyl)pentyl]carbamoyl]-3-[5-(2-naphthyl)pentyloxy]propionic acid
2-Carboxymethoxy-3-[N-[5-(4-fluorophenyl)pentyl]carbamoyl]-3-[5-(2-naphthyl)pentyloxy]propionic acid
2-Carboxymethoxy-3-[N-[5-(3,4-dimethylphenyl)pentyl]carbamoyl]-3-[5-(2-naphthyl)pentyloxy]propionic acid
2-Carboxymethoxy-3-[N-[5-(3,4-dichlorophenyl)pentyl]carbamoyl]-3-[5-(2-naphthyl)pentyloxy]propionic acid
2-Carboxymethoxy-3-[N-[5-(3-chloro-4-methylphenyl)pentyl]carbamoyl]-3-[5-(2-naphthyl)pentyloxy]propionic acid
2-Carboxymethoxy-3-[N-[6-(4-chlorophenyl)hexyl]carbamoyl]-3-[5-(2-naphthyl)pentyloxy]propionic acid
2-Carboxymethoxy-3-[N-[6-(4-methylphenyl)hexyl]carbamoyl]-3-[5-(2-naphthyl)pentyloxy]propionic acid
2-Carboxymethoxy-3-[N-[6-(4-fluorophenyl)hexyl]carbamoyl]-3-[5-(2-naphthyl)pentyloxy]propionic acid
2-Carboxymethoxy-3-[N-[6-(3,4-dimethylphenyl)hexyl]carbamoyl]-3-[5-(2-naphthyl)pentyloxy]propionic acid
2-Carboxymethoxy-3-[N-[6-(3,4-dichlorophenyl)hexyl]carbamoyl]-3-[5-(2-naphthyl)pentyloxy]propionic acid
2-Carboxymethoxy-3-[N-[6-(3-chloro-4-methylphenyl)hexyl]carbamoyl]-3-[5-(2-naphthyl)pentyloxy]propionic acid
2-Carboxymethoxy-3-[N-[7-(4-chlorophenyl)heptyl]carbamoyl]-3-[5-(2-naphthyl)pentyloxy]propionic acid
2-Carboxymethoxy-3-[N-[7-(4-methylphenyl)heptyl]carbamoyl]-3-[5-(2-naphthyl)pentyloxy]propionic acid
2-Carboxymethoxy-3-[N-[7-(4-fluorophenyl)heptyl]carbamoyl]-3-[5-(2-naphthyl)pentyloxy]propionic acid
2-Carboxymethoxy-3-[N-[7-(3,4-dimethylphenyl)heptyl]carbamoyl]-3-[5-(2- naphthyl)pentyloxy]propionic acid
2-Carboxymethoxy-3-[N-[7-(3,4-dichlorophenyl)heptyl]carbamoyl]-3-[5-(2-naphthyl)pentyloxy]propionic acid
2-Carboxymethoxy-3-[N-[7-(3-chloro-4-methylphenyl)heptyl]carbamoyl]-3-[5-(2-naphthyl)pentyloxy]propionic acid
2-Carboxymethoxy-3-[N-[6-(2-naphthyl)hexyl]carbamoyl]-3-[5-(2-naphthyl)pentyloxy]propionic acid
2-Carboxymethoxy-3-[N-[7-(2-naphthyl)heptyl]carbamoyl]-3-[5-(2-naphthyl)pentyloxy]propionic acid
2-Carboxymethoxy-3-[6-(2-naphthyl)hexyloxy]-3-[N-[5-(2-naphthyl)pentyl]carbamoyl]propionic acid
2-Carboxymethoxy-3-[N-[6-(2-naphthyl)hexyl]carbamoyl]-3-[6-(2-naphthyl)hexyloxy]propionic acid
2-Carboxymethoxy-3-[N-[7-(2-naphthyl)heptyl]carbamoyl]-3-[6-(2-naphthyl)hexyloxy]propionic acid
2-Carboxymethoxy-3-[7-(2-naphthyl)heptyloxy]-3-[N-[6-(2-naphthyl)hexyl]carbamoyl]propionic acid
2-Carboxymethoxy-3-[N-[7-(2-naphthyl)heptyl]carbamoyl]-3-[7-(2-naphthyl)heptyloxy]propionic acid
2-Carboxymethoxy-3-[N-[5-(2-naphthyl)pentyl]carbamoyl]-3-(5-(1-naphthyl)pentyloxy]propionic acid
2-Carboxymethoxy-3-[N-[5-(1,3-benzodioxol-5-yl)pentyl]carbamoyl]-3-[5-(2-naphthyl)pentyloxy]propionic acid
2-Carboxymethoxy-3-[N-[5-(2-benzoxazolyl)pentyl]carbamoyl]-3-[5-(2-naphthyl)pentyloxy]propionic acid
2-Carboxymethoxy-3-[N-[6-(2-benzoxazolyl)hexyl]carbamoyl]-3-[5-(2-naphthyl)pentyloxy]propionic acid
2-Carboxymethoxy-3-[N-[7-(2-benzoxazolyl)heptyl]carbamoyl]-3-[5-(2-naphthyl)pentyloxy]propionic acid
2-Carboxymethoxy-3-[N-[5-(2-benzothiazolyl)pentyl]carbamoyl]-3-[5-(2-naphthyl)pentyloxy]propionic acid
2-Carboxymethoxy-3-[N-[5-(2-benzofuranyl)pentyl]carbamoyl]-3-[5-(2-naphthyl)pentyloxy]propionic acid
2-Carboxymethoxy-3-[N-[6-(2-benzofuranyl)hexyl]carbamoyl]-3-(5-(2-naphthyl)pentyloxy]propionic acid
2-Carboxymethoxy-3-[N-[7-(2-benzofuranyl)heptyl]carbamoyl]-3-[5-(2-naphthyl)pentyloxy]propionic acid
2-Carboxymethoxy-3-[5-(1,3-benzodioxol-5-yl)pentyloxy]-3-[N-[5-(2-naphthyl)pentyl]carbamoyl]propionic acid
3-[5-(1,3-benzodioxol-5-yl)pentyloxy]-2-ethoxycarbonylmethoxy-3-[N-[5-(2-naphthyl)pentyl]carbamoyl]propionic acid
2-Carboxymethoxy-3-[5-(2-benzoxazolyl)pentyloxy]-3-[N-[5-(2-naphthyl)pentyl]carbamoyl]propionic acid
2-Ethoxycarbonylmethoxy-3-[5-(2-benzoxazolyl)pentyloxy]-3-[N-[5-(2-naphthyl)pentyl]carbamoyl]propionic acid
2-Carboxymethoxy-3-[5-(2-benzothiazolyl)pentyloxy]-3-[N-[5-(2-naphthyl)pentyl]carbamoyl]propionic acid
2-Ethoxycarbonylmethoxy-3-[5-(2-benzothiazolyl)pentyloxy]-3-[N-[5-(2-naphthyl)pentyl]carbamoyl]propionic acid
2-Ethoxycarbonylmethoxy-3-[5-(2-benzoxazolyl)pentyloxy]-3-[N-[5-(1,3-benzodioxol-5-yl)pentyl]carbamoyl]propionic acid
2-Carboxymethoxy-3-[5-(2-benzoimidazolyl)pentyloxy]-3-[N-[5-(2-naphthyl)pentyl]carbamoyl]propionic acid
2-Carboxymethoxy-3-[5-(5-chloro-2-benzoxazolyl)pentyloxy]-3-[N-[5-(2-naphthyl)pentyl]carbamoyl]propionic acid
2-Carboxymethoxy-3-[5-(2-imidazo[4,5-b]pyridyl)pentyloxy]-3-[N-[5-(2-naphthyl)pentyl]carbamoyl]propionic acid
2-Carboxymethoxy-3-[5-(1,4-benzodioxan-6-yl)pentyloxy]-3-[N-[5-(2-naphthyl)pentyl]carbamoyl]propionic acid

2-Carboxymethoxy-3-[N-[5-(2-naphthyl)pentyl]carbamoyl]-3-[5-[2-(2-propyl)-4-oxazolyl]pentyloxy]propionic acid
2-Carboxymethoxy-3-[N-[5-(2-naphthyl)pentyl]carbamoyl]-3-[5-(2-phenyl-4-oxazolyl)pentyloxy]propionic acid
2-Carboxymethoxy-3-[5-(5-benzofuranyl)pentyloxy]-3-[N-[5-(2-naphthyl)pentyl]carbamoyl]propionic acid
2-Carboxymethoxy-3-[5-(5-benzothiazolyl)pentyloxy]-3-N-[5-(2-naphthyl)pentyl]carbamoyl]propionic acid
2-Carboxymethoxy-3-[5-(5-benzo[b]thienyl)pentyloxy]-3-[N-[5-(2-naphthyl)pentyl]carbamoyl]propionic acid
2-Carboxymethoxy-3-[5-(2,3-dihydro-5-benzo[b]thienyl)pentyloxy]-3-[N-[5-(2-naphthyl)pentyl]carbamoyl]propionic acid
2-Carboxymethoxy-3-[5-(6-imidazo[1,5-a]pyridyl)pentyloxy]-3-[N-[5-(2-naphthyl)pentyl]carbamoyl]propionic acid
2-Carboxymethoxy-3-[5-(7-imidazo[1,2-a]pyridyl)pentyloxy]-3-[N-[5-(2-naphthyl)pentyl]carbamoyl]propionic acid
2-Carboxymethoxy-3-[N-[5-(2-naphthyl)pentyl]carbamoyl]-3-[5-(6-quinoxalyl)pentyloxy]propionic acid
2-Carboxymethoxy-3-[N-[5-(2-naphthyl)pentyl]carbamoyl]-3-[5-(6-quinolyl)pentyloxy]propionic acid
2-Carboxymethoxy-3-[5-(3-furyl)pentyloxy]-3-[N-[5-(2-naphthyl)pentyl]carbamoyl]propionic acid
2-Carboxymethoxy-3-[N-[5-(2-naphthyl)pentyl]carbamoyl]-3-[5-(3-thienyl)pentyloxy]propionic acid
2-Carboxymethoxy-3-[N-[5-(2-naphthyl)pentyl]carbamoyl]-3-[5-(3-pyrrolyl)pentyloxy]propionic acid
2-Carboxymethoxy-3-[5-(4,5-dimethyl-2-thiazolyl)pentyloxy]-3-[N-[5-(2-naphthyl)pentyl]carbamoyl]propionic acid
2-Carboxymethoxy-3-[5-(4,5-dimethyl-2-oxazolyl)pentyloxy]-3-[N-[5-(2-naphthyl)pentyl]carbamoyl]propionic acid
2-Carboxymethoxy-3-[5-(5-benzoxazolyloxy)pentyloxy]-3-[N-[5-(2-naphthyl)pentyl]carbamoyl]propionic acid
2-Carboxymethoxy-3-[N-[5-(2-naphthyl)pentyl]carbamoyl]-3-[5-(7-quinolyloxy)pentyloxy]propionic acid
2-Carboxymethoxy-3-[5-(7-isoquinolyloxy)pentyloxy]-3-[N-[5-(2-naphthyl)pentyl]carbamoyl]propionic acid
2-Carboxymethoxy-3-[5-(4,5-dimethylpyrimidin-2-yl)pentyloxy]-3-[N-[5-(2-naphthyl)pentyl]carbamoyl]propionic acid
2-Carboxymethoxy-3-[N-[5-(2-naphthyl)pentyl]carbamoyl]-3-[5-(4-pyridyl)pentyloxy]propionic acid
2-Carbamoylmethoxy-3-[N-[5-(2-naphthyl)pentyl]carbamoyl]-3-[5-(2-naphthyl)pentyloxy]propionic acid
2-(N,N-Dimethylcarbamoyl)methoxy-3-[N-[5-(2-naphthyl)pentyl]carbamoyl]-3-[5-(2-naphthyl)pentyloxy]propionic acid
2-Morpholinocarbonylmethoxy-3-[N-[5-(2-naphthyl)pentyl]carbamoyl]-3-[5-(2-naphthyl)pentyloxy]propionic acid
3-[N-[5-(2-Naphthyl)pentyl]carbamoyl)-3-[5-(2-naphthyl)pentyloxy]-2-(piperazinocarbonylmethoxy)propionic acid
2-(4-Methylpiperazino)carbonylmethoxy-3-[N-[5-(2-naphthyl)pentyl]carbamoyl]-3-[5-(2-nanthyl)pentyloxy]propionic acid
2-(N-Methoxycarbonylmethylcarbamoyl)methoxy-3-[N-[5-(2-naphthyl)pentyl]carbamoyl]-3-[5-(2-naphthyl)pentyloxy]propionic acid
2-[N-(2-Methoxyethyl)carbamoyl]methoxy-3-[N-[5-(2-naphthyl)pentyl]carbamoyl]-3-[5-(2-naphthyl)pentyloxy]propionic acid
2-Hydrazinocarbonylmethoxy-3-[N-[5-(2-naphthyl)pentyl]carbamoyl]-3-[5-(2-naphthyl)pentyloxy]propionic acid
2-(N-Hydroxycarbamoyl)methoxy-3-[N-[5-(2-naphthyl)pentyl]carbamoyl]-3-[5-(2-naphthyl)pentyloxy]propionic acid
2-(N-Methoxycarbamoyl)methoxy-3-[N-[5-(2-naphthyl)pentyl]carbamoyl]-3-[5-(2-naphthyl)pentyloxy]propionic acid
2-(N-Methoxy-N-methylcarbamoyl)methoxy-3-[N-[5-(2-naphthyl)pentyl]carbamoyl]-3-[5-(2-naphthyl)pentyloxy]propionic acid
2-(3-Morpholino-2-oxopropoxy)-3-[4-(2-naphthoxy)butoxy]-3-[N-[5-(2-naphthyl)pentyl]carbamoyl]propionic acid
2-[(2S-Methoxycarbonylpyrrolidino)carbonyl]methoxy-3-[N-[5-(2-naphthyl)pentyl]carbamoyl]-3-[5-(2-naphthyl)pentyloxy]propionic acid
2-(N-Ethylcarbamoyloxy)-3-[N-methyl-N-[5-(2-naphthyl)pentyl]carbamoyl]-3-[5-(2-naphthyl)pentyloxy]propionic acid
2-(N-Benzyloxycarbamoyl)methoxy-3-[N-[5-(2-naphthyl)pentyl]carbamoyl]-3-[5-(2-naphthyl)pentyloxy]propionic acid
2-(2-Isoxazolidino)carbonylmethoxy-3-[N-[5-(2-naphthyl)pentyl]carbamoyl]-3-[5-(2-naphthyl)pentyloxy]propionic acid
2-(N-Hydroxy-N-methylcarbamoyl)methoxy-3-[N-[5-(2-naphthyl)pentyl]carbamoyl]-3-[5-(2-naphthyl)pentyloxy]propionic acid
2-(N-Methoxycarbamoyl)methoxy-3-[N-[5-(2-naphthyl)pentyl]carbamoyl]-3-[5-(2-naphthyl)pentyloxy]propionic acid
2-Methylsulfonylmethoxy-3-[N-[5-(2-naphthyl)pentyl]carbamoyl]-3-[5-(2-naphthyl)pentyloxy]propionic acid
2,3-Dihydroxypropyloxy-3-[N-methyl-[N-[5-(2-naphthyl)pentyl]carbamoyl]-3-[5-(2-naphthyl)pentyloxy]propionic acid
2-[N-(trans-4-Hydroxycyclohexyl)carbamoyl]methoxy-3-[N-[5-(2-naphthyl)pentyl]carbamoyl]-3-[5-(2-naphthyl)pentyloxy]propionic acid
2-[N-(Methylsulfonyl)carbamoyl]methoxy-3-[N-[5-(2-naphthyl)pentyl]carbamoyl]-3-[5-(2-naphthyl)pentyloxy]propionic acid
2-[N-(Methylphenylsulfonyl)carbamoyl]methoxy-3-[N-[5-(2-naphthyl)pentyl]carbamoyl]-3-[5-(2-naphthyl)pentyloxy]propionic acid

3-[N-[5-(2-Naphthyl)pentyl]carbamoyl]-3-[5-(2-naphthyl)pentyloxy]-2-[N-(sulfamoyl)carbamoyloxy]propionic acid
3-[N-Methyl-N-[5-(2-naphthyl)pentyl]carbamoyl]-2-(2-morpholinoethoxy)-3-[5-(2-naphthyl)pentyloxy]propionic acid
3-[N-[5-(2-Naphthyl)pentyl]carbamoyl]-3-[5-(2-naphthyl)pentyloxy]-2-(pyridinocarbonylmethoxy)propionic acid
3-[N-[5-(2-Naphthyl)pentyl]carbamoyl]-3-[5-(2-naphthyl)pentyloxy]-2-(N-tert-butoxycarbamoyl)methoxypropionic acid
2-[N-(4-Dimethylaminophenyl)carbamoyl]methoxy-3-[N-[5-(2-naphthyl)pentyl]carbamoyl]-3-[5-(2-naphthyl)pentyloxy]propionic acid
2-Acetoxy-3-[N-[5-(2-naphthyl)pentyl]carbamoyl]-3-[5-(2-naphthyl)pentyloxy]propionic acid
2-(2-Hydroxy-3-morpholinopropyloxy)-3-[N-[5-(2-naphthyl)pentyl]carbamoyl]-3-[4-(2-naphthoxy)butoxy]propionic acid
3-[5-(3,4-Dimethylphenyl)pentyloxy]-2-hydroxy-3-[N-[5-(2-naphthyl)pentyl]carbamoyl]propionic acid
3-[5-(3,4-Dimethylphenyl)pentyloxy]-2-hydroxy-3-[N-methyl-N-[5-(2-naphthyl)pentyl]carbamoyl]propionic acid
3-[6-(3,4-Dimethylphenyl)hexyloxy]-2-hydroxy-3-[N-methyl-N-[5-(2-naphthyl)pentyl]carbamoyl]propionic acid
3-[7-(3,4-Dimethylphenyl)heptyloxy]-2-hydroxy-3-[N-methyl-N-[5-(2-naphthyl)pentyl]carbamoyl]propionic acid
2-Acetoxy-3-[5-(3,4-dimethylphenyl)pentyloxy]-3-[N-methyl-N-[5-(2-naphthyl)pentyl]carbamoyl]propionic acid
3-[5-(3,4-Dichlorophenyl)pentyloxy]-2-hydroxy-3-[N-[5-(2-naphthyl)pentyl]carbamoyl]propionic acid
2-Acetoxy-3-[5-(3,4-dichlorophenyl)pentyloxy]-3-[N-[5-(2-naphthyl)pentyl]carbamoyl]propionic acid
3-[5-(3,4-Dichlorophenyl)pentyloxy]-2-hydroxy-3-[N-methyl-N-[5-(2-naphthyl)pentyl]carbamoyl]propionic acid
2-Acetoxy-3-[5-(3,4-dichlorophenyl)pentyloxy]-3-[N-methyl-N-[5-(2-naphthyl)pentyl]carbamoyl]propionic acid
3-[5-(3,4-Dimethylphenyl)pentyloxy]-2-(N-methyl-N-methoxycarbamoyl)methoxy-3-[N-5-(2-naphthyl)pentyl]carbamoyl]propionic acid
3-[5-(3,4-Dimethylphenyl)pentyloxy]-2-(N-methoxycarbamoyl)methoxy-3-[N-[5-(2-naphthyl)pentyl]carbamoyl]propionic acid
3-[5-(3,4-Dimethylphenyl)pentyloxy]-2-(N-methoxycarbamoyl)methoxy-3-[N-methyl-N-[5-(2- naphthyl)pentyl]carbamoyl]propionic acid
2-(N-Benzyloxycarbamoyl)methoxy-3-[5-(3,4-dimethylphenyl)pentyloxy-3-[N-[5-(2-naphthyl)pentyl]carbamoyl]propionic acid
2-(N-Benzyloxycarbamoyl)methoxy-3-[5-(3,4-dimethylphenyl)pentyloxy]-3-[N-methyl-N-[5-(2-naphthyl)pentyl]carbamoyl]propionic acid
2-(2,5-Dimethoxyphenyl)carbonylmethoxy-3-[5-(3,4-dimethylphenyl)pentyloxy]-3-[N-[5-(2-naphthyl)pentyl]carbamoyl]propionic acid
3-[5-(3,4-Dimethylphenyl)pentyloxy]-2-(2-methoxyphenyl)carbonylmethoxy-3-[N-[5-(2-naphthyl)pentyl]carbamoyl]propionic acid
3-[5-(3,4-Dimethylphenyl)pentyloxy]-2-(2-furanyl)carbonylmethoxy-3-[N-[5-(2-naphthyl)pentyl]carbamoyl]propionic acid
3-[5-(3,4-Dimethylphenyl)pentyloxy]-2-(2-thiazolyl)carbonylmethoxy-3-[N-[5-(2-naphthyl)pentyl]carbamoyl]propionic acid
3-[5-(3,4-Dimethylphenyl)pentyloxy]-2-methoxymethoxy-3-[N-methyl-N-[5-(2-naphthyl)pentyl]carbamoyl]propionic acid
3-[5-(3,4-Dimethylphenyl)pentyloxy]-2-(N-(2-hydroxyphenyl)carbamoyl)methoxy-3-[N-methyl-N-[5-(2-naphthyl)pentyl]carbamoyl]propionic acid
3-[5-(3,4-Dimethylphenyl)pentyloxy]-2-[N-(4-hydroxyphenyl)carbamoyl)methoxy-3-[N-methyl-N-[5-(2- naphthyl)pentyl]carbamoyl]propionic acid
3-[N-[5-(1,3-Benzodioxol-5-yl)pentyl]carbamoyl-2-carboxymethoxy-3-[5-(3,4-dimethylphenyl)pentyloxy]propionic acid
3-[N-[5-(1,3-Benzodioxol-5-yl)pentyl]-N-methylcarbamoyl]-2-ethoxycarbonylmethoxy-3-[5-(3,4-dimethylphenyl)pentyloxy]propionic acid
3-[N-[5-(1,3-Benzodioxol-5-yl)pentyl]-N-methylcarbamoyl]-2-ethoxycarboxymethoxy-3-[5-(3,4-dimethylphenyl)pentyloxy]propionic acid
2-Carboxymethoxy-3-[5-(3,4-dimethylphenyl)pentyloxy]-3-[N-methyl-N-[5-(2-naphthyl)pentyl]carbamoyl]propionic acid
3-[5-(3,4-Dimethylphenyl)pentyloxy]-2-ethoxycarbonylmethoxy-3-[N-methyl-N-[5-(2-naphthyl)pentyl]carbamoyl]propionic acid
3-[5-(3,4-Dimethylphenyl)pentyloxy]-3-[N-[5-(2-naphthyl)pentyl]carbamoyl]-2-propyloxycarbonylmethoxypropionic acid
2-Carboxymethoxy-3-[5-(3,4-dichlorophenyl)pentyloxy]-3-[N-methyl-N-[5-(2-naphthyl)pentyl]carbamoyl]propionic acid
3-[5-(3,4-Dichlorophenyl)pentyloxy]-2-ethoxycarbonylmethoxy-3-[N-methyl-N-[5-(2-naphthyl)pentyl]carbamoyl]propionic acid
2-Carboxymethoxy-3-[5-(3,4-dichlorophenyl)pentyloxy]-3-[N-methyl-N-[5-(2-naphthyl)pentyl]carbamoyl]propionic acid
2-Carboxymethoxy-3-[5-(3,4-dimethylphenyl)pentyloxy]-3-[N-methyl-N-[5-(3,4-dimethylphenyl)pentyl]carbamoyl]propionic acid
2-Carboxymethoxy-3-[5-(3,4-dimethylphenyl)pentyloxy]-3-[N-methyl-N-[6-(3,4-dimethylphenyl)hexyl]carbamoyl]propionic acid
2-Carboxymethoxy-3-[5-(3,4-dimethylphenyl)pentyloxy]-3-[N-methyl-N-[7-(3,4-dimethylphenyl)heptyl]carbamoyl]propionic acid
2-Carboxymethoxy-3-[7-(3-chloro-4-methylphenyl)heptyloxy]-3-[N-[5-(2-naphthyl)pentyl]carbamoyl]propionic acid
2-Carboxymethoxy-3-[7-(3,4-dichlorophenyl)heptyloxy]-3-[N-[5-(2-naphthyl)pentyl]carbamoyl]propionic acid

Although there have been many unknown points in the onset mechanism of arteriosclerosis yet, the importance of the oxidation-induced change of low-density lipoprotein (LDL) into oxidized LDL has been pointed out. Described specifically, LDL oxidized in the vascular endothelium is taken up by macrophage. Owing to excessive uptake, however, macrophage has a large amount of cholesterol ester accumulated therein and finally becomes a foam, leading to the rupture of the vascular endothelium. This is considered to be the initial stage of arteriosclerosis. In addition to the control of cholesterol biosynthesis and control of the production amount of LDL thereby, positive inhibition of the oxidation (degeneration) of LDL by an antioxidizing substance such as radical scavenger, control of the generation of active oxygen or the like, which becomes a cause for the oxidation, for example, by chelation of iron ions or the like is presumed to be a desirable countermeasure against the occurrence of arteriosclerosis. From the viewpoints of effectiveness and safety, it is of great significance to lower, like a squalane synthetase inhibitor, the cholesterol level without damaging the biosynthesis of an antioxidizing substance in the body such as ubiquinone or dolichol. Compounds having both the squalane synthetase inhibitory action and antioxidzing action, that is, LDL oxidation (degeneration) inhibitory action are therefore expected to have high effectiveness.
The invention compounds embrace compounds having not only squalane synthetase inhibitory action but also anti-oxidizing action. The following compounds and stereoisomers thereof are typical examples of the compound having both actions.

2-Carboxymethoxy-3-[N-[5-(3,5-dimethyl-4-hydroxyphenyl)pentyl]carbamoyl]-3-[5-(2-naphthyl)pentyloxy]propionic acid
2-Carboxymethoxy-3-[N-[7-(3,5-dimethyl-4-hydroxyphenyl)heptyl]carbamoyl]-3-[5-(2-naphthyl)pentyloxy]propionic acid
2-Carboxymethoxy-3-[N-[5-(2-naphthyl)pentyl]carbamoyl]-3-[5-(3,5-dimethyl-4-hydroxyphenyl)pentyloxy]propionic acid
2-Ethoxycarbonylmethoxy-3-[N-[5-(3,5-dimethyl-4-hydroxyphenyl)pentyl]carbamoyl]-3-[5-(2-naphthyl)pentyloxy]propionic acid.
2-Carboxymethoxy-3-[N-[5-(3,5-di-tert-butyl-4-hydroxyphenyl)pentyl]carbamoyl]-3-[5-(2-naphthyl)pentyloxy]propionic acid
2-Carboxymethoxy-3-[N-[5-(3,4-dihydro-6-hydroxy-2,5,7,8-tetramethyl-2H-1-benzopyran-2-yl)pentyl]carbamoyl]-3-[5-(2-naphthyl)pentyloxy]propionic acid
2-Carboxymethoxy-3-[N-[5-(2-naphthyl)pentyl]carbamoyl]-3-[4-(2-naphthylamino)butoxy]propionic acid
2-Carboxymethoxy-3-[N-[5-(2-naphthyl)pentyl]carbamoyl]-3-[4-(3,4,5-trimethoxyphenylamino)butoxy]propionic acid
2-Carboxymethoxy-3-[N-[5-(2-naphthyl)pentyl]carbamoyl]-3-[4-(1,2-dihydro-2,2,4-trimthylquinolin-6-oxy)butoxy]propionic acid
2-Carboxymethoxy-3-[N-[4-(3,4,5-trimethoxyphenylamino)butyl]carbamoyl]-3-[5-(2-naphthyl)pentyloxy]propionic acid
2-Carboxymethoxy-3-[N-[4-(3,5-dimethylphenylamino)butyl]carbamoyl]-3-[5-(2-naphthyl)pentyloxy]propionic acid
2-Carboxymethoxy-3-[N-[5-(2-naphthyl)pentyl]carbamoyl]-3-[4-(3,5-dimethylphenylamino)butoxy]propionic acid
2-Carboxymethoxy-3-[4-(3-methylphenylamino)butoxy]-3-[N-[5-(2-naphthyl)pentyl]carbamoyl]propionic acid
2-Carboxymethoxy-3-[4-(4-methylphenylamino)butoxy]-3-[N-[5-(2-naphthyl)pentyl]carbamoyl]propionic acid
2-Carboxymethoxy-3-[4-(4-methoxyphenylamino)butoxy]-3-[N-[5-(2-naphthyl)pentyl]carbamoyl]propionic acid
2-Carboxymethoxy-3-[4-(2,4-dimethylphenylamino)butoxy]-3-[N-[5-(2-naphthyl)pentyl]carbamoyl]propionic acid
2-Carboxymethoxy-3-[4-(3,4-dimethylphenylamino)butoxy]-3-[N-[5-(2-naphthyl)pentyl]carbamoyl]propionic acid
2-Carboxymethoxy-3-[4-(3,4-dimethoxylphenylamino)butoxy]-3-[N-[5-(2-naphthyl)pentyl]carbamoyl]propionic acid
2-Carboxymethoxy-3-[4-(1,3-benzodioxol-5-ylamino)butoxy]-3-[N-[5-(2-naphthyl)pentyl]carbamoyl]propionic acid
2-Carboxymethoxy-3-[N-[5-(2-naphthyl)pentyl]carbamoyl]-3-[4-(2,4,6-trimethylphenylamino)butoxy]propionic acid

2-Carboxymethoxy-3-[4-(6-benzothiazolylamino)butoxy]-3-[N-[5-(2-naphthyl)pentyl]carbamoyl]propionic acid

2-Carboxymethoxy-3-[4-(5-indanylamino)butoxy]-3-[N-[5-(2-naphthyl)pentyl]carbamoyl]propionic acid

2-Carboxymethoxy-3-[4-(4-chloro-3-methylphenylamino)butoxy]-3-[N-[5-(2-naphthyl)pentyl]carbamoyl]propionic acid

2-Carboxymethoxy-3-[4-(3-chloro-4-methylphenylamino)butoxy]-3-[N-[5-(2-naphthyl)pentyl]carbamoyl]propionic acid

3-[4-(3-Chloro-4-methylphenylamino)butoxy]-2-ethoxycarbonylmethoxy-3-[N-[5-(2-naphthyl)pentyl]carbamoyl]propionic acid

2-Carboxymethoxy-3-[4-(3-chloro-4-methylphenylamino)butoxy]-3-[N-[5-(4-methylphenyl)pentyl]carbamoyl]propionic acid

2-Carboxymethoxy-3-[4-(3-chloro-4-methylphenylamino)butoxy]-3-[N-[5-(4-chlorophenyl)pentyl]carbamoyl]propionic acid

2-Carboxymethoxy-3-[4-(3-chloro-4-methylphenylamino)butoxy]-3-[N-[5-(4-fluorophenyl)pentyl]carbamoyl]propionic acid

2-Carboxymethoxy-3-[4-(3-chloro-4-methylphenylamino)butoxy]-3-[N-[5-(3,4-dimethylphenyl)pentyl]carbamoyl]propionic acid

2-Carboxymethoxy-3-[4-(3-chloro-4-methylphenylamino)butoxy]-3-[N-[5-(3-chloro-4-methylphenyl)pentyl]carbamoyl]propionic acid

2-Carboxymethoxy-3-[4-(3-chloro-4-methylphenylamino)butoxy]-3-[N-[6-(4-methylphenyl)hexyl]carbamoyl]propionic acid

2-Carboxymethoxy-3-[4-(3-chloro-4-methylphenylamino)butoxy]-3-[N-[6-(4- chlorophenyl)hexyl]carbamoyl]propionic acid

2-Carboxymethoxy-3-[4-(3-chloro-4-methylphenylamino)butoxy]-3-[N-[6-(4-fluorophenyl)hexyl]carbamoyl]propionic acid

2-Carboxymethoxy-3-[4-(3-chloro-4-methylphenylamino)butoxy]-3-[N-[6-(3,4-dimethylphenyl)hexyl]carbamoyl]propionic acid

2-Carboxymethoxy-3-[4-(3-chloro-4-methylphenylamino)butoxy]-3-[N-[6-(3-chloro-4-methylphenyl)hexyl]carbamoyl]propionic acid

2-Carboxymethoxy-3-[4-(3-chloro-4-methylphenylamino)butoxy]-3-[N-[6-(2-naphthyl)hexyl]carbamoyl]propionic acid

2-Carboxymethoxy-3-[4-(3-chloro-4-methylphenylamino)butoxy]-3-[N-[7-(4-methylphenyl)heptyl]carbamoyl]propionic acid

2-Carboxymethoxy-3-[4-(3-chloro-4-methylphenylamino)butoxy]-3-[N-[7-(4-chlorophenyl)heptyl]carbamoyl]propionic acid

2-Carboxymethoxy-3-[4-(3-chloro-4-methylphenylamino)butoxy]-3-[N-[7-(4-fluorophenyl)heptyl]carbamoyl]propionic acid

2-Carboxymethoxy-3-[4-(3-chloro-4-methylphenylamino)butoxy]-3-[N-[7-(3,4-dimethylphenyl)heptyl]carbamoyl]propionic acid

2-Carboxymethoxy-3-[4-(3-chloro-4-methylphenylamino)butoxy]-3-[N-[7-(3-chloro-4-methylphenyl)heptyl]carbamoyl]propionic acid

2-Carboxymethoxy-3-[4-(3-chloro-4-methylphenylamino)butoxy]-3-[N-[7-(2-naphthyl)heptyl]carbamoyl]propionic acid

2-Carboxymethoxy-3-[5-(3-chloro-4-methylphenylamino)pentyloxy]-3-[N-[5-(2-naphthyl)pentyl]carbamoyl]propionic acid

2-Carboxymethoxy-3-[5-(3-chloro-4-methylphenylamino)pentyloxy]-3-[N-[6-(2-naphthyl)hexyl]carbamoyl]propionic acid

2-Carboxymethoxy-3-[5-(3-chloro-4-methylphenylamino)pentyloxy]-3-[N-[7-(2-naphthyl)heptyl]carbamoyl]propionic acid

2-Carboxymethoxy-3-[5-(3-chloro-4-methylphenylamino)pentyloxy]-3-[N-[5-(3,4-dimethylphenyl)pentyl]carbamoyl]propionic acid

2-Carboxymethoxy-3-[5-(3-chloro-4-methylphenylamino)pentyloxy]-3-[N-[6-(3,4-dimethylphenyl)hexyl]carbamoyl]propionic acid

2-Carboxymethoxy-3-[5-(3-chloro-4-methylphenylamino)pentyloxy]-3-[N-[7-(3,4-dimethylphenyl)heptyl]carbamoyl]propionic acid

2-Carboxymethoxy-3-[6-(3-chloro-4-methylphenylamino)hexyloxy]-3-[N-[5-(2- naphthyl)pentyl]carbamoyl]propionic acid

2-Carboxymethoxy-3-[6-(3-chloro-4-methylphenylamino)hexyloxy]-3-[N-[6-(2-naphthyl)hexyl]carbamoyl]propionic acid

2-Carboxymethoxy-3-[6-(3-chloro-4-methylphenylamino)hexyloxy]-3-[N-[7-(2-naphthyl)heptyl]carbamoyl]propionic acid

2-Carboxymethoxy-3-[6-(3-chloro-4-methylphenylamino)hexyloxy]-3-[N-[5-(3,4-dimethylphenyl)pentyl]carbamoyl]propionic acid

2-Carboxymethoxy-3-[6-(3-chloro-4-methylphenylamino)hexyloxy]-3-[N-[6-(3,4-dimethylphenyl)hexyl]carbamoyl]propionic acid

2-Carboxymethoxy-3-[6-(3-chloro-4-methylphenylamino)hexyloxy]-3-[N-(7-(3,4-dimethylphenyl)heptyl]carbamoyl]propionic acid

2-Carboxymethoxy-3-[3-[N-(4-dimethylaminophenyl)carbamoyl]propoxy]-3-[N-[5-(2-naphthyl)pentyl]carbamoyl]propionic acid

2-Carboxymethoxy-3-[3-[N-(4-dimethylaminophenyl)carbamoyl]propoxy]-3-[N-[5-(3,4-dimethylphenyl)pentyl]carbamoyl]propionic acid

2-Carboxymethoxy-3-[4-(3-chloro-4-methylphenylamino)butoxy]-3-[N-methyl-N-[5-(2-naphthyl)pentyl]carbamoyl]propionic acid

3-[4-(3-Chloro-4-methylphenylamino)butoxy]-2-ethoxycarbonylmethoxy-3-[N-methyl-N-[5-(2-naphthyl)pentyl]carbamoyl]propionic acid

2-Carboxymethoxy-3-[4-(3-chloro-4-methylphenylamino)butoxy]-3-[N-[5-(2-naphthyl)pentyl]-N-propylcarbamoyl]propionic acid

2-Carboxymethoxy-3-[4-(3,4-dimethylphenylamino)butoxy]-3-[N-methyl-N-[5-(2-naphthyl)pentyl]carbamoyl]propionic acid

3-[4-(3,4-dimethylphenylamino)butoxy]-2-ethoxycarbonylmethoxy-3-[N-methyl-N-[5-(2-naphthyl)pentyl]carbamoyl]propionic acid

2-Carboxymethoxy-3-[4-(5-indanylamino)butoxy]-3-[N-methyl-N-[5-(2-naphthyl)pentyl]carbamoyl]propionic acid

2-Ethoxycarbonylmethoxy-3-[4-(5-indanylamino)butoxy]-3-[N-methyl-N-[5-(2-naphthyl)pentyl]carbamoyl]propionic acid

2-Carboxymethoxy-3-[4-[N-(3-chloro-4-methylphenyl)-N-methylamino]butoxy]-3-[N-[5-(2-naphthyl)pentyl]carbamoyl]propionic acid

2-Carboxymethoxy-3-[4-[N-(3-chloro-4-methylphenyl)-N-(phenylmethyl)amino]butoxy]-3-[N-[5-(2-naphthyl)pentyl]carbamoyl]propionic acid

2-Carboxymethoxy-3-[4-(3-chloro-4-methylphenylamino)butoxy]-3-[N-[5-(3,4-dichlorophenyl)pentyl]carbamoyl]propionic acid

3-[4-(3-Chloro-4-methylphenylamino)butoxy]-2-hydroxy-3-[N-methyl-N-[5-(2-naphthyl)pentyl]carbamoyl]propionic acid

3-[4-(3,4-Dimethylphenylamino)butoxy]-2-hydroxy-3-[N-methyl-N-[5-(2-naphthyl)pentyl]carbamoyl]propionic acid

2-Hydroxy-3-[4-(5-indanylamino)butoxy]-3-[N-methyl-N-[5-(2-naphthyl)pentyl]carbamoyl]propionic acid

2-Carboxymethoxy-3-[N-[4-(3,4-dimethylphenylamino)butyl]carbamoyl]-3-[5-(2-naphthyl)pentyloxy]propionic acid

2-Carboxymethoxy-3-[N-[5-(3-chloro-4-methylphenylamino)pentyl]carbamoyl]-3-[5-(2-naphthyl)pentyloxy]propionic acid

2-Carboxymethoxy-3-[N-[6-(3-chloro-4-methylphenylamino)hexyl]carbamoyl]-3-[5-(2-naphthyl)pentyloxy]propionic acid

2-Carboxymethoxy-3-[N-[4-(3-chloro-4-methylphenylamino)butyl]carbamoyl]-3-[5-(3,4-dimethylphenyl)pentyloxy]propionic acid

2-Carboxymethoxy-3-[N-[5-(3-chloro-4-methylphenylamino)pentyl]carbamoyl]-3-[5-(3,4-dimethylphenyl)pentyloxy]propionic acid

3-[N-[5-(3-chloro-4-methylphenylamino)pentyl]carbamoyl]-3-[5-(3,4-dimethylphenyl)pentyloxy]-2-ethoxycarbonylmethoxypropionic acid

2-Carboxymethoxy-3-[N-[5-(3-chloro-4-methylphenylamino)pentyl]-N-methylcarbamoyl]-3-[5-(3,4- dimethylphenyl)pentyloxy]propionic acid

3-[N-[5-(3-chloro-4-methylphenylamino)pentyl]-N-methylcarbamoyl]-3-[5-(3,4-dimethylphenyl)pentyloxy]-2-ethoxycarbonylmethoxypropionic acid

2-Carboxymethoxy-3-[N-[6-(3-chloro-4-methylphenylamino)hexyl]carbamoyl]-3-[5-(3,4-dimethylphenyl)pentyloxy]propionic acid

2-Carboxymethoxy-3-[N-[6-(3-chloro-4-methylphenylamino)hexyl]-N-methylcarbamoyl]-3-[5-(3,4-dimethylphenyl)pentyloxy]propionic acid

2-Carboxymethoxy-3-[N-[5-(3,4-dimethylphenylamino)pentyl]-N-methylcarbamoyl]-3-[5-(3,4-dimethylphenyl)pentyloxy]propionic acid

3-[N-[5-(3,4-dimethylphenylamino)pentyl]-N-methylcarbamoyl]-3-[5-(3,4-dimethylphenyl)pentyloxy]-2-ethoxycarbonylmethoxypropionic acid

2-Carboxymethoxy-3-[5-(3,4-dimethylphenyl)pentyloxy]-3-[N-[5-(5-indanylamino)pentyl]-N-methylcarbamoyl]propionic acid

3-[5-(3,4-Dimethylphenyl)pentyloxy]-2-ethoxycarbonylmethoxy-3-[N-[5-(5-indanylamino)pentyl]-N-methylcarbamoyl]propionic acid

3-[N-[5-(3-chloro-4-methylphenylamino)pentyl]-N-methylcarbamoyl]-3-[5-(3,4-dimethylphenyl)pentyloxy]-2-hydroxypropionic acid

3-[N-[5-(3,4-Dimethylphenylamino)pentyl]-N-methylcarbamoyl]-3-[5-(3,4-dimethylphenyl)pentyloxy]-2-hydroxypropionic acid

3-[5-(3,4-Dimethylphenyl)pentyloxy]-2-hydroxy-3-[N-[5-(5-indanylamino)pentyl]-N-methylcarbamoyl]propionic acid

2-Carboxymethoxy-3-[4-(3-chloro-4-methylphenylamino)butoxy]-3-[N-[5-(3-chloro-4-methylphenylamino)pentyl]-N-methylcarbamoyl]propionic acid

2-Carboxymethoxy-3-[4-(3-chloro-4-methylphenylamino)butoxy]-3-[N-[5-(3,4-dimethylphenylamino)pentyl]-N-methylcarbamoyl]propionic acid

2-Carboxymethoxy-3-[4-(3-chloro-4-methylphenylamino)butoxy]-3-[N-[5-(5-indanylamino)pentyl]-N-methylcarbamoyl]propionic acid

2-Carboxymethoxy-3-[N-[5-(3-chloro-4-methylphenylamino)pentyl]-N-methylcarbamoyl]-3-[4-(3,4-dimethylphenylamino)butoxy]propionic acid

2-Carboxymethoxy-3-[4-(3,4-dimethylphenylamino)butoxy]-3-[N-[5-(3,4-dimethylphenylamino)pentyl]-N-methylcarbamoyl]propionic acid

2-Carboxymethoxy-3-[4-(3,4-dimethylphenylamino)butoxy]-3-[N-[5-(5-indanylamino)pentyl]-N-methylcarbamoyl]propionic acid

2-Carboxymethoxy-3-[N-[5-(3-chloro-4-methylphenylamino)pentyl]-N-methylcarbamoyl]-3-[4-(5-indanylamino)butoxy]propionic acid

2-Carboxymethoxy-3-[N-[5-(3,4-dimethylphenylamino)pentyl]-N-methylcarbamoyl]-3-[4-(5-indanylamino)butoxy]propionic acid

2-Carboxymethoxy-3-[4-(5-indanylamino)butoxy]-3-N-[5-(5-indanylamino)pentyl]-N-methylcarbamoyl]propionic acid

3-[4-(3-Chloro-4-methylphenylamino)butoxy]-3-[N-[5-(3-chloro-4-methylphenylamino)pentyl]-N-methylcarbamoyl]-2-hydroxypropionic acid

3-[4-(3-Chloro-4-methylphenylamino)butoxy]-3-[N-[5-(3,4-dimethylphenylamino)pentyl]-N-methylcarbamoyl]-2-hydroxypropionic acid

3-[4-(3-Chloro-4-methylphenylamino)butoxy]-2-hydroxy-3-[N-[5-(5-indanylamino)pentyl]-N-methylcarbamoyl]propionic acid

3-[N-[5-(3-chloro-4-methylphenylamino)pentyl]-N-methylcarbamoyl]-3-[4-(3,4-dimethylphenylamino)butoxy]-2-hydroxypropionic acid

3-[4-(3,4-Dimethylphenylamino)butoxy]-3-[N-[5-(3,4-dimethylphenylamino)pentyl]-N-methylcarbamoyl]-2-hydroxypropionic acid

3-[4-(3,4-Dimethylphenylamino)butoxy]-2-hydroxy-3-[N-[5-(5-indanylamino)pentyl]-N-methylcarbamoyl]propionic acid

3-[N-[5-(3-Chloro-4-methylphenylamino)pentyl]-N-methylcarbamoyl]-2-hydroxy-3-[4-(5-indanylamino)butoxy]propionic acid

3-[N-[5-(3,4-Dimethylphenylamino)pentyl]-N-methylcarbamoyl]-2-hydroxy-3-[4-(5-indanylamino)butoxy]propionic acid

2-Hydroxy-3-[4-(5-indanylamino)butoxy]-3-[N-[5-(5-indanylamino)pentyl]-N-methylcarbamoyl]propionic acid

2-Carboxymethoxy-3-[N-methyl-N-[5-(2-napnthyl)pentyl]carbamoyl]-3-[4-(4-methylphenylamino)butoxy]propionic acid

2-Carboxymethoxy-3-[4-(4-methoxyphenylamino)butoxy]-3-[N-methyl-N-[5-(2-naphthyl)pentyl]carbamoyl]propionic acid

3-[4-(1,3-benzodioxol-5-ylamino)butoxy]-2-carboxymethoxy-3-[N-methyl-N-[5-(2-naphthyl)pentyl]carbamoyl]propionic acid

2-Carboxymethoxy-3-[5-(3,4-dimethylphenyl)pentyloxy]-3-[N-methyl-N-[5-(4-methylphenylamino)pentyl]carbamoyl]propionic acid

2-Carboxymethoxy-3-[5-(3,4-dimethylphenyl)pentyloxy]-3-[N-[5-(4-methoxyphenylamino)pentyl]-N-methylcarbamoyl]propionic acid

2-Carboxymethoxy-3-[5-(3,4-dimethylphenyl)pentyloxy]-3-[N-[5-(1,3-benzodioxol-5-ylamino)pentyl]-N-methylcarbamoyl]propionic acid

The invention compound (1) can be prepared, for example, by Process A, Process B or Process C which will

be described below.

[Process A]

Process for the preparation of Compound (1-1) represented by the formula (1) wherein $X^1$ represents a carboxyl group which may be esterified and $Y^1$ represents -O- or - N($R^1$)-:

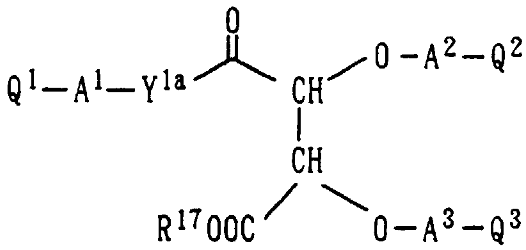

(1 - 1)

(wherein $R^{17}$ represents a hydrogen atom or an ester residue, $Y^{1a}$ represents -O- or -N($R^1$)- and $Q^1$, $Q^2$, $Q^3$, $A^1$, $A^2$, $A^3$ and $R^1$ have the same meanings as described above).

The above-described compound (1-1) can be prepared in accordance with the below-described reaction scheme (A-1) by using, for example, Compound (2) [Journal of Organic Chemistry, **50**, 3462(1985)] or the like.

[Reaction scheme (A-1)]

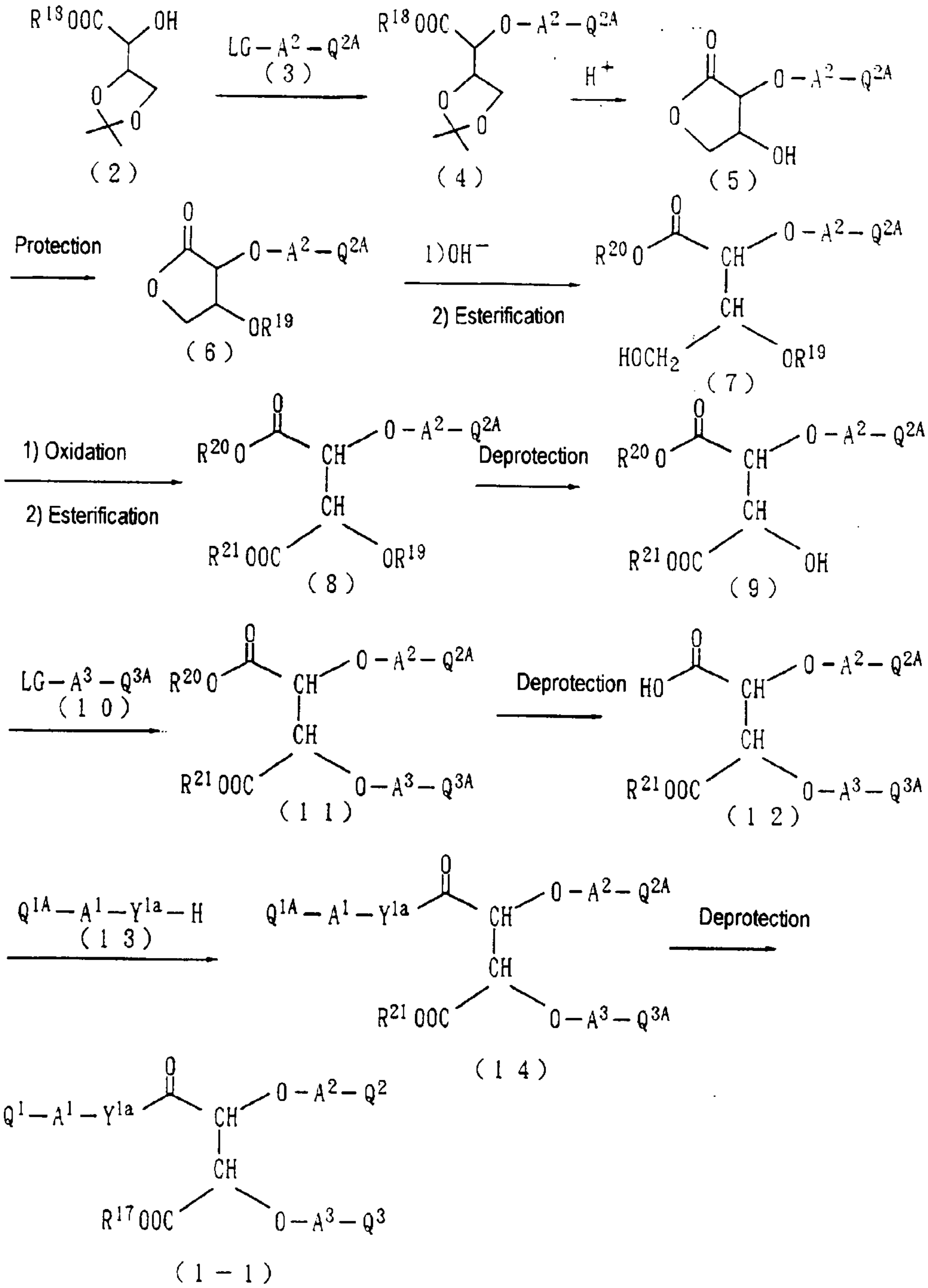


[wherein $R^{18}$, $R^{20}$ and $R^{21}$ each independently represents an ester residue, $R^{19}$ represents a protecting group for the hydroxyl group, $Q^{1A}$ represents $Q^1$ whose functional group may be protected, $Q^{2A}$ represents $Q^2$ whose functional group may be protected, $Q^{3A}$ represents $Q^3$ whose functional group may be protected, LG represents a leaving group and $A^1$, $A^2$, $A^3$, $Y^{1a}$ and $R^{13}$ have the same meanings as described above].

In the above reaction scheme, examples of the ester residue represented by $R^{18}$, $R^{20}$ or $R^{21}$ include alkyl, allyl and aralkyl (such as benzyl, nitrobenzyl, p-methoxybenzyl and diphenylmethyl) groups. Examples of the protecting group for the hydroxyl group include benzyl, nitrobenzyl, p-methoxybenzyl, diphenylmethyl, triphenylmethyl, methoxymethyl, benzyloxymethyl, tert-butyldimethylsilyl and tert-butyldiphenylsilyl groups. Examples of the leaving group include halogen atoms and sulfonyloxy groups such as methanesulfonyloxy and p-toluenesulfonyloxy. Examples of the

protecting group for the functional group of $Q^{1A}$, $Q^{2A}$ or $Q^{3A}$ include protecting groups for the hydroxyl or carboxyl group, such as alkyl, allyl, aralkyl, methoxymethyl, tetrahydropyranyl, triphenylmethyl, tert-butyldimethylsilyl, tert-butyldiphenylsilyl and 2-trimethylsilylethyl groups; and also protecting groups for amino or alkylamino group such as acyl, alkoxycarbonyl and aralkyloxycarbonyl groups.

Preparation of Compound (4) from Compound (2) and Compound (3) or Compound (11) from Compound (9) and Compound (10) is carried out by reacting 1 mole or excess moles, preferably 1 to 2 moles, of the compound of the formula (3) or (10) to 1 mole of the starting material (2) or (9) in an inert solvent in the presence of a base. Examples of the inert solvent include diethyl ether, tetrahydrofuran, dioxane, benzene, toluene, N,N-dimethylformamide and dimethyl sulfoxide and mixtures thereof. Examples of the base include hydrogenated alkali metals such as sodium hydride, lithium hydride and potassium hydride; alkali metal amides such as lithium amide, lithium diisopropylamide, lithium bistrimethylsilylamide, sodium bistrimethylsilylamide and potassium bistrimethylsilylamide, alkyl lithiums such as methyl lithium, butyl lithium, tert-butyl lithium; and alkyl metal alkoxides such as sodium methoxide, sodium ethoxide and potassium tert-butoxide. The base is usually employed in an amount of 1 mole or excess moles, preferably 1 to 3 moles based on the starting material. The reaction temperature usually ranges from -100°C to the boiling point of the solvent to be used in the reaction, preferably -78°C to 80°C. The reaction time usually ranges from 10 minutes to 48 hours, preferably 20 minutes to 24 hours.

Preparation of Compound (5) from Compound (4) is carried out in an inert solvent (methylene chloride, tetrahydrofuran or the like) in the presence of an acid such as hydrochloric acid, acetic acid, trifluoroacetic acid or p-toluenesulfonic acid.

The hydrolysis of Compound (6) which is a lactone is carried out, for example, by acting thereon an aqueous solution of a hydroxide of an alkali metal such as sodium hydroxide, potassium hydroxide or lithium hydroxide or an aqueous solution of a carbonate of an alkali metal such as sodium carbonate or potassium carbonate in a solvent such as tetrahydrofuran or ethanol.

A process for the oxidation of Compound (7) which is a primary alcohol, thereby preparing the corresponding carboxylic acid is carried out by using Jones reagent (Organic Synthesis, IV, 310,1973) or acting an oxidizing agent on Compound (7) in the presence of a nitroxyl radical derivative. The process for the preparation of the corresponding carboxylic acid by acting thereon an oxidizing agent in the presence of a nitroxyl radical derivative can be carried out, for example, by acting sodium hypochlorite, sodium bromite, calcium hypochlorite or the like as an oxidizing agent in the presence of a nitroxyl radical derivative such as 4-benzoyloxy-2,2,6,6-tetramethylpiperidinyl-1-oxy free radical. The nitroxyl radical derivative is added in an amount of 0.5 to 3 mole%, preferably 1 mole% based on the starting material. The oxidizing agent is usually added in an amount of 1 mole to excess moles, preferably 3 to 6 moles based on the raw material. As a solvent, a solvent mixture of a solvent inert to the reaction such as acetonitrile and an aqueous solution of sodium bicarbonate is employed. The reaction temperature usually ranges from -78°C to the boiling point of the solvent used for the reaction, preferably from -20°C to 50°C. The reaction time usually ranges from 10 minutes to 48 hours, preferably from 30 minutes to 24 hours.

As another process for oxidizing a primary alcohol, thereby preparing the corresponding carboxylic acid, the primary alcohol can be converted into the corresponding aldehyde by Swern oxidation (Journal of Organic Chemistry, 43, 2480, 1978) using oxalyl chloride and dimethyl sulfoxide, oxidation by using a Dess-Martin periodinane (Journal of Organic Chemistry, 48, 4155, 1983) or oxidation by using tetra-n-propylammonium perruthenate and 4-methylmorpholin-4-oxide or the like (Synthesis, 639, 1994), followed by the oxidation in the presence of sodium chlorite or the like to convert into the corresponding carboxylic acid.

A process for the preparation of Compound (14) from Compound (12) and Compound (13) is carried out by acting, on 1 mole of the raw material (12), 1 mole or excess moles, preferably 1 to 2 moles of Compound (13) together with a condensing agent in an inert solvent. If necessary, the above reaction may be carried out in the presence of a base such as triethylamine or 4-dimethylaminopyridine and moreover, it is possible to add an N-hydroxy compound such as 1-hydroxybenzotriazole, N-hydroxysuccinimide or N-hydroxyphthalimide or a phenol compound such as 4-nitrophenol, 2,4-dinitrophenol, 2,4,5-trichlorophenol or pentachlorophenol as a reaction accelerator. Examples of the inert solvent include methylene chloride, chloroform, dichloroethane, diethyl ether, tetrahydrofuran, dioxane, benzene, toluene, N,N-dimethylformamide, acetonitrile, acetone and ethyl acetate and mixtures thereof. Examples of the condensing agent include N,N'-dicyclohexylcarbodiimide and 1-ethyl-3-(3-dimethylaminopropyl)carbodiimide hydrochloride. To 1 mole of the raw material (12), 1 mole or excess moles, preferably 1 to 3 moles of the condensing agent is usually employed. The reaction temperature usually ranges from -100°C to the boiling point of the solvent used for the reaction, preferably from -78°C to 80°C. The reaction time usually ranges from 10 minutes to 48 hours, preferably from 30 minutes to 24 hours.

The introduction of a protecting group in the reaction from Compound (6) to Compound (7), esterification of the carboxylic acid in the reaction from Compound (6) to Compound (7) and from Compound (7) to Compound (8), and removal of the protecting group in the reaction from Compound (8) to Compound (9), from Compound (11) to Compound (12) and from Compound (14) to Compound (1-1) each differs depending on the kind of the protecting group or

ester, but can be carried out in accordance with the process as described in the literature [refer to 'Protective Groups in Organic Synthesis, 2nd Edition, written by T.W. Green, P.G.M. Wuts, published by John Wiley & Sons (1991)"] or a process similar thereto. For example, removal is carried out by hydrolysis using an acid or base, hydrogenation in the presence of a palladium-carbon catalyst or Raney nickel, a method using trifluoroacetic acid, or the like method.

Compound (7) in the reaction scheme (A-1) can also be synthesized in accordance with the following reaction scheme (A-2):

[Reaction scheme (A-2)]

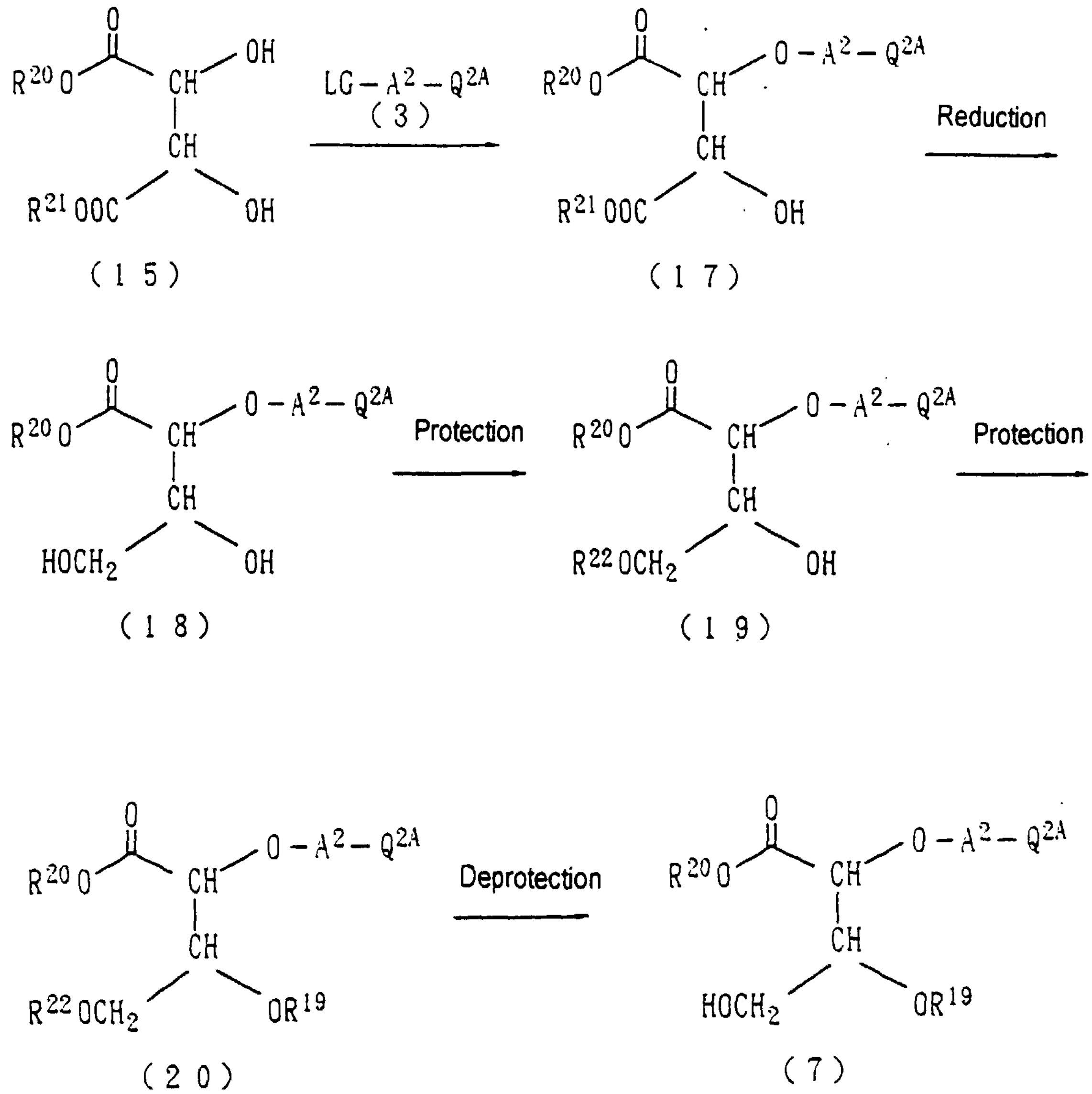


(15) (17) (18) (19) (20) (7)

[wherein $R^{22}$ represents a protecting group for the hydroxyl group and LG, $A^2$, $Q^{2A}$, $R^{19}$, $R^{20}$ and $R^{21}$ have the same meanings as described above].

Conversion (reduction) from Compound (17) to Compound (18) can be carried out, for example, in accordance with the method as described in "Chemistry Letters, 1389-1392(1984)", described specifically, by acting a borane-dimethyl sulfide complex and a catalytic amount of sodium borohydride on Compound (17) in an inert solvent such as tetrahydrofuran. Preparation processes for the steps other than the above-described step can be carried out in accordance with the processes in the reaction scheme (A-1).

Compound (9) in the reaction scheme (A-1) can also be synthesized in accordance with the following reaction scheme (A-3):

[Reaction scheme (A-3)]

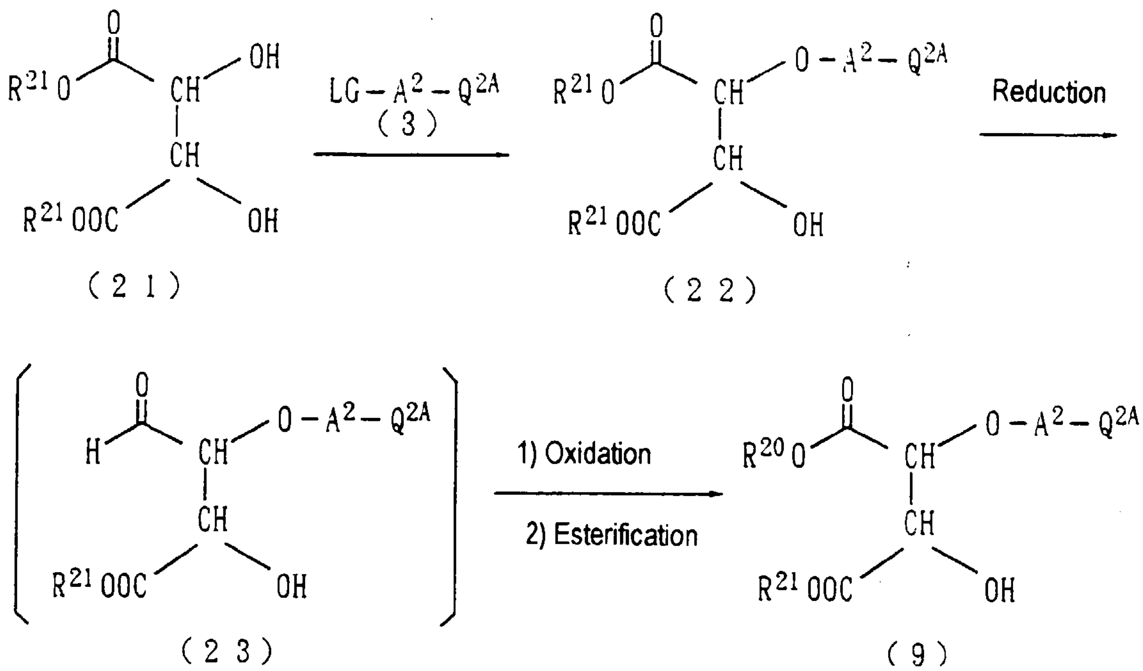


(21) (22) (23) (9)

[wherein LG, $A^2$, $Q^{2A}$, $R^{20}$ and $R^{21}$ have the same meanings as described above].

Preparation of Compound (23) from Compound (22) is carried out by acting 1 mole to excess moles, preferably 1 to 5 moles of a reducing agent on 1 mole of the raw material (22) in an inert solvent in the presence of a Lewis acid. Examples of the inert solvent include tetrahydrofuran, dioxane, benzene, toluene and N,N-dimethylformamide and mixtures thereof. Examples of the Lewis acid include diethylmethoxyborane and it is usually added in an amount of 1 mole to excess moles, preferably 1 to 3 moles to 1 mole of the raw material (22). Examples of the reducing agent include hydrogenated metal complexes such as sodium borohydride, sodium trimethoxyborohydride and diisobutyl sodium hydride. The reaction temperature usually ranges from -78°C to the boiling point of the solvent used for the reaction, preferably from -50°C to 50°C. The reaction time usually ranges from 10 minutes to 48 hours, preferably from 1 hour to 24 hours.

Oxidation of Compound (23), thereby preparing the corresponding carboxylic acid intermediate can be carried out in the conventional manner, described specifically, by acting, on 1 mole of the raw material (23), 1 mole to excess moles, preferably 3 to 30 moles of an oxidizing agent in a liquid mixture of an inert solvent and an aqueous solution of sodium dihydrogenphosphate in the presence of 2-methyl-2-butene. Examples of the inert solvent include tetrahydrofuran, dioxane, benzene, toluene, N,N-dimethylformamide and tert-butanol and mixtures thereof. Examples of the oxidizing agent include sodium chlorite. The reaction temperature usually ranges from -78°C to the boiling point of the solvent used for the reaction, preferably from -20°C to 50°C. The reaction time usually ranges from 1 hour to 5 days, preferably from 3 hours to 3 days.

Preparation processes for the steps other than the above-described step can be carried out in accordance with the processes in the reaction scheme (A-1).

Compound (1-1) can also be prepared in accordance with the below-described reaction scheme (A-4) by using tartaric acid as a raw material.

[Reaction scheme (A-4)]

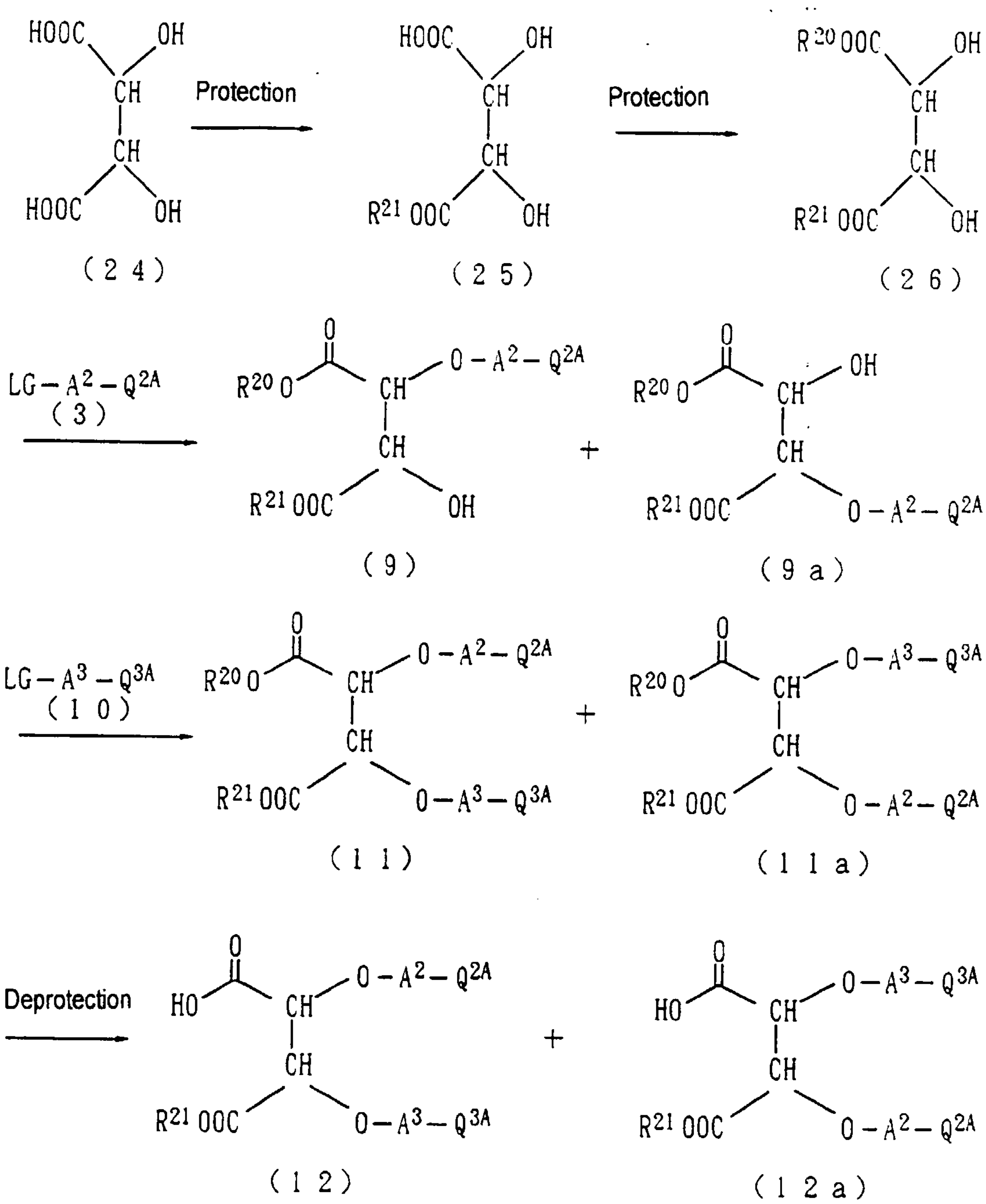

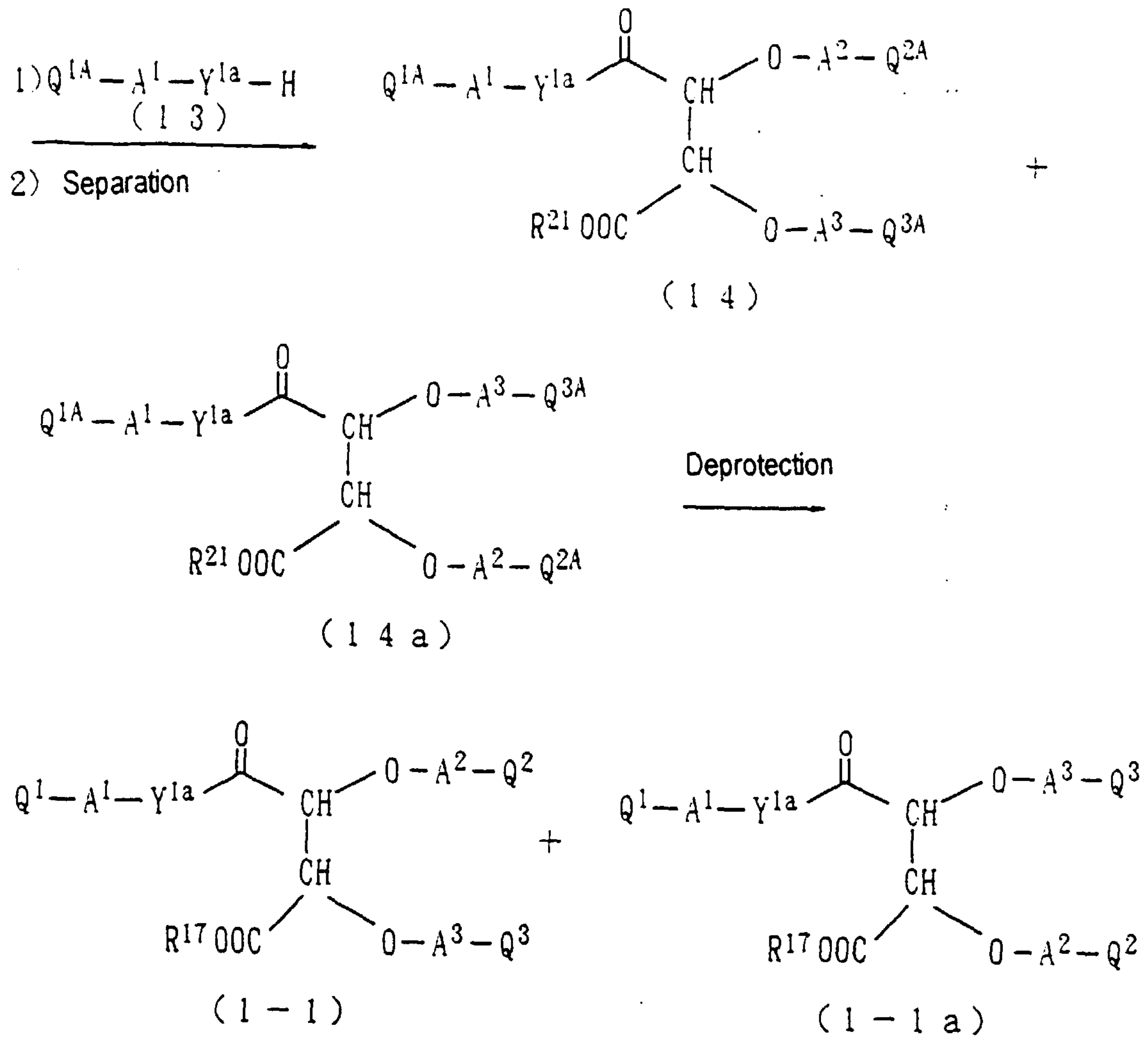


(14)

(14a)

(1-1)

(1-1a)

[wherein $R^{17}$, $R^{20}$, $R^{21}$, LG, $A^1$, $A^2$, $A^3$, $Q^{1A}$, $Q^{2A}$, $Q^{3A}$, $Q^1$, $Q^2$, $Q^3$ and $Y^{1a}$ have the same meaning as described above].

In the reaction scheme (A-4), reaction in each step can be carried out in accordance with the process of the above-described reaction scheme (A-1), (A-2) or (A-3). Compound (14) can be obtained by condensing Compound (13) and a mixture of Compound (12) and Compound (12a) in accordance with the above-described process and then separating arid purifying from Compound (14a) by silica gel column chromatography.

Similar reactions can be carried out by employing, instead of

$LG-A^2-Q^{2A}$ (3),
$LG-A^3-Q^{3A}$ (10) and
$Q^{1A}-A^1-Y^{1a}-H$ (13) used in the above-described reaction schemes (A-1) to (A-4),
$LG-A^{2A}-O-PG^2$ (101),
$LG-A^{3A}-O-PG^3$ (102) and
$PG^1-O-A^{1A}-Y^{1a}-H$ (103)

[wherein $PG^1$, $PG^2$ and $PG^3$ individually represent the protecting group for the hydroxyl group or ester residue and others have the same meanings as described above] as needed. Each intermediate prepared by the reaction can be introduced into the target compound by the elimination of a protecting group (PG) at a proper stage in the subsequent reaction, followed by the reaction as described below. For example, the intermediate (14) in the reaction scheme (A1) can be introduced by the process as shown by the reaction scheme (A5-1).

[Reaction scheme A5-1]

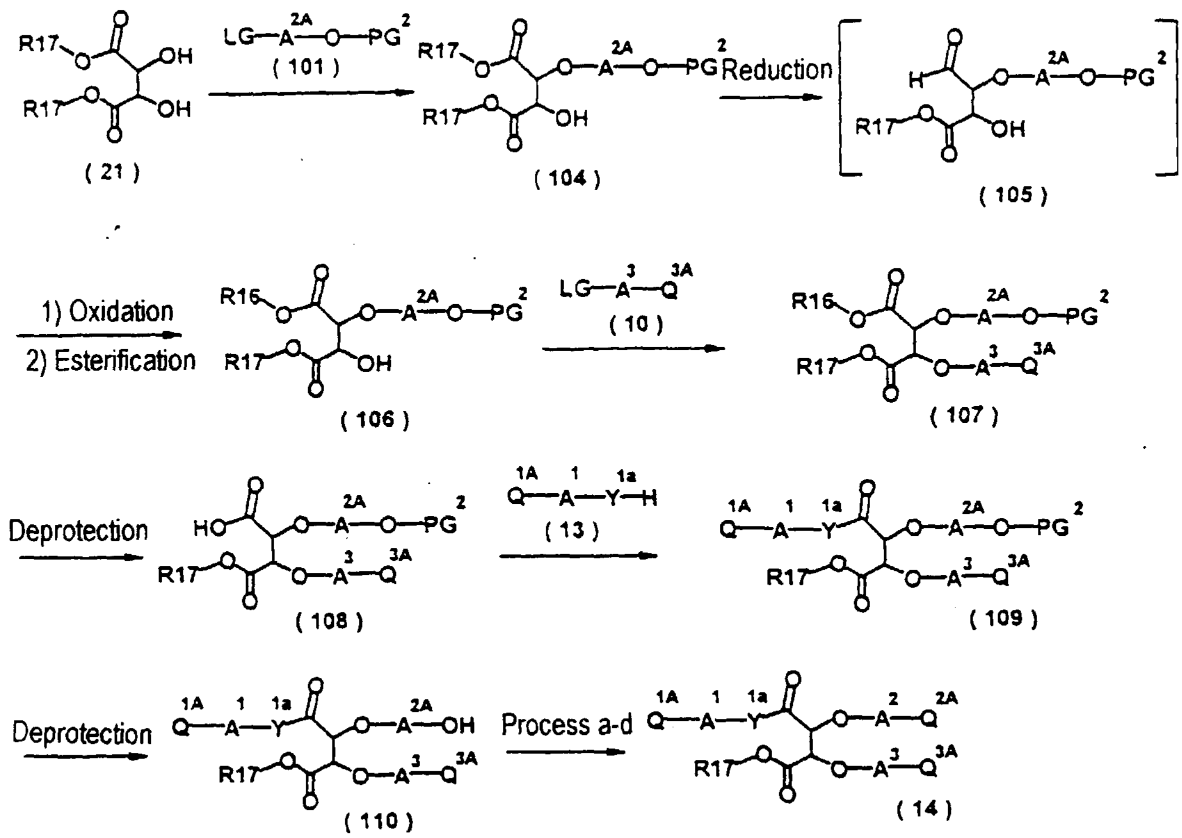


[wherein, each symbol has the same meaning as described above].

In the above reaction scheme, examples of the protecting group for the hydroxyl group represented by $PG^2$ include benzyl, nitrobenzyl, p-methoxybenzyl, diphenylmethyl, triphenylmethyl, methoxymethyl, benzyloxymethyl, tetrahydropyranyl, tert-butyldimethylsilyl, tert-butyldiphenylsilyl and 2-trimethylsilylethyl groups.

Preparation of Compound (104) from Compound (21) is carried out by acting, on 1 mole of the diester (21), 1 mole to excess moles, preferably, 1 to 2 moles of a compound represented by the formula (101) in an inert solvent in the presence of a base. Examples of the inert solvent include diethyl ether, tetrahydrofuran, dioxane, benzene, toluene, N,N-dimethylformamide and dimethyl sulfoxide and mixtures thereof. Examples of the base include hydrogenated alkali metals such as sodium hydride, lithium hydride and potassium hydride, alkali metal amides such as lithium amide, lithium diisopropylamide, lithium bistrimethylsilylamide, sodium bistrimethylsilylamide and potassium bistrimethylsilylamide, alkyl lithiums such as methyl lithium, butyl lithium and tert-butyl lithium, alkyl metal alkoxides such as sodium methoxide, sodium ethoxide and potassium tert-butoxide. The base is usually added in an amount of 1 mole to excess moles, preferably 1 to 3 moles to 1 mole of the raw material (21). The reaction temperature usually ranges from -100°C to the boiling point of the solvent used for the reaction, preferably from -78°C to 80°C. The reaction time usually ranges from 10 minutes to 48 hours, preferably from 20 minutes to 24 hours.

Preparation in each step from Compound (104) to Compound (110) can be carried out in accordance with the process of the above-described reaction scheme (A-1), (A-2) or (A-3).

A specific description will next be made of Process a-d in the above-described reaction scheme.

Process a is, for example, a process (Process a-1), as shown by the below-described reaction scheme (A5-2), for converting the alcohol compound (110) into the corresponding ketone or formyl compound (111) by oxidation, and subjecting it to Wittig reaction to act a phosphonium salt of the formula (112) on the compound (111) in the presence of a base or the reaction to act a phosphonate derivative of the formula (113) on the compound (111) in the presence of a base, thereby introducing the compound (111) into the corresponding vinylene derivative (14-1). Alternatively, an amine derivative (14-2) can be prepared (Process a-2) by acting, on the compound (111), an amine derivative of the

formula (114) and then carrying out reaction using a suitable reducing agent or catalytic reduction.

[Reaction scheme (A5-2)]

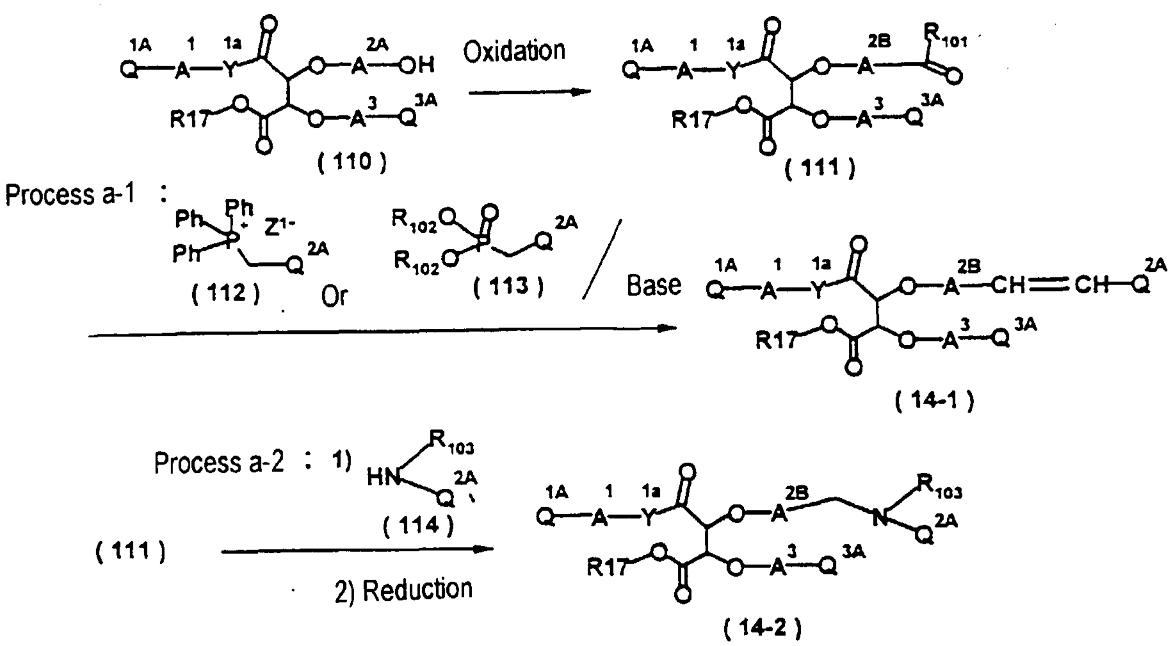


[wherein $Z^1$ represents a chlorine, bromine or iodine atom, $R^{101}$ and $R^{103}$ individually represent a hydrogen atom or a substituted or unsubstituted hydrocarbon group, $R^{102}$ represents a substituted or unsubstituted alkyl group and others have the same meanings as described above].

In the above reaction scheme, the substituted or unsubstituted hydrocarbon group represented by $R^{101}$ or $R^{103}$ is similar to that exemplified above by $R^1$ and examples of the substituted or unsubstituted alkyl group represented by $R^{102}$ include ethyl and 2,2,2-trifluoroethyl groups.

Preparation of Compound (111) from Compound (110) is carried out by oxidation in the conventional manner, for example, that described in the literature such as Swern oxidation using oxalyl chloride and dimethyl sulfoxide, oxidation using a Dess-Martin periodinane or oxidation using tetra-n-propylammonium perruthenate and 4-methylmorpholin-4-oxide.

Preparation of Compound (14-1) from Compound (111) is carried out in the conventional manner, described specifically, by acting 1 to excess moles, preferably 1 to 3 moles, of the compound of the formula (112) or (113) on 1 mole of the carbonyl derivative (111) in an inert solvent in the presence of a base. Examples of the inert solvent include diethyl ether, tetrahydrofuran, dioxane, benzene, toluene, N,N-dimethylformamide and dimethyl sulfoxide and mixtures thereof. Examples of the base include hydrogenated alkali metals such as sodium hydride, lithium hydride and potassium hydride; alkali metal amides such as lithium amide, lithium diisopropylamide, lithium bistrimethylsilylamide, sodium bistrimethylsilylamide and potassium bistrimethylsilylamide; alkyl lithiums such as methyl lithium, butyl lithium and tert-butyl lithium; and alkyl metal alkoxides such as sodium methoxide, sodium ethoxide, sodium 2-methyl-2-butoxide and potassium tert-butoxide. The base is usually added in an amount of 1 mole to excess moles, preferably 1 to 2 moles to 1 mole of the compound (112) or (113). The reaction temperature usually ranges from -100°C to the boiling point of the solvent used for the reaction, preferably from -78°C to 80°C. The reaction time usually ranges from 10 minutes to 48 hours, preferably from 30 minutes to 24 hours. It is preferred to start the reaction by adding the carbonyl derivative (111) after mixing the base and the compound (112) or (113) in advance.

Preparation of Compound (14-2) from Compound (111) is carried out by acting 1 mole or excess moles, preferably 1 to 2 moles, of the amine derivative of the formula (114) on 1 mole of the carbonyl derivative (111) in an inert solvent and then hydrogenating in the presence of a catalyst such as palladium-carbon or acting 1 mole or excess moles, preferably 1 to 3 moles, of a reducing agent such as a hydrogenated metal complex. Examples of the inert solvent include solvents such as dimethoxyethane, tetrahydrofuran, dioxane, benzene, toluene, N,N-dimethylformamide and N,N-dimethylacetamide; and alcohols such as methanol and ethanol; and mixtures thereof. Examples of the hydrogenated metal complex include sodium borohydride, lithium borohydride and sodium cyanoborohydride. The reaction temperature usually ranges from -78°C to the boiling point of the solvent used for the reaction, preferably from -78°C to

80°C. The reaction time usually ranges from 10 minutes to 48 hours, preferably from 30 minutes to 24 hours.

Process b is a process (Process b-1) for preparing Compound (14-3) by the so-called Mitsunobu reaction, that is, the reaction to act a compound of the formula (115), diethyl azodicarboxylate (DEAD) and triphenylphosphine (PPh3) on the alcohol compound (110) as shown in the below-described reaction scheme (A5-3). Alternatively, Compound (14-4) can be prepared by acting a dinitrobenzenesulfonamide derivative represented by the formula (116), together with diethyl azodicarboxylate and triphenylphosphine, on the compound (110) and then acting thioglycolic acid in the presence of a base such as triethylamine (Process b-2).

[Reaction scheme (A5-3)]

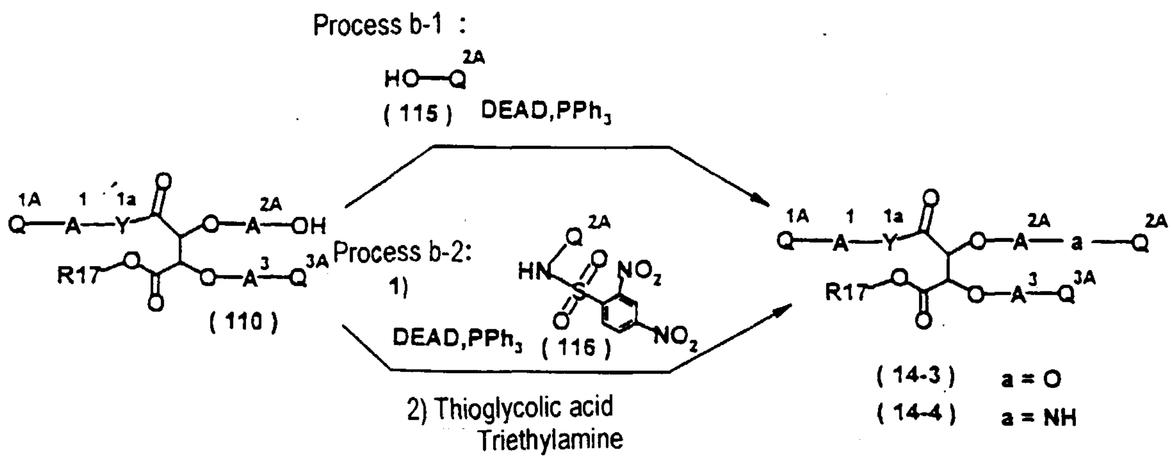


[wherein symbols have the same meanings as described above].

Preparation of Compound (14-3) from Compound (110) is carried out by acting 1 mole or excess moles, preferably 1 to 3 moles, of each of Compound of the formula (115), diethyl azodicarboxylate and triphenyl phosphine to 1 mole of the alcohol compound (110) in an inert solvent. Examples of the inert solvent include solvents such as diethyl ether, tetrahydrofuran, dioxane, benzene, toluene, N,N-dimethylformamide and dimethyl sulfoxide; and mixtures thereof. The reaction temperature usually ranges from - 78°C to the boiling point of the solvent used for the reaction, preferably from -20°C to 60°C. The reaction time usually ranges from 5 minutes to 48 hours, preferably from 30 minutes to 24 hours.

The first step for the preparation of Compound (14-4) from Compound (110) is carried out by acting 1 mole or excess moles, preferably 1 to 3 moles, of each of a compound of the formula (116), diethyl azodicarboxylate and triphenyl phosphine on 1 mole of the alcohol compound (110) in an inert solvent. Examples of the inert solvent include solvents such as diethyl ether, tetrahydrofuran, dioxane, benzene, toluene, N,N-dimethylformamide and dimethyl sulfoxide; and mixtures thereof. The reaction temperature usually ranges from -78°C to the boiling point of the solvent used for the reaction, preferably from -20 to 60°C. The reaction time usually ranges from 5 minutes to 48 hours, preferably from 30 minutes to 24 hours. The second step is carried out by acting 1 mole or excess moles, preferably 1 to 5 moles, of thioglycolic acid on 1 mole of the intermediate, which had been obtained in the above step, in an inert solvent in the presence of a base. Examples of the inert solvent include solvents such as methylene chloride, diethyl ether, tetrahydrofuran, dioxane, benzene, toluene and N,N-dimethylformamide; and mixtures thereof. Examples of the base include alkylamines such as triethylamine, carbonates of an alkali metal such as potassium carbonate and hydroxides of an alkali metal such as lithium hydroxide. The base is added in an amount of 1 mole to excess moles, preferably 1 to 10 moles, to 1 mole of the intermediate. The reaction temperature usually ranges from - 78°C to the boiling point of the solvent used for the reaction, preferably from -20 to 60°C. The reaction time usually ranges from 5 minutes to 36 hours, preferably from 10 minutes to 24 hours.

Process c is, for example, a process (Process c-1 or c-3) for the preparation of Compound (14-3) or (14-5) by activating the hydroxyl group of the alcohol compound (110) with a suitable eliminative group, thereby converting the alcohol compound (110) into Compound (117) and acting a compound of the formula (115) or (118) on the compound (117) in the presence of a base, as shown in the below-described reaction scheme (A5-4). Alternatively, Compound (14-4) can be prepared by acting a dinitrobenzene sulfonamide derivative of the formula (116) on Compound (117) in the presence of a base and then acting thioglycolic acid in the presence of a base such as triethylamine (Process c-2).

[Reaction scheme (A5-4)]

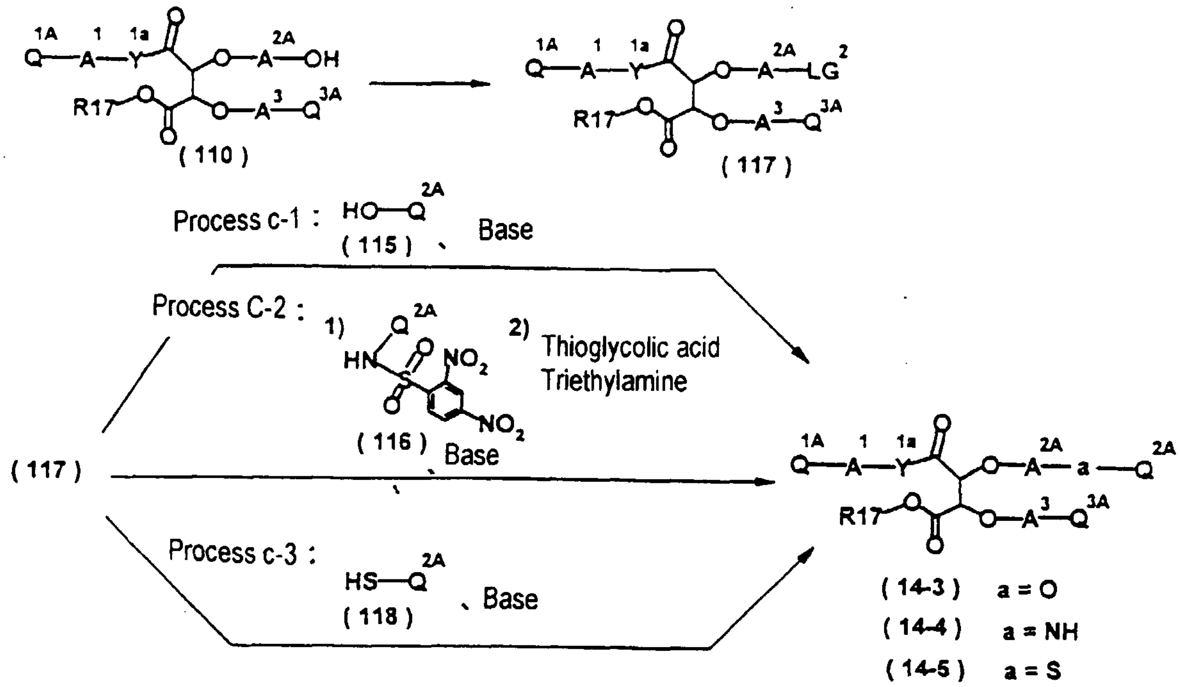


[wherein, LG2 has the same meaning as described in the above-described LG (leaving group) and others have the same meanings as described above].

Preparation of Compound (117) from Compound (110) is carried out in the conventional manner, described specifically, halogenation, methanesulfonyloxy formation, benzenesulfonyloxy formation or p-toluenesulfonyloxy formation. Examples of the halogenation include chlorination with thionyl chloride, bromination with boron tribromide or dibromotriphenyl phosphine and iodination using sodium iodide and trimethylchlorosilane. Methanesulfonyloxy formation, benzenesulfonyloxy formation or p-toluenesulfonyloxy formation is carried out by acting methanesulfonyl chloride, benzenesulfonyl chloride or p-toluenesulfonyl chloride on Compound (117) in the presence of a base, respectively.

Preparation of Compound (14-3), (14-4) or (14-5) from Compound (117) is carried out by acting 1 mole or excess moles, preferably 1 to 3 moles, of the compound of the formula (115), (116) or (117) on 1 mole of Compound (117) in an inert solvent in the presence of a base. Examples of the inert solvent include diethyl ether, tetrahydrofuran, dioxane, benzene, toluene, N,N-dimethylformamide, dimethyl sulfoxide, methylene chloride and acetonitrile and mixtures thereof. Examples of the base include hydrogenated alkali metals such as sodium hydride, lithium hydride and potassium hydride, alkali metal amides such as lithium amide, lithium diisopropylamide, lithium bistrimethylsilylamide, sodium bistrimethylsilylamide and potassium bistrimethylsilylamide, alkyl lithiums such as methyl lithium, butyl lithium and tert-butyl lithium, alkyl metal alkoxides such as sodium methoxide, sodium ethoxide and potassium tert-butoxide, and carbonates of an alkali metal such as sodium carbonate and potassium carbonate. The base is usually added in an amount of 1 mole or excess moles, preferably 1 to 3 moles to 1 mole of the compound (115), (116) or (118). The reaction temperature usually ranges from -50°C to the boiling point of the solvent used for the reaction, preferably from -20°C to 80°C. The reaction time usually ranges from 10 minutes to 48 hours, preferably from 30 minutes to 24 hours. The second step of Process c-2 can be carried out in a similar manner to the second step of Process b-2.

A description will next be made of Process d. In the case where the protecting group PG2 is an ester residue in the above-described compound (109), the compound (110) obtained by the deprotection is represented by the formula (119), while in the other case, Compound (110) can be converted into Compound (119) by oxidation. Process d is, for example, a process which is applied to such a carboxylic acid compound (119). Described specifically, it is, as shown in the reaction scheme (A5-5), a process for preparing an ester derivative (14-6) or amide derivative (14-7) by acting the compound of the formula (115) or (114) on Compound (119) in the presence of a condensing agent.

[Reaction scheme (A5-5)]

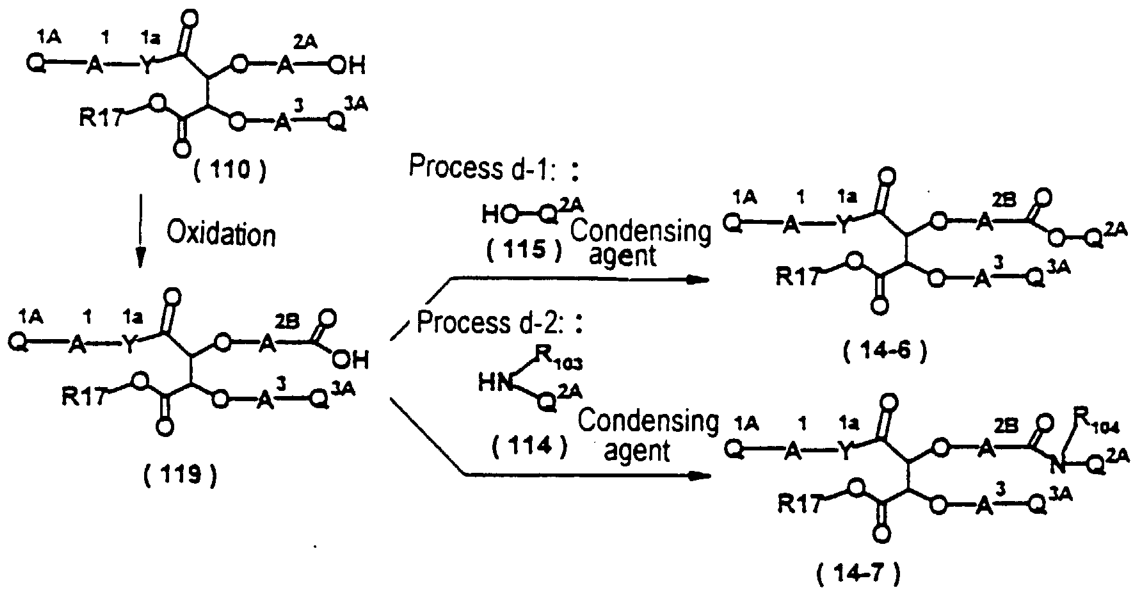

(110) (119) (115) (114) (14-6) (14-7)

[wherein each symbol has the same meaning as described above].

Preparation of Compound (119) from Compound (110) and preparation of Compound (14-6) or (14-7) from Compound (119) are carried out in accordance with the process as shown in the above-described reaction scheme (A-1).

In addition to the process as described above in the reaction scheme (A5-1), the preparation process of Compound (14) by using Compound (101), (102) or (103) as desired will next be shown specifically.

For example, Compound (14) can be prepared by using Compound (103) in accordance with the process as described in the reaction scheme (A6). Described specifically, Compound (14) can be prepared by acting both of Compound (103) and a condensing agent on Compound (11) to prepare Compound (120) and then subjecting the alcohol compound (122) available by the removal of the protecting group PG1 from Compound (120) to any one of Processes a to d. Alternatively, Compound (122) can be prepared by condensation using the protecting-group-free alcohol (121) instead of Compound (103).

[Reaction scheme (A6)]

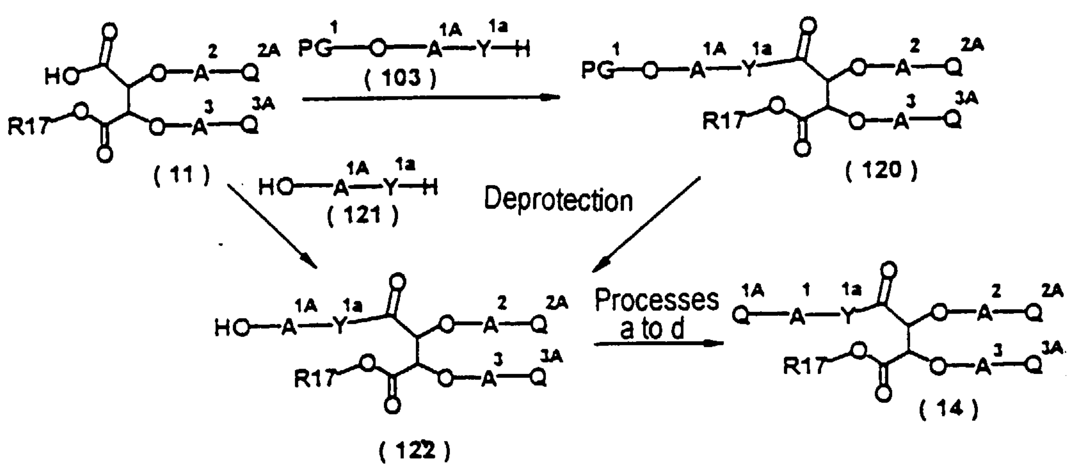

(11) (103) (120) (121) (122) (14)

[wherein PG1 has the same meaning as PG2 and others have the same meanings as described above].

In the above reaction scheme, preparation of each of the steps from Compound (11) to Compound (14) is carried out in accordance with the process in the above-described reaction scheme.

The process for the preparation of Compound (14) by using Compound (102) will be described in the below-described reaction scheme (A7-1). Described specifically, Compound (102) is acted on Compound (9) to convert it to Compound (123). After deprotection, condensation with Compound (13) is carried out, whereby Compound (125) is obtained. The alcohol compound (126) available by the deprotection of Compound (125) is subjected to any one of Processes a to d, whereby Compound (14) can be prepared.

[Reaction scheme (A7-1)]

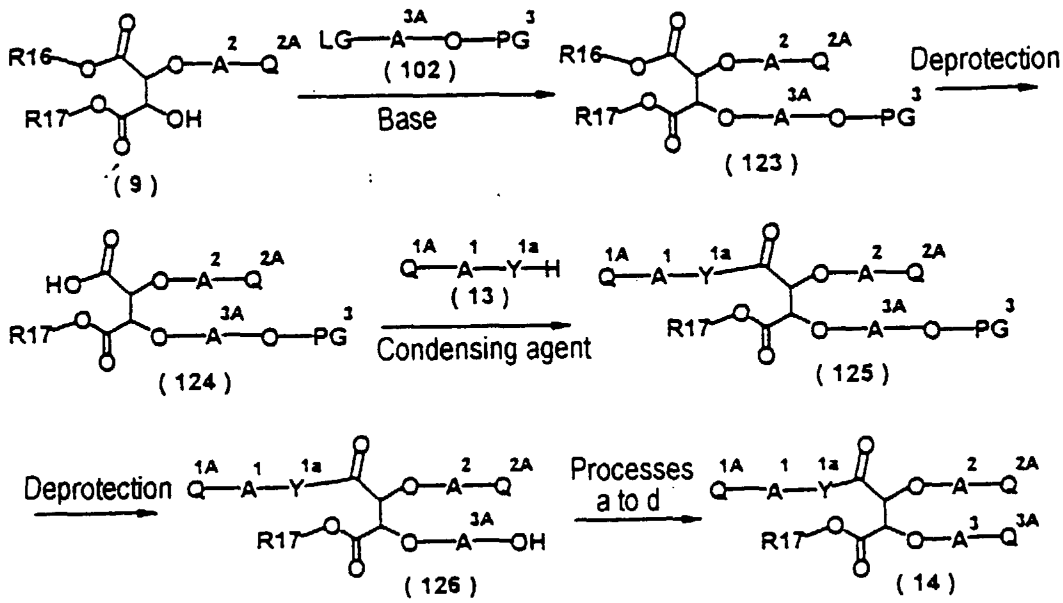


[wherein PG3 has the same meaning as PG2 and others have the same meanings as descried above].

In the above-described reaction scheme, preparation in each of the steps from Compound (9) to Compound (14) is carried out in accordance with the above-described reaction scheme.

Another process for the preparation of Compound (125) of the above reaction scheme (A7-1) will be shown below. Described specifically, Compound (125) can be prepared by protecting the hydroxyl group of Compound (9) with a suitable protecting group in advance, thereby obtaining Compound (127), removing the ester residue $R^{16}$ from Compound (127) to form the corresponding carboxylic acid (128), condensing with Compound (13) to obtain Compound (129), removing the protecting group $R^{15}$ and then introducing Compound (102).

[Reaction scheme (A7-2)]

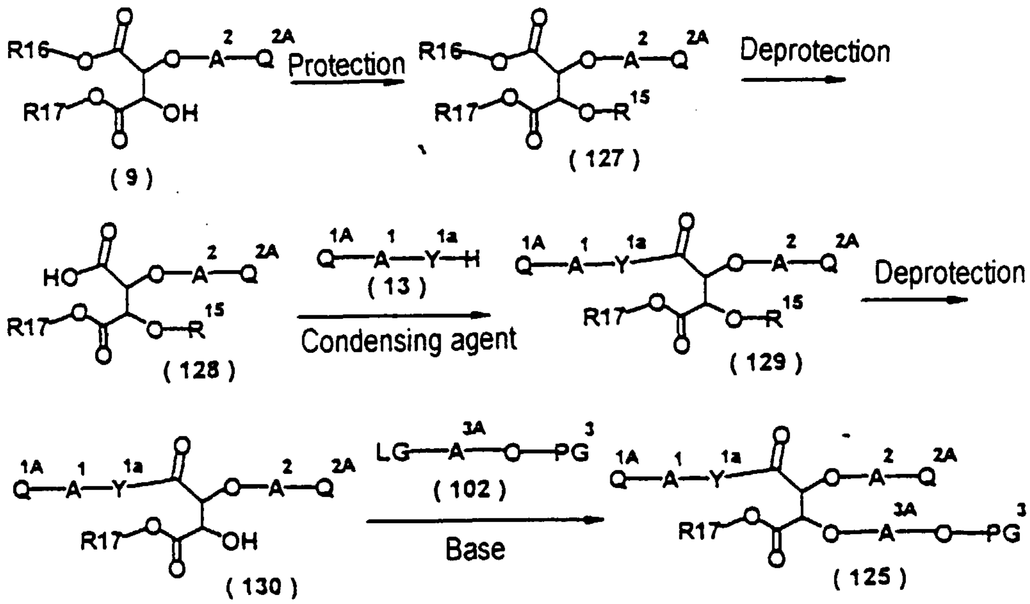


[wherein symbols have the same meanings as described above]

In the above-described reaction scheme, preparation of each of the steps from Compound (9) to Compound (125) can be carried out in accordance with the process described in the above-described reaction scheme.

[Process B]

Process for the preparation of Compound (1-2) which has as $X^1$ a carboxyl group which may be esterified and as $Y^1$ a single bond in the formula (1):

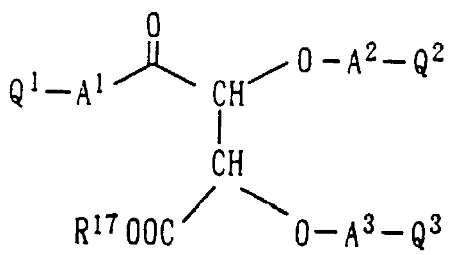

(1 - 2)

[wherein $R^{17}$ represents a hydrogen atom or an ester residue and $A^1$, $A^2$, $A^3$, $Q^1$, $Q^2$ and $Q^3$ have the same meanings as described above].

The compound (1-2) can be prepared, for example, in accordance with the below-described reaction scheme (B).

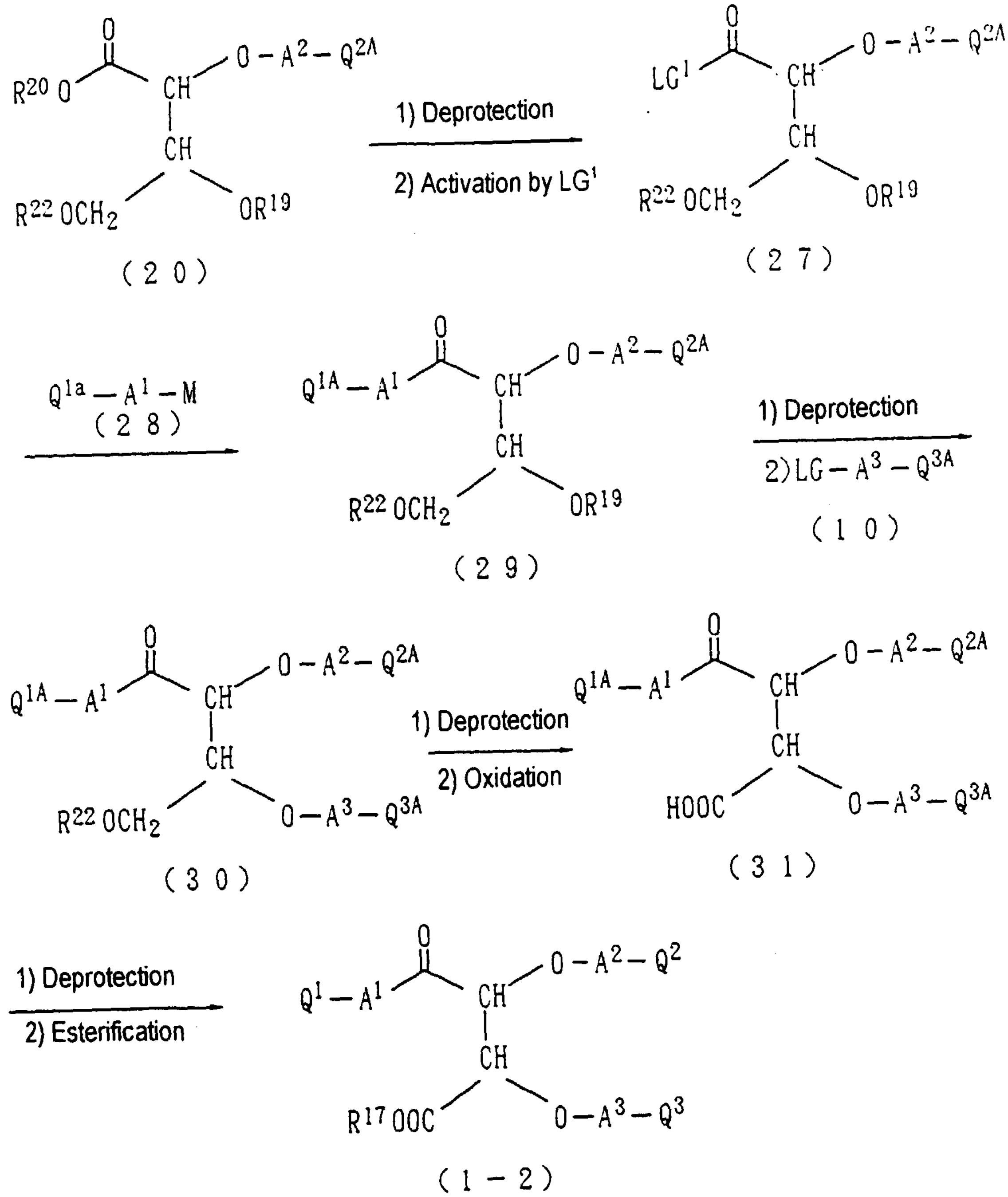


(20) (27) (29) (30) (31) (1-2)

[wherein, $LG^1$ represents an eliminative group such as halogen atom or N-methyl-N-methoxyamino group, M represents an alkali metal, MgBr, MgCl, ZnBr or the like and LG, $R^{15}$, $R^{16}$, $R^{18}$, $A^1$, $A^2$, $A^3$, $Q^{1A}$, $Q^{2A}$, $Q^{3A}$, $Q^1$, $Q^2$ and $Q^3$ have the same meanings as described above].

Preparation of Compound (29) from Compound (27) can be carried out, for example, by acting, on Compound (27), an activated nucleophilic reagent (28) as an alkali metal salt such as lithium, sodium or potassium or a Grignard reagent in an inert solvent (tetrahydrofuran or the like). Preparation processes in the other steps can be carried out similarly to the processes in the above-described reaction schemes (A-1), (A-2), (A-3) and (A-4).

[Process C]

Process for the preparation of Compound (1-3) which has as $X^1$ a tetrazol-5-yl group in the formula (1):

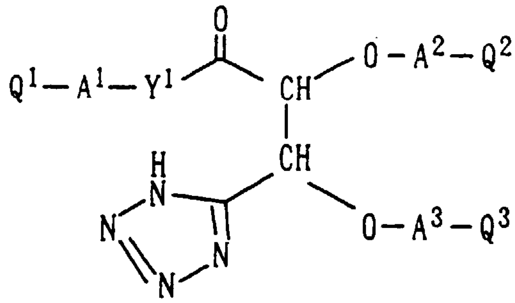

(1 - 3)

(wherein $Y^1$, $A^1$, $A^2$, $A^3$, $Q^1$, $Q^2$ and $Q^3$ have the same meanings as described above).

Compound (1-3) can be prepared, for example, in accordance with the below-described reaction scheme (C).

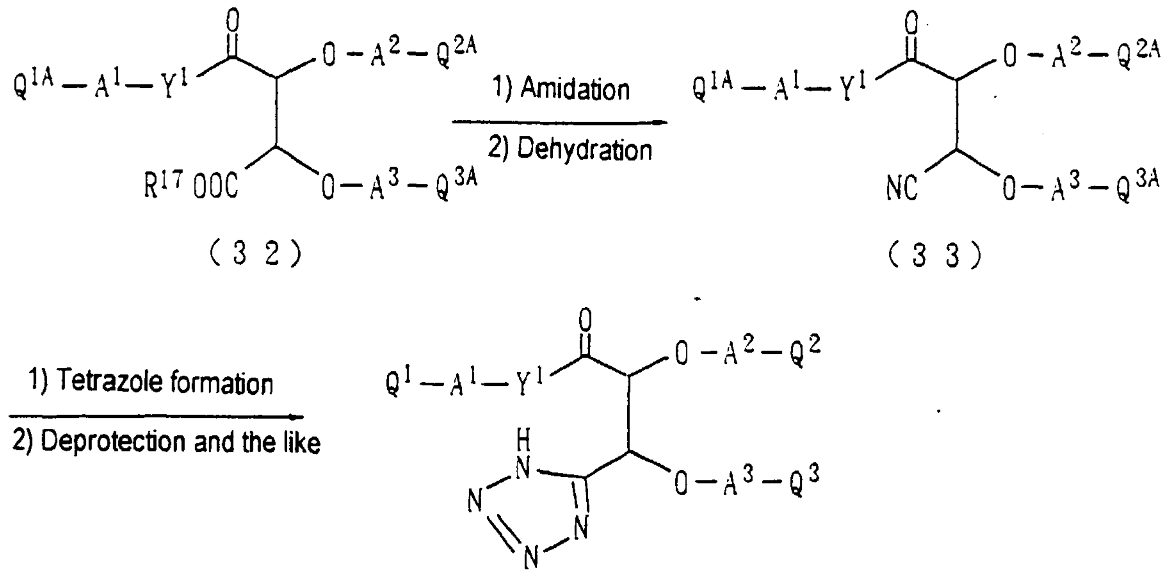


(3 2) (3 3) (1 - 3)

(wherein, $R^{17}$, $Y^1$, $A^1$, $A^2$, $A^3$, $Q^1$, $Q^2$, $Q^3$, $Q^{1A}$, $Q^{2A}$ and $Q^{3A}$ have the same meanings as described above).

Preparation of Compound (1-3) from Compound (32) is carried out, for example, by introducing the $-COOR^{17}$ of Compound (32), as is or after conversion into the free carboxylic acid, into the carbamoyl group by an ordinarily employed process; acting trifluoroacetic anhydride on the resulting compound in the presence of thionyl chloride or a base such as pyridine to convert it into the corresponding nitrile compound (33); and then subjecting Compound (33) to the general tetrazole formation reaction, for example, by acting, on Compound (33), sodium azide in an inert solvent such as N,N-dimethylformamide in the presence of a Lewis acid such as aluminum chloride, ammonium chloride or pyridinium chloride. Deprotection can be carried out in a similar manner to that described in the above-described reaction scheme (A-1).

In the above-described reaction schemes, a compound having a desired steric configuration can be obtained by using as a starting material a compound having a desired steric configuration, for example, L-(+)-tartaric acid, D-(-)-tartaric acid, meso-tartaric acid or the like. Then, it is possible to prepare any one of the compounds of the formula (1) having (2R,3R), (2S,3S), (2R,3S) and (2S,3R) as steric configuration.

Compounds of the formula (1) thus obtained can be converted into their corresponding salts by reacting with a metal hydroxide, organic amine or the like in the conventional manner.

The invention compound (1) or salt thereof has, as will be described later in Examples, excellent squalene synthetase inhibitory action and cholesterol synthesis inhibitory action and also has antioxidizing action so that it is useful as a pharmaceutical such as remedy • preventive for hypercholesterolemia, hyperlipemia or arteriosclerosis.

When the invention compound (1) or salt thereof is used as a pharmaceutical, although the dosage differs

depending on the age, sex or symptoms of each patient, it is preferably administered in an amount ranging from 0.1 mg to 1 g, more preferably 0.5 mg to 100 mg/day per adult.

The daily dosage may be administered once or in two to four portions a day. If necessary, the daily dosage may exceed the above limit.

There is no particular limitation on the manner of administration or dosage form of the pharmaceutical containing the compound (1) or salt thereof. By the ordinarily employed process for the formulation of various preparations, the pharmaceutical in the form suitable for its administering manner may be prepared by incorporating therein a pharmaceutically acceptable carrier according to need.

Examples of the orally dosable formulation include solid formulations such as tablet, powder, granule, pill and capsule and liquid formulations such as liquid and solution, syrup, elixir, suspension and emulsion.

In the case of an injection, it is possible to fill a vial with a solution of the compound (1) or salt thereof, lyophilize it into a solid formulation which can be reconstituted into a liquid preparation immediately before use. A stabilizer, antiseptic, solubilizing agent or the like can be added as needed. It is possible to fill one vial with either of one dose or multidose of the injection.

Examples of the external preparations include liquid and solution, suspension, emulsion, ointment, gel, cream, lotion, aerosol, liniment, ointment, suppository, cataplasm, inhalation and ophthalmic solution.

Solid preparations each contain pharmaceutically acceptable additives as well as active compounds and it can be formulated by selecting, for example, a suitable filler, extender, binder, disintegrator, solubilizing accelerator, humectant, lubricant or the like as needed and mixing them.

## Examples

The present invention will hereinafter be described more specifically by examples. It should however be borne in mind that the present invention is not limited to or by these examples.

Example 1

(2R,3R)-2-Carboxymethoxy-3-[N-[5-(2-naphthyl)pentyl]carbamoyl]-3-[5-(2-naphthyl)pentyloxy]propionic acid

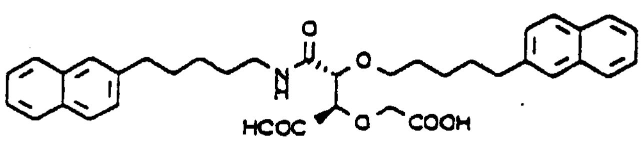

(1) tert-Butyl (2R,3R)-3-benzyloxycarbonyl-3-hydroxy-2-[5-(2-naphthyl)-2-pentenyloxy]propionate and tert-butyl (2R,3R)-3-benzyloxycarbonyl-2-hydroxy-3-[5-(2-naphthyl)-2-pentenyloxy]propionate

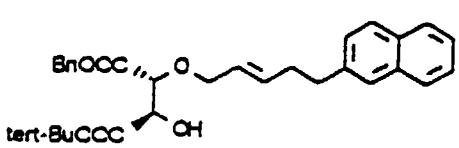

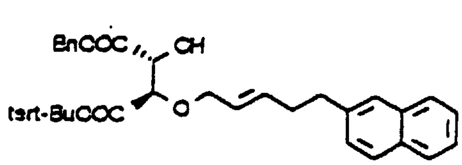

Sodium hydride (60% in oil, 1.60 g) was suspended in N,N-dimethylformamide (120 ml). To the resulting suspension was added benzyl tert-butyl L-tartrate (13.55 g) under cooling with ice water, followed by the dropwise addition of a solution of 5-(2-naphthyl)-2-pentenyl iodide (10.9 g) in N,N-dimethylformamide (50 ml) over about 10 minutes. After stirring at room temperature for 2 hours, the reaction mixture was poured into a mixture of ice water (400 ml) and saturated saline (200 ml), followed by extraction with ethyl acetate. The extract was washed twice with saturated saline and dried over anhydrous sodium sulfate. The solvent was distilled off under reduced pressure. The residue was purified several times by chromatography on a silica gel column (elution with 10 to 20% ethyl acetate-hexane), whereby a mixture (6.42 g) of the title compounds was obtained as an oil.

Mass (EI) m/Z: 490($M^+$).
$^1$H-NMR($CDCl_3$)δ: 1.46,1.48(total 9H,s each), 2.36-2.43(2H,m), 2.78-2.84(2H,m), 3.06,3.13(total 1H,d each,J=8.8/8.3Hz), 3.77,3.89(total 1H,dd each,J=11.7,6.8/11.7,7.3Hz) 4.09,4.21(total 1H,dd each,J=10.7,7.3/11.7,5.9Hz), 4.15,4.30(total 1H,d each,J=2.4/2.4Hz), 4.48,4.59(total 1H,dd each,J=7.8,2.4/8.8,2.4Hz), 5.14,5.21,5.27(total 2H,d each,J=12.2/12.2/12.2Hz), 5.31-5.38,5.51-5.75(total 2H,m each), 7.28-7.46(8H,m), 7.58(1H,s), 7.74-7.80(3H,m).

(2) tert-Butyl (2R,3R)-3-benzyloxycarbonyl-3-tert-butoxycarbonylmethoxy-2-[5-(2-naphthyl)-2-pentenyloxy]propionate and tert-butyl (2R,3R)-3-benzyloxycarbonyl-2-tert-butoxycarbonylmethoxy-3-[5-(2-naphthyl)-2-pentenyloxy]propionate

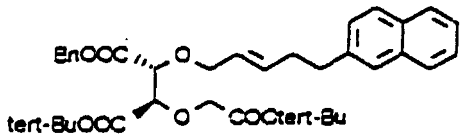
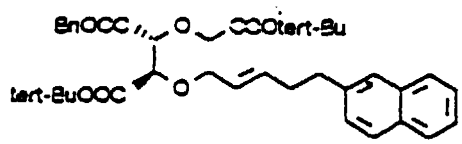

The mixture (1.97 g) obtained in (1) was dissolved in N,N-dimethylformamide (20 ml). To the resulting solution was added sodium hydride (60% in oil, 0.19 g), followed by the dropwise addition of a solution of tert-butyl bromoacetate (1.09 g) in N,N-dimethylformamide (3 ml). After stirring at room temperature for 2 hours, the resulting mixture was poured into a mixture of ice water (100 ml) and saturated saline, followed by extraction with ethyl acetate. The extract was washed twice with saturated saline and dried over anhydrous sodium sulfate. The solvent was then distilled off under reduced pressure. The residue was purified by chromatography on a silica gel column (elution with 9% ethyl acetate-hexane), whereby a mixture (1.22 g) of the title compounds was obtained as an oil.

$^1$H-NMR($CDCl_3$)δ: 1.43,1.44,1.46,1.48(total 18H,s each), 2.34-2.43(4H,m), 2.78-2.83(4H,m), 3.79-3.84,3.87-3.92(total 1H,m each), 4.01,4.13(total 1H,d each,J=16.6/16.1Hz), 4.08-4.12,4.17-4.22(total 1H,m each), 4.25,4.26(total 1H,d each,J=16.1/16.6Hz), 4.25,4.42(total 1H,d each, J=2.9/2.9Hz), 4.48,4.71(total 1H,d each,J=2.9/2.9Hz), 5.13,5.25(total 1H,d each J=12.2/12.2Hz), 5.23,5.28(total 1H,d each,J=12.2/12.2Hz), 5.35-5.45,5.53-5.74(total 2H,m each), 7.27-7.44(8H,m), 7.57(1H,s), 7.74-7.80(3H,m).

(3) tert-Butyl (2R,3R)-3-carboxy-3-tert-butoxycarbonylmethoxy-2-[5-(2-naphthyl)pentyloxy]propionate and tert-butyl (2R,3R)-3-carboxy-2-tert-butoxycarbonylmethoxy-3-[5-(2-naphthyl)pentyloxy]propionate

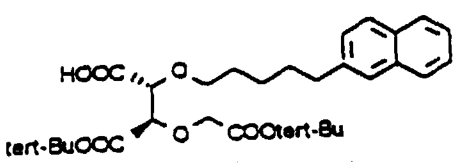
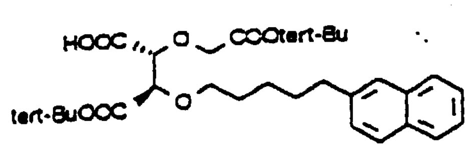

The mixture (0.75 g) obtained in (2) was dissolved in a mixed solution of ethanol (8 ml) and tetrahydrofuran (5 ml). To the resulting solution was added 10% palladium-carbon (0.1 g) and the mixture was stirred for 13.5 hours under a hydrogen atmosphere. After removal of the 10% palladium-carbon catalyst by filtration, the solvent was distilled off from the filtrate under reduced pressure, whereby a mixture (0.56 g) of the title compounds was obtained as an oil.

Mass (FAB+) m/Z: 539$(M+Na)^+$.

$^1$H-NMR($CDCl_3$)δ: 1.43-1.52(20H,m), 1.60-1.73(4H,m), 2.72-2.78(2H,m), 3.40-3.47(1H,m), 3.64-3.69,3.74-3.79(total 1H,m each), 4.02,4.03(total 1H,d each, J=16.1/17.1Hz), 4.28-4.31,4.35-4.41(total 3H,m each), 7.23-7.44(3H,m), 7.57,7.59(total 1H,s each), 7.74-7.79(3H,m).

(4) tert-Butyl (2R,3R)-3-tert-butoxycarbonylmethoxy-3-[N-[5-(2-naphthyl)pentyl]carbamoyl]-2-[5-(2-naphthyl)pentyloxy]propionate and tert-butyl (2R,3R)-2-tert-butoxycarbonylmethoxy-3-[N-[5-(2-naphthyl)pentyl]carbamoyl]-3-[5-(2-naphthyl)pentyloxy]propionate

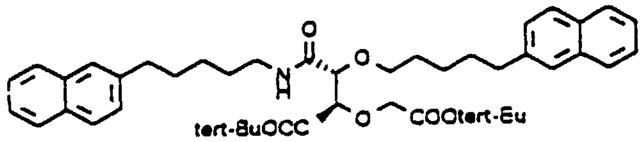

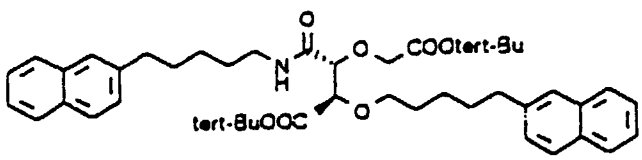

The mixture (0.53 g) obtained in (3) was dissolved in methylene chloride (15 ml), followed by the addition of 5-(2-naphthyl)pentylamine hydrochloride (0.275 g) and 4-dimethylaminopyridine (0.136 g). To the resulting mixture was added 1-ethyl-3-(3-dimethylaminopropyl)carbodiimide hydrochloride (0.257 g) under cooling with ice water. After stirring at room temperature for 21 hours, the solvent was distilled off under reduced pressure. The residue was dissolved in ethyl acetate. The resulting solution was washed with 0.5N hydrochloric acid and saturated saline and then dried over anhydrous sodium sulfate. The solvent was distilled off under reduced pressure. The residue was purified by chromatography on a silica gel column (elution with 25 to 30% ethyl acetate-hexane), whereby tert-butyl (2R,3R)-3-tert-butoxycarbonylmethoxy-3-[N-[5-(2-naphthyl)pentyl]carbamoyl]-2-[5-(2- naphthyl)pentyloxy]propionate (0.22 g) was obtained as an oil from the faster-eluting fractions.

Mass (EI) m/Z: 711$(M^+)$.

$^1$H-NMR($CDCl_3$)δ: 1.37-1.46(4H,m), 1.44(9H,s), 1.51(9H,s), 1.54-1.72(8H,m), 2.71-2.76(4H,m), 3.22-3.29(2H,m), 3.32-3.38(1H,m), 3.73-3.78(1H,m), 3.86(1H,d,J=16.6Hz), 4.26(1H,d,J=16.6Hz), 4.27(1H,d,J=2.9Hz), 4.32(1H,d,J=2.9Hz), 7.26-7.29(2H,m), 7.36-7.43(4H,m), 7.56(2H,s), 7.69-7.77(6H,m), 7.86(1H,dd,J=5.8,5.3Hz).

In addition, tert-butyl (2R,3R)-2-tert-butoxycarbonylmethoxy-3-[N-[5-(2-naphthyl)pentyl]carbamoyl]-3-[5-(2-naphthyl)pentyloxy]propionate (0.21 g) was obtained as an oil from the slower-eluting fractions.

Mass (EI) m/Z: 711$(M^+)$.

$^1$H-NMR($CDCl_3$)δ: 1.34-1.44(4H,m), 1.42(9H,s), 1.48(9H,s), 1.54-1.57(4H,m), 1.64-1.73(4H,m), 2.71-2.76(4H,m), 3.28(2H,dd,J=13.2,6.8Hz), 3.35-3.41(1H,m), 3.42-3.47(1H,m), 3.87(1H,d,J=16.1Hz), 4.15(1H,d,J=1.9Hz), 4.25(1H,d,J=16.1Hz), 4.31(1H,d,J=1.9Hz), 6.68(1H,t,J=5.8Hz), 7.29(2H,d,J=8.3Hz), 7.37-7.44(4H,m), 7.57(2H,s), 7.72-7.78(6H,m).

(5) (2R,3R)-2-Carboxymethoxy-3-[N-[5-(2-naphthyl)pentyl]carbamoyl]-3-[5-(2-naphthyl)pentyloxy]propionic acid

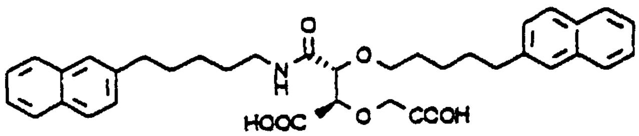

In methylene chloride (2 ml), tert-butyl (2R,3R)-2-tert-butoxycarbonylmethoxy-3-[N-[5-(2-naphthyl)pentyl]carbamoyl]-3-[5-(2-naphthyl)pentyloxy]propionate (0.21 g) obtained in (4) was dissolved, followed by the addition of trifluoroacetic acid (2 ml) under cooling with ice water. After stirring at room temperature for 2.5 hours, the solvent was distilled off under reduced pressure, whereby the title compound (0.17 g) was obtained as an oil.

Mass (FAB+) m/Z: $600(M+H)^+$.
$^1$H-NMR($CDCl_3$)δ: 1.26-1.33(4H,m), 1.45-1.52(4H,m), 1.59-1.67(4H,m), 2.70(4H,m), 3.17-3.27(2H,m), 3.37-3.43(1H,m), 3.46-3.51(1H,m), 4.17(1H,d,J=17.1Hz), 4.28(1H,d,J=17.1Hz), 4.35(1H,s), 4.31(1H,s), 7.01(1H,broad), 7.24(2H,s), 7.35-7.41(4H,m), 7.54(2H,s), 7.70-7.75(6H,m), 10.27(~3H,broad).

Example 2

(2R,3R)-3-Carboxymethoxy-3-[N-[5-(2-naphthyl)pentyl]carbamoyl]-2-[5-(2-naphthyl)pentyloxy]propionic acid

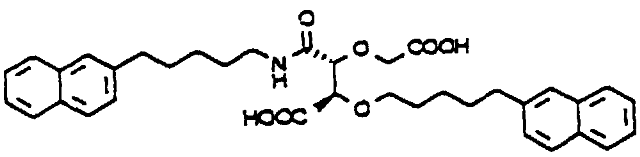

In the same manner as in Example 1-(5), the title compound (0.15 g) as an oil was prepared from tert-butyl (2R,3R)-3-tert-butoxycarbonylmethoxy-3-[N-[5-(2-naphthyl)pentyl]carbamoyl]-2-[5-(2-naphthyl)pentyloxy]propionate (0.18 g) obtained in Example 1-(4).

Mass (FAB+) m/Z: $600(M+H)^+$.
$^1$H-NMR($CDCl_3$)δ: 1.25-1.38(4H,m), 1.46-1.70(8H,m), 2.66-2.72(4H,m), 3.18-3.23(2H,m), 3.32(1H,dd,J=15.6,6.8Hz), 3.70(1H,dd,J=15.6,6.8Hz), 4.16(2H,s), 4.36(1H,d,J=2.4Hz), 4.42(1H,d,J=2.4Hz), 7.22-7.26(2H,m), 7.36-7.42(4H,m), 7.52(1H,s), 7.54(1H,s), 7.67-7.76(6H,m).

Example 3

(2R,3R)-3-Ethoxycarbonylmethoxy-3-[N-[5-(2-naphthyl)pentyl]carbamoyl]-2-[5-(2-naphthyl)pentyloxy]propionic acid

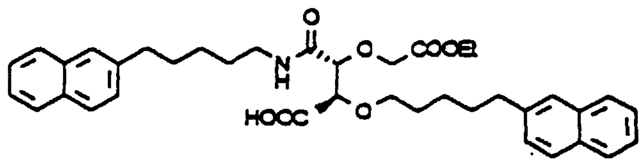

(1) tert-Butyl (2R,3R)-3-benzyloxycarbonyl-2-ethoxycarbonylmethoxy-3-[5-(2-naphthyl)-2-pentenyloxy]propionate and tert-butyl (2R,3R)-3-benzyloxycarbonyl-3-ethoxycarbonylmethoxy-2-[5-(2-naphthyl)-2-pentenyloxy]propionate

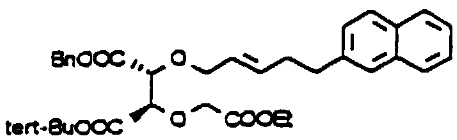

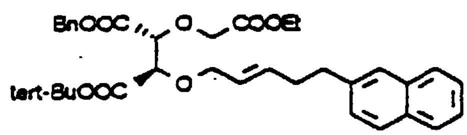

In the same manner as in Example 1-(2) except that tert-butyl bromoacetate was replaced by ethyl bromoacetate, a mixture (2.63 g) of the title compounds as a colorless oil was prepared from a mixture (3.17 g) of tert-butyl (2R,3R)-3-benzyloxycarbonyl-2-hydroxy-3-[5-(2-naphthyl)-2-pentenyloxy]propionate and tert-butyl (2R,3R)-3-benzyloxycarbonyl-3-hydroxy-2-[5-(2-naphthyl)-2-pentenyloxy]propionate obtained in Example 1-(1).

Mass (EI) m/Z: 576($M^+$).
$^1$H-NMR($CDCl_3$)δ: 1.22-1.27(3H,m), 1.46,1.48(total 9H,s each), 2.36-2.43(2H,m), 2.78-2.83(2H,m), 3.79-3.83,3.87-3.92(total 1H,dd each,J=11.7,6.8Hz), 4.08-4.27(4.5H,m), 4.37,4.38(total 1H,d each,J=16.6/16.6Hz), 4.43,4.48,4.69(total 1.5H,d each,J=2.9/2.9/2.9Hz), 5.12,5.22,5.25,5.28(total 2H,d each, J=12.2/12.2/11.2/11.2Hz), 5.34-5.42,5.53-5.74(total 2H,m each), 7.28-7.46(3H,m), 7.57(1H,s), 7.74-7.80(3H,m).

(2) tert-Butyl (2R,3R)-3-carboxy-2-ethoxycarbonylmethoxy-3-[5-(2-naphthyl)pentyloxy]propionate and tert-butyl (2R,3R)-3-carboxy-3-ethoxycarbonylmethoxy-2-[5-(2-naphthyl)pentyloxy]propionate

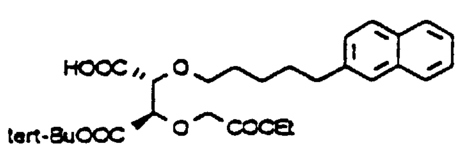

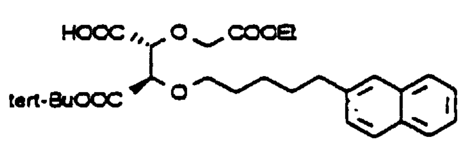

The mixture (2.63 g) obtained in (1) was treated in the same manner as in Example 1-(3), whereby a mixture (2.16 g) of the title compounds was obtained as a colorless oil.

Mass (EI) m/Z: 488($M^+$).

$^1H$-NMR($CDCl_3$)δ: 1.25,1.29(total 3H,t each,J=6.8/7.3Hz), 1.36-1.50(11H,m), 1.62-1.71(4H,m), 2.72-2.78(2H,m), 3.40-3.46(1H,m), 3.64-3.81(1H,m), 4.11-4.29(4H,m), 4.35-4.48(2H,m), 7.27-7.44(3H,m), 7.57,7.59(total 1H,s each), 7.73-7.79(3H,m).

(3) tert-Butyl (2R,3R)-2-ethoxycarbonylmethoxy-3-[N-[5-(2-naphthyl)pentyl]carbamoyl]-3-[5-(2-naphthyl)pentyloxy]propionate and tert-butyl (2R,3R)-3-ethoxycarbonylmethoxy-3-[N-[5-(2-naphthyl)pentyl]carbamoyl]-2-[5-(2-naphthyl)pentyloxy]propionate

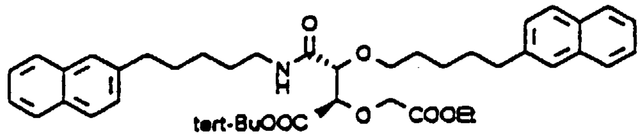

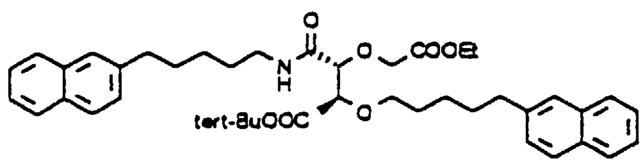

The mixture (3.29 g) obtained in (2) was treated in the same manner as in Example 1-(4). In the chromatography on a silica gel column (elution with 33% ethyl acetate-hexane), tert-butyl (2R,3R)-3-ethoxycarbonylmethoxy-3-[N-[5-(2-naphthyl)pentyl]carbamoyl]-2-[5-(2-naphthyl)pentyloxy]propionate (0.893 g) was obtained as a colorless oil from the faster-eluting fractions.

Mass (EI) m/Z: 683($M^+$).
$^1H$-NMR($CDCl_3$)δ: 1.26(3H,t,J=6.3Hz), 1.35-1.5(4H,m), 1.51(9H,s), 1.5-1.85(8H,m), 2.7-2.9(4H,m), 3.2-3.45(3H,m), 3.7-3.85(1H,m), 3.99(1H,d,J=16.6Hz), 4.12-4.25(2H,m), 4.3-4.5(3H,m), 7.2-7.3(2H,m), 7.34-7.46(4H,m), 7.57(2H,s), 7.65-7.82(6H,m).

In addition, from the slower-eluting fractions, tert-butyl (2R,3R)-2-ethoxycarbonylmethoxy-3-[N-[5-(2-naphthyl)pentyl]carbamoyl]-3-[5-(2-naphthyl)pentyloxy]propionate (1.03 g) was obtained as a colorless oil.

Mass (EI) m/Z: 683($M^+$).
$^1H$-NMR($CDCl_3$)δ: 1.21(3H,t,J=6.8Hz), 1.28-1.43(4H,m), 1.49(9H,s), 1.52-1.63(4H,m), 1.63-1.78(4H,m), 2.70-2.80(4H,m), 3.20-3.33(2H,m), 3.33-3.50(2H,m), 4.01(1H,d,J=16.1Hz), 4.10-4.20(3H,m), 4.34(1H,d,J=2.0Hz), 4.35(1H,d,J=16.1Hz), 6.65(1H,t,J=5.9Hz), 7.28(2H,d,J=8.3Hz), 7.35-7.45(4H,m), 7.57(2H,s), 7.72-7.83(6H,m).

(4) (2R,3R)-3-Ethoxycarbonylmethoxy-3-[N-[5-(2-naphthyl)pentyl]carbamoyl]-2-[5-(2-naphthyl)pentyloxy]propionic acid

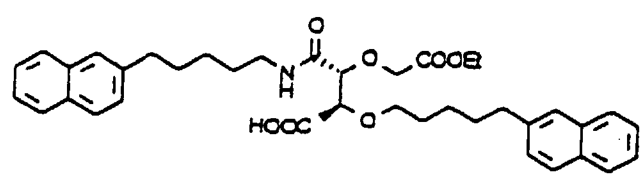

The title compound (0.162 g) as a colorless oil was prepared from the tert-butyl (2R,3R)-3-ethoxycarbonylmethoxy-3-[N-[5-(2-naphthyl)pentyl]carbamoyl]-2-[5-(2-naphthyl)pentyloxy]propionate (0.17 g) obtained in (3) in the

same manner as Example 1-(5).

Mass (FAB+) m/Z: 628(M+H)$^+$.
$^1$H-NMR(CDCl$_3$)δ: 1.25(3H,t,J=7.3Hz), 1.34-1.46(4H,m), 1.54-1.78(8H,m), 2.70-2.80(4H,m), 3.25-3.30(2H,m), 3.45-3.55(1H,m), 3.67-3.76(1H,m), 4.01(1H,d,J=7.1Hz), 4.12-4.23(2H,m), 4.3-4.4(2H,m), 4.48(1H,d,J=1.9Hz), 7.25-7.31(2H,m), 7.37-7.46(4H,m), 7.56(1H,s), 7.57(1H,s), 7.70-7.80(6H,m), 7.96(1H,t,J=5.9Hz).

Example 4

Ethyl (2R,3R)-2-ethoxycarbonylmethoxy-3-[N-[5-(2-naphthyl)pentyl]carbamoyl]-3-[5-(2-naphthyl)pentyloxy]propionic acid

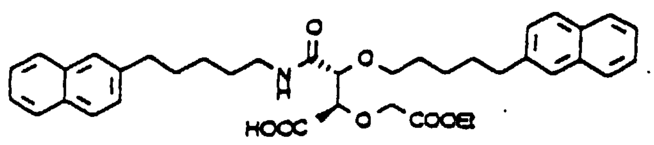

The title compound (0.17 g) as a colorless oil was prepared from tert-butyl (2R,3R)-2-ethoxycarbonylmethoxy-3-[N-[5-(2-naphthyl)pentyl]carbamoyl]-3-[5-(2-naphthyl)pentyloxy]propionate (0.18 g) obtained in Example 3-(3) in the same manner as in Example 1-(5).

Mass (FAB+) m/Z: 628(M+H)$^+$.
$^1$H-NMR(CDCl$_3$)δ: 1.27(3H,t,J=7.3Hz), 1.30-1.41(4H,m), 1.47-1.62(4H,m), 1.64-1.75(4H,m), 2.72-2.80(4H,m), 3.25-3.30(2H,m), 3.42-3.52(1H,m), 3.56-3.64(1H,m), 4.10(1H,d,J=17.5Hz), 4.24(2H,q,J=7.3Hz), 4.32(1H,d,J=17.5Hz), 4.38(1H,d,J=1.9Hz), 4.55(1H,d,J=1.9Hz), 6.85(1H,t,J=5.9Hz), 7.26-7.32(2H,m), 7.36-7.46(4H,m), 7.57(2H,s), 7.71-7.81(6H,m).

Example 5

Ethyl (2R,3R)-3-carboxymethoxy-3-[N-[5-(2-naphthyl)pentyl]carbamoyl]-2-[5-(2-naphthyl)pentyloxy]propionate

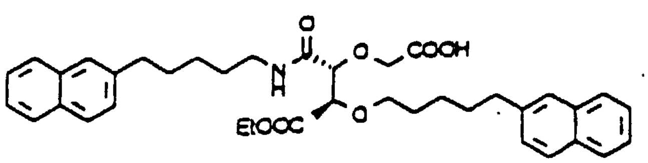

(1) Benzyl (2R,3R)-2-hydroxy-3-ethoxycarbonyl-3-[5-(2-naphthyl)-2-pentenyloxy]propionate and benzyl (2R,3R)-3-hydroxy-3-ethoxycarbonyl-2-[5-(2-naphthyl)-2-pentenyloxy]propionate

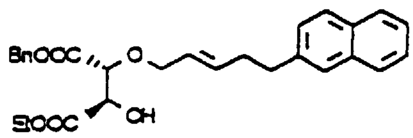

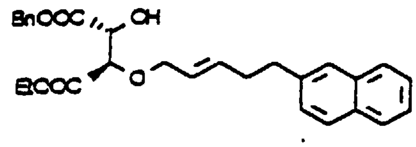

In the same manner as in Example 1-(1) except for the use of benzyl ethyl L-tartrate (2.8 g) instead of benzyl tert-butyl L-tartrate, a mixture (1.88 g) of the title compounds was obtained as a colorless oil.

Mass (EI) m/Z: 462($M^+$).
$^1$H-NMR($CDCl_3$)δ: 1.24,1.29(total 3H,t each,J=6.8/6.8Hz), 2.30-2.50(2H,m), 2.75-2.90(2H,m), 3.07(total 1H,d each,J=9.3/8.3Hz), 3.78,3.89(total 1H,dd each,J=12.2,6.8/11.7,6.8Hz), 4.05-4.30(2.5H,m), 4.32(0.5H,d,J=2.4Hz), 4.57,4.61(total 1H,dd each,J=6.7,2.4/6.8,2.4Hz), 5.16,5.23(total 1H,d each,J=12.2/12.2Hz), 5.24,5.26(total 1H,d each,J=12.2/12.2Hz), 5.26-5.35,5.43-5.52,5.60-5.75(total 2H,m each), 7.27-7.47(8H,m), 7.58(1H,s), 7.75-7.80(3H,m).

(2) Ethyl (2R,3R)-3-benzyloxycarbonyl-3-tert-butoxycarbonylmethoxy-2-[5-(2-naphthyl)-2-pentenyloxy]propionate and ethyl (2R,3R)-3-benzyloxycarbonyl-2-tert-butoxycarbonylmethoxy-3-[5-(2-naphthyl)-2-pentenyloxy]propionate

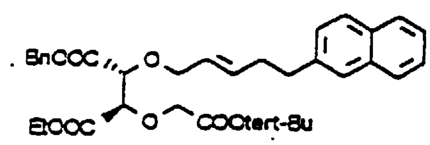

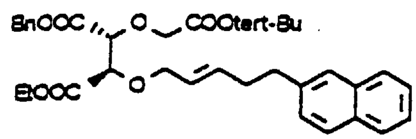

The mixture (1.88 g) obtained in (1) was treated in the same manner as in Example 1-(2), whereby a mixture (0.837 g) of the title compounds was obtained as a colorless oil.

Mass (EI) m/Z: 576($M^+$).
$^1$H-NMR($CDCl_3$)δ: 1.23,1.25(total 3H,t each,J=7.3/7.3Hz), 1.43,1.44(total 9H,s each), 2.34-2.43(2H,m), 2.75-2.83(2H,m), 3.82,3.91(total 1H,dd each,J=11.7,7.3/11.7,6.8Hz), 4.03(0.5H,d,J=17.1Hz), 4.09-4.28(4.5H,m), 4.37,4.43(total 1H,d each,J=3.4/3.4Hz), 4.57,4.63(total 1H,d each,J=3.4/3.4Hz), 5.15,5.25(total 1H,d each,J=12.2/11.7Hz), 5.23,5.28(total 1H,d each,J=12.2/11.7Hz), 5.35-5.42,5.48-5.56,5.63-5.74(total 2H,m each), 7.27-7.37(5H,m), 7.39-7.46(3H,m), 7.58(1H,s), 7.75-7.81(3H,m).

(3) Ethyl (2R,3R)-3-tert-butoxycarbonylmethoxy-3-carboxy-2-[5-(2-naphthyl)pentyloxy]propionate and ethyl (2R,3R)-2-tert-butoxycarbonylmethoxy-3-carboxy-3-[5-(2-naphthyl)pentyloxy]propionate

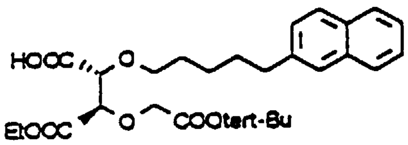

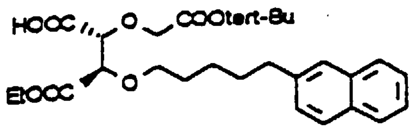

The mixture (0.837 g) obtained in (2) was treated in the same manner as in Example 1-(3), whereby a mixture (0.709 g) of the title compounds was obtained as a colorless oil.

$^1$H-NMR($CDCl_3$)δ: 1.24,1.30(total 3H,t each,J=6.8/6.8Hz), 1.35-1.50(11H,m),1.55-1.70(4H,m), 2.72-2.77(2H,m), 3.34-3.48(1H,m), 3.68-3.79(1H,m), 4.02-4.07(1H,m), 4.13-4.53(5H,m), 7.28-7.32(1H,m), 7.37-7.44(2H,m), 7.57,7.59(total 1H,s each), 7.73-7.78(3H,m).

(4) Ethyl (2R,3R)-3-tert-butoxycarbonylmethoxy-3-N-[5-(2-naphthyl)pentyl]carbamoyl]-2-[5-(2-naphthyl)pentyloxy]propionate and ethyl (2R,3R)-2-tert-butoxycarbonylmethoxy-3-N-[5-(2-naphthyl)pentyl]carbamoyl]-3-[5-(2-naphthyl)pentyloxy]propionate

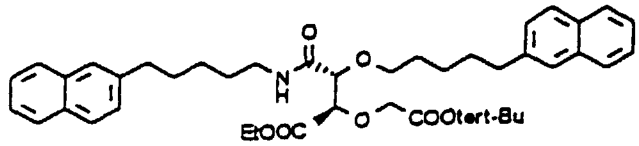

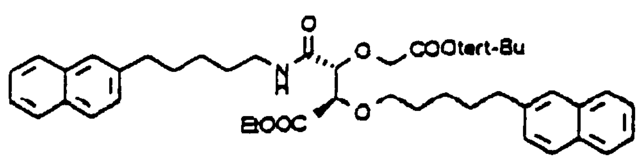

The mixture (0.709 g) obtained in (3) was treated in the same manner as in Example 1-(4). In the chromatography on a silica gel column, ethyl (2R,3R)-3-tert-butoxycarbonylmethoxy-3-[N-[5-(2-naphthyl)pentyl]carbamoyl]-2-[5-(2-naphthyl)pentyloxy]propionate (0.300 g) was obtained as a colorless oil from the faster-eluting fractions.

Mass (FAB+) m/Z: 684$(M+H)^+$.
$^1$H-NMR($CDCl_3$)δ: 1.31(3H,t,J=6.8Hz), 1.37-1.49(13H,m), 1.52-1.75(8H,m), 2.71-2.77(4H,m), 3.22-3.29(2H,m), 3.34-3.38(1H,m), 3.72-3.77(1H,m), 3.86(1H,d,J=16.6Hz), 4.19(1H,d,J=16.6Hz), 4.20-4.30(3H,m), 4.41(1H,d,J=2.9Hz), 7.26-7.29(2H,m), 7.36-7.45(4H,m), 7.56(2H,s), 7.69-7.77(6H,m).

From the faster-eluting fractions, ethyl (2R,3R)-2-tert-butoxycarbonylmethoxy-3-[N-[5-(2-naphthyl)pentyl]carbamoyl]-3-[5-(2-naphthyl)pentyloxy]propionate (0.392 g) was obtained as a colorless oil.

Mass (FAB+) m/Z: 684$(M+H)^+$.
$^1$H-NMR($CDCl_3$)δ: 1.26(3H,t,J=6.8Hz), 1.30-1.45(13H,m), 1.50-1.57(4H,m), 1.64-1.75(4H,m), 2.71-2.76(4H,m), 3.27(2H,dd,J=13.2,6.8Hz), 3.31-3.37(1H,m), 3.43-3.49(1H,m), 3.93(1H,d,J=16.1Hz), 4.16-4.24(4H,m), 4.46(1H,d,J=2.4Hz), 6.69(1H,t,J=5.9Hz), 7.29(2H,d,J=8.3Hz), 7.37-7.44(4H,m), 7.57(2H,s), 7.72-7.78(6H,m).

(5) Ethyl (2R,3R)-3-carboxymethoxy-3-N-[5-(2-naphthyl)pentyl]carbamoyl]-2-[5-(2-naphthyl)pentyloxy]propionate

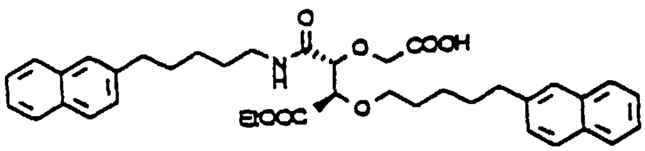

The ethyl (2R,3R)-3-tert-butoxycarbonylmethoxy-3-[N-[5-(2-naphthyl)pentyl]carbamoyl]-2-[5-(2-naphthyl)pentyloxy]propionate (0.210 g) obtained in (4) was treated in the same manner as in Example 1-(5), whereby the title compound (0.20 g) was obtained as a colorless oil.

Mass (FAB+) m/Z: 628(M+H)$^+$.
$^1$H-NMR($CDCl_3$)δ: 1.26(3H,t,J=7.3Hz), 1.30-1.42(4H,m), 1.47-1.70(8H,m), 2.71(4H,t,J=7.8Hz), 3.22(2H,dd,J=13.5,6.8Hz), 3.30-3.39(1H,m), 3.71-3.76(1H,m), 4.02(1H,d,J=17.1Hz), 4.22(2H,q,J=7.3Hz), 4.26(1H,d,J=17.1Hz), 4.36(2H,s), 7.22-7.28(2H,m), 7.36-7.44(4H,m), 7.54(2H,s), 7.62(1H,t,J=5.8Hz), 7.68-7.76(6H,m).

Example 6

Ethyl (2R,3R)-2-carboxymethoxy-3-[N-[5-(2-naphthyl)pentyl]carbamoyl]-3-[5-(2-naphthyl)pentyloxy]propionate

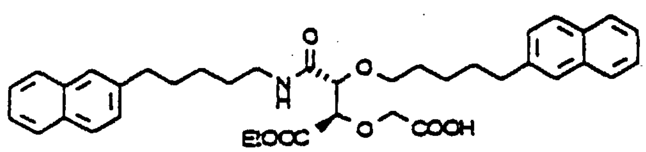

Ethyl (2R,3R)-2-tert-butoxycarbonylmethoxy-3-[N-[5-(2-naphthyl)pentyl]carbamoyl]-3-[5-(2-naphthyl)pentyloxy]propionate (0.219 g) obtained in Example 5-(4) was treated in the same manner as in Example 1-(5), whereby the title compound (0.221 g) was obtained as a colorless oil.

Mass (FAB+) m/Z: 628(M+H)$^+$.
$^1$H-NMR($CDCl_3$)δ: 1.24(3H,t,J=6.8Hz), 1.25-1.35(4H,m), 1.46-1.54(4H,m), 1.62-1.71(4H,m), 2.70-2.75(4H,m), 3.20-3.35(3H,m), 3.39-3.44(1H,m), 4.14-4.23(4H,m), 4.27(1H,d,J=2.4Hz), 4.52(1H,d,J=2.4Hz), 6.90(1H,t,J=5.9Hz), 7.26-7.29(2H,m), 7.36-7.44(4H,m), 7.56(2H,s), 7.71-7.78(6H,m).

Example 7

Ethyl (2R,3R)-2-ethoxycarbonylmethoxy-3-[N-[5-(2-naphthyl)pentyl]carbamoyl]-3-[5-(2-naphthyl)pentyloxy]propionate

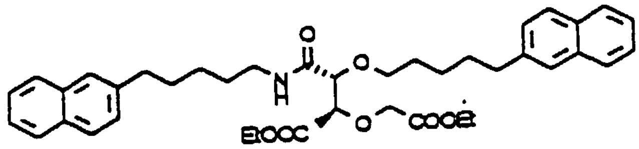

Ethyl (2R,3R)-2-carboxymethoxy-3-[N-[5-(2-naphthyl)pentyl]carbamoyl]-3-[5-(2-naphthyl)pentyloxy]propionate (0.108 g) obtained in Example 6 and ethanol (1 ml) were dissolved in methylene chloride (5 ml). To the resulting solution were added 4-dimethylaminopyridine (0.025 g) and 1-ethyl-3-(3-dimethylaminopropyl)carbodiimide hydrochloride (0.05 g) under cooling with ice water. After stirring for 4 hours, the reaction mixture was washed with saturated saline and dried over anhydrous sodium sulfate. The solvent was then distilled off under reduced pressure. The residue was purified by chromatography on a silica gel column (50% ethyl acetate-hexane), whereby the title compound (0.092 g) was obtained as colorless crystals.

$[\alpha]_D^{27}$ + 35.7° (c=0.95, $CHCl_3$)
Mass (EI) m/Z: 655($M^+$).
$^1$H-NMR($CDCl_3$)δ: 1.22(3H,t,J=6.8Hz), 1.26(3H,t,H=7.3Hz), 1.30-1.41(4H,m), 1.50-1.57(4H,m), 1.62-1.74(4H,m), 2.71-2.76(4H,m), 3.25-3.37(3H,m), 3.43-3.49(1H,m), 4.04(1H,d,J=16.1Hz), 4.12-4.23(5H,m), 4.33(1H,d,J=16.1Hz), 4.47(1H,d,J=2.4Hz), 6.65(1H,t,J=5.8Hz), 7.29(2H,d,J=8.3Hz), 7.36-7.45(4H,m), 7.57(2H,s), 7.72-7.78(6H,m).

Example 8

(3R)-3-[(1R)-7-(2-Naphthyl)-1-[N-[5-(2-naphthyl)pentyl]carbamoyl]-2-oxa-1-heptyl]-1,4-dioxan-2-one

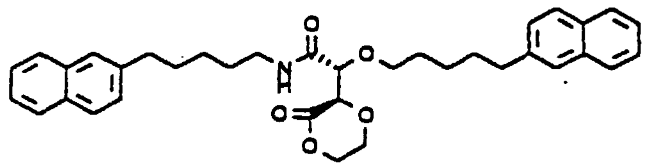

(1) tert-Butyl (2R,3R)-2-(2-hydroxyethoxy)-3-[N-[5-(2-naphthyl)pentyl]carbamoyl]-3-[5-(2-naphthyl)pentyloxy]propionate

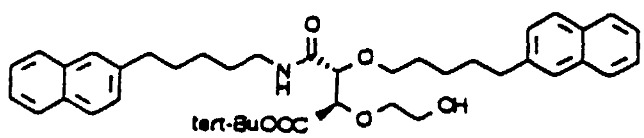

In a mixture of tetrahydrofuran (5 ml) and methanol (1 ml), tert-butyl (2R,3R)-2-ethoxycarbonylmethoxy-3-[N-[5-(2-naphthyl)pentyl]carbamoyl]-3-[5-(2- naphthyl)pentyloxy]propionate (0.217 g) obtained in Example 3-(3) was dissolved. To the resulting solution was added sodium borohydride (0.038 g) under cooling with ice water, and the mixture was stirred at room temperature for 2.5 hours. A dilute aqueous solution of hydrochloric acid was added and the resulting mixture was extracted with ethyl acetate. After washing with water, the extract was washed with saturated saline and dried over anhydrous sodium sulfate. The solvent was distilled off under reduced pressure. The residue was purified by chromatography on a silica gel column (50% ethyl acetate-hexane), whereby the title compound (0.145 g) was obtained as a colorless oil.

Mass (EI) m/Z: 641($M^+$).
$^1$H-NMR($CDCl_3$)δ: 1.30-1.42(4H,m), 1.45-1.55(13H,m), 1.62-1.75(4H,m), 2.74(4H,t,J=7.3Hz), 3.22-3.32(2.5H,m), 3.39-3.49(2H,m), 3.60-3.65(3H,broad), 3.71-3.74(1H,m), 4.22(1H,d,J=2.4Hz), 4.45(1H,d,J=2.4Hz), 6.72(1H,t,J=5.8Hz), 7.28(2H,d,J=8.3Hz), 7.37-7.47(4H,m), 7.57(2H,s), 7.73-7.79(6H,m).

(2) (3R)-3-[(1R)-7-(2-naphthyl)-1-[N-[5-(2-naphthyl)pentyl]carbamoyl]-2-oxa-1-heptyl]-1,4-dioxan-2-on

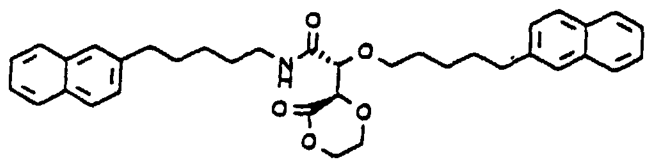

The hydroxyethyl compound (0.157 g) obtained in (1) was dissolved in methylene chloride (8 ml). To the resulting solution was added trifluoroacetic acid (8 ml) under cooling with ice water, and the mixture was stirred at room temperature for 2 hours. The solvent was distilled off under reduced pressure. The residue was purified by chromatography on a silica gel column (50% ethyl acetate-hexane), whereby the title compound (0.11 g) was obtained as colorless crystals.

Melting point: 99 to 100°C
$^1$H-NMR(CDCl$_3$)δ: 1.30-1.40(4H,m), 1.50-1.58(4H,m), 1.65-1.75(4H,m), 2.72-2.78(4H,m), 3.24-3.30(2H,m), 3.48(2H,t,J=6.3Hz), 3.68(1H,ddd,J=12.7,9.8,2.4Hz), 3.79-3.84(1H,m), 4.22(1H,dt,J=8.3,2.9Hz), 4.29-4.35(3H,m), 4.78(1H,d,J=2.0Hz), 6.58(1H,broad), 7.27-7.32(2H,m), 7.37-7.44(4H,m), 7.57(1H,s), 7.58(1H,s), 7.72-7.78(6H,m).

| Elementary analysis for $C_{36}H_{41}NO_5$ | | | |
|---|---|---|---|
| Calculated: | C, 76.16; | H, 7.28; | N, 2.43. |
| Found: | C, 75.96; | H, 7.10; | N, 2.37. |

Example 9

(2R,3R)-3-(2-Hydroxyethoxy)-3-[N-[5-(2-naphthyl)pentyl]carbamoyl]-2-[5-(2-naphthyl)pentyloxy]propionic acid

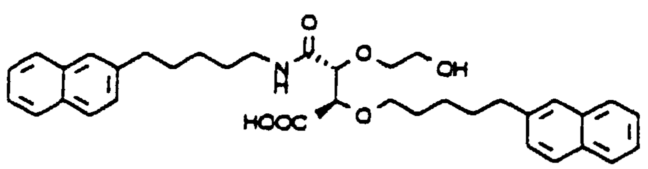

(1) tert-Butyl (2R,3R)-3-(2-hydroxyethoxy)-3-[N-[5-(2-naphthyl)pentyl]carbamoyl]-2-[5-(2-naphthyl)pentyloxy]propionate

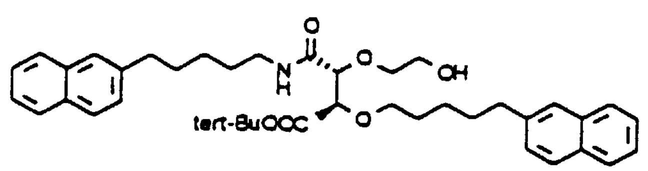

In the same manner as in Example 8-(1), tert-butyl (2R,3R)-3-ethoxycarbonylmethoxy-3-[N-[5-(2-naphthyl)pentyl]carbamoyl]-2-[5-(2-naphthyl)pentyloxy]propionate (0.189 g) obtained in Example 3-(3) was treated, whereby the title compound (0.157 g) was obtained as a colorless oil.

$^1$H-NMR($CDCl_3$)δ: 1.32-1.41(4H,m), 1.47-1.73(17H,m), 2.13(1H,broad), 2.70-2.76(4H,m), 3.14-3.32(3H,m), 3.44-3.56(3H,m), 3.62-3.69(1H,m), 3.76(1H,dt,J=9.3,6.3Hz), 4.22(1H,d,J=2.9Hz), 4.23(1H,d,J=2.9Hz), 6.92(1H,broad), 7.25-7.29(2H,m), 7.37-7.44(4H,m), 7.56(1H,s), 7.57(1H,s), 7.69-7.78(6H,m).

(2) (2R,3R)-3-(2-Hydroxyethoxy)-3-[N-[5-(2-naphthyl)pentyl]carbamoyl]-2-[5-(2-naphthyl)pentyloxy]propionic acid

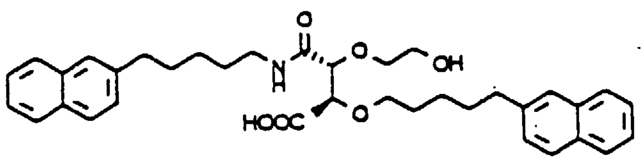

The hydroxyethyl compound (0.157 g) obtained in (1) was treated in the same manner as in Example 8-(2), whereby (2R,3R)-3-(2-hydroxyethoxy)-3-[N-[5-(2-naphthyl)pentyl]carbamoyl]-2-[5-(2- naphthyl)pentyloxy]propionic acid was obtained.

Mass (FAB+) m/Z: 586 $(M+H)^+$.

The resulting compound was dissolved in ethanol (5 ml), followed by the addition of a 1N aqueous solution of sodium hydroxide (0.25 ml). The solvent was distilled off under reduced pressure. Ethanol was added again to the residue to solidify the same. The resulting solid was collected by filtration, whereby sodium (2R,3R)-3-(2-hydroxyethoxy)-3-[N-[5-(2-naphthyl)pentyl]carbamoyl]-2-[5-(2-naphthyl)pentyloxy]propionate (0.054 g) was obtained as an amorphous solid.

$^1$H-NMR($CD_3OD$)δ: 1.28-1.42(4H,m), 1.47-1.75(8H,m), 2.66-2.75(4H,m), 3.05-3.20(2H,m), 3.40-3.75(6H,m), 4.02(1H,d,J=2.4Hz), 4.20(1H,d,J=2.4Hz), 7.24-7.32(2H,m), 7.32-7.42(4H,m), 7.54(1H,s), 7.55(1H,s), 7.65-7.78(6H,m).

| Elementary analysis for $C_{36}H_{42}NNaO_6 \cdot 2H_2O$ | | | |
|---|---|---|---|
| Calculated: | C, 67.17; | H, 7.20; | N, 2.18. |
| Found: | C, 67.33; | H, 7.00; | N, 2.14. |

Example 10

(2R,3R)-2-Ethoxycarbonylmethoxy-3-[N-methyl-N-[5-(2-naphthyl)pentyl]carbamoyl]-3-[5-(2-naphthyl)pentyloxy]propionic acid

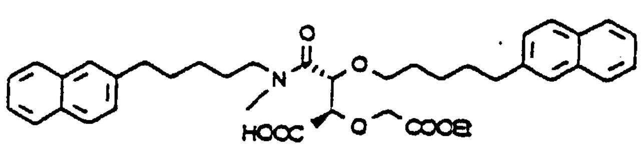

(1) Benzyl (2R,3R)-3-tert-butoxycarbonyl-2-methoxymethoxy-3-[5-(2-naphthyl)pentyloxy]propionate

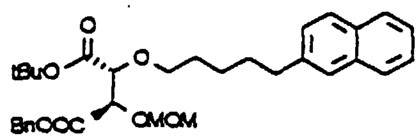

With methylene chloride (25 ml), oxalyl chloride (1.2 ml) was mixed. After cooling the resulting mixture to -78°C, a solution of dimethylsulfoxide (1.6 ml) in methylene chloride (12 ml) was added dropwise to the resulting mixture, and the mixture was stirred for 10 minutes. A solution of tert-butyl (2R,3S)-4-hydroxy-3-methoxymethoxy-2-[5-(2-naphthyl)pentyloxy]butyrate (3.95 g), which had been obtained in Referential Example 4-(3), in methylene chloride (15 ml) was added dropwise to the reaction mixture, and the mixture was stirred for 70 minutes. After the dropwise addition of triethylamine (6 ml), the reaction mixture was heated to 0°C, at which stirring was carried out for 15 minutes. Water was added to the reaction mixture. The resulting mixture was extracted with methylene chloride and dried over anhydrous sodium sulfate. The solvent was distilled off under reduced pressure, whereby tert-butyl (2R,3R)-3-methoxymethoxy-2-[5-(2-naphthyl)pentyloxy]-4-oxobutyrate was obtained as an oil.

The resulting compound was mixed with a solution mixture of an aqueous solution (5 ml) of sodium dihydrogenphosphate (5 g), tert-butanol (30 ml) and 2-methyl-2-butene (2N solution in tetrahydrofuran, 15 ml), followed by stirring at room temperature. To the reaction mixture was added an aqueous solution (5 ml) of sodium chlorite (5 g) over 2 hours in small portions. After extraction with diethyl ether, the extract was washed with water and saturated saline and then dried over anhydrous sodium sulfate. The solvent was distilled off under reduced pressure, whereby (2R,3R)-3-tert-butoxycarbonyl-2-methoxymethoxy-3-[5-(2-naphthyl)pentyloxy]propionic acid was obtained as an oil.

The resulting compound was dissolved in N,N'-dimethylformamide (15 ml). To the resulting mixture were added triethylamine (3 ml) and benzyl bromide (1.6 ml), and the mixture was stirred at room temperature for 12 hours. The reaction mixture was diluted with diethyl ether, washed with water and saturated saline and then dried over anhydrous sodium sulfate. The solvent was distilled off under reduced pressure. The residue was purified by chromatography on a silica gel column (ethyl acetate-hexane 1:4), whereby the title compound (3.15 g) was obtained as an oil.

Mass (EI) m/Z: 536($M^+$).

$^1$H-NMR($CDCl_3$)δ: 1.20-1.80(6H,m), 1.47(9H,s), 2.73(2H,t,J=7.8Hz), 3.17(1H,dt,J=8.8,6.8Hz), 3.35(3H,s), 3.71(1H,dt,J=8.8,6.4Hz), 4.21(1H,d,J=3.4Hz), 4.64(1H,d,J=3.4Hz), 4.68(1H,d,J=6.8Hz), 4.77(1H,d,J=6.8Hz), 5.11(1H,d,J=12.2Hz), 5.20(1H,d,J=12.2Hz), 7.22-7.37(6H,m), 7.39-7.43(2H,m), 7.57(1H,s), 7.67-7.79(3H,m).

(2) Benzyl (2R,3R)-3-tert-butoxycarbonyl-2-hydroxy-3-[5-(2-naphthyl)pentyloxy]propionate

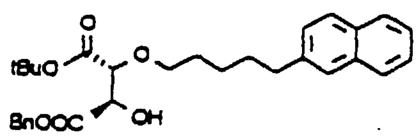

The resulting benzyl ester compound (70.7 mg) obtained in (1) was dissolved in methylene chloride (1 ml). To the resulting solution was added B-bromocatecholborane (30 mg), and the mixture was stirred at room temperature for 45 minutes. Water was added to the reaction mixture. After extraction with methylene chloride, the extract was washed successively with a 2N aqueous solution of sodium hydroxide and saturated saline and then dried over anhydrous sodium sulfate. The solvent was distilled off under reduced pressure. The residue was purified by chromatography on a silica gel column (ethyl acetate-hexane 1:3), whereby the title compound (39 mg) was obtained as an oil.

Mass (EI) m/Z: 492($M^+$).
$^1$H-NMR($CDCl_3$)δ: 1.23-1.72(6H,m), 1.48(9H,s), 2.74(2H,t,J=7.8Hz), 3.03(1H,d,J=9.3Hz), 3.12(1H,dt,J=8.8,6.8Hz), 3.69(1H,dt,J=8.8,6.3Hz), 4.09(1H,d,J=2.5Hz), 4.59(1H,dd,J=9.3,2.5Hz), 5.14(1H,d,J=12.2Hz), 5.20(1H,d,J=12.2Hz), 7.25-(8H,m), 7.58(1H,s), 7.74-7.80(3H,m).

(3) Benzyl (2R,3R)-2-ethoxycarbonylmethoxy-3-[N-methyl-N-[5-(2-naphthyl)pentyl]carbamoyl]-3-[5-(2-naphthyl)pentyloxy]propionate

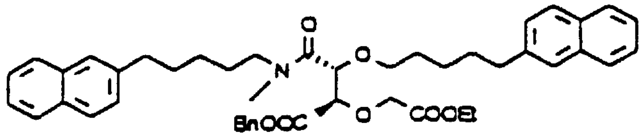

The 2-hydroxy compound (0.569 g) obtained in (2) was dissolved in N,N-dimethylformamide (5 ml), followed by the addition of sodium hydride (60% in oil, 0.06 g). To the resulting mixture was added dropwise ethyl bromoacetate (0.2 ml) and the resulting mixture was stirred at room temperature for 3 hours. The reaction mixture was diluted with diethyl ether, washed with saturated saline and then dried over anhydrous sodium sulfate. The solvent was distilled off under reduced pressure. The residue was purified by chromatography on a silica gel column (ethyl acetate-hexane 1:2), whereby benzyl (2R,3R)-3-tert-butoxycarbonyl-2-ethoxycarbonylmethoxy-3-[5-(2-naphthyl)pentyloxy]propionate was obtained as an oil.

Mass (EI) m/Z: 578($M^+$).
$^1$H-NMR($CDCl_3$)δ: 1.20-1.71(6H,m), 1.23(3H,t,J=7.3Hz), 1.48(9H,s), 3.18(1H,dt,J=8.8,6.8Hz), 3.68(1H,dt,J=8.8,6.8Hz), 4.14(2H,q,J=3.4Hz), 4.20(1H,d,J=3.4Hz), 4.20(1H,d,J=3.4Hz), 4.25(1H,d,J=16.6Hz), 4.38(1H,d,J=16.6Hz), 4.69(1H,d,J=3.4Hz), 5.13(1H,d,J=12.2Hz), 5.19(1H,d,J=12.2Hz), 7.27-7.35(6H,m), 7.35-7.50(2H,m), 7.58(1H,s), 7.73-7.79(3H,m).

The resulting compound was dissolved in methylene chloride (6 ml). To the resulting solution was added trifluoroacetic acid (1.5 ml), and the mixture was stirred at room temperature for 1.5 hours. The reaction mixture was concentrated to dryness reduced pressure, whereby benzyl (2R,3R)-3-carboxy-2-ethoxycarbonylmethoxy-3-[5-(2-naphthyl)pentyloxy]propionate was obtained as an oil.

The resulting compound was dissolved in methylene chloride (5 ml). To the resulting solution were added methyl[5-(2-naphthyl)pentyl]amine (0.30 g), 1-hydroxybenzotriazole (0.03 g) and 1-ethyl-3-(3-dimethylaminopropyl) carbodiimide hydrochloride, and the mixture was stirred at room temperature for 24 hours. The reaction mixture was diluted with ethyl ether, washed with water and saturated saline and then dried over anhydrous sodium sulfate. The solvent was distilled off under reduced pressure. The residue was purified by chromatography on a silica gel column (ethyl acetate-hexane 2:3), whereby the title compound (0.456 g) was obtained as an oil.

Mass (EI) m/Z: 731($M^+$).
$^1$H-NMR(rotamer mixture, $CDCl_3$)δ: 1.23(3H,t,J=7.3Hz), 1.23-1.39(4H,m), 1.47-1.88(8H,m), 2.65-2.79(4H,m), 2.83,3.09(total 3H,s each), 3.15-3.60(4H,m), 4.07-4.19(3H,m), 4.32-4.39(1H,m), 4.48-4.55(2H,m), 5.11(1H,d,J=12.2Hz), 5.18(1H,d,J=12.2Hz), 7.20-7.31(7H,m), 7.33-7.44(4H,m), 7.51-7.57(2H,m), 7.71-7.79(6H,m).

(4) (2R,3R)-2-Ethoxycarbonylmethoxy-3-[N-methyl-N-[5-(2-naphthyl)pentyl]carbamoyl]-3-[5-(2-naphthyl)pentyloxy]propionic acid

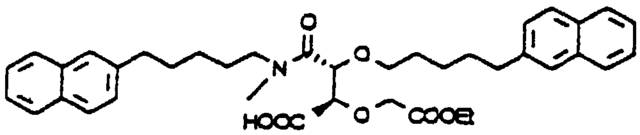

The benzyl ester compound (0.40 g) obtained in (3) was dissolved in a mixture of ethanol (5 ml) and tetrahydrofuran (5 ml). To the resulting solution was added 10% palladium-carbon (0.05 g), and the mixture was stirred at room temperature for 3 days under hydrogen atmosphere. After the removal of 10% palladium-carbon by filtration, the solvent was distilled off under reduced pressure, whereby the title compound (0.10 g) was obtained as an oil.

Mass (FAB+) m/Z: 642(M+H)$^+$.
$^1$H-NMR(rotamer mixture, $CDCl_3$)δ: 1.26,1.27(total 3H,t each,J=7.4Hz), 1.20-1.80(13H,m), 2.70-2.85(4H,m), 2.94,3.09(total 3H,s each), 3.25-3.55(5H,m), 4.10-4.25(2H,m), 4.32-4.34(1H,m), 4.42,4.31(1H,d each,J=5.8Hz), 4.66,4.69(1H,d each,J=5.8Hz), 7.20-7.30(2H,m), 7.35-7.45(4H,m), 7.55-7.60(2H,broad s), 7.70-7.85(6H,m).

Example 11

Disodium (2R,3R)-2-carboxymethoxy-3-[N-methyl-N-[5-(2-naphthyl)pentyl]carbamoyl]-3-[5-(2-naphthyl)pentyloxy]propionate

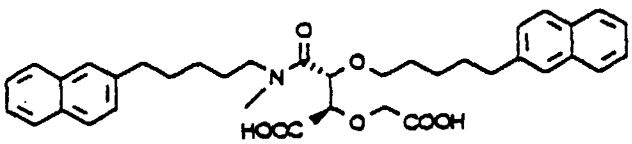

Benzyl (2R,3R)-2-ethoxycarbonylmethoxy-3-[N-methyl-N-[5-(2-naphthyl)pentyl]carbamoyl]-3-[5-(2-naphthyl)pentyloxy]propionate (0.19 g) obtained in Example 10-(4) was dissolved in ethanol (2 ml). To the resulting solution was added a 1N aqueous solution of sodium hydroxide (0.58 ml), and the mixture was stirred at room temperature for 2 days. The solvent was distilled off under reduced pressure. To the residue was added diethyl ether, whereby the title compound (0.096 g) was obtained as amorphous powder.

Mass (FAB+) m/Z: 680(M+Na)$^+$, 658(M+H)$^+$.
$^1$H-NMR(rotamer mixture, $CD_3OD$)δ: 1.24-1.42(4H,m), 1.53-1.80(8H,m), 2.72-2.77(4H,m), 2.90,3.09(total 3H,s each), 3.10-3.50(2H,m), 3.63,3.66(total 1H,d each,J=14.1/14.1Hz), 3.87,3.91(total 1H,s/d,J=1.5Hz), 4.16,4.17(total 1H,d each,J=14.1/14.1Hz), 4.66,4.68(total 1H,d/s,J=1.5Hz), 7.26-7.39(6H,m), 7.56,7.59(total 2H,s each), 7.69-7.76(6H,m).

| Elementary analysis for $C_{37}H_{41}NNa_2O_7 \cdot 3H_2O$ | | | |
|---|---|---|---|
| Calculated: | C, 62.43; | H, 6.66; | N, 1.97. |
| Found: | C, 62.59; | H, 6.59; | N, 1.82. |

Example 12

(2R,3R)-2-Hydroxy-3-[N-methyl-N-[5-(2-naphthyl)pentyl]carbamoyl]-3-[5-(2-naphthyl)pentyloxy]propionic acid

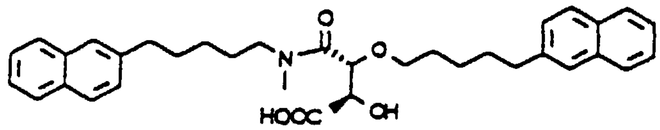

(1) tert-butyl (2R,3R)-3-Carboxy-2-hydroxy-3-[5-(2-naphthyl)pentyloxy]propionate and tert-butyl (2R,3R)-3-carboxy-3-hydroxy-2-[5-(2-naphthyl)pentyloxy]propionate

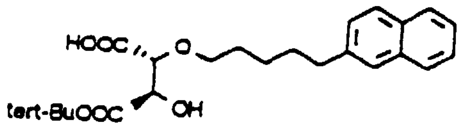

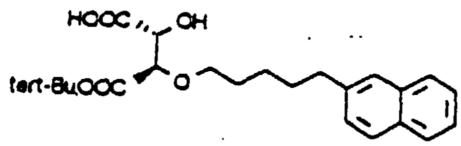

A mixture (1.45 g) of tert-butyl (2R,3R)-3-benzyloxycarbonyl-3-hydroxy-2-[5-(2-naphthyl)pentenyloxy]propionate and tert-butyl (2R,3R)-3-benzyloxycarbonyl-2-hydroxy-3-[5-(2-naphthyl)pentenyloxy]propionate obtained in Example 1-(1) was dissolved in a mixture of ethanol (20 ml) and tetrahydrofuran (8 ml). To the resulting solution was added 10% palladium-carbon (0.2 g), and the mixture was stirred at room temperature for 31 hours under hydrogen atmosphere. After the removal of palladium-carbon by filtration, the filtrate was concentrated to dryness under reduced pressure, whereby a mixture (1.20 g) of tert-butyl (2R,3R)-3-carboxy-3-hydroxy-2-[5-(2-naphthyl)pentyloxy]propionate and tert-butyl (2R,3R)-3-carboxy-2-hydroxy-3-[5-(2-naphthyl)pentyloxy]propionate was obtained as an oil.

Mass (FAB+) m/Z: 425(M+Na)$^+$.
$^1$H-NMR(CDCl$_3$)δ: 1.37-1.46(2H,m), 1.48,1.52(total 9H,s each), 1.61-1.73(4H,m), 2.74(2H,m), 3.33-3.40(1H,m), 3.67-3.77(1H,m), 4.19,4.23(total 1H,d each,J=2.4/2.4Hz), 4.48,4.55(total 1H,d each,J=2.4/2.4Hz), 7.33-7.45(3H,m), 7.58(1H,s), 7.73-7.79(3H,m).

(2) tert-Butyl (2R,3R)-2-hydroxy-3-[N-methyl-N-(5-(2-naphthyl)pentylcarbamoyl]-3-[5-(2-naphthyl)pentyloxy]propionate

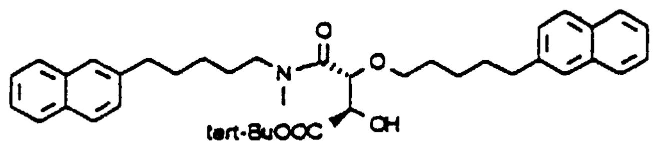

The mixture (0.43 g) obtained in (1), methyl [5-(2-naphthyl)pentyl]amine (0.268 g) and 4-dimethylaminopyridine (0.122 g) were dissolved in methylene chloride (15 ml), followed by the addition of 1-ethyl-3-(3-dimethylaminopropyl)carbodiimide hydrochloride (0.266 g) under cooling with ice water. After stirring at room temperature for 16.5 hours, the reaction mixture was diluted with methylene chloride and added with 0.5N hydrochloric acid. The mixture was then

filtered through Celite. The organic layer was separated from the filtrate, washed with saturated saline and then dried over anhydrous sodium sulfate. The solvent was distilled off under reduced pressure. The residue was purified by chromatography on a silica gel column (elution with 40% ethyl acetate-hexane), whereby the title compound (0.106 g) was obtained as an oil.

Mass (EI) m/Z: 611($M^+$).
$^1$H-NMR(rotamer mixture, $CDCl_3$)δ: 1.33-1.42(4H,m), 1.44,1.48(total 9H,s each), 1.54,-1.72(8H,m), 2.72-2.75(4H,m), 2.92,3.09(total 3H,s each), 3.24-3.58(4H,m), 4.30-4.40(2H,m), 7.28-7.30(2H,m), 7.38-7.44(4H,m), 7.57(1H,s), 7.58(1H,s), 7.73-7.79(6H,m).

(3) (2R,3R)-2-Hydroxy-3-[N-methyl-N-[5-(2-naphthyl)carbamoyl]-3-[5-(2-naphthyl)pentyloxy]propionic acid

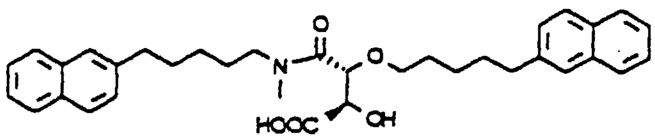

The tert-butyl ester compound (0.106 g) obtained in (2) was treated in the same manner as in Example 1-(5), whereby the title compound (0.10 g) was obtained as an oil.

$^1$H-NMR(rotamer mixture, $CDCl_3$)δ: 1.25-1.36(4H,m), 1.55-1.75(8H,m), 2.72-2.79(4H,m), 2.95,3.04(total 3H,s each), 3.32-3.40(3H,m), 3.50-3.55(1H,m), 4.35,4.37(total 1H,seach), 4.61,4.63(total 1H,d/s,J=2.4Hz), 7.28-7.30(2H,m), 7.37-7.46(4H,m), 7.57(2H,s), 7.73-7.80(6H,m).

The resulting compound was dissolved in ethanol (3 ml), followed by the addition of a 1N aqueous solution (0.17 ml) of sodium hydroxide. The resulting mixture was stirred at room temperature for 2 hours. The reaction mixture was concentrated to dryness under reduced pressure, whereby sodium (2R,3R)-2-hydroxy-3-[N-methyl-N-[5-(2-naphthyl)pentyl]carbamoyl-3-[5-(2-naphthyl)pentyloxy]propionate (0.109 g) was obtained as a viscous oil.

Mass (FAB+) m/Z: 600$(M+Na)^+$, 578$(M+H)^+$
$^1$H-NMR(rotamer mixture, $CD_3OD$)δ: 1.30-1.39(4H,m), 1.55-1.73(8H,m), 2.71-2.74(4H,m), 2.89,3.08(total 3H,s each), 3.26-2.47,3.57-3.65(total 4H,m each), 4.01,4.05(total 1H,d each,J=2.0/2.0Hz), 4.60,4.65(total 1H,d each,J=2.0/2.0Hz), 7.28-7.41(6H,m), 7.57(2H,broad s), 7.69-7.76(6H,m).

Example 13

(2R,3R)-3-Carboxymethoxy-3-[N-[5-(3,5-dimethyl-4-hydroxyphenyl)pentyl]carbamoyl]-3-[5-(2-naphthyl)pentyloxy]propionic acid

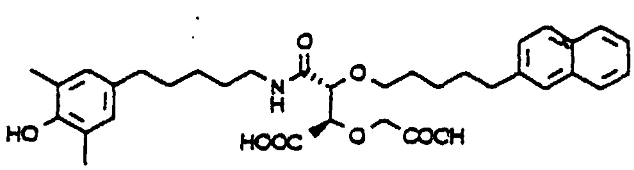

(1) tert-Butyl (2R,3R)-2-tert-butoxycarbonylmethoxy-3-[N-[5-(3,5-dimethyl-4-methoxymethoxyphenyl)pentyl]carbamoyl]-3-[5-(2-naphthyl)pentyloxy]propionate

In the same manner as in Example 1-(4) except for the use of 5-(3,5-dimethyl-4-methoxymethoxyphenyl)pentylamine and a catalytic amount of 1-hydroxybenzotriazole instead of 5-(2-naphthyl)pentylamine hydrochloride and 4-dimethylaminopyridine, respectively, the title compound (0.20 g) was obtained as an oil.

Mass (EI) m/Z: 749($M^+$).
$^1$H-NMR($CDCl_3$)δ: 1.31-1.70(12H,m), 1.43(9H,s), 1.49(9H,s), 2.25(6H,s), 2.46(2H,t,J=7.8Hz), 2.75(2H,t,J=7.3Hz), 3.25(2H,q,J=6.8Hz), 3.39-3.55(2H,m), 3.60(3H,s), 3.88(1H,d,J=15.6Hz), 4.17(1H,d,J=2.5Hz), 4.25(1H,d,J=15.6Hz), 4.32(1H,d,J=2.5Hz), 4.92(2H,s), 6.70(1H,t,J=6.0Hz), 6.79(2H,s), 7.29(1H,dd,J=8.9,1.0Hz), 7.41(2H,dt,J=8.9,1.0Hz), 7.57(1H,s), 7.75(2H,d,J=8.3Hz), 7.79(1H,d,J=8.3Hz).

(2) (2R,3R)-2-Carboxymethoxy-3-[N-[5-(3,5-dimethyl-4-hydroxyphenyl)pentyl]carbamoyl]-3-[5-(2-naphthyl)pentyloxy]propionic acid

The above-described di-tert-butyl ester compound (0.20 g) was treated in the same manner as in Example 1-(5). The residue was then purified by a high-performance liquid column chromatography ("Sensyu Pak ODS-5251-SH", elution with acetonitrile-0.1% trifluoroacetic acid 65:35 (V/V)), whereby the title compound (0.055 g) was obtained as an oil.

Mass (FAB+) m/Z: 594(M+H).
$^1$H-NMR($CDCl_3$)δ: 1.23-1.69(12H,m), 2.18(6H,s), 2.42(2H,t,J=7.3Hz), 2.73(2H,t,J=7.3Hz), 3.23(2H,q,J=6.4Hz), 3.47-3.58(2H,m), 4.10(1H,d,J=17.6Hz), 4.24(1H,d,J=17.6Hz), 4.35(1H,s), 4.55(1H,s), 6.72(2H,s), 6.95(1H,t,J=5.6Hz), 7.28(1H,d,J=8.3Hz), 7.40(2H,dt,J=6.8,1.0Hz), 7.57(1H,s), 7.73(1H,d,J=8.3Hz), 7.74(1H,d,J=8.3Hz), 7.76(1H,d,J=8.3Hz).

Example 14

(2R,3R)-2-[N-[5-(2-Naphthyl)pentyl]carbamoyl]methoxy-3-[5-(2-naphthyl)pentyloxy]succinic acid

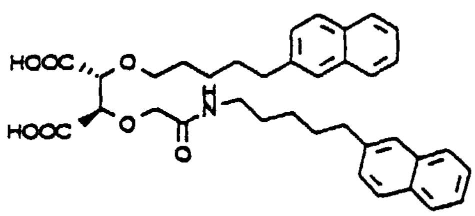

(1) Diethyl (2R,3R)-2-hydroxy-3-[5-(2-naphthyl)-2-pentenyloxy]succinate

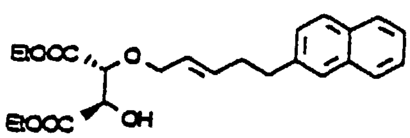

In N,N-dimethylformamide (120 ml), 60% sodium hydride (1.60 g) was suspended. To the resulting suspension was added a solution of diethyl L-tartrate (8.25 g) in N,N-dimethylformamide (10 ml) under cooling with ice water, followed by the dropwise addition of a solution of 5-(2-naphthyl)-2-pentenyl iodide (9.0 g) in N,N-dimethylformamide (40 ml) over about 7 minutes. After stirring at room temperature for one hour, the reaction mixture was poured into a mixture of ice water (500 ml) and saturated saline (200 ml). The resulting mixture was extracted with ethyl acetate. The extract was washed twice with saturated saline and then dried over anhydrous sodium sulfate. The solvent was distilled off under reduced pressure. The residue was purified by chromatography on a silica gel column (elution with 25 to 30% ethyl acetate-hexane), whereby the title compound (6.51 g) was obtained as an oil.

Mass (EI) m/Z: 400($M^+$).
$^1$H-NMR($CDCl_3$)δ: 1.26(3H,t,J=7.4Hz), 1.31(3H,t,J=7.3Hz), 2.42-2.47(2H,m), 2.80-2.87(2H,m), 3.06(1H,d,J=8.3Hz), 3.89(1H,dd,J=11.7,7.3Hz), 4.20-4.31(6H,m), 4.55(1H,dd,J=8.8,2.4Hz), 5.46-5.53(1H,m), 5.69-5.77(1H,m), 7.31(1H,dd,J=8.3,1.5Hz), 7.40-7.47(2H,m), 7.59(1H,s), 7.76-7.81(3H,m).

(2) Diethyl (2R,3R)-2-hydroxy-3-[5-(2-naphthyl)pentyloxy]succinate

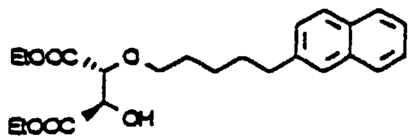

The 3-[5-(2-naphthyl)-2-pentenyloxy] compound (7.7 g) obtained in (1) was dissolved in ethyl acetate (80 ml). To the resulting solution was added 10% palladium-carbon (0.8 g), and the mixture was stirred at room temperature for 17.5 hours under hydrogen atmosphere. After the removal of palladium-carbon by filtration, the filtrate was concentrated to dryness under reduced pressure, whereby the title compound (6.75 g) was obtained as an oil.

Mass (EI) m/Z: 402($M^+$).
$^1$H-NMR($CDCl_3$)δ: 1.23-1.49(8H,m), 1.55-1.74(4H,m), 2.76(2H,t,J=6.8Hz), 3.06(1H,d,J=7.8Hz), 3.26-3.31(1H,m), 3.75-3.81(1H,m), 4.17-4.32(5H,m), 4.57(1H,m), 7.31(1H,dd,J=8.3,1.5Hz), 7.38-7.46(2H,m), 7.59(1H,s), 7.74-7.80(3H,m).

(3) (2R,3R)-2-Hydroxy-3-[5-(2-naphthyl)pentyloxy]succinic acid

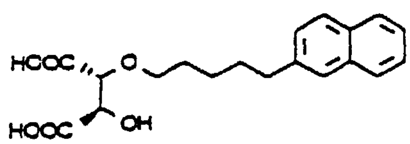

The 3-[5-(2-naphthyl)pentyloxy] compound (5.70 g) obtained in (2) was dissolved in ethanol (60 ml), followed by the dropwise addition of a 2N aqueous sodium hydroxide solution (18 ml). After stirring at room temperature for 1.25 hours, methanol and water were added to the reaction mixture to dissolve the insoluble matter therein. An ion exchange resin ("DOWEX$^{TX}$ 50W-X4, $H^+$ type") was added to make the resulting solution acidic. The ion exchange resin was filtered off, followed by washing with ethanol. The filtrate and washing were combined together and then concentrated under reduced pressure. Toluene was added to the residue to powder the same, whereby the title compound (4.33 g) was obtained as pale yellow crystals.

Mass (FAB+) m/Z: 347$(M+H)^+$.
$^1$H-NMR($CD_3OD$)δ: 1.42-1.49(2H,m), 1.59-1.75(4H,m), 2.77(2H,t,J=7.3Hz), 3.32-3.38(1H,m), 3.74-3.79(1H,m), 4.31(1H,d,J=2.4Hz), 4.54(1H,d,J=2.4Hz), 7.32-7.41(3H,m), 7.61(1H,s), 7.73-7.78(3H,m).

| Elementary analysis for $C_{19}H_{22}O_6$ | | |
|---|---|---|
| Calculated: | C, 65.89; | H, 6.40. |
| Found: | C, 65.54; | H, 6.34. |

(4) Dibenzyl (2R,3R)-2-hydroxy-3-[5-(2-naphthyl)pentyloxy]succinate

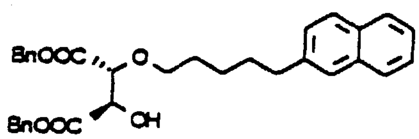

The succinic acid compound (3.0 g) obtained in (3) was dissolved in N,N-dimethylformamide (30 ml). To the resulting solution was added triethylamine (3.05 ml) under cooling with ice water, followed by the dropwise addition of benzyl bromide (2.6 ml). After stirring at room temperature for 21 hours, triethylamine (1.2 ml) and benzyl bromide (0.5 ml) were added again and the resulting mixture was stirred for 19 hours. The reaction mixture was poured into dilute hydrochloric acid, followed by extraction with ethyl acetate. The extract was washed with saturated saline and then dried over anhydrous sodium sulfate. The solvent was distilled off under reduced pressure. The residue was purified by chromatography on a silica gel column (elution with 30% ethyl acetate-hexane), whereby the title compound (3.19 g) was obtained as an oil.

Mass (FAB+) m/Z: 527$(M+H)^+$.
$^1$H-NMR($CDCl_3$)δ: 1.23-1.36(2H,m), 1.46-1.53(2H,m), 1.60-1.68(2H,m), 2.72(2H,t,J=7.3Hz), 3.08(1H,d,J=8.8Hz), 3.10-3.16(1H,m), 3.65-3.70(1H,m), 4.26(1H,d,J=2.0Hz), 4.64(1H,dd,J=8.8,2.4Hz), 5.14(1H,d,J=12.2Hz), 5.19(1H,d,J=12.2Hz), 5.20(1H,d,J=12.2Hz), 5.23(1H,d,J=12.2Hz), 7.26-7.34(11H,m), 7.37-7.45(2H,m), 7.57(1H,s), 7.76-7.80(3H,m).

(5) Dibenzyl (2R,3R)-2-tert-butoxycarbonylmethoxy-3-[5-(2-naphthyl)pentyloxy]succinate

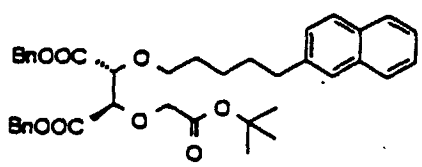

The dibenzyl ester compound (2.08 g) obtained in (4) was treated in the same manner as in Example 1-(2), whereby the title compound (0.88 g) was obtained as an oil.

Mass (FAB+) m/Z: 641$(M+H)^+$.
$^1$H-NMR($CDCl_3$)δ: 1.25-1.38(2H,m), 1.42(9H,s), 1.49-1.68(4H,m), 2.71(2H,t,J=7.8Hz), 3.15-3.21(1H,m), 3.64-3.69(1H,m), 4.04(1H,d,J=16.6Hz), 4.24(1H,d,J=16.6Hz), 4.37(1H,d,J=2.9Hz), 4.63(1H,d,J=2.9Hz), 5.10(1H,d,J=12.2Hz), 5.17(1H,d,J=12.2Hz), 5.20(1H,d,J=12.2Hz), 5.25(1H,d,J=12.2Hz), 7.26-7.35(9H,m), 7.37-7.44(4H,m), 7.56(1H,s), 7.73-7.79(3H,m).

(6) Dibenzyl (2R,3R)-2-Carboxymethoxy-3-[5-(2-naphthyl)pentyloxy]succinate

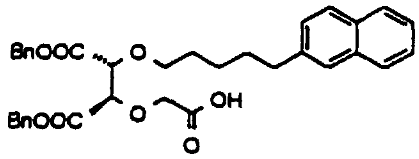

The 2-tert-butoxycarbonylmethoxy compound (0.88 g) obtained in (5) was treated in the same manner as in Example 1-(5), whereby the title compound (0.87 g) was obtained as an oil.

Mass (FAB+) m/Z: 585$(M+H)^+$.
$^1$H-NMR($CDCl_3$)δ: 1.22-1.35(2H,m), 1.45-1.54(2H,m), 1.60-1.68(2H,m), 2.72(2H,t,J=7.3Hz), 3.14-3.20(1H,m), 3.66-3.72(1H,m), 4.09(1H,d,J=16.6Hz), 4.11(1H,d,J=16.6Hz), 4.36(1H,d,J=3.4Hz), 4.49(1H,d,J=3.4Hz), 5.11(1H,d,J=11.7Hz), 5.14(1H,d,J=12.2Hz), 5.18(1H,d,J=12.2Hz), 5.24(1H,d,J=11.7Hz), 7.25-7.33(11H,m), 7.38-7.45(2H,m), 7.57(1H,s), 7.74-7.79(3H,m).

(7) Dibenzyl (2R,3R)-2-[N-[5-(2-naphthyl)pentyl]carbamoyl]methoxy-3-[5-(2-naphthyl)pentyloxy]succinate

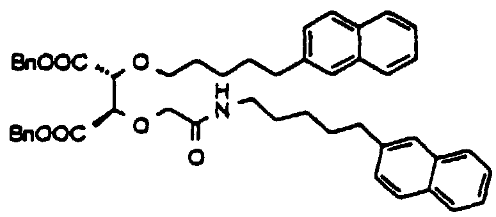

The 2-carboxymethoxy compound (0.465 g) obtained in (6) was treated in the same manner as in Example 1-(4), whereby the title compound (0.40 g) was obtained as an oil.

Mass (FAB+) m/Z: 780$(M+H)^+$.
$^1$H-NMR($CDCl_3$)δ: 1.23-1.40(2H,m), 1.46-1.57(4H,m), 1.63-1.73(4H,m), 2.69-2.76(4H,m), 3.14-3.22(3H,m), 3.66-3.72(1H,m), 3.86(1H,d,J=15.1Hz), 4.08(1H,d,J=15.1Hz), 4.35(1H,d,J=3.4Hz), 4.38(1H,d,J=3.4Hz), 5.09(1H,d,J=12.2Hz), 5.12(1H,d,J=12.2Hz), 5.15(1H,d,J=12.2Hz), 5.20(1H,d,J=12.2Hz), 6.99(1H,t,J=5.4Hz), 7.26-7.34(12H,m), 7.37-7.44(4H,m), 7.56(1H,s), 7.58(1H,s), 7.72-7.78(6H,m).

(8) (2R,3R)-2-[N-[5-(2-Naphthyl)pentyl]carbamoyl]methoxy-3-[5-(2-naphthyl)pentyloxy]succinic acid

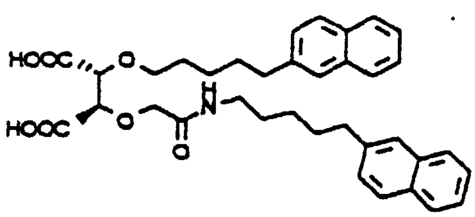

The 2-carbamoylmethoxy compound (0.39 g) obtained in (7) was dissolved in ethanol (5 ml) and tetrahydrofuran (1 ml). To the resulting solution was added 10% palladium-carbon (0.08 g), and the mixture was stirred at room temperature for 18.5 hours under hydrogen atmosphere. The palladium-carbon was filtered off and the filtrate was concentrated to dryness under reduced pressure, whereby the title compound (0.274 g) was obtained as an oil.

Mass (FAB+) m/Z: 622$(M+Na)^+$, 600$(M+H)^+$.
$^1$H-NMR($CDCl_3$)δ: 1.31-1.39(4H,m), 1.48-1.55(2H,m), 1.57-1.70(6H,m), 2.68-2.73(4H,m), 3.19-3.24(2H,m), 3.38-3.43(1H,m), 3.68-3.74(1H,m), 4.14(1H,d,J=16.1Hz), 4.20(1H,d,J=16.1Hz), 4.36(1H,d,J=2.9Hz), 4.39(1H,d,J=2.9Hz), 5.01(~4H,broad), 7.25-7.28(2H,m), 7.35-7.43(4H,m), 7.55(2H,s), 7.70-7.77(6H,m).

The resulting compound (0.256 g) was dissolved in ethanol (5 ml). To the resulting solution was added a 2N aqueous sodium hydroxide solution (0.43 ml), and the mixture was stirred for 30 minutes. Under reduced pressure, the solvent was distilled off and the residue was powdered with ethanol, whereby disodium (2R,3R)-2-[N-[5-(2-naphthyl)pentyl]carbamoyl]methoxy-3-[5-(2-naphthyl)pentyloxy]succinate (0.095 g) was obtained as a powder.

Mass (FAB+) m/Z: 666$(M+Na)^+$, 644$(M+H)^+$.
$^1$H-NMR($CDCl_3$)δ: 1.37-1.45(4H,m), 1.57-1.78(8H,m), 2.75-2.80(4H,m), 3.19-3.23(2H,m), 3.3-3.34(1H,m), 3.68-3.74(1H,m), 3.90(1H,d,J=16.1Hz), 4.17(1H,d,J=2.4Hz), 4.18(1H,d,J=16.1Hz), 4.19(1H,d,J=2.4Hz), 7.31-7.43(6H,m), 7.61(2H,s), 7.72-7.78(6H,m).

| Elementary analysis for $C_{36}H_{39}NNa_2O_7 \cdot H_2O$ | | | |
|---|---|---|---|
| Calculated: | C, 65.34; | H, 6.24; | N, 2.12 |
| Found: | C, 64.92; | H, 6.24; | N, 2.03 |

Example 15

tert-Butyl (2R,3R)-2-tert-butoxycarbonylmethoxy-3-carboxy-3-[5-(2-naphthyl)pentyloxy]propionate

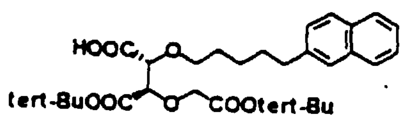

(1) tert-Butyl (2R,3R)-3-tert-butoxycarbonyl-3-hydroxy-2-[5-(2-naphthyl)pentyloxy]propionate

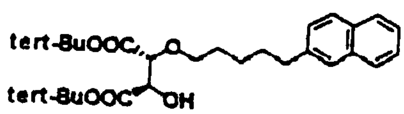

Process A) The dicarboxylic acid compound (2.0 g) obtained in Example 14-(3) was dissolved in methylene chloride (30 ml). To the resulting solution was added O-tert-butyl-N,N'-diisopropylisourea (5.8 g), and the mixture was stirred at room temperature for 2 days. The insoluble matter was filtered off and the filtrate was concentrated under reduced pressure. The residue was dissolved in ethyl acetate. The resulting solution was washed with 1N hydrochloric acid and saturated saline and dried over anhydrous sodium sulfate. The solvent was distilled off under

reduced pressure. The residue was purified by chromatography on a silica gel column (elution with 15% ethyl acetate-hexane), whereby the title compound (1.1 g) was obtained as a colorless oil.

Mass (FAB+) m/Z: 459(M+H)$^+$.
$^1$H-NMR($CDCl_3$)δ: 1.38-1.50(2H,m), 1.48(9H,s), 1.50(9H,s), 1.60-1.74(4H,m), 2.75(2H,t,J=7.8Hz), 3.01(1H,d,J=8.3Hz), 3.29(1H,dt,J=8.8,6.8Hz), 3.74(1H,dt,J=8.8,6.8Hz), 4.07(1H,d,J=2.4Hz), 4.44(1H,dd,J=8.3,2.4Hz), 7.31(1H,dd,J=8.3,1.5Hz), 7.38-7.45(2H,m), 7.58(1H,s), 7.74-7.80(3H,m).

Process b) Sodium hydride (60% in oil, 9.12 g) was suspended in N,N-dimethylformamide (600 ml), followed by the dropwise addition of a solution of di-tert-butyl L-(+)-tartrate (59.8 g) in N,N-dimethylformamide (100 ml) under cooling with ice water. After 5 minutes, a solution of 5-(2-naphthyl)-2-pentenyl iodide (61.2 g) in N,N-dimethylformamide (100 ml) was added dropwise and the resuiting mixture was stirred at room temperature for 2 hours. The reaction mixture was poured into ice water (1000 ml) and ethyl acetate (1000 ml) under stirring. To the resulting mixture was added saturated saline to separate the organic layer. The organic layer was washed with saturated saline and dried over anhydrous sodium sulfate. The solvent was distilled off under reduced pressure. The residue was purified by chromatography on a silica gel column (elution with 20% ethyl acetate-hexane), whereby tert-butyl (2R,3R)-3-tert-butoxycarbonyl-3-hydroxy-2-[5-(2-naphthyl)pent-2-en-1-yloxy]propionate (44.7 g) was obtained as a pale yellow oil.

$^1$H-NMR($CDCl_3$)δ: 1.47(9H,s), 1.50(9H,s), 2.41-2.47(2H,m), 2.85(2H,t,J=7.3Hz), 3.03(1H,d,J=7.8Hz), 3.85-3.90(1H,m), 4.13(1H,d,J=2.4Hz), 4.18-4.22(1H,m), 4.44(1H,dd,J=7.8,2.4Hz), 5.54-5.61(1H,m), 5.71-5.78(1H,m), 7.31(1H,dd,J=8.3,2.0Hz), 7.39-7.46(2H,m), 7.59(1H,s), 7.75-7.80(3H,m).

The resulting compound (22.0 g) was dissolved in ethyl acetate (300 ml). To the resulting solution was added 10% palladium-carbon (1 g), and the mixture was stirred at room temperature for 9.5 hours under hydrogen atmosphere. The palladium-carbon was filtered off and the filtrate was concentrated to dryness under reduced pressure, whereby the title compound (21.1 g) was obtained as a colorless oil.

(2) Benzyl (2R,3R)-3-tert-butoxycarbonyl-3-hydroxy-2-[5-(2-naphthyl)pentyloxy]propionate

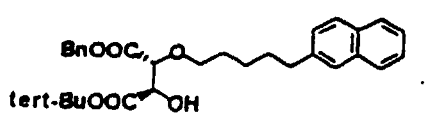

The di-tert-butyl ester compound (21.1 g) obtained in (1) was dissolved in N,N-dimethylformamide (65 ml). To the resulting solution was added dropwise diethylmethoxyborane (1M solution in tetrahydrofuran, 54 ml) under cooling with ice water. After 25 minutes, sodium borohydride (2.09 g) was added to the reaction mixture, followed by stirring at room temperature for 2.5 hours. The reaction mixture was poured into a mixture of sodium dihydrogenphosphate (108 g) and water (350 ml), which mixture had been cooled with ice water in advance, followed by the addition of tert-butanol (43 ml) and 2-methyl-2-butene (110 ml). Sodium chlorite (80%, 102 g) and water (220 ml) were added and the resulting mixture was stirred at room temperature for 15 hours. Water and ethyl acetate were added to the reaction mixture to separate the organic layer. The resulting organic layer was washed with 1N hydrochloric acid and then dried over anhydrous sodium sulfate. The solvent was distilled off under reduced pressure. To the residue were added toluene and triethylamine, followed by azeotropic distillation. The residue was then dissolved in N,N-dimethylformamide (200 ml). To the resulting solution were added triethylamine (13.9 g) and benzyl bromide (19.7 g) under cooling with ice water. The resulting mixture was stirred at room temperature for 21 hours and then concentrated under reduced pressure. The residue was diluted with diethyl ether, washed with 1N hydrochloric acid, a 5% aqueous solution of sodium bicarbonate and water and then dried over anhydrous sodium sulfate. The solvent was distilled off under reduced pressure and the residue was purified by chromatography on a silica gel column (elution with 15 to 20% ethyl acetate-hexane), whereby the title compound (13.95 g) was obtained as a pale yellow oil.

Mass (EI) m/Z: 492(M$^+$).
$^1$H-NMR($CDCl_3$)δ: 1.35-1.46(2H,m), 1.47(9H,s), 2.74(2H,t,J=7.8Hz), 3.08(1H,d,J=8.3Hz), 3.28-3.33(1H,m), 3.71-3.76(1H,m), 4.24(1H,d,J=2.0Hz), 4.48(1H,dd,J=7.8,2.0Hz), 5.21(1H,d,J=12.1Hz), 5.26(1H,d,J=12.1Hz), 7.29-7.44(8H,m), 7.58(1H,s), 7.74-7.80(3H,m).

(3) tert-Butyl (2R,3R)-3-benzyloxycarbonyl-2-tert-butoxycarbonylmethoxy-3-[5-(2-naphthyl)pentyloxy]propionate

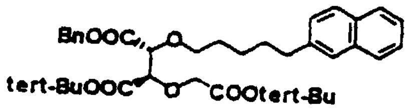

The diester (2.03 g) obtained in (2) was dissolved in tetrahydrofuran (20 ml). To the resulting solution was added dropwise potassium bistrimethylsilylamide (0.5M solution in toluene, 8.4 ml) at -70°C or lower. Then, a solution of tert-butyl bromoacetate (1.05 g) in tetrahydrofuran (5 ml) was added dropwise, followed by heating to room temperature. After the reaction mixture was poured into a mixture of 0.5N hydrochloric acid and ethyl acetate, the organic layer was separated. After the resulting organic layer was washed with saturated saline and dried over anhydrous sodium sulfate, the solvent was distilled off under reduced pressure. The residue was purified by chromatography on a silica gel column (elution with 15% ethyl acetate-hexane), whereby the title compound (1.98 g) was obtained as a colorless oil.

Mass (EI) m/Z: 606($M^+$).
$^1$H-NMR($CDCl_3$)δ: 1.50(9H,s), 1.52(9H,s), 1.45-1.85(6H,m), 2.79(2H,t,7.81Hz), 3.25-3.45(1H,m), 3.70-3.85(1H,m), 4.06(1H,d,J=6.6Hz), 4.33(1H,d,J=6.6Hz), 4.42(1H,d,J=2.9Hz), 4.53(1H,d,J=2.9Hz), 7.28-7.45(8H,m), 7.57(1H,s), 7.73-7.80(3H,m).

(4) tert-Butyl (2R,3R)-2-tert-butoxycarbonylmethoxy-3-carboxy-3-[5-(2-naphthyl)pentyloxy]propionate

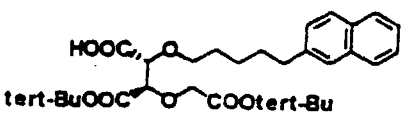

The triester (1.86 g) obtained in (3) was dissolved in ethyl acetate (30 ml), followed by the addition of 10% palladium-carbon (0.1 g). The resulting mixture was stirred at room temperature for 12.5 hours under hydrogen atmosphere. After palladium-carbon was filtered off, the filtrate was concentrated to dryness under reduced pressure, whereby the title compound (1.63 g) was obtained as a pale yellow oil.

Mass (FAB+) m/Z: 599$(M+Na)^+$, 517$(M+H)^+$.
$^1$H-NMR($CDCl_3$)δ: 1.38-1.51(2H,m), 1.45(9H,s), 1.48(9H,s), 1.62-1.74(4H,m), 2.75(2H,t,J=7.5Hz). 3.45(1H,dt,J=9.3,6.8Hz), 3.67(1H,dt,K=9.3,6.8Hz), 4.02(1H,d,J=16.1Hz), 4.31(1H,d,J=16.1Hz), 4.36(1H,d,J=2.7Hz), 4.37(1H,d,J=2.7Hz), 7.30(1H,dd,J=8.3,1.5Hz), 7.38-7.45(2H,m), 7.57(1H,s), 7.74-7.80(3H,m).

Example 16

The 3-carboxy compound (0.20 g) obtained in Example 15-(4) was dissolved in methylene chloride (20 ml), followed by the addition of 5-(1,3-benzodioxol-5-yl)pentylamine (0.096 g) obtained in Referential Example 6, 1-hydroxybenzotriazole (0.053 g) and 1-ethyl-3-(3-dimethylaminopropyl)carbodiimide hydrochloride (0.114 g) under cooling with ice water. The resulting mixture was stirred at room temperature for 16 hours. The reaction mixture was washed with dilute hydrochloric acid and saturated saline and then dried over anhydrous sodium sulfate. The solvent was then distilled off under reduced pressure. The residue was purified by chromatography on a silica gel column (25% ethyl acetate-hexane), whereby the compound of Example 16-a (0.144 g) was obtained as a colorless oil.
Compounds of Examples 16-b and 16-c were obtained by reacting in the same manner as in the above-described reaction except for the use of, instead of 5-(1,3-benzodioxol-5-yl)pentylamine, the corresponding amine derivatives of Referential Examples 7 and 8, respectively.

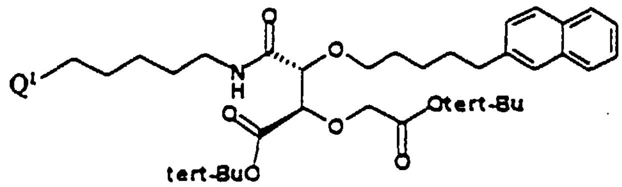

| Example | $Q^1$ | Mass(FAB+) m/Z: | $^1$H-NMR($CDCl_3$) δ : |
|---|---|---|---|
| 16-a | 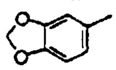 | 706(M+H)$^+$ | 1.22-1.73(30H,m),2.49(2H,t,J=7.6Hz),2.75 (2H,t,J=7.6Hz),3.20-3.30(2H,m),3.40-3.55(2H,m),3.88(1H,d,J=15.6Hz),4.17(1H,d,J=2.4Hz),5.90(2H,s),6.58(1H,d,J=7.8Hz),6.64(1H,d,J=1.5Hz),6.67-6.73(2H,m),7.28-7.82(7H,m). |
| 16-b | 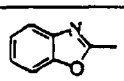 | 703(M+H)$^+$ | 1.32-1.73(28H,m),1.83-1.92(2H,m),2.74 (2H,t,J=7.6Hz),2.91(2H,t),3.20-3.32(2H,m),3.38-3.53(2H,m),3.88(1H,d,J=16.1Hz),4.17 (1H,d,J=2.4Hz),4.26(1H,d,J=16.1Hz),4.32(1H,d,J=2.4Hz),6.72(1H,t,J=5.8Hz),7.26-7.80(11H,m). |
| 16-c | 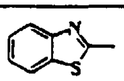 | 719(M+H)$^+$ | 1.30-1.70(28H,m),1.81-1.91(2H,m),2.75 (2H,t,J=7.8Hz),3.09(2H,t,J=6.8Hz),3.25-3.55(4H,m),3.88(1H,d,J=16.1Hz),4.17(1H,d,J=2.4Hz),4.26(1H,d,J=16.1Hz),4.32(1H,d,J=2.4Hz),6.85-6.75(1H,broad),7.27-8.00(11H,m). |

Example 17

The di-tert-butyl-ester compound (0.144 g) of Example 16-a was dissolved in methylene chloride (6 ml), followed by the addition of trifluoroacetic acid (4 ml). The resulting mixture was stirred at room temperature for 2 hours. The reaction mixture was concentrated to dryness under reduced pressure, whereby the compound (0.136 g) of Example 17-a was obtained as a colorless oil.

In a similar reaction to that described above, Compounds of Examples 17-b and 17-c were obtained.

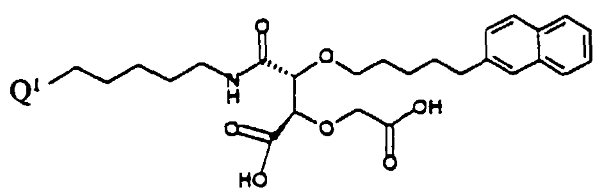

| Example | $Q^1$ | Mass (FAB+) m/Z: | $^1$H-NMR δ: |
|---|---|---|---|
| 17-a | 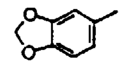 | 594(M+H)$^+$ | (Disodium salt, $CD_3OD$) 1.20-1.75(12H,m),2.44(2H,t,J=7.3Hz),2.74(2H,t,J=7.6Hz),3.05-3.52(4H,m),3.79(1H,d,J=14.2Hz), 3.90(1H,d,J=14.2Hz),4.01(1H,s),4.17(1H,s),5.82(2H,s),6.54(1H,d,J=7.8Hz),6.60(1H,s),6.63(1H,d,J=7.8Hz),7.26-7.80(7H,m). |
| 17-b | 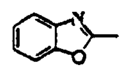 | 591(M+H)$^+$ | ($CDCl_3$)1.30-1.90(12H,m),2.70-2.76(2H,m),2.95-3.05(2H,m),3.20-3.30(2H,m),3.45-3.60(2H,m),4.23(1H,d,J=17.1Hz),4.28-4.35(2H,m),4.55(1H,broad),6.95-7.05(1H,broad),7.30-7.80(11H,m). |
| 17-c | 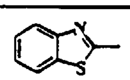 | 607(M+H)$^+$ | ($CDCl_3$)1.22-1.92(12H,m),2.68-2.78(4H,m),3.20-3.30(2H,m),3.42-3.60(2H,m),4.20-4.40(4H,m), 7.00-7.10(1H,broad),7.30-8.10(11H,m). |

Example 18

tert-Butyl (2R,3R)-3-benzyloxycarbonyl-2-tert-butoxycarbonylmethoxy-3-(4-oxobutoxy)propionate

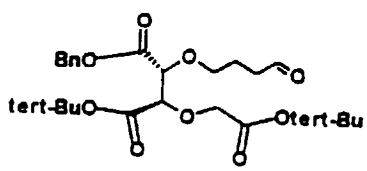

(1) tert-Butyl (2R,3R)-3-tert-butoxycarbonyl-2-[4-(tert-butyldiphenylsilyloxy)-2-butenyloxy]-3-hydroxypropionate

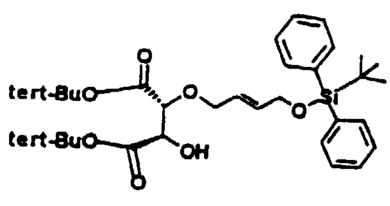

A solution of di-tert-butyl L-(+)-tartrate (65.4 g) in N,N-dimethylformamide (350 ml) was stirred under ice cooling, followed by the addition of sodium hydride (60% in oil, 9.98 g). The resulting mixture was stirred for 15£ minutes. A solution of 4-tert-butyldimethylsilyloxy-1-iodo-2-butene (108.8 g) of Referential Example 9 in N,N-dimethylformamide (100 ml) was added dropwise and the resulting mixture was stirred at room temperature for 6.5 hours. After the solvent

was distilled off under reduced pressure, water was added to the residue and the resulting mixture was diluted with diethyl ether. The organic layer was washed with saturated saline and dried over anhydrous magnesium sulfate. The residue obtained by distilling off the solvent under reduced pressure was purified by chromatography on a silica gel column (elution with 16% ethyl acetate-hexane), whereby the title compound (62.1 g) was obtained as a yellow oil.

Mass (FAB+) m/Z: 571(M+H)$^+$.
$^1$H-NMR($CDCl_3$)δ: 1.05(9H,s), 1.48(9H,s), 1.51(9H,s), 3.06(1H,d,J=8.3Hz), 3.92(1H,dd,J=11.9,5.1Hz), 4.16(1H,d,J=2.4Hz), 4.18-4.22(2H,m), 4.31(1H,dd,J=12.2,4.9Hz), 4.46(1H,dd,J=8.3,2.4Hz), 5.75-5.87(2H,m), 7.36-7.44(6H,m), 7.65-7.67(4H,m).

(2) tert-Butyl (2R,3R)-3-tert-butoxycarbonyl-2-[4-(tert-butyldiphenylsilyloxy)butoxy]-3-hydroxypropionate

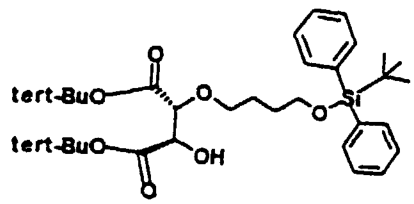

The 2-butenyloxy compound (62.1 g) obtained in (1) was dissolved in methanol (500 ml). To the resulting solution was added 10% palladium-carbon (3.0 g), and the mixture was stirred for 3 days under hydrogen atmosphere. After the 10% palladium-carbon was filtered off, the solvent was distilled off from the filtrate under reduced pressure, whereby the title compound (56.8 g) was obtained as a yellow oil.

Mass (FAB+) m/Z: 573(M+H)$^+$.
$^1$H-NMR($CDCl_3$)δ: 1.01(9H,s), 1.47(9H,s), 1.48(9H,s), 1.53-1.70(4H,m), 2.98(1H,d,J=8.3Hz), 3.25-3.29(1H,m), 3.47(2H,d,J=5.9Hz), 3.71-3.77(1H,m), 4.05(1H,d,J=2.4Hz), 4.41(1H,dd,J=8.3,2.4Hz), 7.34-7.42(6H,m), 7.63(4H,dd,J=7.8,1.5Hz).

(3) Benzyl (2R,3R)-3-tert-butoxycarbonyl-2-[4-(tert-butyldiphenylsilyloxy)butoxy]-3-hydroxypropionate

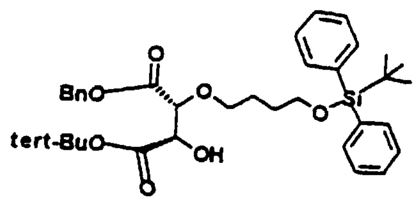

The di-tert-butyl ester compound (132.3 g) obtained in (2) was treated in the same manner as in Example 15-(2), whereby the title compound (78.3 g) was obtained as a colorless oil.

Mass (FAB+) m/Z: 607(M+H)$^+$.
$^1$H-NMR($CDCl_3$)δ: 1.03(9H,s), 1.48(9H,s), 1.52-1.72(4H,m), 3.03(1H,d,J=8.3Hz), 3.29-3.34(1H,m), 3.62-3.65(2H,m), 3.75(1H,dd,J=15.1,6.4Hz), 4.24(1H,d,J=2.0Hz), 4.48(1H,dd,J=8.3,2.4Hz), 5.21(1H,d,J=12.2Hz), 7.32-7.43(11H,m), 7.63-7.65(4H,m).

(4) tert-Butyl (2R,3R)-3-benzyloxycarbonyl-2-tert-butoxycarbonylmethoxy-3-[4-(tert-butyldiphenylsilyloxy)butoxy)propionate

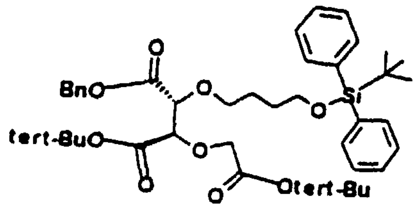

The diester compound (40.1 g) obtained in (3) was treated in the same manner as in Example 15-(3), whereby the title compound (42.4 g) was obtained as a colorless oil.

$^1$H-NMR($CDCl_3$)δ: 1.02(9H,s), 1.44(9H,s), 1.47(9H,s), 1.56-1.63(2H,m), 1.66-1.72(2H,m), 3.29-3.34(1H,m), 3.61-3.64(2H,m), 3.70-3.74(1H,m), 4.00(1H,d,J=16.6Hz), 4.27(1H,d,J=16.6Hz), 4.36(1H,d,J=2.9Hz), 4.47(1H,d,J=2.9Hz), 5.23(1H,d,J=12.2Hz), 5.28(1H,d,J=12.2Hz), 7.32-7.43(11H,m), 7.63-7.65(4H,m).

(5) tert-Butyl (2R,3R)-3-benzyloxycarbonyl-2-tert-butoxycarbonylmethoxy-3-(4-hydroxybutoxy)propionate

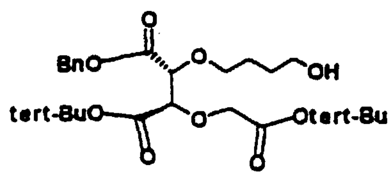

To a solution of the triester compound (30.1 g) obtained in (4) in tetrahydrofuran (200 ml) were added dropwise acetic acid (7.2 ml) and then tetrabutylammonium fluoride (1M solution in tetrahydrofuran, 83.6 ml) under ice cooling, and the mixture was stirred at room temperature for 36 hours. The residue obtained by distilling off the solvent under reduce pressure was purified by chromatography on a silica gel column (elution with 50% ethyl acetate-hexane), whereby the title compound (17.0 g) was obtained as a colorless oil.

$^1$H-NMR($CDCl_3$)δ: 1.40(9H,s), 1.42(9H,s), 1.61-1.70(4H,m), 3.41(1H,dt,J=8.8,5.9Hz), 3.61-3.69(2H,m), 3.73-3.78(1H,m), 4.00(1H,d,J=16.6Hz), 4.26(1H,d,J=16.6Hz), 4.37(1H,d,J=3.4Hz), 4.58(1H,d,J=3.4Hz), 5.24(1H,d,J=12.2Hz), 5.29(1H,d,J=12.2Hz), 7.33-7.39(3H,m), 7.42-7.45(2H,m).

(6) tert-Butyl (2R,3R)-3-benzyloxycarbonyl-2-tert-butoxycarbonylmethoxy-3-(4-oxobutoxy)propionate

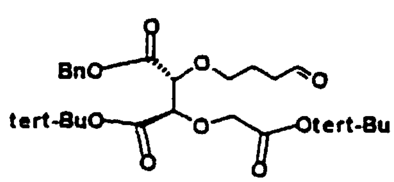

Oxalyl chloride (2.0 ml) was dissolved in methylene chloride (100 ml). To the resulting solution was slowly

added dropwise a solution of dimethylsulfoxide (2.15 ml) in methylene chloride (10 ml) to maintain the temperature at -65°C or lower. After stirring for 10 minutes, a solution of the alcohol compound obtained in (5) in methylene chloride (40 ml) was added dropwise to the reaction mixture, followed by stirring for 30 minutes. Triethylamine (12 ml) was then added dropwise, and the whole was brought to room temperature. The reaction mixture was washed successively with dilute hydrochloric acid and saturated saline and dried over anhydrous sodium sulfate. The solvent was distilled off under reduced pressure. The residue was purified by chromatography on a silica gel column (elution with 50% ethyl acetate-hexane), whereby the title compound (4.60 g) was obtained as a colorless oil.

$^1$H-NMR($CDCl_3$)δ: 1.44(9H,s), 1.48(9H,s), 1.81-1.95(2H,m), 2.59(2H,t,J=7.1Hz), 3.41-3.47(1H,m), 3.66-3.71(1H,m), 3.99(1H,d,J=16.6Hz), 4.25(1H,d,J=16.6Hz), 4.36(1H,d,J=2.9Hz), 4.45(1H,d,J=2.9Hz), 5.24(1H,d,J=12.0Hz), 5.27(1H,d,J=12.0Hz), 7.31-7.38(3H,m), 7.42-7.52(2H,m), 9.72(1H,s).

Example 19

In tetrahydrofuran (3 ml), (3,4-dimethylphenylmethyl)triphenylphosphonium chloride (0.55 g) prepared in Referential Example 10 was suspended, followed by the dropwise addition of potassium bistromethylsilylamide (0.5M solution in toluene, 2.6 ml) at room temperature. After stirring for 10 minutes, a solution of the aldehyde compound (0.30 g) of Example 18-(6) in tetrahydrofuran (3 ml) was added and the resulting mixture was stirred for 1 hour. The reaction mixture was diluted with diethyl ether, washed with water and then dried over anhydrous sodium sulfate. The solvent was distilled off under reduced pressure. The residue was purified by chromatography on a silica gel column (elution with 17% ethyl acetate-hexane), whereby tert-butyl (2R,3R)-3-benzyloxycarbonyl-2-tert-butoxycarbonylmethoxy-3-[5-(3,4-dimethylphenyl)pent-4-en-1-yloxy)propionate (0.30 g) was obtained as a colorless oil.

$^1$H-NMR($CDCl_3$)δ: 1.43,1.44,1.46,1.48(total 18H,s each), 1.71-1.79(2H,m), 2.17-2.25(7H,m), 2.30-2.38(1H,m), 3.32-3.40(1H,m), 3.71-3.79(1H,m), 4.00,4.01(total 1H,d each,J=16.6/16.6Hz), 4.25,4.27(total 1H,d each,J=16.6/16.6Hz), 4.37,4.38(total 1H,d each,J=3.4,2.9Hz), 4.46,4.47(total 1H,d each,J=2.9,3.4Hz), 5.22-5.30(2H,m), 5.50-5.57,6.60-6.13(total 1H,m each), 6.28-6.34(1H,m), 6.97-7.09(3H,m), 7.30-7.36(3H,m), 7.40-7.43(2H,m).

The resulting compound (0.30 g) was dissolved in ethanol (3 ml). To the resulting solution was added 10% palladium-carbon (0.03 g), and the mixture was stirred for 17 hours under hydrogen atmosphere. From the reaction mixture, the 10% palladium-carbon was filtered off and the solvent was distilled off under reduced pressure, whereby (2R,3R)-3-tert-butoxycarbonyl-3-tert-butoxycarbonylmethoxy-2-[5-(3,4-dimethylphenyl)pentyloxy)propionic acid was obtained as a colorless oil. The resulting compound was dissolved in methylene chloride (3 ml). To the resulting solution were added 5-(2-naphthyl)pentylamine hydrochloride (0.25 g), 1-hydroxybenzotriazole (0.020 g) and 1-ethyl-3-(3-dimethylaminopropyl)carbodiimide hydrochloride (0.250 g), and the mixture was stirred at room temperature for 16 hours. The reaction mixture was washed with dilute hydrochloric acid and saturated saline and dried over anhydrous sodium sulfate. The solvent was then distilled off under reduced pressure. The residue was purified by chromatography on a silica gel column (25% ethyl acetate-hexane), whereby the compound (0.15 g) of Example 19-a was obtained as a colorless oil.

In the same manner as in the above-described example except for the use of, instead of (3,4-dimethylphenylmethyl)triphenylphosphonium chloride, the corresponding phosphonium salts of Referential Example 10, reaction was effected, whereby compounds of Example 19-b to 19-o were obtained.

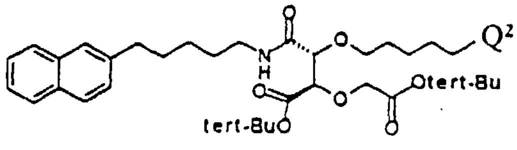

| Example | $Q^2$ | Mass(EI) m/Z: | $^1$H-NMR($CDCl_3$)δ : |
|---|---|---|---|
| 19-a | 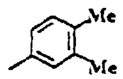 | 690($M^+$+1), 689($M^+$) | 1.25-1.60(28H,m),1.70-1.75(2H,m),2.20(3H,s),2.21(3H,s),2.50(2H,t,J=7.6Hz),2.76(2H,t,J=7.6Hz),3.38-3.45(4H,m),3.88(1H,d,J=16.1Hz),4.16(1H,d,J=2.0Hz),4.23(1H,d,J=16.1Hz),4.30(1H,d,J=2.0Hz),6.68-6.74(1H,m),6.87(1H,d,J=7.3Hz),6.91(1H,s),7.01(1H,d,J=7.3Hz),7.26-7.32(1H,m),7.37-7.45(2H,m),7.58(1H,s),7.73-7.79(3H,m). |
| 19-b | 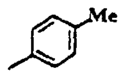 | 675($M^+$) | 1.25-1.60(28H,m),1.70-1.85(2H,m),2.30(3H,s),2.52(2H,t,J=7.6Hz),2.76(2H,t,J=7.6Hz),3.27-3.48(4H,m),3.87(1H,d,J=15.7Hz),4.15(1H,d,J=2.0Hz),4.24(1H,d,J=15.7Hz),4.31(1H,d,J=2.0Hz),6.68-6.71(1H,m),7.01-7.07(4H,m),7.29(1H,d,J=2.0Hz),7.37-7.45(2H,m),7.58(1H,s),7.73-7.79(3H,m). |
| 19-c | 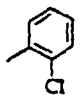 | 695($M^+$) | 1.30-1.60(28H,m),1.65-1.76(2H,m),2.68(2H,t,J=7.8Hz),2.76(2H,t,J=7.6Hz),3.27-3.32(2H,m),3.33-3.48(2H,m),3.88(1H,d,J=16.1Hz),4.16(1H,d,J=2.4Hz),4.24(1H,d,J=16.1Hz),4.31(1H,d,J=2.4Hz),6.71(1H,t,J=5.6Hz),7.08-7.17(3H,m),7.29-7.31(2H,m),7.37-7.45(2H,m),7.58(1H,s), 7.73-7.79(3H,m). |
| 19-d | 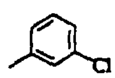 | 695($M^+$) | 1.23-1.60(28H,m),1.65-1.74(2H,m), 2.51-2.58(2H,m),2.76(2H,t,J=7.6Hz),3.27-3.32(2H,m),3.33-3.50(2H,m),3.88(1H,d,J=16.1Hz),4.15(1H,d,J=2.4Hz),4.24(1H,d,J=16.1Hz),4.30(1H,d,J=2.4Hz),6.70(1H,broad),7.00(1H,d,J=7.3Hz),7.12-7.19(3H,m),7.29-7.31(2H,m),7.35-7.45(2H,m),7.58 (1H,s), 7.73-7.79(3H,m). |
| 19-e | 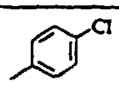 | 695($M^+$) | 1.30-1.70(28H,m),1.70-1.80(2H,m),2.52(2H,t,J=7.6Hz),2.76(2H,t,J=7.6Hz),3.27-3.48(4H,m),3.87(1H,d,J=16.1Hz),4.15(1H,d,J=2.0Hz),4.24(1H,d,J=16.1Hz),4.30(1H,d,J=2.0Hz),6.68-6.70(1H,m),7.05(2H,d,J=8.3Hz),7.21(2H,d,J=8.3Hz),7.30(1H,d,J=8.3Hz),7.38-7.45(2H,m),7.58(1H,s),7.73-7.79(3H,m). |
| 19-f | 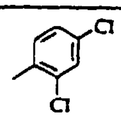 | 729($M^+$) | 1.25-1.65(28H,m),1.70-1.80(2H,m),2.64(2H,t,J=7.8Hz),2.76(2H,t,J=7.3Hz),3.38-3.45(4H,m),3.87(1H,d,J=16.1Hz),4.15(1H,d,J=2.4Hz),4.24(1H,d,J=16.1Hz),4.31(1H,d,J=2.4Hz),6.68-6.71(1H,m),7.13(1H,d,J=2.4Hz),7.15(1H,d,J=2.4Hz |

| | | | |
|---|---|---|---|
| | | | ),7.29-7.33(2H,m),7.39-7.45(2H,m),7.58(1H,s),7.73-7.79(3H,m). |
| 19-g | 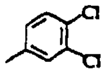 | 729(M⁺) | 1.23-1.31(2H,m),1.37-1.60(26H,m),1.68-1.76(2H,m),2.51(2H,t,J=7.8Hz),2.76(2H,t,J=7.8Hz),3.27-3.32(2H,m),3.35-3.47(2H,m),3.87(1H,d,J=16.1Hz),4.15(1H,d,J=2.4Hz),4.24(1H,d,J=16.1Hz),4.30(1H,d,J=2.4Hz),6.69(1H,broad),6.96(1H,dd,J=8.3,2.0Hz),7.22(1H,d,J=2.0Hz),7.29-7.31(2H,m),7.38-7.45(2H,m),7.58(1H,s), 7.73-7.79(3H,m). |
| 19-h | 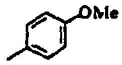 | 691(M⁺) | 1.23-1.60(28H,m),1.68-1.76(2H,m),2.50(2H,t,J=7.8Hz),2.76(2H,t,J=7.6Hz),3.27-3.32(2H,m),3.35-3.48(2H,m),3.76(3H,s),3.88(1H,d,J=15.6Hz),4.16(1H,d,J=2.0Hz),4.24(1H,d,J=15.6Hz),4.30(1H,d,J=2.0Hz),6.70(1H,t,J=5.9Hz),6.80(2H,d,J=8.6Hz),7.04(2H,d,J=8.6Hz),7.30(1H,dd,J=8.3,1.5Hz),7.37-7.45(2H,m),7.58(1H,s), 7.73-7.79(3H,m). |
| 19-i | 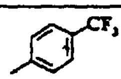 | 729(M⁺) | 1.24-1.60(28H,m),1.68-1.75(2H,m),2.61(2H,t,J=7.8Hz),2.76(2H,t,J=7.6Hz),3.24-3.30(2H,m),3.32-3.50(2H,m),3.87(1H,d,J=16.1Hz),4.16(1H,d,J=2.4Hz),4.24(1H,d,J=16.1Hz),4.30(1H,d,J=2.4Hz),6.70(1H,t,J=5.4Hz),7.23(2H,d,J=8.1),7.30(1H,dd,J=8.3,1.5Hz),7.34-7.45(2H,m),7.50(2H,d,J=8.1Hz),7.58 (1H,s),7.73-7.79(3H,m). |
| 19-j | 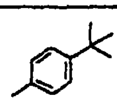 | 717(M⁺) | 1.24-1.60(37H,m),1.68-1.76(2H,m),2.53(2H,t,J=7.8Hz),2.76(2H,t,J=7.6Hz),3.27-3.50(4H,m),3.88(1H,d,J=16.1Hz),4.16(1H,d,J=2.4Hz),4.24(1H,d,J=16.1Hz),4.30(1H,d,J=2.4Hz),6.71(1H,t,J=5.9Hz),7.07(2H,d,J=8.3Hz),7.27-7.29(3H,m),7.37-7.45(2H,m),7.58(1H,s),7.73-7.79(3H,m). |
| 19-k | 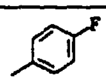 | 679(M⁺) | 1.25-1.60(28H,m),1.70-1.85(2H,m),2.53(2H,t,J=7.8Hz),2.76(2H,t,J=7.8Hz),3.27-3.48(4H,m),3.87(1H,d,J=15.6Hz),4.15(1H,d,J=2.0Hz),4.24(1H,d,J=15.6Hz),4.30(1H,d,J=2.0Hz),6.68-6.69(1H,m),6.91-7.09(4H,m),7.29-7.58(4H,m),7.73-7.79(3H,m). |
| 19-l | 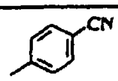 | 686(M⁺) | 1.30-1.65(28H,m),1.75-1.80(2H,m),2.61(2H,t,J=7.8Hz),2.76(2H,t,J=7.8Hz),3.27-3.52(4H,m),3.86(1H,d,J=16.1Hz),4.16(1H,d,J=2.0Hz),4.25(1H,d,J=15.6Hz),4.30(1H,d,J=2.0Hz),6.68-6.70(1H,m),7.21-7.31(3H,m),7.40-7.50(2H,m),7.52-7.58(3H,m),7.73-7.79(3H,m). |
| 19-m | 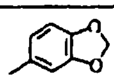 | (FAB+) 706(M+H)⁺ | 1.25-1.80(30H,m),2.47(2H.,t,J=7.6Hz),2.77(2H,t,J=7.6Hz),3.28-3.43(4H,m),3.88 |

| | | | |
|---|---|---|---|
| | | 728(M+Na)' | (1H,d,J=16.1Hz),4.16(1H,d,J=2.4Hz),4.25(1H,d,J=16.1Hz),4.32(1H,d,J=2.4Hz),5.89(2H,s),6.57(1H,d,J=7.8Hz),6.63(1H,s,),6.66-6.72(2H,m),7.27-7.82(7H,m). |
| 19-n | 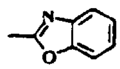 | (FAB+) 703(M+H)' | 1.35-1.75(28H,m),1.80-1.90(2H,m),2.76(12H,t,J=7.6Hz),2.88(2H,t,J=7.6Hz),3.25-3.51(4H,m),3.87(1H,d,J=15.6Hz),4.16(1H,d,J=2.0Hz),4.25(1H,d,J=16.1Hz),4.32(1H,d,J=2.0Hz),6.70(1H,t,J=5.8Hz),7.26-7.80(11H,m). |
| 19-o | 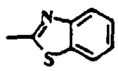 | (FAB+) 719(M+H)' | 1.30-1.90(30H,m),2.74(2H,t,J=7.6Hz),3.06(1H,t,J=7.6Hz),3.26-3.50(4H,m),3.87(1H,d,J=16.1Hz),4.16(1H,d,J=2.4Hz),4.24(1H,d,J=16.1Hz),4.31(1H,d,J=2.4Hz),6.70(1H,broad),7.27-7.97(11H,m). |

Example 20

The di-tert-butyl ester compound (0.15 g) obtained in Example 19-a was dissolved in methylene chloride (2 ml). To the resulting solution was added trifluoroacetic acid (1 ml), and the mixture was stirred at room temperature for 3 hours. The reaction mixture was concentrated to dryness under reduced pressure and the residue was dissolved in ethanol (2 ml). To the resulting solution was added 2N sodium hydroxide (0.22 ml). After concentration under reduced pressure, the solid was collected by filtration, whereby the compound (0.08 g) of Example 20-a was obtained as a colorless solid.

The compounds of Examples 19-b to 19-o were subjected to the reaction similar to the above, whereby the compounds of Examples 20-b to 20-o were obtained, respectively.

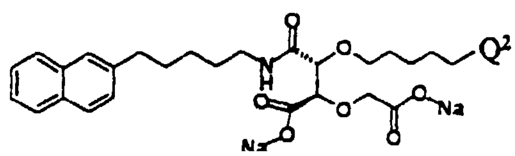

| Example | $Q^2$ | Mass (FAB+) m/Z: | Elementary analysis Calculated: Found: C H N %(S) |
|---|---|---|---|
| 20-a | 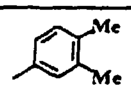 | 644(M+Na)' 622(M+H)' | for C34H41NNa2O7 · 6H2O<br>55.96 7.32 1.92<br>55.50 7.28 1.77 |
| 20-b | 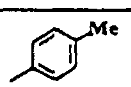 | 630(M+Na)' | for C33H39NNa2O7 · 4H2O<br>58.39 6.56 1.79<br>58.31 6.96 2.06 |
| 20-c | 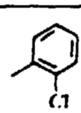 | 650(M+Na)' | for C32H36ClNNa2O7 · 1.5H2O<br>58.67 6.00 2.13<br>58.47 5.96 2.03 |

| | | | |
|---|---|---|---|
| 20-d | 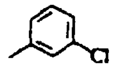 | $650(M+Na)^+$ | for $C_{32}H_{36}ClNNa_2O_7 \cdot 4H_2O$<br>55.21 6.37 2.01<br>54.81 6.27 1.94 |
| 20-e | 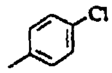 | $650(M+Na)^+$ | for $C_{32}H_{36}ClNNa_2O_7 \cdot 2.5H_2O$<br>57.10 6.14 2.08<br>57.14 5.82 1.88 |
| 20-f | 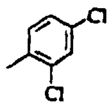 | (EI)<br>$618(M^+)$ | Oil |
| 20-g | 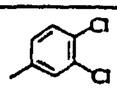 | $684(M^+)$ | for $C_{32}H_{35}Cl_2NNa_2O_7 \cdot 3H_2O$<br>53.63 5.76 1.96<br>54.07 5.37 1.91 |
| 20-h | 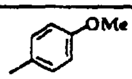 | $646(M+Na)^+$<br>$624(M+H)^+$ | for $C_{33}H_{39}NNa_2O_8 \cdot 4H_2O$<br>56.97 6.80 2.01<br>57.20 6.40 1.93 |
| 20-i | 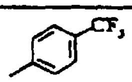 | $684(M+Na)^+$<br>$662(M+H)^+$ | for $C_{33}H_{36}F_3NNa_2O_7 \cdot 5H_2O$<br>52.73 6.16 1.86<br>53.24 5.66 1.86 |
| 20-j | 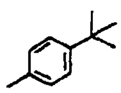 | $672(M+Na)^+$<br>$650(M+H)^+$ | for $C_{36}H_{45}NNa_2O_7 \cdot 6H_2O$<br>57.05 7.58 1.84<br>57.34 7.47 1.82 |
| 20-k | 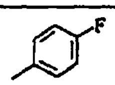 | $634(M+Na)^+$<br>$612(M+H)^+$ | for $C_{32}H_{36}FNNa_2O_7 \cdot 2.5H_2O$<br>57.48 6.18 2.09<br>57.46 5.94 1.50 |
| 20-l | 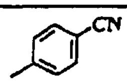 | $641(M+Na)^+$<br>$619(M+H)^+$ | for $C_{33}H_{36}N_2Na_2O_7 \cdot 5.5H_2O$<br>55.07 6.58 3.89<br>55.45 6.19 3.43 |
| 20-m | 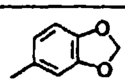 | $594(M+H)^+$<br>(free acid) | 1H-NMR (free acid, $CDCl_3$) δ:1.22-1.60(10H,m), 1.67-1.76(2H,m),2.48(2H,t,J=7.6Hz),2.75 (2H,t,J=7.3Hz),3.23-3.35(2H,m),3.40-3.55 (2H,m),4.15(1H,d,J=17.5Hz),4.28-4.38(2H,m), 4.59(1H.broad s),5.87(2H,s),6.58(1H,dd, J=7.8,1.5Hz),6.63(1H,d,J=1.5Hz),6.69(1H,d,J =7.8Hz),7.00(1H,broad s),7.27-7.80(7H,m). |
| 20-n | 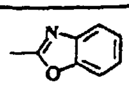 | $635(M+H)^+$ | for $C_{33}H_{36}N_2Na_2O_8 \cdot 1.75H_2O$<br>59.50 5.98 4.21<br>59.73 5.90 3.93 |
| 20-o | 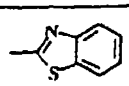 | $651(M+H)^+$ | for $C_{33}H_{36}N_2Na_2O_7S$<br>60.72 5.87 4.29 (4.91)<br>60.72 6.02 3.96 (4.68) |

Example 21

tert-Butyl (2R,3R)-2-tert-butoxycarbonylmethoxy-3-[N-[5-(2-naphthyl)pentyl]carbamoyl]-3-(4-oxobutoxy)propionate

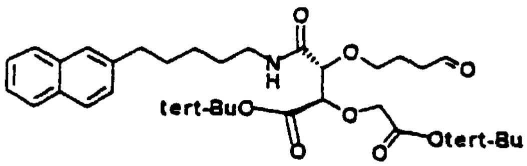

(1) tert-Butyl (2R,3R)-2-tert-butoxycarbonylmethoxy-3-(4-hydroxybutoxy)-3-[N-[5-(2-naphthyl)pentyl]carbamoyl]propionate

The alcohol compound (0.50 g) of Example 18-5 was dissolved in tetrahydrofuran (10 ml). To the resulting solution was added 10% palladium-carbon (0.05 g), and the mixture was stirred at room temperature for 2 hours under hydrogen atmosphere. The 10% palladium-carbon was filtered off and the filtrate was concentrated to dryness under reduced pressure, whereby tert-butyl (2R,3R)-2-tert-butoxycarbonylmethoxy-3-carboxy-3-(4-hydroxybutoxy))propionate was obtained as a colorless oil.

$^1$H-NMR($CDCl_3$)δ: 1.46(9H,s), 1.50(9H,s), 1.69-1.80(2H,m), 1.82-1.89(2H,m), 3.55-3.59(1H,m), 3.63-3.77(3H,m), 3.97(1H,d,J=15.6Hz), 4.30(1H,d,J=15.6Hz), 4.32(1H,d,J=2.5Hz), 4.36(1H,d,J=2.5Hz).

The resulting compound was dissolved in methylene chloride (10 ml). To the resulting solution were added 5-(2-naphthyl)pentylamine hydrochloride (0.26 g), 1-hydroxybenzotriazole (0.05g) and 1-ethyl-3-(3-dimethylaminopropyl)carbodiimide hydrochloride (0.24 g), and the mixture was stirred at room temperature for 16 hours. The reaction mixture was washed with dilute hydrochloric acid and saturated saline and dried over anhydrous sodium sulfate. The solvent was then distilled off under reduced pressure. The residue was purified by chromatography on a silica gel column (3% methanol-methylene chloride), whereby the title compound (0.515 g) was obtained as a colorless oil.

$^1$H-NMR($CDCl_3$)δ: 1.42(9H,s), 1.50(9H,s), 1.54-1.63(8H,m), 1.73(2H,dt,J=7.8,7.3Hz), 2.77(2H,t,J=7.8Hz), 3.31(2H,dt,J=6.4,5.9Hz), 3.36-3.45(1H,m), 3.48-3.53(1H,m), 3.60(2H,q,J=5.4Hz), 3.87(1H,d,J=16.1Hz), 4.17(1H,d,J=2.0Hz), 4.23(1H,d,J=16.1Hz), 4.30(1H,d,J=2.0Hz), 6.84(1H,t,J=5.9Hz), 7.31(1H,dd,J=8.3,1.6Hz), 7.40(1H,dt,J=7.8,1.5Hz), 7.44(1H,dt,J=7.8,1.5Hz), 7.60(1H,s), 7.76(1H,d,J=8.3Hz), 7.78(1H,d,J=7.8Hz), 7.79(1H,d,J=7.8Hz).

(2) tert-Butyl (2R,3R)-2-tert-butoxycarbonylmethoxy-3-[N-[5-(2-naphthyl)pentyl]carbamoyl]-3-(4-oxobutoxy)propionate

The alcohol compound (0.587 g) obtained in (1) was treated in the same manner as in Example 18-(6), whereby the title compound (0.444 g) was obtained as a pale yellow oil.

$^1$H-NMR($CDCl_3$)δ: 1.37-1.45(12H,m), 1.49(9H,s), 1.53-1.63(2H,m), 1.70-1.85(4H,m), 2.42-2.47(2H,m), 2.77(2H,t,J=7.3Hz), 3.25-3.40(2H,m), 3.45-3.51(2H,m), 3.87(1H,d,J=16.1Hz), 4.15(1H,d,J=2.4Hz), 4.21(1H,d,J=16.1Hz), 4.29(1H,d,J=2.4Hz), 6.70(1H,broad t,J=5.5Hz), 7.31(1H,dd,J=8.3,1.5Hz), 7.38-7.46(2H,m), 7.59(1H,s), 7.74-7.80(3H,m).

Example 22

(2R,3R)-2-Carboxymethoxy-3-[5-(3-chloro-4-methylphenyl)pentyloxy]-3-[N-[5-(2-naphthyl)pentyl]carbamoyl]propionic acid

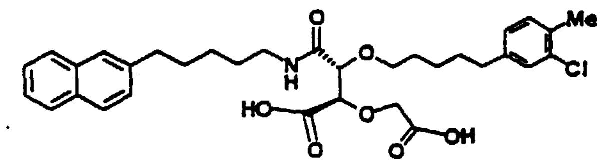

The aldehyde (0.20 g) obtained in Example 21 was treated in the same manner as in Example 19-a except for the use of (3-chloro-4-methylphenylmethyl)triphenylphosphonium chloride instead of (3,4-dimethylphenylmethyl)triphenylphosphonium chloride, whereby tert-butyl (2R,3R)-2-tert-butoxycarbonylmethoxy-3-[5-(3-chloro-4-methylphenyl)pentyloxy]-3-[N-[5-(2-naphthyl)pentyl]carbamoyl]propionate (0.15 g) was obtained as a colorless oil.

Mass (EI) m/Z: 709($M^+$).
$^1$H-NMR($CDCl_3$)δ: 1.25-1.90(30H,m), 2.32(3H,s), 2.49(2H,t,J=7.3Hz), 2.76(2H,t,J=7.3Hz), 3.30-3.65(4H,m), 3.86(1H,d,J=2.0Hz), 4.10-4.30(1H,m), 4.35-4.45(2H,m), 6.65-6.75(1H,m), 6.85-7.20(3H,m), 7.26-7.50(3H,m), 7.58(1H,s), 7.77-7.79(3H,m).

The resulting compound was treated in the same manner as in Example 20, whereby the disodium salt (0.095 g) of the title compound was obtained as a colorless solid.

Mass (FAB+) m/Z: 664$(M+Na)^+$, 642$(M+H)^+$.
$^1$H-NMR($CDCl_3$)δ: 1.20-1.85(12H,m), 2.31(3H,s), 2.48(2H,t,J=7.3Hz), 2.75(2H,t,J=7.3Hz), 3.20-3.60(4H,m), 4.15(1H,d,J=17.6Hz), 4.25(1H,d,J=17.6Hz), 4.34(1H,s), 4.58(1H,s), 5.80-6.10(3H,broad s), 6.80-7.25(3H,m), 7.29(1H,d,J=1.4Hz), 7.35-7.50(2H,m), 7.56(1H,s), 7.72-7.94(3H,m).

| Elementary analysis for $C_{33}H_{38}ClNNa_2O_7 \cdot 2.5H_2O$ | | | |
|---|---|---|---|
| Calculated: | C, 57.68; | H, 6.31; | N, 2.04. |
| Found: | C, 57.82; | H, 5.91; | N, 1.97. |

Example 23

The alcohol compound (0.25 g) obtained in Example 18-(5), N-(3,4-dimethylphenyl)-2,4-dinitrobenzene sulfonamide (0.18 g) of Referential Example 11-a and triphenylphosphine (0.16 g) were dissolved in tetrahydrofuran (10 ml), followed by the dropwise addition of diethyl azodicarboxylate (40% solution in toluene, 0.43 ml) under cooling with ice water. After stirring for 5 hours, the solvent was distilled off under reduced pressure. The residue was purified by chromatography on a silica gel column (25% ethyl acetate-hexane), whereby tert-butyl (2R,3R)-3-benzyloxycarbonyl-2-tert-butoxycarbonylmethoxy-3-[4-(N- (3,4-dimethylphenyl)-N-(2,4-dinitrophenylsulfonyl)amino]butoxy]propionate (0.34 g) was obtained as a colorless oil.

$^1$H-NMR($CDCl_3$)δ: 1.44(9H,s), 1.47(9H,s), 1.49-1.65(4H,m), 2.21(3H,s), 2.24(3H,s), 3.36(1H,dd,J=8.8,5.9Hz), 3.66-3.81(3H,m), 3.96(1H,d,J=16.6Hz), 4.24(1H,d,J=16.6Hz), 4.35(1H,d,J=2.9Hz), 4.45(1H,d,J=2.9Hz), 5.23(1H,d,J=12.2Hz), 5.27(1H,d,J=12.2Hz), 6.82(1H,d,J=8.3Hz), 6.96(1H,s), 7.06(1H,d,J=8.3Hz), 7.29-7.34(3H,m), 7.40-7.43(2H,m), 7.72(1H,d,J=8.8Hz), 8.27(1H,d,J=8.8Hz), 8.40(1H,s).

The resulting compound (0.34 g) was dissolved in methylene chloride (5 ml). To the resulting solution were

added thioglycolic acid (0.05 ml) and triethylamine (0.2 ml), and the mixture was stirred at room temperature for 0.5 hour. The reaction mixture was washed with a 5% aqueous solution of sodium bicarbonate and dilute hydrochloric acid and then dried over anhydrous sodium sulfate. The solvent was distilled off under reduced pressure. The residue was purified by chromatography on a silica gel column (20% ethyl acetate-hexane), whereby tert-butyl (2R,3R)-3-benzyloxycarbonyl-2-tert-butoxycarbonylmethoxxy-3-[4-(3,4-dimethylphenylamino)butoxy]propionate (0.25 g) was obtained as a colorless oil.

$^1$H-NMR($CDCl_3$)δ: 1.44(9H,s), 1.47(9H,s), 1.63-1.69(4H,m), 2.13(3H,s), 2.18(3H,s), 3.06(2H,t,J=6.4Hz), 3.36-3.42(1H,m), 3.72-3.77(1H,m), 4.00(1H,d,J=16.6Hz), 4.27(1H,d,J=16.6Hz), 4.38(1H,d,J=2.9Hz), 4.48(1H,d,J=2.9Hz), 5.25(1H,d,J=12.2Hz), 5.29(1H,d,J=12.2Hz), 6.35(1H,dd,J=8.3,2.0Hz), 6.41(1H,d,J=2.0Hz), 6.90(1H,d,J=8.3Hz), 7.31-7.38(3H,m), 7.41-7.44(2H,m).

The resulting compound (0.25 g) was dissolved in ethanol (3 ml). To the resulting solution was added 10% palladium-carbon (0.03 g), and the mixture was stirred for 17 hours under hydrogen atmosphere. The 10% palladium-carbon was filtered off and the solvent was distilled off under reduced pressure, whereby tert-butyl (2R,3R)-2-tert-butoxycarbonylmethoxy-3-carboxy-3-[4-(3,4-dimethylphenylamino)butoxy]propionate was obtained as a colorless oil.

$^1$H-NMR($CDCl_3$)δ: 1.43(9H,s), 1.50(9H,s), 1.71-1.88(4H,m), 2.14(3H,s), 2.17(3H,s), 3.23-3.35(2H,m), 3.52-3.59(1H,m), 3.77-3.85(1H,m), 3.94(1H,d,J=16.1Hz), 4.21(1H,d,J=16.1Hz), 4.25(1H,d,J=2.0Hz), 4.53(1H,d,J=2.0Hz), 6.97(1H,d,J=7.8Hz), 7.05(1H,s), 7.06(1H,d,J=7.8Hz).

The resulting compound was dissolved in methylene chloride (3 ml). To the resulting solution were added 5-(2-naphthyl)pentylamine hydrochloride (0.12 g), 1-hydroxybenzotriazole (0.02 g) and 1-ethyl-3-(3-dimethylaminopropyl)carbodiimide hydrochloride (0.11 g), and the mixture was stirred at room temperature for 16 hours. The reaction mixture was washed with dilute hydrochloric acid and saturated saline and then dried over anhydrous sodium sulfate. The solvent was distilled off under reduced pressure. The residue was purified by chromatography on a silica gel column (25% ethyl acetate-hexane), whereby the compound of Example 23-a (0.136 g) was obtained as a colorless oil.

In the same manner as in the above-described example except for the use of, instead of the N-(3,4-dimethylphenyl)-2,4-dinitrobenzenesulfonamide, the corresponding sulfonamide derivatives of Referential Example 11, the reaction was effected, whereby compounds of Examples 23-b to 23-i were obtained.

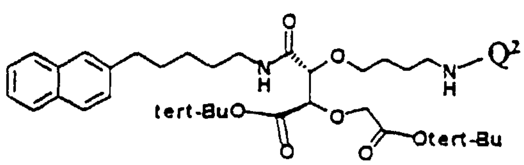

| Example | $Q^2$ | $^1$H-NMR($CDCl_3$)δ :<br>Mass |
|---|---|---|
| 23-a | 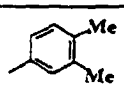 | 1.33-1.43(2H, m), 1.42(9H, s), 1.49(9H, s), 1.56-1.64(6H, m), 1.66-1.75(2H, m), 2.13(3H, s), 2.17(3H, s), 2.75(2H, dd, J=7.8,7.3Hz), 3.03(2H, t, J=6.4Hz), 3.26-3.32(2H, m), 3.38-3.42(1H, m), 3.46-3.51(1H, m), 3.88(1H, d, J=16.1Hz), 4.17(1H, d, J=2.0Hz), 4.25(1H, d, J=16.1Hz), 4.31(1H, d, J=2.0Hz), 6.35(1H, dd, J=8.3,2.4Hz), 6.40(1H, s), 6.74(1H, t, J=5.9Hz), 6.90(1H, d, J=8.3Hz), 7.30(1H, dd, J=8.8,2.0 Hz), 7.39(1H, dt, J=6.4,1.5Hz), 7.43(1H, dt, J=6.4,1.5Hz), 7.58(1H, s), 7.75(1H, d, J=7.3Hz), 7.76(1H, d, J=7.3Hz), 7.78(1H, d, J=8.8Hz). |
| 23-b | 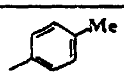 | 1.35-1.44(2H, m), 1.42(9H, s), 1.48(9H, s), 1.53-1.75(8H, m), 2.21(3H, s), 2.75(2H, t, J=7.3Hz), 3.03(2H, t, J=6.8Hz), 3.24-3.32(2H, m), 3.37-3.43(1H, m), 3.46-3.52(1H, m), 3.88(1H, d, J=16.1Hz), 4.17(1H, d, J=2.4Hz), 4.25(1H, d, J=16.1Hz), 4.31(1H, d, J=2.4Hz), 6.50(2H, d, J=8.3Hz), 6.74(1H, t, J=5.9Hz), 6.95(2H, d, J=8.3Hz), 7.30(1H, dd, J=8.3,1.5Hz), 7.39(1H, dt, J=6.8,1.5Hz), 7.43(1H, dt, J=6.8,1.5Hz), 7.58(1H, s), 7.74(1H, d, J=8.3Hz), 7.76(1H, d, J=6.8Hz), 7.78(1H, d, J=8.8Hz).<br>Mass(EI)m/z:676(M⁺). |
| 23-c | 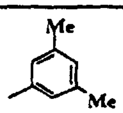 | 1.26-1.73(28H, m), 2.21(6H, s), 2.75(2H, t, J=7.3Hz), 3.04(2H, t, J=6.8Hz), 3.25-3.34(2H, m), 3.36-3.51(3H, m), 3.83-3.92(1H, m), 4.14-4.35(3H, m), 6.22(2H, d, J=8.3Hz), 6.35(1H, d, J=8.8Hz), 6.72(1H, s), 7.25-7.32(1H, m), 7.26-7.45(2H, m), 7.59(1H, d, J=8.8Hz), 7.70-7.80(3H, m).<br>Mass(EI) m/z:690(M⁺). |

| | | |
|---|---|---|
| 23-d | 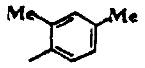 | 1.33-1.44(2H, m), 1.42(9H, s), 1.49(9H, s), 1.53-1.75(8H, m), 2.08(3H, s), 2.21(3H, s), 2.76(2H, t, J=7.3Hz), 3.08(2H, t, J=5.9Hz), 3.30(2H, q, J=6.8Hz), 3.39-3.44(1H, m), 3.46-3.53(1H, m), 3.88(1H, d, J=16.1Hz), 4.18(1H, d, J=2.4Hz), 4.25(1H, d, J=16.1Hz), 4.32(1H, d, J=2.4Hz), 6.48(1H, d, J=7.8Hz), 6.71(1H, t, J=5.9Hz), 6.86(1H, s), 6.90(1H, d, J=8.3Hz), 7.30(1H, dd, J=8.3,1.5Hz), 7.41(2H, ddd, J=7.8,6.8,1.5 Hz), 7.58(1H, s), 7.75(2H, t, J=6.8Hz), 7.78(1H, d, J=8.8Hz). |
| 23-e | 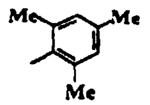 | 1.23-1.26(2H, m), 1.36-1.51(4H, m), 1.42(9H, s), 1.49(9H, s), 1.54-1.71(4H, m), 2.20(3H, s), 2.21(6H, s), 2.75(2H, dd, J=7.8,7.3Hz), 2.86(2H, bs), 3.27-3.34(1H, m), 3.37-3.53(1H, m,), 3.87(1H, d, J=16.1Hz), 4.07-4.14(1H, m), 4.17(1H, s), 4.24(1H, d, J=16.1Hz), 4.32(1H, s), 6.75(1H, bs), 6.78(2H, s), 7.29(1H, d, J=8.3Hz), 7.39(2H, t, J=7.3Hz), 7.57(1H, s), 7.74(2H, d, J=6.8Hz), 7.76(1H, d, J=8.8Hz). |
| 23-f | 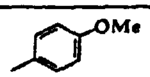 | 1.34-1.44(2H, m), 1.42(9H, s), 1.49(9H, s), 1.53-1.76(8H, m), 2.75(2H, dd, J=7.8,7.3Hz), 3.02(2H, t, J=6.4Hz), 3.24-3.34(2H, m), 3.38-3.45(1H, m), 3.47-3.54(1H, m), 3.72(3H, s), 3.88(1H, d, J=15.6Hz), 4.17(1H, d, J=2.4Hz), 4.25(1H, d, J=15.6Hz), 4.31(1H, d, J=2.4Hz), 6.55(2H, d, J=8.8Hz), 6.75(2H, d, J=8.8Hz), 7.28(1H, dd, J=8.3,1.5Hz), 7.39(1H, dt, J=7.3,1.5Hz), 7.43(1H, dt, J=7.3,1.5Hz), 7.58(1H, s), 7.74(1H, d, J=7.8Hz), 7.76(1H, d, J=6.8Hz), 7.78(1H, d, J=8.8Hz). |
| | | Mass(EI)m/z:692($M^+$). |
| 23-g | 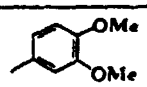 | 1.35-1.42(2H, m), 1.42(9H, s), 1.49(9H, s), 1.53-1.64(6H, m), 1.67-1.75(2H, m), 2.75(2H, t, J=7.3Hz), 3.02(2H, t, J=6.4Hz), 3.26-3.35(2H, m), 3.38-3.45(1H, m), 3.46-3.53(1H, m), 3.77(3H, s), 3.81(3H, s), 3.88(1H, d, J=16.1Hz), 4.18(1H, d, J=2.4Hz), 4.25(1H, d, J=16.1Hz), 4.31(1H, d, J=2.4Hz), 6.10(1H, dd, J=8.8,2.0Hz), 6.22(1H, d, J=2.0Hz), 6.71(1H, d, J=8.8Hz), 6.75(1H, t, J=5.9Hz), 7.29(1H, d, J=8.3Hz), 7.39(1H, t, J=6.8Hz), 7.43(1H, t, J=6.8Hz), 7.58(1H, s), 7.74(1H, d, J=7.8Hz), 7.75(1H, d,J=6.8Hz), 7.77(1H, d, J=8.3Hz). |
| | | Mass(EI)m/z:722($M^+$). |

| | | |
|---|---|---|
| 23-h | 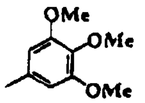 | 1.36-1.46(2H, m), 1.41(9H, s), 1.48(9H, s), 1.58-1.75(8H, m), 2.76(2H, t, J=7.3Hz), 3.18(1H, t, J=5.9Hz), 3.25-3.33(3H, m), 33.36-3.44(1H, m), 3.47-3.53(1H, m), 3.76(3H, s), 3.82(6H, s), 3.87(1H, d, J=16.1Hz), 4.17(1H, d, J=2.4Hz), 4.24(1H, d, J=16.1Hz), 4.31(1H, d, J=2.4Hz), 5.87(2H, s), 70(1H, t, J=5.9Hz), 7.30(1H, dd, J=8.3,1.5Hz), 7.39(1H, dt, J=7.8,1.5Hz), 7.44(1H, dt, J=7.8,1.5Hz), 7.58(1H, s), 7.74(1H, d, J=8.3Hz), 7.76(1H, d, J=6.8Hz), 7.78(1H, d, J=8.8Hz). |
| | | Mass(EI)m/z:752(M$^+$). |
| 23-i | 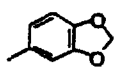 | 1.-1.44(2H, m), 1.42(9H, s), 1.49(9H, s), 1.52-1.63(6H, m), 1.67-1.76(2H, m), 2.76(2H, t, J=7.3Hz), 2.99(2H, t, J=6.4Hz), 3.24-3.34(2H, m), 3.37-3.44(1H, m), 3.45-3.52(1H, m), 3.88(1H, d, J=16.1Hz), 4.17(1H, d, J=2.4Hz), 4.29(1H, d, J=16.1Hz), 4.31(1H, d, J=2.4Hz), 5.82(2H, s), 6.00(1H, dd, J=8.3,2.4Hz), 6.22(1H, d, J=2.4Hz), 6.63(1H, d, J=8.3Hz), 6.74(1H, t, J=5.9Hz), 7.30(1H, dd, J=8.3,1.5 Hz), 7.40(1H, dt, J=7.8,1.5Hz), 7.44(1H, dt, J=7.8,1.5Hz), 7.58(1H, s), 7.75(1H, d, J=7.8Hz), 7.76(1H, d, J=6.8Hz), 7.78(1H, d, J=8.8Hz). |
| | | Mass(EI)m/z:706(M$^+$). |

Example 24

The compound (0.136 g) of Example 23-a was dissolved in methylene chloride (3 ml). To the resulting solution was added trifluoroacetic acid (2 ml), and the mixture was stirred at room temperature for 3 hours. The reaction mixture was concentrated to dryness under reduced pressure. The residue was purified by high-performance liquid column chromatography ("Sensyu Pak ODS-5251-SH", elution with acetonitrile-0.1% trifluoroacetic acid 65:35(V/V)), whereby the compound of Example 24-a (0.076 g) was obtained as a pale yellow solid.

The compounds of Examples 23-b to 23-i were reacted in the same manner as in the above-described example, whereby compounds of Examples 24-b to 24-i were obtained, respectively.

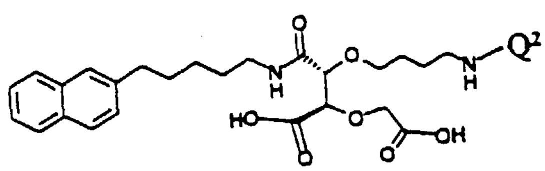

| Example | $Q^2$ | $^1$H-NMR($CDCl_3$)δ :<br>Elementary analysis Calculated:<br>Found:<br>C H N F |
|---|---|---|
| 24-a | 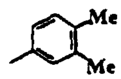 | 1.28-1.37(2H,m),1.46-1.58(4H,m),1.66-1.86(4H,m),2.20 (3H,s),2.21(3H,s)2.73(2H,t,J=7.3Hz),3.19-3.36(5H,m),3.57-3.64(1H,m),4.02(1H,d,J=16.6Hz),4.22(1H,d,J=16.6Hz),4.35(1H,s), 4.48(1H,s),7.07(1H,bs),7.11(1H,d, J=8.3Hz), 7.19(1H, d, J=8.3Hz), 7.22(1H, s), 7.28(1H, dd, J=8.3,1.5Hz), 7.37 (1H,dt,J=6.8,1.5Hz),7.41(1H,dt,J=6.8,1.5Hz),7.56(1H,s),7.72(1H,d,J=8.3Hz),7.74(1H,d,J=7.8Hz),7.76(1H,d,J=7.8Hz).<br>for $C33H42N2O7 \cdot 1.25H_2O \cdot 0.6CF3COOH$<br>61.34 6.79 4.18 5.11.<br>61.71 6.75 3.73 5.12 |
| 24-b | 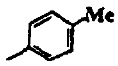 | 1.32-1.40(2H, m), 1.42-4.59(4H, m), 1.63-1.87(4H, m), 2.30(3H, s), 2.73(2H, t, J=7.3Hz), 3.22-3.34(5H, m), 3.55-3.62(1H, m), 4.03(1H, d, J=16.6Hz), 4.20(1H, J=16.6Hz), 4.33(1H, s), 4.48(1H, s), 7.16(2H, d, J=7.8Hz), 7.28(1H, d, J=8.3Hz), 7.34-7.43(3H, m), 7.56(1H, s), 7.72(1H, d, J=7.8Hz), 7.74(1H, d, J=7.3Hz), 7.76(1H, d, J=7.3Hz).<br>for $C32H39N2O7 \cdot 1H_2O \cdot 0.65\ CF3COOH$<br>60.99 6.40 4.27 5.65.<br>61.22 6.38 4.22 5.76. |
| 24-c | 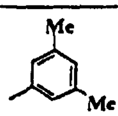 | 1.27-1.37(2H, m), 1.43-1.57(4H, m), 1.63-1.72(2H, m), 2.27(6H, s), 2.72(2H, t, J=7.3Hz), 3.19-3.30(4H, m), 3.27-3.33(1H, m), 3.54-3.61(1H, m), 4.04(1H, d, J=16.1Hz), 4.20(1H, d, J=16.1Hz), 4.34(1H, s), 4.49(1H, s), 6.95(1H, s), 7.08(2H, s), 7.15(1H, broad s), 7.27(1H, d, J=7.8Hz), 7.36(1H, t, J=8.3Hz), 7.41(1H, t, J=8.3Hz), 7.56(1H, s), 7.71(1H, d, J=7.8Hz), 7.73(1H, d, J=6.8Hz), 7.75(1H, d, J=7.8Hz).<br>for $C33H42N2O7 \cdot 1H_2O \cdot 0.7\ CF3COOH$<br>61.07 6.66 4.14 5.90.<br>60.82 6.39 4.14 5.61 |

| | | |
|---|---|---|
| 24-d | 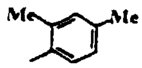 | 1.34-1.39(2H, m), 1.52-1.58(4H, m), 1.68-1.93(4H, m), 2.23(3H, s), 2.32(3H, s), 2.75(2H, t, J=7.3Hz), 3.09-3.19(2H, m), 3.25-3.37(3H, m), 3.58-3.64(1H, m), 3.97(1H, d, J=16.6Hz), 4.27(1H, d, J=16.6Hz), 4.37(1H, s), 4.44(1H, s), 6.96(1H, s), 6.98(1H, s), 7.30(1H, d, J=8.3Hz), 7.37(1H, t, J=6.8Hz), 7.41(1H, t, J=6.8Hz), 7.58(1H, s), 7.73-7.77(3H, m). |
| | | for C33H42N207 · 0.5$H_2O$ · 0.5 CF3COOH<br>63.34 6.80 4.35 4.42.<br>63.51 6.69 4.24 4.13. |
| 24-e | 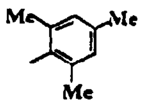 | 1.33-1.39(2H, m), 1.54-1.63(4H, m), 1.71(2H, dt, J=7.3 and 7.8Hz), 1.92-2.03(2H, m), 2.26(3H, s), 2.40(3H, s), 2.76(2H, dd, J=7.3 and 7.8Hz), 3.05-3.15(1H, m), 3.16-3.23(1H, m), 3.27(2H, q, J=6.4Hz), 3.42-3.48(1H, m), 3.54-3.61(1H, m), 3.80(1H, d, J=16.1Hz), 4.03(1H, d, J=16.1Hz), 4.45(2H, s), 6.90(2H, s), 7.31(1H, dd, J=1.5 and 8.8Hz), 7.40(2H, dd, J=5.9 and 8.8Hz), 7.59(1H, s), 7.74(2H, d, J=8.3Hz), 7.77(1H, d, J=8.8Hz). |
| | | for C34H44N207 · 0.5$H_2O$ · 0.75 CF3COOH<br>62.21 6.71 4.08 6.22.<br>62.21 6.73 3.98 5.82. |
| 24-f | 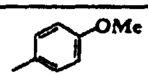 | 1.28-1.37(2H, m), 1.46-1.57(4H, m), 1.64-1.83(6H, m), 2.73(2H, t, J=7.3Hz), 3.18-3.34(5H, m), 3.55-3.62(1H, m), 3.74(3H, s), 4.05(1H, d, J=17.1Hz), 4.19(1H, d, J=17.1Hz), 4.33(1H, s), 4.50(1H, s), 6.87(2H, d, J=8.8Hz), 7.15(1H, broad s), 7.28(1H, dd, J=1.5 and 8.3Hz), 7.37(1H, dt, J=1.5 and 6.8Hz), 7.41(1H, dt, J=1.5 and 6.8Hz), 7.42(2H, d, J=8.8Hz), 7.56(1H, s), 7.72(1H, d, J=7.8Hz), 7.74(1H, d, J=5.9Hz), 7.76(1H, d, J=8.3Hz). |
| | | for C32H40N208 · 1$H_2O$ · 0.75 CF3COOH<br>58.81 6.30 4.09 6.25.<br>58.83 6.10 3.98 6.25. |
| 24-g | 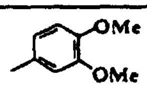 | 1.30-1.40(2H, m), 1.51-1.62(4H, m), 1.67-1.90(4H, m), 2.75(2H, t, J=7.3Hz), 3.20-3.36(5H, m), 3.59-3.66(1H, m), 3.83(3H, s), 3.84(3H, s), 4.04(1H, J=15.6Hz), 4.28(1H, d, J=15.6Hz), 4.36(1H, s), 4.49(1H, s), 6.78(1H, d, J=7.8Hz), 6.96-7.05(2H, m), 7.11(1H, s), 7.27(1H, d, J=8.3Hz), 7.38(1H, t, J=6.8Hz), 7.42(1H, t, J=6.8Hz), 7.57(1H, s), 7.73(1H, d, J=7.8Hz), 7.75(1H, d, J=7.3Hz), 7.77(1H, d, J=7.3Hz). |
| | | for C33H41N209 · 2$H_2O$ · 0.6CF3COOH<br>57.52 6.44 3.92 4.79.<br>57.61 6.06 4.10 4.47. |

| | | |
|---|---|---|
| 24-h | 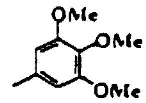 | 1.28-1.38(2H, m), 1.44-1.59(4H, m), 1.63-1.75(2H, m), 2.72(2H, t, J=7.8Hz), 3.20-3.34(4H, m), 3.55-3.62(1H, m), 3.77-3.83(1H, m), 3.81(3H, s), 3.82(6H, s), 4.11(1H, d, J=16.6Hz), 4.21(1H, d, J=16.6Hz), 4.33(1H, s), 4.53(1H, s), 6.85(2H, s), 7.21(1H, broad s), 7.27(1H, d, =8.8Hz), 7.36(1H, t, J=6.8Hz), 7.40(1H, t, J=6.8Hz), 7.55(1H, s), 7.72(1H, d, J=7.8Hz), 7.73(1H, d, J=7.8Hz), 7.75(1H, J=8.8Hz). |
| | | for C34H44N2O10 · $1H_2O$ · 0.7 CF3COOH<br>57.01 6.29 3.74 6.08<br>57.06 5.85 3.55 6.09. |
| 24-i | 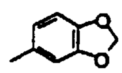 | 1.29-1.38(2H, m), 1.45-1.58(4H, m), 1.65-1.84(4H, m), 2.73(2H, t, J=7.3Hz), 3.19-3.35(5H, m), 3.56-3.64(1H, m), 4.07(1H, d, J=16.6Hz), n4.21(1H, d, J=16.6Hz), 4.33(1H, s), 4.51(1H, s), 5.95(2H, s), 6.76(1H, d, J=7.8Hz), 6.99(1H, d, J=7.8Hz), 7.02(1H, s), 7.28(1H, d, J=8.8Hz), 7.37(1H, t, J=6.3Hz), 7.41(1H, t, J=6.3Hz), 7.56(1H, s), 7.72(1H, d, J=7.8Hz), 7.74(1H, d, J=6.3Hz), 7.76(1H, d, J=8.8Hz). |
| | | for C32H38N2O9 · $1H_2O$ · 0.8 CF3COOH<br>57.33 5.84 3.98 6.48.<br>57.20 5.72 3.78 6.41. |

Example 25

The alcohol compound (0.15 g) of Example 21-(1), N-(3-chloro-4-methylphenyl)-2,4-dinitrobenzenesulfonamide (0.10 g) of Referential Example 11-j and triphenylphosphine (0.09 g) were dissolved in tetrahydrofuran (3 ml), followed by the dropwise addition of diethyl azodicarboxylate (40% solution in toluene, 0.15 ml) under cooling with ice water. After stirring for 12 hours, the solvent was distilled off under reduced pressure. The residue was purified by chromatography on a silica gel column (30% ethyl acetate-hexane), whereby tert-butyl (2R,3R)-2-tert-butoxycarbonylmethoxy-3-[4-[N-(3-chloro-4-methylphenyl)-N- (2,4-dinitrobenzenesulfonyl)amino]butoxy]-3-[N-[5-(2-naphthyl)pentyl]carbamoyl]propionate (0.18 g) was obtained as a colorless oil.

$^1$H-NMR($CDCl_3$)δ: 1.37-1.50(4H,m), 1.42(9H,s), 1.47(9H,s), 1.52-1.60(4H,m), 1.68-1.76(2H,m), 2.35(3H,s), 2.76(2H,t,J=7.8Hz), 3.29(2H,q,J=6.8Hz), 3.32-3.38(1H,m), 3.45-3.50(1H,m), 3.68-3.82(2H,m), 3.87(1H,d,J=15.6Hz), 4.14(1H,d,J=2.4Hz), 4.22(1H,d,J=15.6Hz), 4.30(1H,d,J=2.4Hz), 6.74(1H,t,J=5.9Hz), 6.97(1H,dd,J=8.3,2.0Hz), 7.17(1H,d,J=2.0Hz), 7.30(1H,dd,J=8.8,1.5Hz), 7.38(1H,dt,J=6.8,1.5Hz), 7.42(1H,dt,J=6.8,1.5Hz), 7.57(1H,s), 7.72-7.78(4H,m), 8.26(1H,dd,J=8.8,2.0Hz), 8.40(1H,d,J=2.0Hz).

The resulting compound (0.18 g) was dissolved in methylene chloride (3 ml). To the resulting solution were added thioglycolic acid (0.04 ml) and triethylamine (0.12 ml), and the mixture was stirred at room temperature for 0.5 hour. The reaction mixture was washed with a 5% aqueous sodium bicarbonate solution and dilute hydrochloric acid and then dried over anhydrous sodium sulfate. The solvent was distilled off under reduced pressure. The residue was purified by chromatography on a silica gel column (50% ethyl acetate-hexane), whereby the compound of Example 25-j (0.128 g) was obtained as a colorless oil.

In the same manner as in the above-described example except for the use of, instead of N-(3-chloro-4-methylphenyl)-2,4-dinitrobenzenesulfonamide, the corresponding sulfonamide derivatives of Referential Example 11, the reaction was effected, whereby the compounds of Example 25-k to 25-m were obtained, respectively.

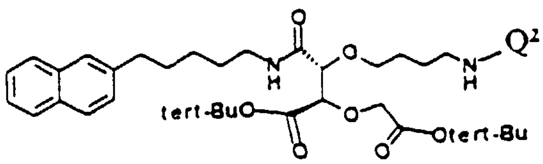

| Example | $Q^2$ | $^1$H-NMR($CDCl_3$)δ : |
|---|---|---|
| 25-j | 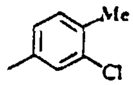 | 1.35-1.43(2H, m), 1.42(9H, s), 1.48(9H, s), 1.54-1.64(6H, m), 1.67-1.76(2H, m), 2.22(3H, s), 2.75(2H, dd, J=7.8, 7.3Hz), 3.00(2H, dd, J=6.4, 5.9Hz), 3.26-3.51(4H, m), 3.62(1H, bs), 3.88(1H, d, J=16.1Hz), 4.17(1H, d, J=2.4Hz), 4.24(1H, d, J=16.1Hz), 4.31(1H, d, J=2.4Hz), 6.39(1H, dd, J=7.8, 2.4Hz), 6.58(1H, d, J=2.4Hz), 6.74(1H, t, J=5.9Hz), 6.95(1H, d, J=7.8Hz), 7.30(1H, ddd, J=7.8, 6.8, 1.5Hz), 7.43(1H, ddd, J=7.8, 6.8, 1.5Hz), 7.58(1H, s), 7.74(1H, d, J=7.8Hz), 7.76(1H, d, J=8.8Hz), 7.78(1H, d, J=8.8Hz). |
| 25-k | 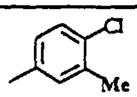 | 1.30-1.45(2H, m), 1.42(9H, s), 1.48(9H, s), 1.52-1.61(6H, m), 1.67-1.75(2H, m), 2.22(3H, s), 2.75(2H, t, J=7.3Hz), 3.00(2H, t, J=6.8Hz), 3.24-3.34(2H, m), 3.37-3.41(1H, m), 3.45-3.50(1H, m), 3.87(1H, d, J=16.1Hz), 4.17(1H, d, J=2.4Hz), 4.25(1H, d, J=16.1Hz), 4.30(1H, d, J=2.4Hz), 6.39(1H, dd, J=8.3, 2.4Hz), 6.58(1H, d, J=2.4Hz), 6.75(1H, t, J=5.9Hz), 6.95(1H, d, J=8.3Hz), 7.30(1H, dd, J=8.3,1.5Hz), 7.39(1H, dt, J=5.4,1.5Hz),7.43(1H,dt,J=5.4,1.5Hz),7.58(1H,s),7.74(1H,d, J=8.3Hz),7.76(1H,d, J=8.8Hz),7.78(1H,d, J=8.8Hz). |
| 25-l | 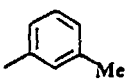 | 1.31-1.49(4H, m), 1.42(9H, s), 1.49(9H, s), 1.59-1.66(4H, m), 1.67-1.76(2H, m), 2.26(3H, s), 2.76(2H, t, J=7.3Hz), 3.05(2H, J=6.8Hz), 3.25-3.32(2H, m), 3.37-3.44(1H, m), 3.46-3.53(1H, m), 3.88(1H, d, J=16.1Hz), 4.18(1H, d, J=2.0Hz), 4.25(1H, d, J=16.1Hz), 4.31(1H, d, J=2.0Hz), 6.39(1H, d, J=7.3Hz), 6.40(1H, s), 6.50(1H, d, J=7.3Hz), 6.75(1H, t, J=5.4Hz), 7.04(1H, t, J=7.3Hz), 7.30(1H, d, J=8.3Hz), 7.40(1H, t, J=6.8Hz), 7.43(1H, t, J=6.4Hz), 7.58(1H, s), 7.74(1H, d, J=8.3Hz), 7.76(1H, d, J=8.8Hz), 7.78(1H, d, J=8.8Hz). |
| 25-m | 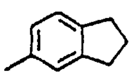 | 1.34-1.79(10H, m), 1.42(9H, s), 1.49(9H, s), 1.97-2.05(2H, m), 2.74-2.83(6H, m), 3.05(2H, t, J=6.4Hz), 3.26-3.32(2H, m), 3.39-3.50(2H, m), 3.88(1H, d, J=16.1Hz), 4.18(1H, d, J=2.4Hz), 4.25(1H, d, J=16.1Hz), 4.31(1H, d, J=2.4Hz), 6.39(1H, dd, J=7.8, 2.0Hz), 6.49(1H, s), 6.75(1H, t, J=5.9Hz), 7.00(1H, d, J=7.8Hz), 7.30(1H, dd, J=8.3,1.5Hz), 7.40(1H, dt, J=6.4,1.5Hz), 7.44(1H, dt, J=6.4, 1.5Hz), 7.58(1H, s), 7.74(1H, d, J=8.3Hz), 7.76(1H, d, J=8.3Hz), 7.78(1H, d, J=8.3Hz). |

Example 26

The compound (0.128 g) of Example 25-j was dissolved in methylene chloride (2 ml). To the resulting solution was added trifluoroacetic acid (1 ml), and the mixture was stirred at room temperature for 3 hours. The reaction mixture was concentrated to dryness under reduced pressure. The residue was purified by high-performance liquid column chromatography ("Sensyu Pak ODS-5251-SH", elution with acetonitrile-0.1% trifluoroacetic acid 65:35 (V/V)), whereby the compound (0.08 g) of Example 26-j was obtained as a pale yellow solid.

The compounds of Examples 25-k to 25-m were subjected to the reaction similar to that of the above-described Example, whereby compounds of Examples 26-k to 26-m were obtained, respectively.

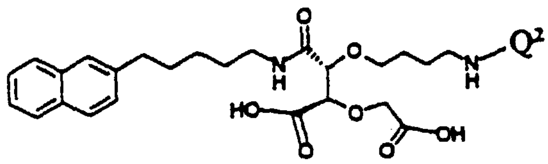

| Example | $Q^2$ | $^1H$-NMR($CDCl_3$) δ : <br> Elementary analysis Calculated: Found: <br> C H N F (Cl) |
|---|---|---|
| 26-j | 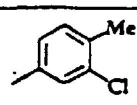 | 1.29-1.38(2H, m), 1.47-1.58(4H, m), 1.65-1.80(4H, m), 2.31(3H, s), 2.73(2H, dd, J=7.8, 7.3Hz), 3.19-3.36(5H, m), 3.54-3.61(1H, m), 4.07(1H, d, J=17.1Hz), 4.22(1H, d, J=17.1Hz), 4.34(1H, s). 4.50(1H, s), 7.13(1H, bs), 7.18-7.23(2H, m), 7.28(1H, dd, J=8.3,1.5Hz), 7.37(1H, dd, J=7.8,6.8Hz), 7.41(1H, dd, J=7.8,6.8Hz), 7.44(1H, s), 7.56(1H, s), 7.72(1H, d, J=8.3Hz), 7.73(1H, J=6.8Hz), 7.76(1H, d, J=6.8Hz). <br> for C32H39ClN2O7 · 1.1H2O · 0.6 CF3COOH <br> 58.01 6.13 4.08 4.98 (5.16) <br> 58.31 6.15 3.76 4.68 (5.22) |

| | | |
|---|---|---|
| 26-k | 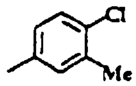 | 1.25-1.36(2H, m), 1.45-1.57(4H, m), 1.64-1.79(4H, m), 2.31(3H, s), 2.72(2H, t, J=7.3Hz), 3.29-3.33(5H, m), 3.53-3.41(1H, m), 4.09(1H, d, J=16.6Hz), 4.19(1H, d, J=16.6Hz), 4.32(1H, s), 4.52(1H, s), 7.21(1H, d, J=7.8Hz), 7.26-7.28(2H, m), 7.37(1H, t, J=6.4Hz), 7.41(1H, t, J=6.4Hz), 7.49(1H, s), 7.55(1H, s), 7.72(1H, d, J=7.8Hz), 7.74(1H, d, J=8.8Hz), 7.75(1H, d, J=8.8Hz). |
| | | for C32H39ClN2O9 · $1.3H_2O$ · 0.75 CF3COOH<br>56.82 6.03 3.96 6.04 (5.01)<br>56.83 6.03 3.76 6.08 (5.28) |
| 26-l | 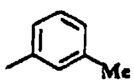 | 1.28-1.38(2H, m), 1.42-1.60(4H, m), 1.63-1.88(4H, m), 2.32(3H, s), 3.20-3.33(5H, m), 3.55-3.62(1H, m), 4.03(1H, d, J=16.1Hz), 4.20(1H, d, J=16.1Hz), 4.34(1H, s), 4.49(1H, s), 7.13-7.17(2H, m), 7.26-7.29(3H, m), 7.36(1H, t, J=8.8Hz), 7.41(1H, t, J=8.8Hz), 7.56(1H, s), 7.72(1H, d, J=7.8Hz), 7.74(1H, d, J=7.8Hz), 7.76(1H, d, J=7.8Hz). |
| | | for C32H40N2O7 · $1.4H_2O$ · 0.65 CF3COOH<br>60.23 6.62 4.22 5.58<br>59.93 6.52 3.99 5.46 |
| 26-m | 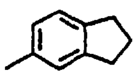 | 1.32-1.38(2H, m), 1.52-1.60(4H, m), 1.66-1.83(4H, m), 2.05(2H, t, J=7.3Hz), 2.74(2H, dd, J=7.8,7.3Hz), 2.85(2H, t, J=7.3Hz), 2.86(2H, t, J=7.3Hz), 3.24-3.31(5H, m), 3.58-3.65(1H, m), 4.03(1H, d, J=16.6Hz), 4.23(1H, d, J=16.6Hz), 4.35(1H, s, ), 4.49(1H, s), 7.07(1H, bs), 7.20(2H, s), 7.28(1H, d, J=2.0Hz), 7.30(1H, d, J=4.4Hz), 7.37(1H, dd, J=7.8,6.8Hz), 7.41(1H, dd, J=7.8,6.8Hz), 7.57(1H, s), 7.72-7.77(3H, m). |
| | | for C34H42N2O7 · $1.8H_2O$ · 0.75 CF3COOH<br>61.14 6.89 3.80 5.80<br>60.92 6.54 3.76 5.48 |

Example 27

tert-Butyl (2R,3R)-2-ethoxycarbonylmethoxy-3-[N-[5-(2-naphthyl)pentyl]carbamoyl]-3-(4-oxobutoxy)propionate

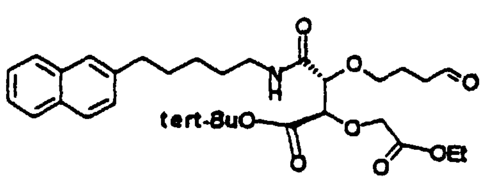

The diester compound (23.6 g) obtained in Example 18-(3) was treated in the same manner as in Example 18-(4) except for the use of ethyl bromoacetate instead of tert-butyl bromoacetate, whereby tert-butyl (2R,3R)-3-benzyloxycarbonyl-3-[4-(tert-butyldiphenylsilyloxy)butoxy]-2-ethoxycarbonylmethoxypropionate (24.1 g) was obtained as a colorless oil.

Mass (FAB+) m/Z: 715(M+Na)$^+$.
$^1$H-NMR($CDCl_3$)δ: 1.02(9H,s), 1.24(3H,t,J=7.1Hz), 1.47(9H,s), 1.55-1.62(1H,m), 1.66-2.04(2H,m), 3.29-3.34(1H,m), 3.62(2H,t,J=6.1Hz), 3.70-3.75(1H,m), 4.12-4.19(3H,m), 4.37(1H,d,J=16.6Hz), 4.38(1H,d,J=2.9Hz), 4.48(1H,d,J=2.9Hz), 5.23(1H,d,J=12.0Hz), 5.27(1H,d,J=12.0Hz), 7.31-7.41(11H,m), 7.63-7.65(4H,m).

The resulting silyloxy compound (22.7 g) was treated in the same manner as in Example 18-(5), whereby tert-butyl (2R,3R)-3-benzyloxycarbonyl-2-ethoxycarbonylmethoxy-3-(4-hydroxybutoxy)propionate (14.0 g) was obtained as a colorless oil.

Mass (FAB+) m/Z: 455(M+H)$^+$.
$^1$H-NMR($CDCl_3$)δ: 1.25(3H,t,J=7.1Hz), 1.48(9H,s), 1.62-1.70(4H,m), 3.41(1H,dt,J=9.3,5.9Hz), 3.56-3.63(2H,m), 3.75(1H,dt,J=8.8,5.9Hz), 4.11-4.19(3H,m), 4.35-4.40(2H,m), 4.48(1H,d,J=2.9Hz), 5.23(1H,d,J=12.2Hz), 5.29(1H,d,J=12.2Hz), 7.33-7.39(3H,m), 7.41-7.43(2H,m).

The resulting alcohol compound (30.8 g) was treated in the same manner as in Example 21-(1), whereby tert-butyl (2R,3R)-2-ethoxycarbonylmethoxy-3-(4-hydroxybutoxy)-3-[N-[5-(2-naphthyl)pentyl]carbamoyl]propionate was obtained.

Mass (EI) m/Z: 559(M)$^+$.
$^1$H-NMR($CDCl_3$)δ: 1.23(3H,t,J=7.1Hz), 1.39-1.46(4H,m), 1.50(9H,s), 1.52-1.63(6H,m), 1.73(2H,m), 2.77(2H,t,J=7.8Hz), 3.24-3.39(2H,m), 3.40-3.45(1H,m), 3.48-3.52(1H,m), 3.58-3.62(1H,m), 4.01(1H,d,J=16.1Hz), 4.15(2H,m), 4.19(1H,d,J=2.4Hz), 4.33(1H,d,J=2.9Hz), 4.35(1H,d,J=16.1Hz), 6.77-6.80(1H,m), 7.32(1H,dd,J=8.3,1.5Hz), 7.39-7.46(2H,m), 7.60(1H,s), 7.75-7.80(3H,m).

The resulting alcohol compound (14.28 g) was treated in the same manner as in Example 18-(6), whereby the title compound (12.4 g) was obtained as a colorless oil.

Mass (EI) m/Z: 557(M$^+$).
$^1$H-NMR($CDCl_3$)δ: 1.23(3H,t,J=7.3Hz), 1.36-1.44(2H,m), 1.50(9H,s), 1.56-1.62(2H,m), 1.70-1.78(1H,m), 1.80-1.86(1H,m), 2.42-2.47(2H,m), 2.78(1H,t,J=7.6Hz), 3.26-3.40(3H,m), 3.46-3.51(1H,m), 4.00(1H,d,J=16.1Hz), 4.10-4.18(3H,m), 4.32(1H,d,J=2.9Hz), 4.34(1H,d,J=15.6Hz), 6.63(1H,t,J=5.4Hz), 7.31-7.33(1H,m), 7.39-7.46(2H,m), 7.60(1H,s), 7.75-7.80(3H,m), 9.70(1H,s).

Example 28

(2R,3R)-3-[5-(3,4-Dimethylphenyl)pentyloxy]-2-ethoxycarbonylmethoxy-3-[N-[5-(2-naphthyl)pentyl]carbamoyl]propionic acid

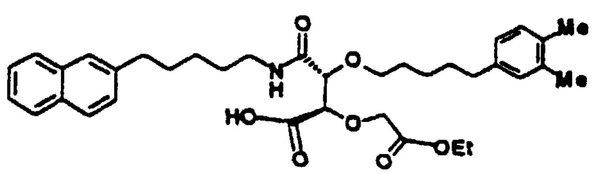

(1) tert-Butyl (2R,3R)-3-[5-(3,4-dimethylphenyl)pentyloxy]-2-ethoxycarbonylmethoxy-3-[N-(5-(2-naphthyl)pentyl]carbamoyl]propionate

The aldehyde compound (0.6 g) obtained in Example 27 was treated in the same manner as in Example 29, whereby the title compound (0.47 g) was obtained as a colorless oil.

tert-Butyl (2R,3R)-3-[5-(3,4-dimethylphenyl)pent-4-en-1-yloxy]-2-ethoxycarbonylmethoxy-3-[N-[5-(2-naphthyl)pentyl]carbamoyl]propionate

Mass (EI) m/Z: 659($M^+$).
$^1$H-NMR($CDCl_3$)δ: 1.22(3H,t,J=7.1Hz), 1.34-1.41(2H,m), 1.49(9H,s), 1.48-1.60(2H,m), 1.67-1.76(2H,m), 2.17-2.22(8H,m), 2.74(2H,t,J=7.8Hz), 3.21-3.30(2H,m), 3.42-3.55(2H,m), 4.02,4.03(total 1H,d each,J=16.1/16.6Hz), 4.09-4.18(2H,m), 4.19(1H,d,J=2.4Hz), 4.33,4.35(total 1H,d each,J=16.1/16.1Hz), 4.34(1H,d,J=2.0Hz), 6.07(1H,dt,J=16.1,6.8Hz), 6.29(1H,d,J=16.1Hz), 6.69(1H,t,J=5.9Hz), 6.97-7.08(3H,m), 7.28-7.30(2H,m), 7.38-7.45(2H,m), 7.57(1H,s), 7.73-7.79(3H,m).

tert-Butyl (2R,3R)-3-[5-(3,4-dimethylphenyl)pentyloxy]-2-ethoxycarbonylmethoxy-3-[N-[5-(2-naphthyl)pentyl}carbamoyl]propionate

Mass (EI) m/Z: 661($M^+$).
$^1$H-NMR($CDCl_3$)δ: 1.20-1.32(5H,m), 1.34-1.43(2H,m), 1.49(9H,s), 1.50-1.60(6H,m), 1.68-1.76(2H,m), 2.20(3H,s), 2.21(3H,s), 2.50(2H,t,J=7.8Hz), 2.76(2H,t,J=7.6Hz), 3.23-3.50(4H,m), 4.02(1H,d,J=16.1Hz), 4.12-4.19(3H,m), 4.33(1H,d,J=2.0Hz), 4.34(1H,d,J=16.1Hz), 6.67(1H,t,J=5.9Hz), 6.87(1H,d,J=7.6Hz), 6.91(1H,s), 7.01(1H,d,J=7.6Hz), 7.28-7.31(1H,m), 7.37-7.45(2H,m), 7.58(1H,s), 7.73-7.79(3H,m).

(2) (2R,3R)-3-[5-(3,4-dimethylphenyl)pentyloxy]-2-ethoxycarbonylmethoxy-3-[N-[5-(2-naphthyl)pentyl]carbamoyl]propionic acid

The diester compound (0.22 g) obtained in (1) was dissolved in methylene chloride (1 ml). To the resulting solution was added trifluoroacetic acid (0.5 ml), and the mixture was stirred at room temperature for 1 hour. The residue obtained by concentration to dryness under reduced pressure was subjected to column of "DIAION® HP20" and eluted with acetonitrile. The residue obtained by concentration under reduced pressure was dissolved in diethyl ether. The resulting solution was dried over anhydrous sodium sulfate and then concentrated to dryness under reduced pressure, whereby the title compound (0.18 g) was obtained as a colorless oil.

Mass (EI) m/Z: 605($M^+$).
$^1$H-NMR($CDCl_3$)δ: 1.25-1.43(7H,m), 1.50-1.62(6H,m), 1.70-1.78(2H,m), 2.20(3H,s), 2.22(3H,s), 2.51(2H,t,J=7.6Hz), 2.77(2H,t,J=7.6Hz), 3.27-3.32(2H,m), 3.45-3.51(2H,m), 4.10(1H,d,J=17.1Hz), 4.25(2H,q,J=7.1Hz), 4.34(1H,d,J=17.1Hz), 4.37(1H,d,J=2.0Hz), 4.54(1H,d,J=2.0Hz), 6.80(1H,t,J=5.9Hz), 6.88(1H,d,J=7.6Hz), 6.92(1H,s), 7.02(1H,d,J=7.6Hz), 7.30(1H,dd,J=8.3,1.5Hz), 7.38-7.46(2H,m), 7.58(1H,s), 7.74-7.80(3H,m).

Example 29

(2R,3R)-3-[5-(2-Benzoxazolyl)pentyloxy]-2-ethoxycarbonylmethoxy-3-[N-[5-(2-naphthyl)pentyl]carbamoyl]propionic acid

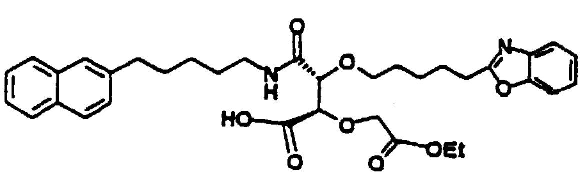

The treatment was carried out in the same manner as in Example 28 except for the use of 2-(benzoxazolylmethyl)triphenylphosphonium bromide instead of (3,4-dimethylphenylmethyl)triphenylphosphonium chloride, whereby the title compound was obtained as a colorless oil.

(1) tert-Butyl (2R,3R)-3-[5-(2-benzoxazolyl)-4-pentenyloxy]-2-ethoxycarbonylmethoxy-3-[N-[5-(2-naphthyl)pentyl]carbamoyl]propionate

$^1$H-NMR($CDCl_3$)δ: 1.23(3H,t,J=7.3Hz), 1.37-1.42(2H,m), 1.55(9H,s), 1.52-1.59(2H,m), 1.67-1.79(4H,m), 2.28-2.36(2H,m), 2.75(2H,t,J=7.6Hz), 3.25-3.39(2H,m), 3.45(1H,dt,J=9.3,6.4Hz), 3.55(1H,dt,J=9.3,6.2Hz), 4.02(1H,d,J=16.1Hz), 4.16(2H,m), 4.21(1H,d,J=2.4Hz), 4.34(1H,d,J=2.0Hz), 4.37(1H,d,J=16.1Hz), 6.42(1H,d,J=15.6Hz), 6.63-6.66(1H,m), 6.91-6.99(1H,m), 7.28-7.31(2H,m), 7.39-7.47(4H,m), 7.58(1H,s), 7.66(1H,dd,J=5.9,3.4Hz), 7.73-7.79(3H,m).

(2) tert-Butyl (2R,3R)-3-[5-(2-benzoxazolyl)pentyloxy]-2-ethoxycarbonylmethoxy-3-[N-[5-(2-naphthyl)pentyl]carbamoyl]propionate

Mass (EI) m/Z: 674($M^+$).
$^1$H-NMR($CDCl_3$)δ: 1.21-1.28(3H,m), 1.37-1.45(4H,m), 1.49(9H,s), 1.51-1.64(2H,m), 1.67-1.75(2H,m), 1.81-1.89(2H,m), 2.75(2H,t,J=7.6Hz), 2.89(2H,t,J=7.6Hz), 3.24-3.36(2H,m), 3.39(1H,dt,J=9.8,6.6Hz), 3.47(1H,dt,J=9.8,6.5Hz), 4.00(1H,d,J=16.1Hz), 4.10-4.16(2H,m), 4.18(1H,d,J=2.4Hz), 4.33(1H,d,J=2.4Hz), 4.35(1H,d,J=16.1Hz), 6.67(1H,t,J=5.9Hz), 7.27-7.31(2H,m), 7.33-7.47(4H,m), 7.58(1H,s), 7.63-7.66(1H,m), 7.73-7.79(3H,m).

(3) (2R,3R)-3-[5-(2-Benzoxazolyl)pentyloxy]-2-ethoxycarbonylmethoxy-3-[N-[5-(2-naphthyl)pentyl]carbamoyl]propionic acid

Mass (FAB+) m/Z: 619$(M+H)^+$.
$^1$H-NMR($CDCl_3$)δ: 1.26(3H,t,J=7.3Hz), 1.36-1.44(3H,m), 1.56-1.61(4H,m), 1.70-1.77(2H,m), 1.82-1.91(2H,m), 2.77(2H,t,J=7.6Hz), 2.96(2H,dt,J=7.3,3.4Hz), 3.30(2H,q,J=6.8Hz), 3.44-3.48(1H,m), 3.57-3.62(1H,m), 4.18-4.28(4H,m), 4.35(1H,d,J=2.4Hz), 4.56(1H,d,J=2.4Hz), 6.74(1H,t,J=5.9Hz), 7.29-7.32(2H,m), 7.38-7.49(4H,m), 7.59(1H,s), 7.67-7.69(1H,m), 7.74-7.80(3H,m).

Example 30

(2R,3R)-2-Carboxymethoxy-10-(2-naphthyl)-3-[5-(2-naphthyl)pentyloxy]-4-oxodacanoic acid

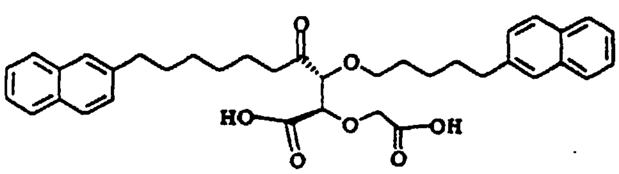

(1) tert-Butyl (2R,3R)-3-benzyloxycarbonyl-2-tert-butyldimethylsilyloxy-3-[5-(2-naphthyl)pentyloxy]propionate

The compound (1.05 g) obtained in Example 15-(2) was dissolved in N,N-dimethylformamide (10 ml). To the resulting solution were added imidazole (0.29 g) and tert-butyl chlorodimethylsilane (0.61 g), and the mixture was stirred at room temperature for 3 days. The reaction mixture was diluted with ethyl acetate, washed successively with 0.5N hydrochloric acid and water, and dried over anhydrous sodium sulfate. The solvent was distilled off under reduced pressure. The residue was purified by chromatography on a silica gel column (elution with 3% ethyl acetate-hexane), whereby the title compound (1.25 g) was obtained as a colorless oil.

Mass (FAB+) m/Z: 629(M+Na)+.

$^1$H-NMR($CDCl_3$)δ: 0.02(3H,s), 0.11(3H,s), 0.87(9H,s), 1.34-1.40(2H,m), 1.45(9H,s), 1.57-1.70(4H,m), 2.72(2H,t,J=7.6Hz), 3.34-3.40(1H,m), 3.62-3.68(1H,m), 4.25(1H,d,J=4.2Hz), 4.52(1H,d,J=4.2Hz), 5.11(1H,d,J=12.5Hz), 5.21(1H,d,J=12.5Hz), 7.28-7.45(8H,m), 7.57(1H,s), 7.73-7.78(3H,m).

(2) tert-Butyl (2R,3R)-2-tert-butyldimethylsilyloxy-3-(N-methoxy-N-methylcarbamoyl)-3-[5-(2-naphthyl)pentyloxy]propionate

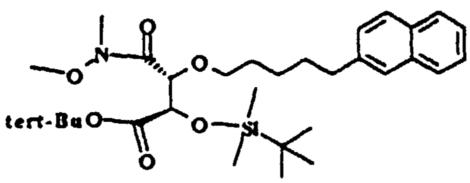

To a solution of the silylether compound (0.38 g) obtained in (1) in ethanol (8 ml) was added a catalytic amount of 10% palladium-carbon, and the mixture was stirred at room temperature for 15 hours under hydrogen atmosphere. The reaction mixture was diluted with methylene chloride and the palladium-carbon was filtered off. The solvent was distilled off under reduced pressure, whereby tert-butyl (2R,3R)-2-tert-butyldimethylsilyloxy-3-carboxy-3-[5-(2-naphthyl)pentyloxy]propionate (0.33 g) was obtained as a colorless oil. The resulting compound was dissolved in tetrahydrofuran (8 ml). To the resulting solution were successively added N-methylmorpholine (0.08 ml) and isobutyl chloroformate (0.1 ml) at -15°C, and the mixture was stirred at the same temperature for 7 minutes. Then, N,O-dimethylhydroxylamine hydrochloride (0.069 g) and a solution of N-methylmorpholine (0.08 ml) in N,N-dimethylformamide (5 ml) were added in portions and the resulting mixture was warmed gradually to room temperature. After stirring for one hour, the reaction mixture was diluted with diethyl ether and 10% hydrochloric acid and the water layer was extracted thrice with diethyl ether. The organic layers combined were washed with saturated saline and dried over anhydrous magnesium sulfate. The solvent was then distilled off under reduced pressure. The residue was purified by chromatography on a silica gel column (elution with 17% ethyl acetate-hexane), whereby the title compound (0.24 g) was obtained as a colorless oil.

$^1$H-NMR($CDCl_3$)δ: 0.05(6H,s), 0.79(9H,s), 1.34(9H,s), 1.20-1.70(6H,m), 2.64(2H,t,J=7.3Hz), 3.08(3H,broad s), 3.20-3.55(2H,m), 3.60(3H,s), 4.26(1H,d,J=6.3Hz), 4.42(1H,d,J=6.4Hz), 7.18-7.75(7H,m).

(3) tert-Butyl (2R,3R)-2-hydroxy-3-(N-methoxy-N-methylcarbamoyl)-3-[5-(2-naphthyl)pentyloxy]propionate

To a solution of the silylether compound (0.23 g) obtained in (2) in tetrahydrofuran (8 ml) was added tetrabutylammonium fluoride (1M solution in tetrahydrofuran, 1 ml), and the mixture was stirred at room temperature for 12 hours. The reaction mixture was diluted with ethyl acetate and then added with water. The water layer was extracted thrice with ethyl acetate. The organic layers combined were washed with saturated saline The solvent was distilled off under reduced pressure.

The residue was purified by chromatography on a silica gel column (elution with 25% ethyl acetate-hexane), whereby the title compound (0.16 g) was obtained as a colorless oil.

$^1$H-NMR($CDCl_3$)δ: 1.30-1.78(6H,m), 1.50(9H,s), 2.75(2H,t,J=7.8Hz), 3.10(1H,d,J=9.3Hz), 3.20-3.30(1H,m), 3.25(3H,s), 3.58-3.70(1H,m), 3.73(3H,s), 4.44-4.50(2H,m), 7.27-7.85(7H,m).

(4) tert-Butyl (2R,3R)-2-tert-butoxycarbonylmethoxy-3-(N-methoxy-N-methylcarbamoyl)-3-[5-(2-naphthyl)pentyloxy]propionate

To a solution of the 2-hydroxy compound (0.15 g) obtained in (3) in N,N-dimethylformamide (3 ml) were successively added sodium hydride (60% in oil, 0.017 g) and tert-butyl bromoacetate (0.1 ml) at room temperature, and the mixture was stirred at the same temperature for 12 hours. The reaction mixture was diluted with ethyl acetate and added with 10% hydrochloric acid. The aqueous layer was then extracted thrice with ethyl acetate. The organic layers combined were washed with saturated saline. The solvent was distilled off under reduced pressure. The residue was purified by chromatography on a silica gel column (elution with 20% ethyl acetate-hexane), whereby the title compound (0.125 g) was obtained as a colorless oil.

$^1$H-NMR($CDCl_3$)δ: 1.35-1.73(6H,m), 1.44(9H,S), 1.48(9H,s), 2.76(2H,t,J=7.3Hz), 3.25(3H,broad s), 3.32-3.42(1H,m), 3.55-3.68(1H,m), 3.72(3H,s), 4.00-4.60(4H,m), 7.26-7.83(7H,m).

(5) tert-Butyl (2R,3R)-2-tert-butoxycarbonylmethoxy-10-(2-naphthyl)-3-[5-(2-naphthyl)pentyloxy]4-oxodecanoate

To tetrahydrofuran (5 ml) were added metal magnesium (0.48 g) and a catalytic amount of iodine, and a solution of 6-(2-naphthyl)hexyl bromide (0.58 g) in tetrahydrofuran (2 ml) was added to the resulting mixture at room temperature under stirring. The reaction mixture was heated up to 60°C and at the same temperature, it was stirred for 5 minutes. Then, the reaction mixture was cooled to room temperature, at which stirring was conducted for further 3 hours, whereby 6-(2-naphthyl)hexylmagnesium bromide was prepared. To a solution of the hydroxamate (0.045 g) obtained in (4) in tetrahydrofuran (3 ml) was added the 6-(2-naphthyl)hexylmangesium bromide solution (2 ml) was added at room temperature, at which stirring was conducted for 4 hours. After the addition of 10% hydrochloric acid, the resulting mixture was diluted with ethyl acetate. The aqueous layer was extracted three times with ethyl acetate. The organic layers combined were washed successively with a saturated aqueous solution of sodium bicarbonate and saturated saline and dried over magnesium sulfate. The solvent was then distilled off under reduced pressure. The residue was purified by chromatography on a silica gel column (elution with 7% ethyl acetate-hexane), whereby the title compound (0.0013 g) was obtained as a colorless oil.

Mass (FD) m/Z: 710(M)$^+$.
$^1$H-NMR($CDCl_3$)δ: 1.71-1.78(14H,m), 2.60-2.90(6H,m), 3.40-3.70(2H,m), 4.00-4.60(4H,m), 7.25-7.83(14H,m).

(6) (2R,3R)-2-Carboxymethoxy-10-(2-naphthyl)-3-[5-(2-naphthyl)pentyloxy]-4-oxodacanoic acid

To a solution of the di-tert-butyl ester (0.0011 g) obtained in (5) in methylene chloride (5 ml) was added trifluoroacetic acid (0.3 ml), and the mixture was stirred at room temperature for 12 hours. After the completion of the reaction, the solvent was distilled off under reduced pressure, whereby the title compound (0.0009 g) was obtained as a colorless oil.

Mass (FAB+) m/Z: 621(M+Na)$^+$.
$^1$H-NMR($CDCl_3$)δ: 1.20-1.65(14H,m), 1.39(9H,s), 1.41(9H,s), 2.49-2.78(6H,m), 3.23-3.50(2H,m), 3.81(1H,d,J=16.1Hz), 4.19(1H,d,J=16.1Hz), 3.98(1H,d,J=2.9Hz), (1H,d,J=2.9Hz), 7.20-7.75(14H,m).

Example 31

(2R,3R)-3-[5-(3,4-Dimethylphenyl)pentenyloxy]-2-hydroxy-3-[N-methyl-N-[5-(2-naphthyl)pentyl]carbamoyl]propionic acid

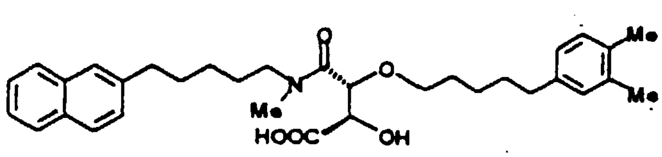

(1) tert-Butyl (2R,3R)-3-tert-butoxycarbonyl-2-[5-(3,4-dimethylphenyl)pent-2-en-1-yloxy]-3-hydroxypropionate

In N,N-dimethylformamide (300 ml), di-tert-butyl L-(+)-tartrate (19.7 g) was dissolved, followed by the addition of sodium hydride (60% in oil, 3.0 g) under cooling with ice water and stirring. After 5 minutes, a solution of the 5-(3,4-dimethylphenyl)pent-2-en-1-yl iodide (22.5 g) of Referential Example 13 in N,N-dimethylformamide (100 ml) was added dropwise over 15 minutes. While heating to room temperature, the reaction mixture was stirred for 80 minutes and then poured into an ice-water-cooled mixture of saturated saline and ethyl acetate. The organic layer was separated, washed

twice with saturated saline and dried over anhydrous sodium sulfate. The solvent was then distilled off under reduced pressure. The residue was purified by chromatography on a silica gel column (elution with 15% ethyl acetate-hexane), whereby the title compound (14.0 g) was obtained as a colorless oil.

Mass (FAB+) m/Z: 457$(M+Na)^+$.
$^1$H-NMR($CDCl_3$)δ: 1.49(9H,s), 1.50(9H,s), 2.22(3H,s), 2.23(3H,s), 2.29-2.35(2H,m), 2.59-2.63(2H,m), 3.04(1H,d,J=8.3Hz), 3.85-3.90(1H,m), 4.12(1H,d,J=2.4Hz), 4.17-4.22(1H,m), 4.44(1H,dd,J=8.3,2.4Hz), 5.45-5.58(1H,m), 5.61-5.75(1H,m), 6.89(1H,dd,J=7.8,1.5Hz), 6.93(1H,s), 7.03(1H,d,J=7.8Hz).

(2) tert-Butyl (2R,3R)-3-tert-butoxycarbonyl-2-[5-(3,4-dimethylphenyl)pentyloxy]-3-hydroxypropionate

The olefin compound (14.0 g) obtained in (1) was dissolved in ethyl acetate (200 ml). To the resulting solution was added 10% palladium-carbon (0.8 g), and the mixture was stirred at room temperature for 14.5 hours under hydrogen atmosphere. The palladium-carbon was filtered off and the filtrate was dried over anhydrous sodium sulfate. The solvent was then distilled off under reduced pressure, whereby the title compound (13.97 g) was obtained as a colorless oil.

MS (EI) m/Z: 436$(M)^+$.
$^1$H-NMR($CDCl_3$)δ: 1.34-1.41(2H,m), 1.45-1.65(22H,m), 2.22(3H,s), 2.23(3H,s), 2.52(2H,t,J=7.8Hz), 3.01(1H,d,J=8.8Hz), 3.26-3.31(1H,m), 3.69-3.75(1H,m), 4.06(1H,t,J=0.98Hz), 4.41-4.44(1H,m), 6.89(1H,d,J=7.6Hz), 6.93(1H,s), 7.02(1H,d,J=7.6Hz).

(3) Benzyl (2R,3R)-3-tert-Butoxycarbonyl-2-[5-(3,4-dimethylphenyl)pentyloxy]-3-hydroxypropionate

The di-tert-butyl ester (13.93 g) obtained in (2) was dissolved in N,N-dimethylformamide (45 ml), followed by the dropwise addition of diethyl methoxyborane (1M solution in tetrahydrofuran, 37 ml) over 15 minutes under cooling with ice water. After 40 minutes, sodium borohydride (1.45 g) was added and the resulting mixture was stirred at room temperature for 4.5 hours. The reaction mixture was poured into a ice-water-cooled mixture of sodium dihydrogenphosphate (75 g) and water (250 ml), followed by the addition of tert-butanol (30 ml) and 2-methyl-2-butene (76 ml). An aqueous solution (150 ml) of sodium chlorite (80%, 75 g) was added and the resulting mixture was stirred at room temperature for 15.5 hours. Water (400 ml) and ethyl acetate (400 ml) were added to separate the organic layer. The resulting organic layer was washed with 1N hydrochloric acid (700 ml) and dried over anhydrous sodium sulfate. The solvent was then distilled off under reduced pressure. To the residue were added toluene and triethylamine, followed by azeotropic distillation. The residue was dissolved in N,N-dimethylformamide (150 ml). To the resulting solution were added triethylamine (13 ml) and benzyl bromide (10.9 g) under cooling with ice water, and the mixture was stirred at room temperature for 20.5 hours. The reaction mixture was diluted with ethyl acetate, washed with 1N hydrochloric acid, a 5% aqueous solution of sodium bicarbonate and saturated saline and then dried over anhydrous sodium sulfate. The solvent was distilled off under reduced pressure. The residue was purified by chromatography on a silica gel column (elution with 15% ethyl acetate-hexane), whereby the title compound (6.35 g) was obtained as a pale yellow oil.

MS (FAB+) m/Z: 493$(M+Na)^+$.
$^1$H-NMR($CDCl_3$)δ: 1.30-1.40(2H,m), 1.48(9H,s), 1.55-1.63(4H,m), 2.21(3H,s), 2.23(3H,s), 2.50(2H,t,J=7.8Hz), 3.09(1H,d,J=8.3Hz), 3.31(1H,dt,J=8.8,6.8Hz), 3.73(1H,dt,J=8.8,6.4Hz), 4.24(1H,d,J=2.4Hz), 4.48(1H,d,J=8.3,2.4Hz), 5.21(1H,d,J=12.2Hz), 5.26(1H,d,J=12.2Hz), 6.88(1H,d,J=7.6Hz), 6.88(1H,d,J=7.6Hz), 6.92(1H,s), 7.02(1H,d,J=7.6Hz), 7.32-7.38(5H,m).

(4) tert-Butyl (2R,3R)-3-benzyloxycarbonyl-2-tert-butyldimethylsilyloxy-3-[5-(3,4-dimethylphenyl)pentyloxy]propionate

The hydroxy compound (1.58 g) obtained in (3) was dissolved in N,N-dimethylformamide (25 ml). To the resulting solution were added imidazole (0.46 g) and tert-butyl chlorodimethylsilane (0.91 g), and the mixture was stirred at room temperature for 27 hours. The reaction mixture was poured into water and extracted with ethyl acetate. The extract was washed with dilute hydrochloric acid and saturated saline and then dried over anhydrous sodium sulfate. The solvent was distilled off under reduced pressure. The residue was purified by chromatography on a silica gel column (elution with 3% ethyl acetate-hexane), whereby the title compound (1.56 g) was obtained as a colorless oil.

MS (FAB+) m/Z: 607$(M+Na)^+$, 585$(M+H)^+$.
$^1$H-NMR($CDCl_3$)δ: 0.017(3H,s), 0.112(3H,s), 0.88(9H,s), 1.25-1.34(2H,m), 1.45(9H,s), 1.51-1.61(4H,m), 2.21(3H,s), 2.22(3H,s), 2.48(2H,t,J=7.6Hz), 3.36(1H,dt,J=9.3,7.1Hz), 3.64(1H,dt,J=9.3,7.1Hz),

4.25(1H,d,J=3.9Hz), 4.52(1H,d,J=3.9Hz), 5.11(1H,d,J=12.2Hz), 5.21(1H,d,J=12.2Hz), 6.87(1H,d,J=7.8Hz), 6.91(1H,s), 7.02(1H,d,J=7.8Hz), 7.31-7.37(5H,m).

(5) (2R,3R)-3-tert-Butoxycarbonyl-3-tert-butyldimethylsilyloxy-2-[5-(3,4-dimethylphenyl)pentyloxy]propionic acid

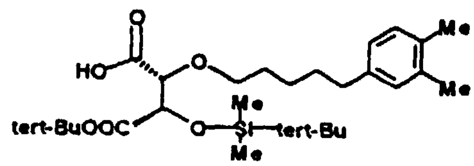

The diester compound (1.50 g) obtained in (4) was dissolved in ethyl acetate (20 ml). To the resulting solution was added 10% palladium-carbon (0.09 g), and the mixture was stirred at room temperature for 17 hours under hydrogen atmosphere. The palladium-carbon was filtered off and the filtrate was dried over anhydrous sodium sulfate. The solvent was distilled off under reduced pressure, whereby the title compound (1.35 g) was obtained as a colorless oil.

MS (FAB+) m/Z: 495$(M+H)^+$.
$^1$H-NMR($CDCl_3$)δ: 0.04(3H,s), 0.11(3H,s), 0.88(9H,s), 1.30-1.38(2H,m), 1.48(9H,s), 1.56-1.66(4H,m), 2.21(3H,s), 2.23(3H,s), 2.52(2H,t,J=7.8Hz), 3.48-3.60(2H,m), 4.29(1H,d,J=2.9Hz), 4.49(1H,d,J=2.9Hz), 6.88(1H,d,J=7.8Hz), 6.92(1H,s), 7.02(1H,d,J=7.8Hz).

(6) tert-Butyl (2R,3R)-2-tert-butyldimethylsilyloxy-3-[5-(3,4-dimethylphenyl)pentyloxy]-3-[N-methyl-N-[5-(2-naphthyl)pentyl]carbamoyl]propionate

The propionic acid (0.25 g) obtained in (5), the methyl[5-(2-naphthyl)pentyl]amine (free base, 0.15 g) of Referential Example 14-(2) and 1-hydroxybenzotriazole (23 mg) were dissolved in methylene chloride (10 ml), followed by the addition of 1-ethyl-3-(3-dimethylaminopropyl)carbodiimide hydrochloride (0.29 g) under cooling with ice water and stirring. After stirring for 14.5 hours at room temperature, the reaction mixture was concentrated under reduced pressure. Water was added to the residue and the resulting mixture was extracted with ethyl acetate. The extract was washed successively with dilute hydrochloric acid and saturated saline and then dried over anhydrous sodium sulfate. The solvent was distilled off under reduced pressure. The residue was purified by chromatography on a silica gel column (elution with 10% ethyl acetate-hexane), whereby the title compound (0.25 g) was obtained as a colorless oil.

MS (FAB+) m/Z: 726$(M+Na)^+$, 704$(M+H)^+$.
$^1$H-NMR($CDCl_3$, rotamer mixture)δ: 0.032,0.035(total 3H,s each), 0.089,0.096(total 3H,s each), 0.89(9H,s), 1.25-1.40(4H,m), 1.43,1.44(total 9H,s each), 1.51-1.60(6H,m), 1.68-1.76(2H,m), 2.20(3H,s), 2.21(3H,s), 2.50(2H,t,J=7.6Hz), 2.76(2H,t,J=7.6Hz), 2.87,3.14(total 3H,s each), 3.02-3.08,3.27-3.61,3.74-3.81(total 4H,m each), 4.34,4.36(1H,d, each,J=5.9/4.9Hz), 4.44,4.48(1H,d, each,J=5.9/4.9Hz), 6.86-6.88(1H,m), 6.91(1H,s), 6.99-7.02(1H,m), 7.30(1H,dd,J=8.3,1.5Hz), 7.37-7.51(2H,m), 7.58(1H,s), 7.73-7.79(3H,m).

(7) tert-Butyl (2R,3R)-3-[5-(3,4-dimethylphenyl)pentyloxy]-2-hydroxy-3-[N-methyl-N-[5-(2-naphthyl)pentyl]carbamoyl]propionate

The 2-tert-butyldimethylsilyloxy compound (0.22 g) obtained in (6) was dissolved in tetrahydrofuran (6 ml). To the resulting solution were added acetic acid (38 g) and tetrabutylammonium fluoride (1M solution in tetrahydrofuran, 0.56 ml), and the mixture was stirred at room temperature for 2 days. The solvent was then distilled off under reduced pressure. The residue was purified by chromatography on a silica gel column (elution with 35% ethyl acetate-hexane), whereby the title compound (0.16 g) was obtained as a colorless oil.

MS (FAB+) m/Z: 512$(M+Na)^+$, 590$(M+H)^+$.
$^1$H-NMR($CDCl_3$, rotamer mixture)δ: 1.30-1.40(4H,m), 1.40-1.64(15H,m), 1.70-1.76(2H,m), 2.21(3H,s), 2.22(3H,s), 2.50(2H,t,J=7.6Hz), 2.76(2H,t,J=7.1Hz), 2.92,3.12(total 3H,s each), 3.24-3.33(2H,m), 3.41-3.59(2H,m), 4.30-4.41(2H,m), 6.87(1H,d,J=7.3Hz), 6.91(1H,s), 7.01(1H,d,J=7.8Hz), 7.30(1H,d,J=8.1Hz), 7.38-7.45(2H,m), 7.58(1H,s), 7.74-7.79(3H,m).

(8) (2R,3R)-3-[5-(3,4-dimethylphenyl)pentyloxy]-2-hydroxy-3-[N-methyl-N-[5-(2-naphthyl)pentyl]carbamoyl]propionic acid

The tert-butyl ester compound (153 mg) obtained in (7) was dissolved in methylene chloride (1.5 ml). To the resulting solution was added trifluoroacetic acid (1.5 ml), and the mixture was stirred at room temperature for 5 hours. The solvent was then distilled off under reduced pressure. To the residue was added toluene and the resulting mixture was subjected to azeotropic distillation three times. After drying under reduced pressure, the title compound (133 mg) was obtained as a colorless oil.

MS (FAB+) m/Z: 556(M+Na)$^+$, 534(M+H)$^+$.
$^1$H-NMR($CDCl_3$, rotamer mixture)δ: 1.26-1.40(4H,m), 1.51-1.66(6H,m), 1.68-1.78(2H,m), 2.20(3H,s), 2.22(3H,s), 2.49-2.52(2H,m), 2.74-2.80(2H,m), 2.95,3.06(total 3H,s each), 3.29-3.41(3H,m), 3.48-3.55(1H,m), 4.34,4.39(total 1H,d each,J=2.9/2.9Hz), 4.57,4.60(total 1H,d each,,J=2.9/2.9Hz), 6.87(1H,d,J=7.8Hz), 6.92(1H,s), 7.01(1H,d,J=7.8Hz), 7.30(1H,d,J=8.3Hz), 7.38-7.45(2H,m), 7.58(1H,s), 7.75-7.80(3H,m).

Example 32

(2R,3R)-3-[4-(3-Chloro-4-methylphenylamino)butoxy]-2-ethoxycarbonylmethoxy-3-[N-methyl-N-[5-(2-naphthyl)pentyl]carbamoyl]propionic acid

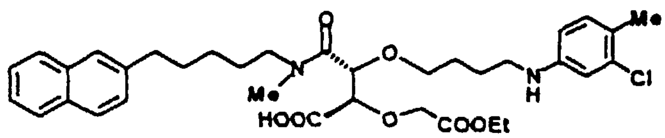

(1) tert-Butyl (2R,3R)-3-[4-(tert-butyldiphenylsilyloxy)butoxy]-2-ethoxycarbonylmethoxy-3-[N-methyl-N-[5-(2-naphthyl)pentyl]carbamoyl]propionate

The benzyl (2R,3R)-3-tert-butoxycarbonyl-2-[4-(tert-butyldiphenylsilyloxy)butoxy]-3-ethoxycarbonylmethoxypropionate (2.5 g) obtained in Example 27 was dissolved in tetrahydrofuran (50 ml). To the resulting solution was added 10% palladium-carbon (0.5 g), and the mixture was stirred at room temperature for 4.5 hours under hydrogen atmosphere. The palladium-carbon was filtered off and the solvent was distilled off under reduced pressure, whereby (2R,3R)-3-tert-butoxycarbonyl-2-[4-(tert-butyldiphenylsilyloxy)butoxy]-3-ethoxycarbonylmethoxypropionic acid was obtained as a colorless oil.

The resulting compound was dissolved in methylene chloride (50 ml), followed by the addition of the methyl [5-(2-naphthyl)pentyl]amine (free base, 0.82 g) of Referential Example 14-12) and 1-hydroxybenzotriazole (0.1 g). To the resulting mixture was added 1-ethyl-3-(3-dimethylaminopropyl)carbodiimide hydrochloride (1.04 g) under cooling with ice water and stirring. After stirring at room temperature for 3 days, water was added, followed by extraction with methylene chloride. The extract was washed with saturated saline and dried over anhydrous sodium sulfate. The solvent was then distilled off under reduced pressure. The residue was purified by chromatography on a silica gel column (elution with 15% ethyl acetate-hexane), whereby the title compound (1.67 g) was obtained as an oil.

Mass (EI) m/Z: 811(M)$^+$.
$^1$H-NMR($CDCl_3$, rotamer mixture)δ: 1.03(9H,s), 1.23(3H,t,J=7.3Hz), 1.32-1.40(2H,m), 1.45,1.46(total 9H,s each), 1.54-1.76(8H,m), 2.76(2H,dd,J=7.8,7.3Hz), 2.90,3.16(total 3H,s each), 3.26-3.62(4H,m), 3.63(2H,t,J=6.4Hz), 4.06(1H,d,J=16.1Hz), 4.11-4.20(2H,m), 4.29-4.35(1H,m), 4.39(1H,d,J=16.1Hz), 4.50(2/3H,d,J=4.4Hz), 4.52(1/3H,d,J=5.8Hz), 7.27-7.44(9H,m), 7.58(1H,s), 7.64(4H,d,J=5.9Hz), 7.73(1H,d,J=8.3Hz), 7.75(1H,d,J=8.8Hz), 7.76(1H,d,J=8.8Hz).

(2) tert-Butyl (2R,3R)-2-ethoxycarbonylmethoxy-3-(4-hydroxybutoxy)-3-[N-methyl-N-[5-(2-naphthyl)pentyl]carbamoyl]propionate

The tert-butyldiphenylsilyloxy compound (1.67 g) obtained in (1) was treated in the same manner as in Example 18-(5), whereby the title compound (1.05 g) was obtained as an oil.

Mass (EI) m/Z: 573(M)$^+$.
$^1$H-NMR($CDCl_3$, rotamer mixture)δ: 1.25(3H,t,J=6.8Hz), 1.30-1.41(2H,m), 1.45,1.47(total 9H,s each), 1.52-1.68(6H,m), 1.70-1.78(2H,m), 2.77(2H,t,J=7.8Hz), 2.91,3.16(total 3H,s each), 3.28-3.60(6H,m), 4.07-4.21(3H,m), 4.30-4.38(4/3H,m), 4.38(2/3H,d,J=16.6Hz), 4.50(2/3H,d,J=5.4Hz), 4.52(1/3H,d,J=5.4Hz), 7.32(1H,d,J=8.3Hz), 7.38-7.47(2H,m), 7.60(1H,s), 7.75(1H,d,J=8.3Hz), 7.77(1H,d,J=7.8Hz), 7.79(1H,d,J=7.8Hz).

(3) tert-Butyl (2R,3R)-3-[4-(3-chloro-4-methylphenylamino)butoxy]-2-ethoxycarbonylmethoxy-3-[N-methyl-N-[5-(2-naphthyl)pentyl]carbamoyl]propionate

The alcohol compound (0.25 g) obtained in (2) was treated in the same manner as in Example 25, whereby the crude title compound (0.42 g) was obtained as an oil.

Mass (EI) m/Z: 696(M)$^+$.
$^1$H-NMR($CDCl_3$, rotamer mixture)δ: 1.24(3H,t,J=7.3Hz), 1.32-1.39(2H,m), 1.44,1.46(total 9H,s each), 1.50-1.67(6H,m), 1.69-1.76(2H,m), 2.22(3H,s), 2.76(2H,t,J=7.3Hz), 2.91,3.15(total 3H,s each), 3.26-3.68(6H,m), 4.09(2/3H,d,J=16.1Hz), 4.14-4.22(7/3H,m), 4.33(1H,d,J=4.9Hz), 4.36(1/3H,d,J=16.1Hz), 4.38(2/3H,d,J=16.1Hz), 4.50(2/3H,d,J=4.9Hz), 4.52(1/3H,d,J=4.9Hz), 6.37(1/3H,dd,J=7.8,2.0Hz), 6.39(2/3H,d,J=8.3,2.4Hz), 6.58(1H,s), 6.59(1H,broad s), 6.94(2/3H,d,J=8.3Hz), 6.95(1/3H,d,J=7.8Hz), 7.30(1H,d,J=8.3Hz), 7.39(1H,dt,J=6.8,2.0Hz), 7.42(1H,dt,J=6.8,2.0Hz), 7.58(1H,s), 7.73-7.79(3H,m).

(4) (2R,3R)-3-[4-(3-chloro-4-methylphenylamino)butoxy]-2-ethoxycarbonylmethoxy-3-[N-methyl-N-[5-(2-naphthyl)pentyl]carbamoyl]propionic acid

The diester compound (0.42 g) obtained in (3) was treated in the same manner as in Example 26, whereby the title compound (0.048 g) was obtained as a pale yellow oil.

Mass (FAB+) m/Z: 641(M+H)$^+$.
$^1$H-NMR($CDCl_3$, rotamer mixture)δ: 1.19(3H,t,J=6.8Hz), 1.34-1.45(2H,m), 1.60-1.80(8H,m), 2.31,2.34(total 3H,s each), 2.77,2.79(total 2H,t each,J=7.8/7.8Hz), 3.00,3.13(total 3H,s each), 3.10-3.16(6H,m), 3.94(1/3H,d,J=17.1Hz), 3.96(2/3H,d,J=15.8Hz), 4.03(2/3H,d,J=15.8Hz), 4.10(2H,q,J=6.8Hz), 4.40(1/3H,d,J=2.9Hz), 4.50(2/3H,d,J=2.4Hz), 4.70(1H,broad s), 7.19(2/3H,d,J=7.8Hz), 7.22(1/3H,d,J=8.3Hz), 7.28-7.35(2H,m), 7.51(1H,s), 7.58(2/3H,s), 7.59(1/3H,s), 7.74(1H,d,J=8.3Hz), 7.76(1H,d,J=8.3Hz), 7.78(1H,d,J=8.3Hz).

(5) (2R,3R)-2-Carboxymethoxy-3-[4-(3-chloro-4-methylphenylamino)butoxy]-3-[N-methyl-N-[5-(2-naphthyl)pentyl]carbamoyl]propionic acid

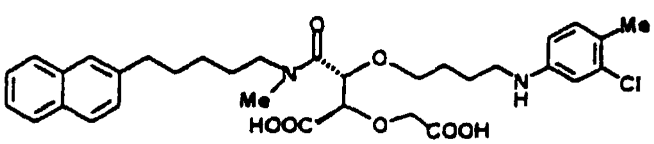

The monoethyl ester compound obtained in (4) was treated in the same manner as in Example 39-(4), followed by purification by high-performance liquid column chromatography ("Sensyu Pak ODS-5251-SH", elution with acetonitrile-0.1% trifluoroacetic acid 65:35) instead of column chromatography of "DIAION® HP20", whereby the title compound was obtained as a pale yellow amorphous solid.

MS(FAB+) m/Z: 613(M+H)$^+$.

| Elementary analysis for $C_{33}H_{41}ClN_2O_7 \cdot 0.7CF_3COOH \cdot 1.2H_2O$ | | | | | |
|---|---|---|---|---|---|
| Calculated: | C, 57.82; | H, 6.22; | Cl, 4.96; | F, 5.58; | N, 3.92. |
| Found: | C, 58.11; | H, 6.10; | Cl, 5.66; | F, 5.36; | N, 3.51. |

Example 33

(1) (4R,5R)-4-tert-Butoxycarbonyl-3,6-dioxa-5-[N-[5-(2-naphthyl)pentyl]carbamoyl-11-(2-naphthyl)undecanoic acid

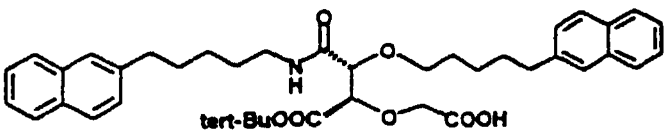

The tert-butyl (2R,3R)-2-ethoxycarbonylmethoxy-3-[N-[5-(2-naphthyl)pentyl]carbamoyl]-3-[5-(2- naphthyl)pentyloxy]propionate (0.85 g) obtained in Example 3-(3) was dissolved in ethanol (5 ml). To the resulting solution was added a 1N aqueous sodium hydroxide solution (1.4 ml) at room temperature, and the mixture was stirred for 12 hours. The solvent was then distilled off under reduced pressure. To the residue were added a 10% aqueous solution of hydrochloric acid and methylene chloride and the water layer was extracted thrice with methylene chloride. The organic layers were combined and dried over anhydrous magnesium sulfate. The solvent was distilled off under reduced pressure, whereby the title compound (0.67 g) was obtained as a colorless oil. The resulting compound was provided for the subsequent reaction without purification.
(2) The carboxylic acid (110 mg) obtained in (1) was dissolved in methylene chloride (5 ml), followed by the addition of morpholine (35 mg), 1-hydroxybenzotriazole (50 mg) and 1-ethyl-3-(3-dimethylaminopropyl)carbodiimide hydrochloride (35.5 mg) under cooling with ice water. The resulting mixture was stirred for 18 hours. To the reaction mixture were added a 10% aqueous solution of hydrochloric acid and methylene chloride. The water layer was extracted thrice with methylene chloride. The organic layers combined were washed successively with a saturated aqueous solution of sodium bicarbonate and saturated saline and then dried over anhydrous magnesium sulfate. The solvent was distilled off under reduced pressure. The residue was purified by chromatography on a silica gel column (elution with 20% ethyl acetate-hexane), whereby the diamide compound (55 mg) of Example 33-a was obtained as a colorless oil.
In the same manner as in the above-described reaction except for the use of, instead of morpholine, the corresponding amine derivatives, reaction was effected, whereby the compounds of Examples 33-b to 33-k were synthesized, respectively.

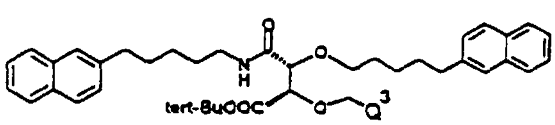

| Example | $Q^3$ | $^1H$-NMR($CDCl_3$)$\delta$ : |
|---|---|---|
| 33-a | 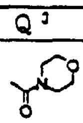 | 1.27-1.80(12H,m),1.48(9H,s),2.73(4H,broad t),3.08-3.70 (12H, m),4.06(1H,d,J=12.2Hz),4.15(1H,d,J=2.4Hz),4.34(1H,d,J=2.4Hz ),4.40(1H,d,J=12.2Hz),6.70(1H,broad t),7.25-7.80(14H, m). |
| 33-b | 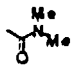 | 1.28-1.75(12H,m),2.70-2.80(4H,m),2.88(3H,s),2.92(3H,s), 3.08-3.50(4H,m),4.07(1H,d,J=13.2Hz),4.16(1H,d,J=2.4Hz),4.34 (1H,d,J=2.4Hz),4.40(1H,d,J=13.2Hz), 6.80(1H,broad t),7.21-7.80(14H,m). |

| | | |
|---|---|---|
| 33-c | 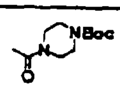 | 1.25-1.75(12H,m),1.45(9H,s),1.48(9H,s),2.74(4H,broad t), 3.05-3.60(12H,m),4.07(1H,d,J=12.7Hz),4.14(1H,d,J=2.4Hz), 4.34(1H,d,J=2.4Hz),4.39(1H,d,J=12.7Hz),6.65(1H,broad t),7.25-7.81(14H, m). |
| 33-d | 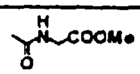 | 1.30-1.77(12H,m),1.49(9H,s),2.68-2.79(4H,m),3.13-3.50(4H, m),3.67(3H,s),4.04(2H,d,J=6.4Hz),4.05(1H,d,J=6.6Hz), 4.21(1H,d, J=2.9Hz), 4.26(1H, d,J=6.6Hz), 4.46(1H,d,J=2.9Hz), 6.78(1H, broad t), 7.25-7.85(14H, m). |
| 33-e | 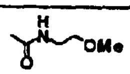 | 1.22-1.75(12H,m),1.49(9H,s),2.73(4H,broad t),3.31(3H,s), 3.12-3.55(8H,m),3.98(1H,d,J=16.1Hz),4.19(1H,d,J=2.9Hz), 4.21(1H, d,J=16.1Hz),4.38(1H,d,J=2.9Hz),6.70(1H,broad t), 7.23-7.81(14H,m). |
| 33-f | 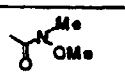 | 1.27-1.76(12H,m),1.49(9H,s),2.68-2.79(4H,m),3.12(3H,s), 3.13-3.52(4H,m),3.61(3H,s),4.18(1H,d,J=2.4Hz),4.22(1H,d, J=15.6 Hz),4.37(1H,d,J=2.4Hz),4.54(1H,d,J=15.6Hz),6.80-6.88(1H,m), 7.21-7.80(14H,m). |
| 33-g | 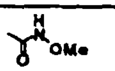 | 1.25-1.77(12H,m),1.50(9H,s),2.75(4H,t,J=7.3Hz),3.20-3.53 (4H,m),3.78(3H,s),4.10(1H,d,J=17.6Hz),4.21(1H,d,J=2.4Hz), 4.25(1H,d,J=17.6Hz),4.42(1H,d,J=2.4Hz),6.75(1H,broad t), 7.25-7.82(14H,m),10.01(1H,s). |
| 33-h | 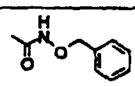 | 1.25-1.75(12H,m),1.47(9H,s),2.74(4H,t,J=8.3Hz),3.00-3.50 (4H, m),4.08(1H,d,J=16.6Hz),4.15(1H,d,J=2.9Hz),4.24 (1H,d,J= 16.6Hz),4.34(1H,d,J=2.9Hz),4.93(1H,d,J=11.2Hz),4.97(1H,d,J= 11.2 Hz),6.65(1H, broad t),7.25-7.80(14H,m),10.01(1H,s). |
| 33-i | 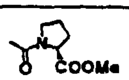 | 1.25-2.20(16H,m),1.47(2.3H,s),1.48(6.7H,s),2.69-2.79(4H,m), 3.05-3.63(6H,m),3.67(3H,s),4.05(0.75H,d,J=13.2Hz),4.44 (0.75H,d,J=13.2Hz),4.08-4.28(1H,m),4.15(0.75H,d,J=2.4Hz), 4.38(0.75H,d, J=2.4Hz),4.47(0.75H,broad dd),4.87(0.25H,broad dd),6.70(0.25H,broad t),6.77(0.75H,broad t),7.23-7.83(14H,m). |
| 33-j | 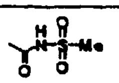 | 1.15-1.80(12H,m),1.49(9H,s),3.70-3.80(4H,m),3.15-3.50 (7H,m),4.10(1H,d,J=16.6Hz),4.22(1H,d,J=16.6Hz),4.22(1H,broad s), 4.44(1H,broad s), 6.65(1H,broad t), 7.20-7.80(14H,m). |
| 33-k | 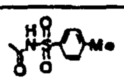 | 1.22-1.80(12H,m),1.48(9H,s),2.38(3H,s),2.65-2.85(4H,m), 3.20-3.52(4H,m),3.89(1H,d,J=17.6Hz),4.08(1H,d,J=17.6Hz), 4.20(1H,d,J=2.9Hz),4.37(1H,d,J=2.9Hz),6.68(1H,broad t), 7.20-8.00(18H,m),10.01(1H,s). |

Example 34

The tert-butyl ester compounds of Example 33 were treated in the same manner as in Example 17, whereby the compounds of Examples 34-a to 34-k were obtained, respectively.

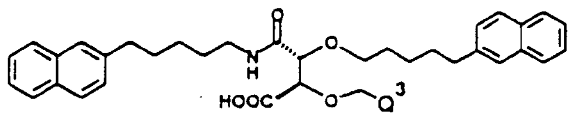

| Example | $Q^3$ | $^1$H-NMR($CDCl_3$) δ : |
|---|---|---|
| 34-a | 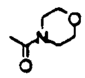 | 1.20-1.80(12H,m),2.68-2.84(4H,m),3.12-3.78(12H,m),4.18(1H, d,J=16.1Hz),4.52(1H,d,J=16.1Hz),4.46(1H,d,J=2.0Hz),4.53(1H, d,J=2.0Hz),6.90(1H,broad t),7.22-7.81(14H,m). |
| 34-b | 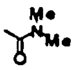 | 1.20-1.78(12H,m),2.72-2.79(4H,broad t),2.79(3H,s),2.98(3H, s),3.18-3.75(4H,m),4.14(1H,d,J=16.6Hz),4.46(1H,broad d), 4.52(1H,d,J=16.6Hz),4.50(1H,broad d),6.97(1H,broad t),7.20-7.82(14H, m). |
| 34-c | 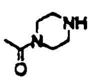 | 1.13-1.78(12H,m),2.55-2.49(4H,m),2.95-4.60(16H,m),7.00-8.00(15H,m) |
| 34-d | 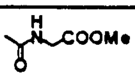 | 1.25-1.78(12H,m),2.75(4H,broad t),3.15-3.60(4H,m), 3.69(3H, s),4.04(2H,d,J=5.4Hz),4.17(1H,d,J=16.6Hz),4.23(1H,d,J= 16.6Hz),4.29(1H,d,J=2.9Hz),4.55(1H,d,J=2.9Hz),6.90(1H, broad t),7.20-7.80(14H,m). |
| 34-e | 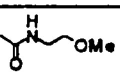 | 1.22-1.75(12H,m),2.70-2.80(4H,m),3.31(3H,s),3.10-3.70(8H;m) 4.11(1H,d,J=16.1Hz),4.21(1H,d,J=16.1Hz),4.31(1H,d,J=2.9Hz), 4.49(1H,d,J=2.9Hz),6.80-6.95(2H,m),7.20-7.80(14H,m). |
| 34-f | 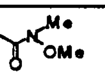 | 1.25-1.77(12H,m),2.70-2.80(4H,m),3.22(3H,s),3.61(3H,s), 3.20-3.75(4H,m),4.25(1H,d,J=17.6Hz),4.45(1H,d,J=1.5Hz), 4.52(1H,d,J=1.5Hz),4.68(1H,d,J=17.6Hz),6.90(1H,broad t),7.20-7.80(14H, m). |
| 33-g | 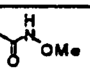 | 1.20-2.25(12H,m),2.62-2.80(4H,m),3.18-3.69(4H,m),3.76(3H, s),4.10-4.76(4H,m),6.85(1H,m),7.20-7.80(14H,m). |
| 34-h | 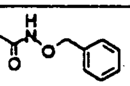 | 1.20-1.75(12H,m),2.65-2.80(4H,m),3.03-3.67(4H,m),4.10-5.15 (6H,m),6.60-6.92(2H,m),7.20-7.80(14H,m). |
| 34-i | 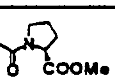 | 1.27-2.23(16H,m),1.47(2.3H,s),2.70-2.80(4H,m),3.18-3.75(6H, m),3.73(2.4H,s),3.78(0.6H,s),4.05-4.57(5H,m),6.80-6.93(1H,m),7.20-7.83(14H,m). |
| 34-j | 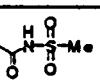 | 0.80-1.80(12H,m),2.70-2.80(4H,m),3.15-3.60(4H,m),4.10-4.30(3H,m),4.50-4.60(1H,broad s),6.75-6.85(1H,m),7.20-7.81(14H,m). |
| 34-k | 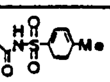 | 1.22-1.78(12H,m),2.33(3H,s),2.65-2.80(4H,m),3.18-3.59(4H, m),4.08(2H,broad s),4.27(1H,broad s),4.54(1H,broad s), 6.82(1H,broad t),7.17-7.95(18H,m),10.41(1H,broad s). |

Example 35

(1) tert-Butyl (2R,3R)-2-benzyloxycarbonylmethoxy-3-[5-(3,4-dimethylphenyl)pentyloxy]-3-[N-methyl-N-[5-(2-naphthyl)pentyl]carbamoyl]propionate

The tert-Butyl (2R,3R)-3-[5-(3,4-dimethylphenyl)pentyloxy]-2-hydroxy-3-[N-methyl-N-[5-(2-naphthyl)pentyl]carbamoyl]propionate (0.19 g) obtained in Example 31-(7) was dissolved in tetrahydrofuran (5 ml), followed by the dropwise addition of potassium bistrimethylsilylamide (0.5M solution in tetrahydrofuran, 0.85 ml) under cooling to -78°C and stirring. After heating to 0°C and stirring for 10 minutes, the reaction mixture was cooled again to -78°C. Benzyl bromoacetate (0.1 ml) was added and the resulting mixture was stirred for 2 hours. To the reaction mixture was added a 10% aqueous solution of hydrochloric acid, followed by extraction with ethyl acetate. The extract was washed with water and then dried over anhydrous sodium sulfate. The solvent was distilled off under reduced pressure. The residue was purified by chromatography on a silica gel column (elution with 20% ethyl acetate-hexane), whereby the title compound (0.22 g) was obtained as a colorless oil.

MS (FAB+) m/Z: 760$(M+Na)^+$.
$^1$H-NMR($CDCl_3$, rotamer mixture)δ: 1.22-1.78(21H,m), 2.18-2.23(6H,m), 2.43-2.79(4H,m), 2.87(0.9H,s), 3.12(2.1H,s), 3.13-3.80(4H,m), 4.05-4.52(4H,m), 5.08-5.18(2H,m), 6.83-7.85(15H,m).

(2) (4R,5R)-tert-Butoxycarbonyl-11-(3,4-dimethylphenyl)-3,6-dioxa-5-[N-methyl-N-[5-(2-naphthyl)pentyl]carbamoyl]undecanoic acid

The benzyl ester (0.22 g) obtained in (1) was dissolved in ethanol (5 ml). To the resulting solution was added 10% palladium-carbon (0.01 g), and the mixture was stirred at room temperature for 5 hours under hydrogen atmosphere. The palladium-carbon was filtered off and the filtrate was dried over anhydrous sodium sulfate. The solvent was then distilled off under reduced pressure, whereby the title compound (0.15 g) was obtained as a colorless oil.

MS (FAB+) m/Z: 670$(M+Na)^+$.
$^1$H-NMR($CDCl_3$, rotamer mixture)δ: 1.25-1.82(21H,m), 2.21(3H,s), 2.45-2.57(2H,m), 2.70-2.82(2H,m), 2.95(2H,s), 3.08(4H,s), 3.20-3.62(4H,m), 4.08-4.65(4H,m), 6.81-7.82(10H,m).

(3) tert-Butyl (2R,3R)-3-[5-(3,4-dimethylphenyl)pentyloxy]-2-(N-methoxycarbamoyl)methoxy-3-[N-methyl-N-[5-(2-naphthyl)pentyl]carbamoyl]propionate

The carboxylic acid (99 mg) obtained in (2), O-methylhydroxylamine hydrochloride (20 mg), 4-dimethylaminopyridine (28 mg) and 1-hydroxybenzotriazole (5 mg) were dissolved in methylene chloride (6 ml). To the resulting solution was added 1-ethyl-3-(3-dimethylaminopropyl)carbodiimide hydrochloride (33 mg), and the mixture was stirred at room temperature for 16 hours. The reaction mixture was concentrated under reduced pressure. To the residue was added dilute hydrochloric acid, and the resulting mixture was extracted with methylene chloride. The extract was washed with saturated saline and dried over anhydrous sodium sulfate. The solvent was then distilled off under reduced pressure. The residue was purified by chromatography on a silica gel column (elution with 75% ethyl acetate-hexane), whereby the title compound (60 mg) was obtained as a colorless oil.

$^1$H-NMR($CDCl_3$, rotamer mixture)δ: 1.10-1.90(12H,m), 2.10-2.30(6H,m), 2.40-2.57(2H,m), 2.66-2.83(2H,m), 2.94(1H,s), 3.09(2H,s), 3.20-3.70(7H,m), 4.00-4.60(4H,m), 6.75-7.82(10H,m), 10.50-10.67(1H,m).

(4) (2R,3R)-3-[5-(3,4-Dimethylphenyl)pentyloxy]-2-(N-methoxycarbamoyl)methoxy-3-[N-methyl-N-[5-(2-naphthyl)pentyl]carbamoyl]propionic acid

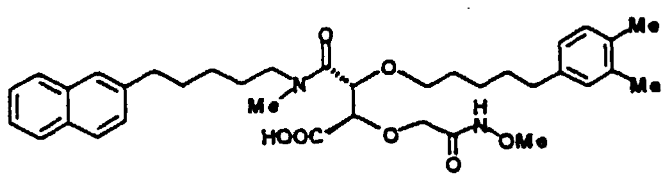

To a solution (2 ml) of the tert-butyl ester (58 mg) obtained in (3) in methylene chloride (2 ml) was added trifluoroacetic acid (1 ml), and the mixture was stirred at room temperature for 12 hours. The reaction mixture was concentrated to dryness under reduced pressure. The residue was purified by high-performance liquid column chromatography ("Sensyu Pak ODS-5251-SH", elution with acetonitrile-1% aqueous trifluoroacetic acid = 8:2 V/V), whereby the title compound (27 mg) was obtained as a colorless oil.

MS (FAB+) m/Z: 621(M+H)$^+$.
$^1$H-NMR(CDCl$_3$, rotamer mixture)δ: 1.20-1.80(12H,m), 2.19-2.24(6H,m), 2.43-2.58(2H,m), 2.70-2.81(2H,m), 2.89-3.12(3H,m), 3.20-3.90(7H,m), 4.08-4.72(4H,m), 6.80-7.86(10H,m).

Example 36

(2R,3R)-3-[5-(3,4-Dichlorophenyl)pentyloxy]-2-ethoxycarbonylmethoxy-3-[N-[5-(2-naphthyl)pentyl]carbamoyl]propionic acid

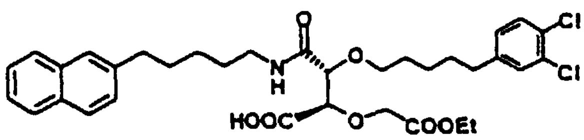

The treatment was carried out in the same manner as in Example 28 except for the use of (3,4-dichlorophenylmethyl)triphenylphosphonium bromide instead of (3,4-dimethylphenylmethyl)triphenylphosphonium chloride, whereby the title compound was obtained as a colorless oil.

(1) tert-Butyl (2R,3R)-3-[5-(3,4-dichlorophenyl)pentyloxy]-2-ethoxycarbonylmethoxy-3-[N-[5-(2-naphthyl)pentyl]carbamoyl]propionate

Mass (EI) m/Z: 701(M)$^+$.
$^1$H-NMR(CDCl$_3$)δ: 1.23(3H,t,J=7.4Hz), 1.49(9H,s), 1.27-1.65(10H,m), 1.65-1.74(2H,m), 2.51(2H,t,J=7.4Hz), 2.76(2H,t,J=7.4Hz), 3.25-3.55(3H,m), 3.90(1H,d,J=6.1Hz), 4.10-4.20(3H,m), 4.30-4.40(2H,m), 6.65(1H,t,J=5.8Hz), 6.95(1H,dd,J=8.3,2.0Hz), 7.21-7.45(6H,m), 7.58(1H,s), 7.73-7.79(3H,m).

(2) (2R,3R)-3-[5-(3,4-Dichlorophenyl)pentyloxy]-2-ethoxycarbonylmethoxy-3-[N-[5-(2-naphthyl)pentyl]carbamoyl]propionic acid

Mass (EI) m/Z: 645(M)$^+$.
$^1$H-NMR(CDCl$_3$)δ: 1.28(3H,t,J=7.3Hz), 1.35-1.80(12H,m), 2.53(2H,t,J=7.3Hz), 2.77(2H,t,J=7.3Hz), 3.28-3.33(2H,m), 3.47-3.60(2H,m), 4.09(1H,d,J=17.6Hz), 4.25(2H,q,J=7.3Hz), 4.30-4.40(2H,m), 4.55(1H,d,J=1.5Hz), 6.75-6.90(1H,m), 6.98(1H,dd,J=8.3,2.0Hz), 7.20-7.35(3H,m), 7.35-7.50(2H,m), 7.58(1H,s), 7.70-7.85(3H,m).

Example 37

Disodium (2R,3R)-2-carboxymethoxy-3-[5-(3,4-dimethylphenyl)pentyloxy]-3-[N-methyl-N-[5-(2-naphthyl)pentyl]carbamoyl]propionate

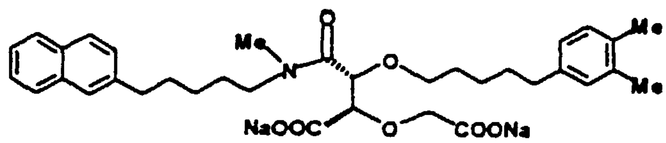

(1) tert-Butyl (2R,3R)-3-benzyloxycarbonyl-2-tert-butoxycarbonyloxy-3-[5-(3,4-dimethylphenyl)pentyloxy]propionate

The benzyl (2R,3R)-3-tert-butoxycarbonyl-2-[5-(3,4-dimethylphenyl)pentyloxy]-3-hydroxypropionate (1.20 g) obtained in Example 31-(3) was dissolved in tetrahydrofuran (12 ml), followed by the dropwise addition of potassium bistrimethylsilylamide (1M solution in toluene, 5.2 ml) over 5 minutes under cooling to -70°C or lower and stirring. After 5 minutes, a solution of tert-butyl bromoacetate (0.64 g) in tetrahydrofuran (3 ml) was added dropwise over 5 minutes. The cooling bath was then taken off. The reaction mixture was stirred for 75 minutes while heating to room temperature and poured into a mixture of 0.5N hydrochloric acid and ethyl acetate. The organic layer was separated, washed with saturated saline and dried over anhydrous sodium sulfate. The solvent was then distilled off under reduced pressure. The residue was purified by chromatography on a silica gel column (elution with 10% ethyl acetate-hexane), whereby the title compound (0.93 g) was obtained as a colorless oil.

MS (FAB+) m/Z: 607$(M+Na)^+$, 585$(M+H)^+$.
$^1$H-NMR($CDCl_3$)δ: 1.32-1.39(2H,m), 1.44(9H,s), 1.47(9H,s), 1.51-1.65(4H,m), 2.21(3H,s), 2.22(3H,s), 2.49(2H,t,J=7.8Hz), 3.28-3.33(1H,m), 3.67-3.73(1H,m), 4.00(1H,d,J=16.6Hz), 4.26(1H,d,J=16.6Hz), 4.36(1H,d,J=2.9Hz), 4.46(1H,d,J=2.9Hz), 5.24(1H,d,J=12.0Hz), 5.27(1H,d,J=12.0Hz), 6.88(1H,d,J=7.6Hz), 6.92(1H,s), 7.02(1H,d,J=7.6Hz), 7.31-7.37(3H,m), 7.41-7.44(2H,m).

(2) (2R,3R)-3-tert-Butoxycarbonyl-3-tert-butoxycarbonylmethoxy-2-[5-(3,4-dimethylphenyl)pentyloxy]propionic acid

In the same manner as in Example 31-(5), the title compound as a colorless oil was prepared from 3-benzyloxycarbonyl compound obtained in (1).

MS (FAB+) m/Z: 495$(M+H)^+$.
$^1$H-NMR($CDCl_3$)δ: 1.27-1.68(24H,m), 2.22(3H,s), 2.23(3H,s), 2.52(2H,t,J=7.6Hz), 3.41-3.50(1H,m), 3.60-3.70(1H,m), 4.00(1H,d,J=16.1Hz), 4.33(1H,d,J=16.1Hz), 4.36(1H,s), 6.88(1H,d,J=7.3Hz), 6.92(1H,s), 7.02(1H,d,J=7.3Hz).

(3) tert-Butyl (2R,3R)-2-tert-butoxycarbonylmethoxy-3-[5-(3,4-dimethylphenyl)pentyloxy]-3-[N-methyl-N-[5-(2-naphthyl)pentyl]carbamoyl]propionate

In the same manner as in Example 31-(6), the title compound (139 mg) as a colorless oil was prepared from the propionic acid (103 mg) obtained in (2).

MS (FAB+) m/Z: 726$(M+Na)^+$.
$^1$H-NMR($CDCl_3$, rotamer mixture)δ: 1.30-1.80(30H,m), 2.15-2.25(6H,m), 2.42-2.54(2H,m), 2.70-2.80(2H,m), 2.91,3.19(total 3H,s each), 3.25-3.63(4H,m), 3.87-4.50(4H,m), 6.80-7.83(10H,m).

(4) Disodium (2R,3R)-2-carboxymethoxy-3-[5-(3,4-dimethylphenyl)pentyloxy]-3-[N-methyl-N-[5-(2-naphthyl)pentyl]carbamoyl]propionate

The diester compound (139 mg) obtained in (3) was dissolved in methylene chloride (3 ml). To the resulting

solution was added trifluoroacetic acid (0.6 ml), and the mixture was stirred at room temperature for 18 hours. The residue obtained by concentration to dryness under reduced pressure was dissolved in ethanol (2 ml). To the resulting solution was added 1N sodium hydroxide (0.4 ml), and the resulting mixture was concentrated to dryness under reduced pressure. The residue was purified by chromatography on a column of "DIAION® HP20" (elution with acetonitrile-containing water), whereby the title compound (76 mg) was obtained as a colorless solid.

MS (FAB+) m/Z: 658$(M+Na)^+$, 636$(M+H)^+$.
$^1$H-NMR($CDCl_3$, rotamer mixture)δ: 1.20-1.82(12H,m), 2.04-2.25(6H,m), 2.40-2.80(4H,m), 2.82-3.14(3H,m), 3.24-4.71(8H,m), 6.72-7.81(10H,m).

| Elementary analysis for $C_{35}H_{43}NNa_2O_7 \cdot 0.4H_2O$ | | | |
|---|---|---|---|
| Calculated: | C, 65.39; | H, 6.87; | N, 2.18. |
| Found: | C, 65.55; | H, 6.97; | N, 2.06. |

Example 38

(2R,3R)-3-[5-(3,4-Dimethylphenyl)pentyloxy]-2-ethoxycarbonylmethoxy-3-[N-methyl-N-[5-(2-naphthyl)pentyl]carbamoyl]propionic acid

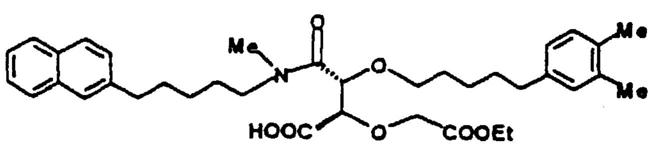

(1) tert-Butyl (2R,3R)-3-[5-(3,4-dimethylphenyl)pentyloxy]-2-ethoxycarbonylmethoxy-3-[N-methyl-N-[5-(2-naphthyl)pentyl]carbamoyl]propionate

The 2-hydroxy compound (204 mg) obtained in Example 31-(7) was dissolved in tetrahydrofuran (6 ml), followed by the dropwise addition of potassium bistrimethylsilylamide (0.5M solution in tetrahydrofuran, 0.8 ml) under cooling to -78°C and stirring. After stirring for 20 minutes, ethyl bromoacetate (0.1 ml) was added. The ice bath was taken off and the reaction mixture was warmed to 0°C. The reaction mixture was added with 10% hydrochloric acid and then extracted with ethyl acetate. The extract was washed successively with a saturated aqueous solution of sodium bicarbonate and saturated saline and then, dried over anhydrous sodium sulfate. The solvent was distilled off under reduced pressure. The residue was purified by chromatography on a silica gel column (elution with 33% ethyl acetate-hexane), whereby the title compound (203 mg) was obtained as a colorless oil.

MS (FAB+) m/Z: 698$(M+Na)^+$, 620$(M\text{-tert-Bu}+H)^+$.
$^1$H-NMR($CDCl_3$, rotamer mixture)δ: 1.22-1.27(3H,m), 1.31-1.41(4H,m), 1.45,1.47(total 9H,s each), 1.52-1.64(6H,m), 1.67-1.76(2H,m), 2.20(3H,s), 2.21(3H,s), 2.48-2.52(2H,m), 2.74-2.79(2H,m), 2.90,3.13(total 3H,s each), 3.25-3.66(4H,m), 4.07(1H,dd,J=16.6Hz), 4.14-4.21(2H,m), 4.30-4.33(1H,m), 4.36,4.39(total 1H,d each,J=16.6/16.6Hz), 4.48,4.51(total 1H,d each,J=4.9,5.9Hz), 6.87(1H,d,J=7.3Hz), 6.92(1H,s), 7.01(1H,d,J=7.3Hz), 7.31(1H,dd,J=8.3,2.0Hz), 7.58(1H,s), 7.74-7.79(3H,m).

(2) (2R,3R)-3-[5-(3,4-Dimethylphenyl)pentyloxy]-2-ethoxycarbonylmethoxy-3-[N-methyl-N-[5-(2-naphthyl)pentyl]carbamoyl]propionic acid

In the same manner as in Example 28-2, the title compound (180 mg) as a colorless oil was prepared from the

diester compound (197 mg) obtained above.

MS (FAB+) m/Z: 642$(M+Na)^+$, 620$(M+H)^+$.

$^1$H-NMR($CDCl_3$, rotamer mixture)δ: 1.25-1.42(7H,m), 1.52-1.61(6H,m), 1.70-1.79(2H,m), 2.20(3H,s), 2.22(3H,s), 2.48-2.53(2H,m), 2.74-2.80(2H,m), 2.96,3.11(total 3H,s each), 3.30-3.50(4H,m), 4.22,4.25(total 2H,q each, J=7.3/7.3Hz), 4.351,4.357(total 2H,s each), 4.42,4.44(total 1H,d each,J=6.1/6.1Hz), 4.66,4.70(total 1H,d each,J=6.1/6.1Hz), 6.87-6.89(1H,m), 6.92(1H,s), 7.01(1H,d,J=7.3Hz), 7.30(1H,d,J=8.3Hz), 7.37-7.46(2H,m), 7.58(1H,s), 7.74-7.80(3H,m).

Example 39

Disodium (2R,3R)-2-carboxymethoxy-3-[5-(3,4-dichlorophenyl)pentyloxy]-3-[N-methyl-N-[5-(2-naphthyl)pentyl]carbamoyl]propionate

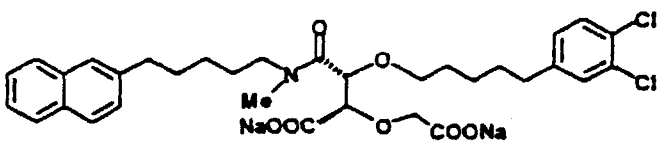

(1) tert-Butyl (2R,3R)-2-ethoxycarbonylmethoxy-3-[N-methyl-N-[5-(2-naphthyl)pentyl]carbamoyl]-3-(4-oxobutoxy)propionate

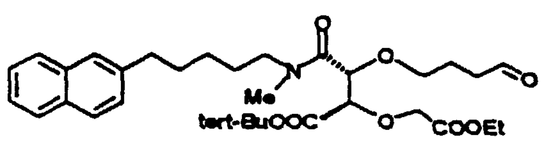

In the same manner as in Example 18-(6), the title compound as a colorless oil was prepared from the tert-butyl (2R,3R)-2-ethoxycarbonylmethoxy-3-(4-hydroxybutoxy)-3-(N-methyl-N-[5-(2-naphthyl)pentyl]carbamoyl]propionate obtained in Example 32-(2).

$^1$H-NMR($CDCl_3$)δ: 1.25(3H,t,J=7.1Hz), 1.33-1.41(2H,m), 1.44,1.47(total 9H,s each), 1.55-1.66(1H,m), 1.70-1.77(2H,m), 1.82-1.88(3H,m), 2.50(2H,t,J=7.3Hz), 2.74-2.81(2H,m), 2.90,3.14(total 3H,s each), 3.25-3.63(3H,m), 3.73-3.76(1H,m), 4.10(0.9H,d,J=16.1Hz), 4.13-4.20(2.1H,m), 4.31-4.39(2H,m), 4.48-4.51(1H,m), 7.32(1H,d,J=8.3Hz), 7.38-7.46(2H,m), 7.59(1H,s), 7.74-7.80(3H,m), 9.71,9.73(total 1H,s each).

(2) tert-Butyl (2R,3R)-3-[5-(3,4-dichlorophenyl)pentyloxy]-2-ethoxycarbonylmethoxy-3-[N-methyl-N-[5-(2-naphthyl)pentyl]carbamoyl]propionate

In tetrahydrofuran (25 ml), (3,4-dichlorophenylmethyl)triphenylphosphonium bromide (530 mg) was dissolved, followed by the dropwise addition of potassium bistrimethylsilylamide (0.5M solution in tetrahydrofuran, 0.84 ml) under cooling to -78°C and stirring. After stirring for 5 minutes, a solution of the 3-(4-oxobutoxy) compound (300 mg) obtained in (1) in tetrahydrofuran (5 ml) was added dropwise to the reaction mixture. The resulting mixture was then stirred for 4 hours while warming to room temperature. The reaction mixture was diluted with ethyl acetate, washed successively with water and saturated saline and dried over anhydrous magnesium sulfate. The solvent was then distilled off under reduced pressure. The residue was purified by chromatography on a silica gel column (elution with 33% ethyl acetate-hexane), whereby tert-butyl (2R,3R)-3-[5-(3,4-dichlorophenyl)pent-4-en-1-yloxy]-2-ethoxycarbonylmethoxy-3-[N-methyl-N-[5-(2-naphthyl)pentyl]carbamoyl]propionate (217 mg) was obtained as a colorless oil.

MS (FAB+) m/Z: 736$(M+Na)^+$, 714$(M+H)^+$.
$^1$H-NMR ($CDCl_3$, rotamer mixture)δ: 1.17-1.27(3H,m), 1.27-1.80(17H,m), 2.18-2.38(2H,m), 2.70-2.80(2H,m), 2.90,2.91,3.13,3.17(total 3H,s each), 3.21-3.68(4H,m), 4.02-4.42(5H,m), 4.44-4.55(1H,m), 5.60-5.70,6.10-6.30(total 2H,m each), 7.00-7.82(10H,m).

The resulting compound (217 mg) was dissolved in tetrahydrofuran (40 ml). To the resulting solution was added 10% palladium-carbon (20 mg), and the mixture was stirred for 3 hours under hydrogen atmosphere. The palladium-carbon was filtered off and the filtrate was concentrated to dryness under reduced pressure. The residue was purified by chromatography on a silica gel column (elution with 50% ethyl acetate-hexane), whereby the title compound (168 mg) was obtained as a colorless oil.

MS (FAB+) m/Z: 716$(M+H)^+$.
$^1$H-NMR($CDCl_3$, rotamer mixture)δ: 1.20-1.80(24H,m), 2.45-2.56(2H,m), 2.70-2.80(2H,m), 2.91,3.16(total 3H,s each), 3.23-3.63(4H,m), 4.01-4.23(3H,m), 4.28-4.52(3H,m), 6.90-7.00(1H,m), 7.16-7.32(3H,m), 7.35-7.47(2H,m), 7.58(1H,s), 7.72-7.83(3H,m).

(3) (2R,3R)-3-[5-(3,4-dichlorophenyl)pentyloxy]-2-ethoxycarbonylmethoxy-3-[N-methyl-N-[5-(2-naphthyl)pentyl]carbamoyl]propionic acid

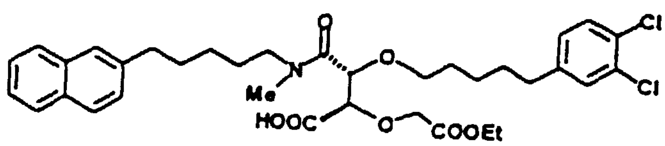

The diester compound (168 mg) obtained in (2) was dissolved in methylene chloride (5 ml). To the resulting solution was added trifluoroacetic acid (0.5 ml), and the mixture was stirred at room temperature for 8 hours. The reaction mixture was concentrated to dryness under reduced pressure, whereby the title compound (161 mg) was obtained as a colorless oil.

MS (FAB+) m/Z: 682$(M+Na)^+$, 660$(M+H)^+$.
$^1$H-NMR($CDCl_3$, rotamer mixture)δ: 1.19-1.81(15H,m), 2.45-2.60(2H,m), 2.70-2.80(2H,m), 2.97,3.15(total 3H,s each), 3.26-3.53(4H,m), 4.16-4.41(4H,m), 4.45-4.50(1H,m), 4.64,4.66(total 1H,d each,J=6.3Hz), 6.90-7.00(1H,m), 7.20-8.00(9H,m).

(4) Disodium (2R,3R)-2-carboxymethoxy-3-[5-(3,4-dichlorophenyl)pentyloxy]-3-[N-methyl-N-[5-(2-naphthyl)pentyl]carbamoyl]propionate

The monoester compound (57 mg) obtained in (3) was dissolved in tetrahydrofuran (3 ml). To the resulting solution was added 1N sodium hydroxide (0.18 ml), and the mixture was stirred at room temperature for 16 hours. The solvent was then distilled off under reduced pressure. The residue was purified by chromatography on the column of "DIAION® HP-20" (elution with acetonitrile-containing water), whereby the title compound (55 mg) was obtained as a colorless solid.

MS (FAB+) m/Z: 698$(M+Na)^+$, 676$(M+H)^+$.
$^1$H-NMR($CD_3OD$, rotamer mixture)δ: 1.13-1.73(12H,m), 2.42-2.52(2H,m), 2.63-2.73(2H,m), 2.83,3.02(total 3H,s each), 3.20-3.45(4H,m), 3.50-3.60(1H,m), 3.79,3.82(total 1H,broad s each), 4.02-4.12(1H,m), 4.55-4.61(1H,m), 6.95-7.03(1H,m), 7.21-7.35(4H,m), 7.52(1H,s), 7.61-7.70(3H,m).

| Elementary analysis for $C_{33}H_{37}Cl_2NNa_2O \cdot 1.5H_2O$ | | | | |
|---|---|---|---|---|
| Calculated: | C, 56.34; | H, 5.73; | Cl, 10.08; | N, 1.99. |
| Found: | C, 56.12; | H, 5.80; | Cl, 10.30; | N, 1.74. |

Example 40

(2R,3R)-3-[5-(3,4-Dichlorophenyl)pentyloxy]-2-hydroxy-3-[N-methyl-N-[5-(2-naphthyl)pentyl]carbamoyl]propionic acid

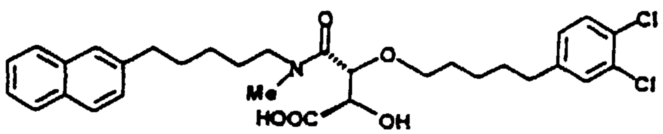

(1) tert-Butyl (2R,3R)-3-benzyloxycarbonyl-2-hydroxy-3-(4-hydroxybutoxy)propionate

The benzyl (2R,3R)-3-tert-butoxycarbonyl-2-[4-(tert-butyldiphenylsilyloxy)butoxy]-3-hydroxypropionate (657 mg) obtained in Example 18-(3) was dissolved in tetrahydrofuran (5 ml). To the resulting solution was added acetic acid (0.1 ml) and tetrabutylammonium fluoride (1M solution in tetrahydrofuran, 1.5 ml) under cooling with ice water, and the mixture was stirred at room temperature for 24 hours. The solvent was then distilled off under reduced pressure. The residue was purified by chromatography on a silica gel column (elution with 75% ethyl acetate-hexane), whereby the title compound (377 mg) was obtained as a colorless oil.

MS (EI) m/Z: 368($M^+$).
$^1$H-NMR($CDCl_3$)δ: 1.49(9H,s), 1.51-1.72(6H,m), 3.37-3.41(1H,m), 3.62-3.65(2H,m), 3.76-3.80(1H,m), 4.25(1H,d,J=2.5Hz), 4.43-4.60(1H,m), 5.22(1H,d,J=12.2Hz), 5.28(1H,d,J=12.2Hz), 7.30-7.45(5H,m).

(2) tert-Butyl (2R,3R)-3-benzyloxycarbonyl-2-hydroxy-3-(4-oxobutoxy)propionate

In the same manner as in Example 18-(6), the slightly crude title compound (2.63 g) was prepared from the alcohol compound (3.75 g) obtained in (1).

MS (EI) m/Z: 367($M^+$+1).
$^1$H-NMR($CDCl_3$)δ: 1.49(9H,s), 1.80-1.97(2H,m), 2.52-2.62(2H,m), 3.09(1H,d,J=8.3Hz), 3.35-3.48(1H,m), 3.65-3.78(1H,m), 4.24(1H,d,J=2.5Hz), 4.50(1H,dd,J=7.8,2.5Hz), 5.20(1H,d,J=12.2Hz), 5.25(1H,d,J=12.2Hz), 7.30-7.45(5H,m), 9.73(1H,s).

(3) Benzyl (2R,3R)-3-tert-butoxycarbonyl-2-[5-(3,4-dichlorophenyl)pent-4-en-1-yloxy]-3-hydroxypropionate

The 3-(4-oxobutoxy) compound (2.63 g) obtained in (2) was subjected to Wittig reaction in the same manner as in Example 39-(2), whereby the title compound (2.82 g) was obtained as a colorless oil.

MS (EI) m/Z: 508($M^+$).
$^1$H-NMR($CDCl_3$)δ: 1.47(9H,s), 1.49(9H,s), 1.69-1.80(2H,m), 2.25-2.45(2H,m), 3.09(1H,m), 3.25-3.45(1H,m), 3.72-3.84(1H,m), 4.22-4.27(1H,m), 4.46-4.52(1H,m), 5.15-5.35(2H,m), 5.65-5.75(1H,m), 6.10-6.36(1H,m), 7.05-7.50(8H,m).

(4) (2R,3R)-3-Benzyloxycarbonyl-2-(tert-butyldimethylsilyloxy)-3-[5-(3,4-dichlorophenyl)pent-4-en-1-yloxy]propionate

In the same manner as in Example 31-(4), the title compound (3.42 g) as a colorless oil was prepared from the hydroxy compound (2.82 g) obtained in (3).

MS (EI) m/Z: 622($M^+$).
$^1$H-NMR($CDCl_3$)δ: 0.01(3H,s), 0.11(3H,s), 0.77(9H,s), 0.78(9H,s), 1.34,1.36(total 9H,s each), 1.55-1.75(2H,m), 2.05-2.30(2H,m), 3.30-3.3.45(1H,m), 3.55-3.70(1H,m), 4.16-4.19(1H,m), 4.43-4.46(1H,m), 4.98-5.02(1H,m), 5.65-5.75(1H,m), 6.05-6,25(1H,m), 6.95-7.40(8H,m).

(5) (2R,3R)-3-tert-Butoxycarbonyl-3-(tert-butyldimethylsilyloxy)-2-[5-(3,4-dichlorophenyl)pentyloxy]propionic acid

In the same manner as in Example 31-(5), the title compound (1.50 g) was prepared from the diester compound (1.80 g) obtained in (4).

MS (EI) m/Z: 535($M^+$+1).
$^1$H-NMR($CDCl_3$)δ: 0.08(3H,s), 0.10(3H,s), 0.76(10H,s), 1.20-1.30(2H,m), 1.36(9H,s), 1.46-1.60(4H,m), 2.43(2H,t,J=7.3Hz), 3.35-3.45(1H,m), 3.46-3.52(1H,m), 4.17(1H,d,J=2.9Hz), 4.39(1H,d,J=2.9Hz), 6.87(1H,m), 7.08-7.18(2H,m).

(6) tert-Butyl (2R,3R)-2-(tert-butyldimethylsilyloxy)-3-[5-3,4-dichlorophenyl)pentyloxy]-3-[N-methyl-N-[5-(2-naphthyl)pentyl]carbamoyl]propionate

In the same manner as in Example 31-(6), the title compound (1.46 g) as a colorless oil was prepared from the propionic acid (1.50 g) obtained in (5).

MS (EI) m/Z: 744($M^+$+1).
$^1$H-NMR($CDCl_3$, rotamer mixture)δ: 0.08(3H,s), 0.14(3H,s), 0.86(9H,s), 1.15-1.40(4H,m), 1.41(9H,s), 1.45-1.65(8H,m), 1.65-1.80(2H,m), 2.40-2.55(2H,m), 2.65-2.75(2H,m), 2.85(3H,s), 3.10(3H,s), 3.20-3.60(2H,m), 4.25-4.50(2H,m), 6.85-6.95(1H,m), 7.15-7.30(3H,m), 7.35-7.45(2H,m), 7.56(1H,s), 7.60-7.70(3H,m).

(7) tert-Butyl (2R,3R)-3-[5-(3,4-dichlorophenyl)pentyloxy]-2-hydroxy-3-[N-methyl-N-[5-(2-naphthyl)pentyl]carbamoyl]propionate

In the same manner as in Example 31-(7), the title compound (1.02 g) as a colorless oil was prepared from the amide compound (1.32 g) obtained in (6).

MS (EI) m/Z: 629($M^+$).
$^1$H-NMR($CDCl_3$, rotamer mixture)δ: 1.15-1.80(23H,m), 2.40-2.55(2H,m), 2.70-2.85(2H,m), 2.93(3H,s), 3.11(3H,s), 3.20-3.40(4H,m), 4.25-4.50(2H,m), 6.90-7.00(1H,m), 7.15-7.50(5H,m), 7.59(1H,s), 7.75-7.80(3H,m).

(8) (2R,3R)-3-[5-(3,4-Dichlorophenyl)pentyloxy]-2-hydroxy-3-[N-methyl-N-[5-(2-naphthyl)pentyl]carbamoyl]propionic acid

In the same manner as in Example 31-(8), the title compound (300 mg) as a colorless oil was prepared from the tert-butyl ester compound (353 mg) obtained in (7).

MS (FAB+) m/Z: 574$(M+H)^+$.
$^1$H-NMR($CDCl_3$, rotamer mixture)δ: 1.25-1.85(14H,m), 2.45-2.60(2H,m), 2.75-2.85(2H,t,J=7.8Hz), 2.97(3H,s), 3.07(3H,s), 3.30-3.50(3H,m), 4.34-4.38(1H,m), 4.60-4.62(1H,m), 6.90-7.00(1H,m), 7.20-7.35(4H,m), 7.35-7.60(2H,m), 7.58(1H,s), 7.72-7.80(3H,m).

Example 41

(2R,3R)-2-Carboxymethoxy-3-[N-[5-(3-chlro-4-methylphenylamino)pentyl]carbamoyl-3-[5-(3,4-dimethylphenyl)pentyloxy]propionic acid

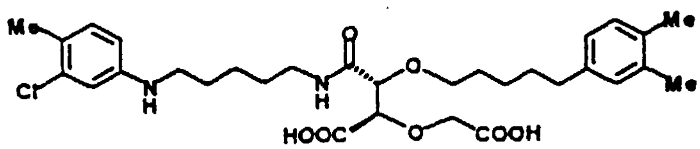

(1) tert-Butyl (2R,3R)-2-tert-butoxycarbonylmethoxy-3-[5-(3,4-dimethylphenyl)pentyloxy]-3-[N-(5-hydroxypentyl)carbamoyl]propionate

The (2R,3R)-3-tert-butoxycarbonyl-3-tert-butoxycarbonylmethoxy-2-[5-(3,4-dimethylphenyl)pentyloxy]propionic acid (174 mg) obtained in Example 37-(2), 5-hydroxypentylamine (45 mg) and 4-dimethylaminopyridine (54 mg) were dissolved in methylene chloride (10 ml). To the resulting solution was added 1-ethyl-3-(3-dimethylaminopropyl)carbodiimide hydrochloride (84 mg), and the mixture was stirred at room temperature for 18 hours. The reaction mixture was diluted with methylene chloride, washed successively with 1N hydrochloric acid and saturated saline and dried over anhydrous sodium sulfate. The solvent was then distilled off under reduced pressure. The residue was purified by chromatography on a silica gel column (elution with 2 to 3% methanol-methylene chloride), whereby the title compound (130 mg) was obtained as a pale yellow oil.

MS (FAB+) m/Z: 580$(M+H)^+$.
$^1$H-NMR($CDCl_3$)δ: 1.26-1.65(12H,m), 1.44(9H,s), 1.50(9H,s), 2.22(3H,s), 2.23(3H,s), 2.52(2H,t,J=7.6Hz), 3.26(1H,m), 3.37(1H,m), 3.43(1H,m), 3.53(1H,m), 3.62(2H,dd,J=11.7,5.9Hz), 3.88(1H,d,J=15.9Hz), 4.18(1H,d,J=2.0Hz), 4.27(1H,d,J=15.9Hz), 4.32(1H,d,J=2.0Hz), 6.76(1H,t,J=5.9Hz), 6.88(1H,d,J=7.6Hz), 6.93(1H,s), 7.03(1H,d,J=7.6Hz).

(2) tert-Butyl (2R,3R)-2-tert-butoxycarbonylmethoxy-3-[N-[5-[N-(3-chloro-4-methylphenyl)-N-(2,4-dinitrophenylsulfonyl)amino]pentyl]carbamoyl]-3-[5-(3,4-dimethylphenyl)pentyloxy]propionate

The alcohol compound (130 mg) obtained in (1) was treated with N-(3-chloro-4-methylphenyl)-2,4-dinitrobenzenesulfonamide, triphenylphosphine and diethyl azodicarboxylate in the same manner as in Example 25, whereby the title compound (80 mg) was obtained as a yellow oil.

MS (FAB+) m/Z: 933$(M+H)^+$.
$^1$H-NMR($CDCl_3$)δ: 1.24-1.64(12H,m), 1.42(9H,s), 1.50(9H,s), 2.22(3H,s), 2.23(3H,s), 2.37(3H,s), 2.51(2H,t,J=7.8Hz), 3.26(2H,m), 3.43(1H,m), 3.52(1H,m), 3.75(2H,t,J=7.1Hz), 3.88(1H,d,J=15.6Hz), 4.17(1H,d,J=2.0Hz), 4.25(1H,d,J=15.6Hz), 4.32(1H,d,J=2.0Hz), 6.73(1H,t,J=5.9Hz), 6.87(1H,d,J=7.8Hz), 6.92(1H,s), 7.00(2H,m), 7.20(2H,m), 7.74(1H,d,J=8.8Hz), 8.29(1H,dd,J=8.8,2.0Hz), 8.44(1H,d,J=2.4Hz).

(3) tert-Butyl (2R,3R)-2-tert-butoxycarbonylmethoxy-3-[N-[5-(3-chloro-4-methylphenylamino)pentyl]carbamoyl]-3-[5-(3,4-dimethylphenyl)pentyloxy]propionate

The 2,4-dinitrophenylsulfonyl compound (80 mg) obtained in (2) was treated with thioglycolic acid and triethylamine in the same manner as in Example 25, whereby the title compound (29 mg) was obtained as a pale yellow oil.

MS (FAB+) m/Z: 703$(M+H)^+$.
$^1$H-NMR($CDCl_3$)δ: 1.26-1.73(12H,m), 1.43(9H,s), 1.50(9H,s), 2.22(3H,s), 2.23(6H,s), 2.51(2H,t,J=7.8Hz), 3.05(2H,t,J=7.1Hz), 3.27(1H,m), 3.35(1H,m), 3.43(1H,m), 3.51(1H,m), 3.63(1H,broad), 3.88(1H,d,J=5.9Hz), 4.18(1H,d,J=2.4Hz), 4.27(1H,d,J=15.9Hz), 4.32(1H,d,J=2.4Hz), 6.40(1H,dd,J=8.3,2.4Hz), 6.58(1H,d,J=2.4Hz), 6.76(1H,t,J=5.9Hz), 6.87(1H,d,J=7.6Hz), 6.92(1H,s), 6.96(1H,d,J=8.3Hz), 7.02(1H,d,J=7.6Hz).

(4) (2R,3R)-2-Carboxymethoxy-3-[N-(5-(3-chloro-4-methylphenylamino)pentyl]carbamoyl]-3-[5-(3,4-dimethylphenyl)pentyloxy]propionic acid

In the same manner as in Example 26, the title compound (18 mg) as a pale yellow oil was prepared from the diester compound (29 mg) obtained in (3).

MS (FAB+) m/Z: 591(M+H)$^+$.
$^1$H-NMR($CDCl_3$)δ: 1.26-1.69(12H,m), 2.20(6H,s), 2.34(3H,s), 2.47(2H,m), 3.04-3.49(6H,m), 4.25-4.54(4H,m), 5.49(3H,broad), 6.86-7.49(7H,m).

Example 42

(2R,3R)-2-Carboxymethoxy-3-[N-[5-(3-chloro-4-methylphenylamino)pentyl]-N-methylcarbamoyl]-3-[5-(3,4-dimethylphenyl)pentyloxy]propionic acid

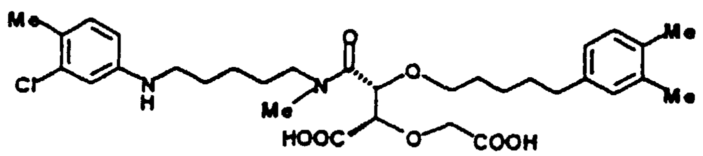

(1) tert-Butyl (2R,3R)-2-tert-butoxycarbonylmethoxy-3-[5-(3,4-dimethylphenyl)pentyloxy]-3-[N-(5-hydroxypentyl)-N-methylcarbamoyl]propionate

The treatment was carried out in the same manner as in Example 41-(1) except for the use of (5-hydroxypentyl)methylamine (47 mg) of Referential Example 15 instead of 5-hydroxypentylamine, whereby the title compound (110 mg) was obtained.

MS (FAB+) m/Z: 594(M+H)$^+$.
$^1$H-NMR($CDCl_3$, rotamer mixture)δ: 1.25-1.72(12H,m), 1.45(9H,s), 1.47(9H,s), 2.22(3H,s), 2.23(3H,s), 2.51(2H,t,J=7.8Hz), 2.91,3.20(total 3H,s each), 3.35-3.65(6H,m), 3.94-4.52(4H,m), 6.88(1H,d,J=7.3Hz), 6.93(1H,s), 7.02(1H,d,J=7.3Hz).

(2) tert-Butyl (2R,3R)-2-tert-butoxycarbonylmethoxy-3-[N-[5-[N-(3-chloro-4-methylphenyl)-N-(2,4-dinitrophenylsulfonyl)amino]pentyl-N-methylcarbamoyl]-3-[5-(3,4-dimethylphenyl)pentyloxy]propionate

The alcohol compound (100 mg) obtained in (1) was treated with N-(3-chloro-4-methylphenyl)-2,4-dinitrobenzenesulfonamide, triphenylphosphine and diethyl azodicarboxylate in the same manner as in Example 25, whereby the title compound (101 mg) was obtained as a yellow oil.

MS (FAB+) m/Z: 947(M+H)$^+$.
$^1$H-NMR($CDCl_3$, rotamer mixture)δ: 1.26-1.61(12H,m), 1.43(9H,s), 1.47(9H,s), 2.22(3H,s), 2.23(3H,s), 2.37(3H,s), 2.51(2H,t,J=7.6Hz), 2.91,3.18(total 3H,s each), 3.20-3.77(6H,m), 3.93-4.49(4H,m), 6.87(1H,d,J=7.3Hz), 6.92(1H,s), 6.98-7.03(2H,m), 7.20(2H,d,J=7.8Hz), 7.74(1H,d,J=8.6Hz), 8.30(1H,dd,J=8.6,2.1Hz), 8.44(1H,d,J=2.1Hz).

(3) tert-Butyl (2R,3R)-2-tert-butoxycarbonylmethoxy-3-[N-[5-(3-chloro-4-methylphenylamino)pentyl]-N-methylcarbamoyl]-3-[5-(3,4-dimethylphenyl)pentyloxy]propionate

The 2,4-dinitrophenylsulfonyl compound (101 mg) obtained in (2) was treated with thioglycolic acid and triethylamine in the same manner as in Example 25, whereby the title compound (35 mg) was obtained as a pale yellow oil.

MS (FAB+) m/Z: 717(M+H)$^+$.

$^1$H-NMR($CDCl_3$, rotamer mixture)δ: 1.29-1.62(12H,m), 1.44(9H,s), 1.47(9H,s), 2.22(3H,s), 2.23(6H,s), 2.50(2H,t,J=7.8Hz), 2.91,3.19(total 3H,s each), 3.02-3.61(6H,m), 3.66(1H,broad), 3.92-4.49(4H,m), 6.40(1H,dd,J=8.3,2.0Hz), 6.59(1H,t,J=2.7Hz), 6.87(1H,d,J=7.8Hz), 6.92(1H,s), 6.96(1H,d,J=8.3Hz), 7.02(1H,d,J=7.8Hz).

(4) (2R,3R)-2-carboxymethoxy-3-[N-[5-(3-chloro-4-methylphenylamino)pentyl]-N-methylcarbamoyl]-3-[5-(3,4-dimethylphenyl)pentyloxy]propionic acid

In the same manner as in Example 26, the title compound (25 mg) as a pale yellow amorphous substance was prepared from the diester compound (35 mg) obtained in (3).

MS (FAB+) m/Z: 605$(M+H)^+$.
$^1$H-NMR($CD_3OD$, rotamer mixture)δ: 1.24-1.70(12H,m), 2.19(3H,s), 2.21(3H,s), 2.35(3H,s), 2.49(2H,m), 2.93,3.17(total 3H,s each), 3.17-3.67(6H,m), 4.11-4.30(4H,m), 4.44(1H,broad), 4.50(1H,broad), 4.64(1H,broad), 6.83-7.35(6H,m).

| Elementary analysis for $C_{32}H_{45}ClN_2O_7 \cdot CF_3CO_2H$ | | | |
|---|---|---|---|
| Calculated: | C, 56.78; | H, 6.45; | N, 3.90. |
| Found: | C, 56.48; | H, 6.78; | N, 3.93. |

Example 43

(2R,3R)-3-[4-(3-Chloro-4-methylphenylamino)butoxy]-2-hydroxy-3-[N-methyl-[N-[5-(2-naphthyl)pentyl]carbamoyl]propionic acid

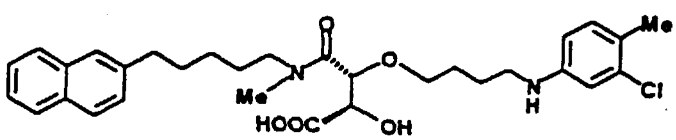

(1) tert-Butyl (2R,3R)-3-[4-(tert-butyldiphenylsilyloxy)butoxy]-2-hydroxy-3-[N-methyl-[N-[5-(2-naphthyl)pentyl]carbamoyl]propionate

The benzyl (2R,3R)-3-tert-butoxycarbonyl-2-[4-(tert-butyldiphenylsilyloxy)butoxy]-3-hydroxypropionate (1.0 g) obtained in Example 18-(3) was dissolved in ethanol (50 ml). To the resulting solution was added 10% palladium-carbon (0.1 g), and the mixture was stirred at room temperature for 10 hours under hydrogen atmosphere. The palladium-carbon was filtered off and the filtrate was concentrated to dryness under reduced pressure, whereby (2R,3R)-3-tert-butoxycarbonyl-2-[4-(tert-butyldiphenylsilyloxy)butoxy]-3-hydroxypropionic acid (0.994 g) was obtained as a colorless oil.

MS(FAB+) m/Z: 539 $(M+Na)^+$, 517$(M+H)^+$.

The resulting propionic acid (0.994 g) was dissolved in methylene chloride (30 ml). To the resulting solution were added the methyl [5-(2-naphthyl)pentyl]amine hydrochloride (761 mg) of Referential Example 14, triethylamine (0.4 ml), 1-hydroxybenzotriazole (260 mg) and 1-ethyl-3-(3-dimethylaminopropyl)carbodiimide hydrochloride (553 mg) under cooling with ice water, and the mixture was stirred at room temperature for 14 hours. The reaction mixture was washed with water and then dried over anhydrous sodium sulfate. The solvent was distilled off under reduced pressure. The residue was purified by chromatography on a silica gel column (elution with 33% ethyl acetate-hexane), whereby

the title compound (890 mg) was obtained as a colorless oil.

MS (FAB+) m/Z: 726(M+H)$^+$.
$^1$H-NMR($CDCl_3$, rotamer mixture)δ: 1.03(9H,s), 1.30-1.79(19H,m), 2.77(2H,t,J=7.6Hz), 2.93,3.12(total 3H,s each), 3.24-3.67(6H,m), 4.25-4.44(2H,m), 7.27-7.82(17H,m).

(2) tert-Butyl (2R,3R)-2-hydroxy-3-(4-hydroxybutoxy)-3-(N-methyl-[N-[5-(2-naphthyl)pentyl]carbamoyl]propionate

In the same manner as in Example 18-(5), the title compound (494 mg) as a colorless oil was prepared from the 4-(tert-butyldiphenylsilyloxy)butoxy compound obtained in (1).

MS (FAB+) m/Z: 510(M+Na)$^+$, 488(M+H)$^+$.
$^1$H-NMR($CDCl_3$, rotamer mixture)δ: 0.94(1H,t,J=7.3Hz), 1.28-1.82(19H,m), 2.67-2.85(3H,m), 2.94,3.11(total 3H,s each), 3.26-3.68(6H,m), 4.25-4.47(2H,m), 7.26-7.84(7H,m).

(3) tert-Butyl (2R,3R)-3-[4-[N-(3-chloro-4-methylphenyl)-N-(2,4-dinitrophenylsulfonyl)amino]butoxy]-2-hydroxy-3-[N-methyl-[N-[5-(2-naphthyl)pentyl]carbamoyl]propionate

The alcohol compound (398 mg) obtained in (2) was treated with N-(3-chloro-4-methylphenyl)-2,4-dinitrobenzenesulfonamide, triphenylphosphine and diethyl azodicarboxylate in the same manner as in Example 25, whereby the title compound (482 mg) was obtained as a pale yellow oil.

MS (FAB+) m/Z: 841(M+H)$^+$.
$^1$H-NMR($CDCl_3$, rotamer mixture)δ: 1.29-1.80(19H,m), 2.72-2.83(2H,m), 2.94,3.09(total 3H,s each), 3.22-3.58(4H,m), 3.68-3.83(2H,m), 4.25-4.44(2H,m), 6.97(1H,d,J=8.3Hz), 7.15-7.82(10H,m), 8.27(1H,d,J=8.8Hz), 8.41(1H,s).

(4) tert-Butyl (2R,3R)-3-[4-(3-chloro-4-methylphenylamino)butoxy-2-hydroxy-3-[N-methyl-[N-[5-(2-naphthyl)pentyl]carbamoyl]propionate

The 2,4-dinitrophenylsulfonyl compound (582 mg) obtained in (3) was treated with thioglycolic acid and triethylamine in the same manner as in Example 25, whereby the title compound (348 mg) was obtained as a pale yellow oil.

MS (FAB+) m/Z: 633(M+Na)$^+$, 611(M+H)$^+$.
$^1$H-NMR($CDCl_3$, rotamer mixture)δ: 1.31-1.81(19H,m), 2.23(3H,s), 2.77(2H,t,J=6.8Hz), 2.94,3.11(total 3H,s each), 2.98-3.08(2H,m), 3.24-3.71(5H,m), 4.25-4.44(2H,m), 6.34-6.43(1H,m), 6.58(1H,s), 6.96(1H,d,J=8,3Hz), 7.31(1H,d,J=8.3Hz), 7.35-7.51(2H,m), 7.58(1H,s), 7.71-7.80(3H,m).

(5) (2R,3R)-3-[4-(3-Chloro-4-methylphenylamino)butoxy-2-hydroxy-3-[N-methyl-[N-[5-(2-naphthyl)pentyl]carbamoyl]propionic acid

The ester compound (91 mg) obtained in (4) was dissolved in methylene chloride (5 ml). To the resulting solution was added trifluoroacetic acid (1 ml), and the mixture was stirred at room temperature for 7 hours. The reaction mixture was concentrated to dryness under reduced pressure. The residue was purified by chromatography on the column of "DIAION® HP-20" (elution with 80% aqueous acetonitrile), whereby the title compound (85 mg) was obtained as a colorless viscous oil.

MS (FAB+) m/Z: 555(M+H)$^+$.
$^1$H-NMR($CD_3OD$, rotamer mixture)δ: 1.07(1H,t,J=6.8Hz), 1.42-1.98(10H,m), 2.35(3H,s), 2.95(2H,t,J=7.6Hz), 3.07,3.30(total 3H,s each), 3.12-3.20(2H,m), 3.45-3.75(4H,m), 4.50-4.55(1H,m), 4.64,4.71(total 1H,d each,J=3.4Hz), 6.63-6.70(1H,m), 6.81(1H,s), 7.10-7.15(1H,m), 7.45-7.95(7H,m).

| Elementary analysis for $C_{31}H_{39}ClN_2O_5 \cdot 0.5H_2O$ | | | |
|---|---|---|---|
| Calculated: | C, 66.00; | H, 7.13; | N, 4.97. |

(continued)

| Elementary analysis for $C_{31}H_{39}ClN_2O_5 \cdot 0.5H_2O$ | | | |
|---|---|---|---|
| Found: | C, 66.09; | H, 7.21; | N, 5.08. |

Example 44

(2R,3R)-3-[N-[5-(3-Chloro-4-methylphenylamino)pentyl]-N-methylcarbamoyl]-3-[5-(3,4-dimethylphenyl)pentyloxy]-2-hydroxypropionic acid

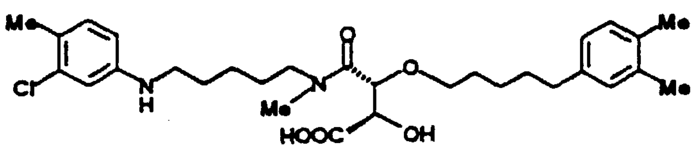

(1) tert-Butyl (2R,3R)-2-tert-butyldimethylsilyloxy-3-[5-(3,4-dimethylphenyl)pentyloxy]-3-[N-(5-hydroxypentyl)-N-methylcarbamoyl]propionate

The treatment was carried out in the same manner as in Example 31-(6) except for the use of the (5-hydroxypentyl)methylamine (117 mg) of Referential Example 15 instead of methyl[5-(2-naphthyl)pentyl]amine, whereby the title compound (440 mg) was obtained as a pale yellow oil.

MS (FAB+) m/Z: 616$(M+Na)^+$, 594$(M+H)^+$.
$^1$H-NMR($CDCl_3$, rotamer mixture)δ: 0.04(3H,s), 0.11(3H,s), 0.90(9H,s), 1.26-1.64(12H,m), 1.46(9H,s), 2.23(3H,s), 2.24(3H,s), 2.52(2H,m), 3.06-3.52(4H,m), 2.90,3.19(taotal 3H,s each), 3.58-3.65(2H,m), 4.39(1H,m), 4.46-4.50(1H,m), 6.89(1H,d,J=7.8Hz), 6.94(1H,s), 7.04(1H,d,J=7.8Hz).

(2) tert-Butyl (2R,3R)-2-tert-butyldimethylsilyloxy-3-[N-[5-[N-(3-chloro-4-methylphenyl)-N-(2,4-dinitrophenylsulfonyl)amino)pentyl]-N-methylcarbamoyl]-3-[5-(3,4-dimethylphenyl)pentyloxy]propionate

The alcohol compound (195 mg) obtained in (1) was treated with N-(3-chloro-4-methylphenyl)-2,4-dinitrobenzenesulfonamide, triphenylphosphine and diethyl azodicarboxylate in the same manner as in Example 25, the title compound (167 mg) was obtained as a yellow oil.

MS (FAB+) m/Z: 969$(M+Na)^+$, 947$(M+H)^+$.
$^1$H-NMR($CDCl_3$, rotamer mixture)δ: 0.03(3H,s), 0.10(3H,s), 0.89(9H,s), 1.24-1.62(12H,m), 1.45(9H,s), 2.22(3H,s), 2.23(3H,s), 2.37(3H,s), 2.47-2.53(2H,m), 3.03-3.54(4H,m), 2.87,3.17(total 3H,s each), 3.76(2H,t,J=7.1Hz), 4.36(1H,d,J=4.9Hz), 4.47(1H,d,J=4.9Hz), 6.87-6.89(1H,m), 6.92(1H,s), 6.99-7.03(2H,m), 7.20(2H,d,J=2.0Hz), 7.73(1H,d,J=8.8Hz), 8.29(1H,dd,J=8.8,2.4Hz), 8.44(1H,d,J=2.4Hz).

(3) tert-Butyl (2R,3R)-2-tert-butyldimethylsilyloxy-3-[N-[5-(3-chloro-4-methylphenylamino)pentyl]-N-methylcarbamoyl]-3-[5-(3,4-dimethylphenyl)pentyloxy]propionate

The 2,4-dinitrophenylsulfonyl compound (167 mg) obtained in (2) was treated with thioglycolic acid and triethylamine in the same manner as in Example 25, whereby the title compound (110 mg) was obtained as a pale yellow oil.

MS (FAB+) m/Z: 739$(M+Na)^+$, 717$(M+H)^+$.
$^1$H-NMR($CDCl_3$, rotamer mixture)δ: 0.04(3H,s), 0.11(3H,s), 0.90(9H,s), 1.29-1.64(12H,m), 1.45(9H,s), 2.22(3H,s), 2.23(3H,s), 2.24(3H,s), 2.50(2H,t,J=7.6Hz), 3.02-3.64(6H,m), 2.89,3.17(total 3H,s each), 4.32-4.39(1H,m), 4.45(4.50(1H,m), 6.40(1H,d,J=8.1,2.4Hz), 6.59(1H,t,J=2.4Hz), 6.88(1H,d,J=7.3Hz), 6.92(1H,s), 6.97(1H,d,J=8.1Hz), 7.02(1H,d,J=7.3Hz).

(4) tert-Butyl (2R,3R)-3-[N-[5-[3-chloro-4-methylphenylamino)pentyl]-N-methylcarbamoyl]-3-[5-(3,4-dimethylphenyl)pentyloxy]-2-hydroxypropionate

The 2-tert-butyldimethylsilyloxy compound (110 mg) obtained in(3) was dissolved in tetrahydrofuran (5 ml). To the resulting solution was added tetrabutylammonium fluoride (1M solution in tetrahydrofuran, 1.53 ml) under cooling with ice water, and the mixture was stirred at room temperature for 3 hours. The reaction mixture was separated in methylene chloride-aqueous saturated ammonium chloride. The water layer was extracted twice with methylene chloride. The organic layers were combined, washed successively with 1N hydrochloric acid and saturated saline and then dried over anhydrous sodium sulfate. The residue was purified by chromatography on a silica gel column (elution with 66% ethyl acetate-hexane), whereby the title compound (73 mg) was obtained as a pale yellow oil.

MS (FAB+) m/Z: 625$(M+Na)^+$, 603$(M+H)^+$.
$^1$H-NMR($CDCl_3$, rotamer mixture)δ: 1.34-1.41(3H,m), 1.50(9H,s), 1.54-1.65(9H,m), 2.22(3H,s), 2.23(3H,s), 2.51(2H,t,J=7.8Hz), 3.05(2H,t,J=6.6Hz), 2.94,3.15(total 3H,s each), 3.30-3.36(2H,m), 3.47-3.58(2H,m), 4.37(1H,m), 4.42(1H,m), 6.40(1H,dd,J=8.1,2.4Hz), 6.58(1H,d,J=2.4Hz), 6.88(1H,d,J=7.8Hz), 6.92(1H,s), 6.96(1H,d,J=8.1Hz), 7.02(1H,d,J=7.8Hz).

(5) (2R,3R)-3-[N-[5-(3-Chloro-4-methylphenylamino)pentyl]-N-methylcarbamoyl]-3-[5-(3,4-dimethylphenyl)pentyloxy]-2-hydroxypropionic acid

In the same manner as in Example 26, the title compound (61 mg) as a pale yellow amorphous substance was prepared from the ester compound (73 mg) obtained in (4).

MS (FAB+) m/Z: 569$(M+Na)^+$, 547$(M+H)^+$.
$^1$H-NMR($CDCl_3$, rotamer mixture)δ: 0.92-1.73(12H,m), 2.21(3H,s), 2.22(3H,s), 2.32(3H,s), 2.36(2H,t,J=7.6Hz), 2.93,3.08(total 3H,s each), 2.82-3.40(5H,m), 4.05-4.11(1H,m), 4.54(1H,m), 4.60(1H,broad), 6.82(1H,d,J=7.8Hz), 6.88(1H,s), 7.00(1H,d,J=7.8Hz), 7.22(1H,d,J=7.8Hz), 7.37(1H,d,J=7.8Hz), 7.56(1H,s), 8.37(2H,broad).

| Elementary analysis for $C_{30}H_{43}ClN_2O_5 \cdot CF_3CO_2H \cdot 0.5H_2O$ | | | |
|---|---|---|---|
| Calculated: | C, 57.35; | H, 6.77; | N, 4.18. |
| Found: | C, 57.15; | H, 6.72; | N, 3.99. |

Example 45

(2R,3R)-3-[N-[5-(3,4-Dimethylphenylamino)pentyl]-N-methylcarbamoyl]-3-[5-(3,4-dimethylphenyl)pentyloxy]-2-hydroxypropionic acid

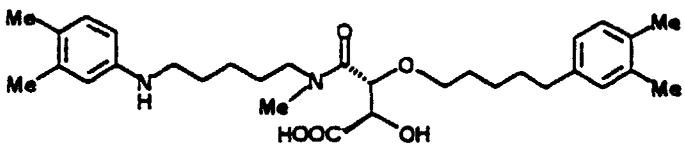

The treatment was carried out in the same manner as in Example 44 except for the use of N-(3,4-dimethylphenyl)-2,4-dinitrobenzenesulfonamide instead of N-(3-chloro-4-methylphenyl)-2,4-dinitrobenzenesulfonamide, whereby the title compound was obtained as a pale yellow amorphous substance.

MS (FAB+) m/Z: 527$(M+H)^+$.
$^1$H-NMR($CDCl_3$, rotamer mixture)δ: 0.87-1.63(12H,m), 2.21(6H,s), 2.22(3H,s), 2.24(3H,s), 2.34(2H,t,J=7.8Hz),

2.92,3.08(total 3H,s each), 2.80-3.30(5H,m), 4.12(1H,m), 4.56(1H,broad), 4.59(1H,broad), 6.81(1H,d,J=7.8Hz), 6.87(1H,s), 7.00(1H,d,J=7.8Hz), 7.14(1H,d,J=7.8Hz), 7.31(1H,d,J=7.8Hz), 7.34(1H,s).

Example 46

(2R,3R)-3-[5-(3,4-Dimethylphenyl)pentyloxy]-2-hydroxy-3-[N-[5-(5-indanylamino)pentyl]-N-methylcarbamoyl]propionic acid

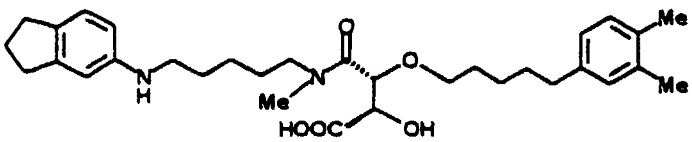

The treatment was carried out in the same manner as in Example 44 except for the use of N-(5-indanyl)-2,4-dinitrobenzenesulfonamide instead of N-(3-chloro-4-methylphenyl)-2,4-dinitrobenzenesulfonamide, whereby the title compound was obtained as a pale yellow amorphous substance.

MS (FAB+) m/Z: 539$(M+H)^+$.

$^1$H-NMR($CDCl_3$, rotamer mixture)δ: 0.87-1.63(12H,m), 2.05(2H,dt,J=14.7,7.3Hz), 2.21(3H,s), 2.22(3H,s), 2.35(2H,t,J=7.6Hz), 3.08(3H,s), 2.79-3.31(9H,m), 4.15(1H,m), 4.57-4.60(2H,m), 6.81(1H,d,J=7.3Hz), 6.87(1H,s), 7.00(1H,d,J=7.3Hz), 7.22(1H,d,J=7.8Hz), 7.34(1H,d,J=7.8Hz), 7.42(1H,s).

Example 47

(2R,3R)-2-Carboxymethoxy-3-[4-(3,4-dimethylphenylamino)butoxy]-3-[N-methyl-N-(5-(2-naphthyl)pentyl]carbamoyl]propionic acid

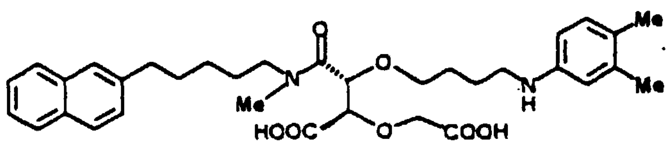

(1) tert-Butyl (2R,3R)-2-tert-butoxycarbonylmethoxy-3-[4-(tert-butyldiphenylsilyloxy)butoxy]-3-[N-methyl-N-(5-(2-naphthyl)pentyl]carbamoyl]propionate

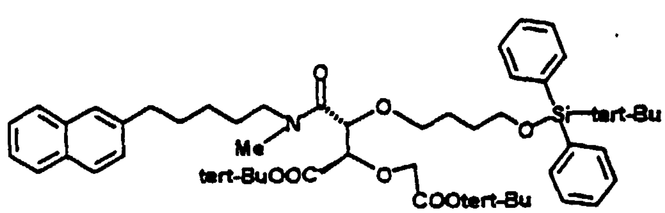

The tert-butyl (2R,3R)-3-[4-(tert-butyldiphenylsilyloxy)butoxyl-2-hydroxy-3-[N-methyl-[N-[5-(2-naphthyl)pentyl]carbamoyl]propionate (1.2 g) obtained in Example 43-(1) was treated with tert-butyl bromoacetate in the same manner as in Example 37-(1), whereby the title compound (1.5 g) was obtained as a colorless oil.

MS (FAB+) m/Z: 840(M+H)$^+$.
$^1$H-NMR(CDCl$_3$, rotamer mixture)δ: 1.02(9H,s), 1.27-1.75(28H,m), 2.76(2H,t,J=7.8Hz), 2.89,3.17(total 3H,s each), 3.29-3.66(6H,m), 3.92,4.03(total 1H,d each,J=16.1/16.6Hz), 4.20-4.33(2H,m), 4.48,4.50(total 1H,d each,J=4.4/5.8Hz), 7.27-7.80(17H,m).

(2) tert-Butyl (2R,3R)-2-tert-butoxycarbonylmethoxy-3-(4-hydroxybutoxy)-3-[N-methyl-N-(5-(2-naphthyl)pentyl]carbamoyl]propionate

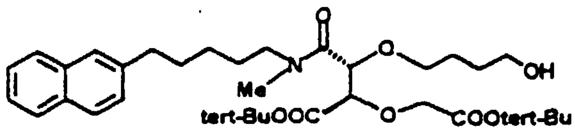

In the same manner as in Example 18-(5), the title compound (0.96 g) as a colorless oil was prepared from the 4-(tert-butyldiphenylsilyloxy)butoxy compound (1.5 g) obtained in (1).

MS (FAB+) m/Z: 602(M+H)$^+$.
$^1$H-NMR(CDCl$_3$, rotamer mixture)δ: 1.31-1.80(28H,m), 2.74-2.83(2H,m), 2.91,3.16(total 3H,s each), 3.30-3.68(6H,m), 3.95,4.04(total 1H,d each,J=16.1/16.1Hz), 4.22,4.25(total 1H,d each,J=16.1,16.1Hz), 4.29-4.35(1H,m), 4.47-4.53(1H,m), 7.31(1H,d,J=8.3Hz), 7.35-7.50(2H,m), 7.60(1H,s), 7.72-7.88(3H,m).

(3) tert-Butyl (2R,3R)-2-tert-butoxycarbonylmethoxy-3-[4-[N-(3,4-dimethylphenyl)-N-(2,4-dinitrophenylsulfonyl)amino]butoxy]-3-[N-methyl-N-(5-(2-naphthyl)pentyl]carbamoyl]propionate

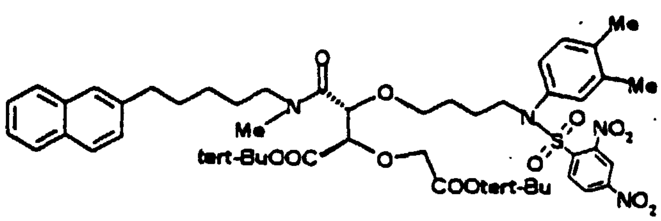

The alcohol compound (0.20 g) obtained in (2) was treated with triphenylphosphine and diethyl azodicarboxylate in the same manner as in Example 25 except for the use of N-(3,4-dimethylphenyl)-2,4-dinitrobenzenesulfonamide instead of N-(3-chloro-4-methylphenyl)-2,4-dinitrobenzenesulfonamide, whereby the title compound (0.24 g) was obtained as a pale yellow oil.

MS (FAB+) m/Z: 957(M+Na)$^+$.
$^1$H-NMR(CDCl$_3$, rotamer mixture)δ: 1.30-1.87(28H,m), 2.19(3H,s), 2.22(3H,s), 2.72-2.91(2H,m), 2.89,3.13(total 3H,s each), 3.24-3.60(4H,m), 3.73(2H,t,J=6.8Hz), 3.92,4.01(total 1H,J=16.1/16.1Hz), 4.16-4.31(2H,m), 4.45,4.48(total 1H,d each,J=4.9/5.9Hz), 6.80(1H,d,J=7.8Hz), 6.94(1H,s), 7.01-7.06(1H,m), 7.27-7.33(1H,m), 7.36-7.46(2H,m), 7.56-7.80(5H,m), 8.17,8.23(1H,m), 8.35-8.40(1H,m).

(4) tert-Butyl (2R,3R)-2-tert-butoxycarbonylmethoxy-3-[4-(3,4-dimethylphenylamino)butoxy]-3-[N-methyl-N-[5-(2-naphthyl)pentyl]carbamoyl]propionate

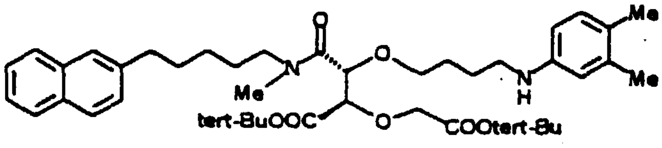

The 2,4-dinitrophenylsulfonyl compound (0.24 g) obtained in (3) was treated with thioglycolic acid and triethylamine in the same manner as in Example 25, whereby the title compound (0.19 g) was obtained as a pale yellow oil.

MS (FAB+) m/Z: 705(M+H)$^+$.
$^1$H-NMR($CDCl_3$, rotamer mixture)δ: 1.30-1.78(28H,m), 2.13(3H,s), 2.17(3H,s), 2.72-2.80(2H,m), 2.90,3.16(total 3H,s each), 3.02-3.08(2H,m), 3.28-3.67(4H,m), 3.94,4.03(total 1H,d each,J=16.1/16.6Hz), 4.20-4.32(2H,m), 4.49,4.51(total 1H,d each,J=4.9/5.9Hz), 6.32-6.38(1H,m), 6.41(1H,s), 6.90(1H,d,J=8.3Hz), 7.31(1H,dd,J=8.8,1.5Hz), 7.36-7.47(2H,m), 7.58(1H,s), 7.72-7.81(3H,m).

(5) (2R,3R)-2-Carboxymethoxy-3-[4-(3,4-dimethylphenylamino)butoxy]-3-[N-methyl-N-(5-(2-naphthyl)pentyl]carbamoyl]propionic acid

In the same manner as in Example 43-(5), the title compound (0.15 g) as a colorless viscous oil was prepared from the diester compound (0.19 g) obtained in (4).

$^1$H-NMR($CD_3OD$, rotamer mixture)δ: 1.40-1.60(4H,m), 1.77-2.08(6H,m), 2.37,2.40,2.43,2.60(total 6H,s each), 2.92,3.00(total 2H,t each,J=7.3/7.3Hz), 3.12,3.30(total 3H,s each), 3.50-3.80(4H,m), 4.00-4.08(4H,m), 4.39,4.47(total 1H,broad s each), 4.91,4.94(total 1H,broad s each), 7.29-2.98(10H,m).

| Elementary analysis for $C_{34}H_{44}N_2O_7 \cdot 1/3H_2O$ | | | |
|---|---|---|---|
| Calculated: | C, 68.21; | H, 7.53; | N, 4.68. |
| Found: | C, 68.04; | H, 7.60; | N, 4.74. |

Example 48

(2R,3R)-2-Carboxymethoxy-3-[4-(5-indanylamino)butoxy]-3-[N-methyl-N-[5-(2-naphthyl)pentyl]carbamoyl]propionic acid

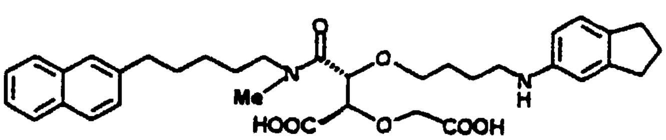

The reaction was effected in the same manner as in Example 47 except for the use of N-(5-indanyl)-2,4-dinitrobenzenesulfonamide instead of N-(3,4-dimethylphenyl)-2,4-dinitrobenzenesulfonamide, whereby the title compound was obtained as a colorless viscous oil.

MS (FAB+) m/Z: 605$(M+H)^+$.

$^1$H-NMR($CD_3OD$, rotamer mixture)δ: 1.42-2.32(12H,m), 2.88-3.24(6H,m), 3.12,3.30(total 3H,s each), 3.42-3.80(4H,m), 3.98-4.10(4H,m), 4.36-4.50(1H,m), 4.85-4.98(1H,m), 7.32-7.98(10H,m).

| Elementary analysis for $C_{35}H_{44}N_2O_7 \cdot 1/2H_2O$ | | | |
|---|---|---|---|
| Calculated: | C, 68.50; | H, 7.39; | N, 4.56. |
| Found: | C, 68.56; | H, 7.35; | N, 4.62. |

Example 49

(2R,3R)-3-[4-(3,4-Dimethylphenylamino)butoxy]-2-ethoxycarbonylmethoxy-3-[N-methyl-N-[5-(2-naphthyl)pentyl]carbamoyl]propionic acid

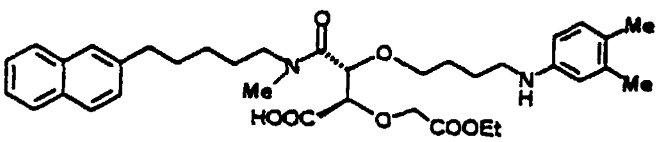

The reaction was effected in the same manner as in Example 32 except for the use of N-(3,4-dimethylphenyl)-2,4-dinitrobenzenesulfonamide instead of N-(3-chloro-4-methylphenyl)-2,4-dinitrobenzenesulfonamide, whereby the title compound was obtained as a colorless viscous oil.

MS (FAB+) m/Z: 621$(M+H)^+$.

$^1$H-NMR($CDCl_3$, rotamer mixture)δ: 1.15-1.22(3H,m), 1.24-2.00(10H,m), 2.21,2.23,2.26,2.27(total 6H,s each), 2.73-2.82(2H,m), 3.01,3.12(total 3H,s each), 3.15-3.78(6H,m), 3.88(1H,d,J=16.6Hz), 3.96(1H,d,J=16.6Hz), 4.07(2H,q,J=7.0Hz), 4.39,4.49(total 1H,broad each), 4.72,4.72(total 1H,broad each), 7.10-7.83(10H,m).

Example 50

(2R,3R)-2-Ethoxycarbonylmethoxy-3-[N-methyl-N-[5-(2-naphthyl)pentyl]carbamoyl]-3-[4-(3-methylphenylamino)butoxy]propionic acid

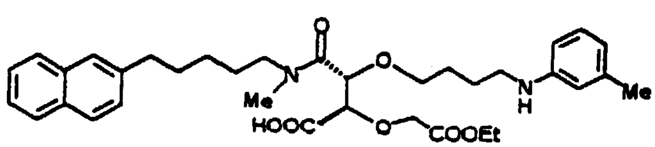

The reaction was effected in the same manner as in Example 32 except for the use of N-(3-methylphenyl)-2,4-dinitrobenzenesulfonamide instead of N-(3-chloro-4-methylphenyl)-2,4-dinitrobenzenesulfonamide, whereby the title compound was obtained as a colorless viscous oil.

MS (FAB+) m/Z: 607$(M+H)^+$.

$^1$H-NMR($CDCl_3$)δ: 1.05-2.00(13H,m), 2.34,2.38(total 3H,s each), 2.72-2.84(2H,m), 3.01,3.12(total 3H,s each), 3.18-3.80(6H,m), 3.85(1H,d,J=16.1Hz), 3.93(1H,d,J=16.1Hz), 4.06(2H,q,J=6.8Hz), 4.39,4.49(total 1H,broad

each), 4.70-4.76(1H,m), 7.15-7.82(11H,m).

Example 51

(2R,3R)-2-Ethoxycarbonylmethoxy-3-[4-(5-indanylamino)butoxy]-3-[N-methyl-N-[5-(2-naphthyl)pentyl]carbamoyl]propionic acid

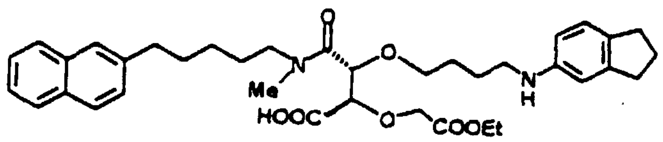

The reaction was effected in the same manner as in Example 32 except for the use of N-(5-indanyl)-2,4-dinitrobenzenesulfonamide instead of N-(3-chloro-4-methylphenyl)-2,4-dinitrobenzenesulfonamide, whereby the title compound was obtained as a pale yellow viscous oil.

MS (FAB+) m/Z: 633$(M+H)^+$.
$^1$H-NMR($CDCl_3$, rotamer mixture)δ: 1.16-1.82(13H,m), 1.98-2.08(2H,m), 2.72-2.88(6H,m), 2.96,3.12(total 3H,s each), 3.03-3.15(2H,m), 3.30-3.63(4H,m), 4.02-4.40(5H,m), 4.68-4.73(1H,m), 6.76(1H,d,J=7.3Hz), 6.85(1H,s), 7.07(1H,d,J=7.3Hz), 7.30(1H,d,J=8.3Hz), 7.37-7.48(2H,m), 7.59(1H,s), 7.72-7.83(3H,m).

Example 52

(2R,3R)-3-[N-[5-(3-Chloro-4-methylphenylamino)pentyl]-N-methylcarbamoyl]-3-[5-(3,4-dimethylphenyl)pentyloxy]-2-ethoycarbonylmethoxypropionic acid

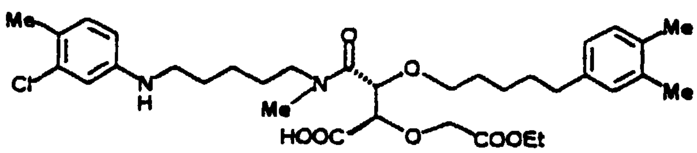

(1) Benzyl (2R,3R)-3-tert-butoxycarbonyl-2-[5-(3,4-dimethylphenyl)pentyloxy]-3-ethoxycarbonylmethoxypropionate

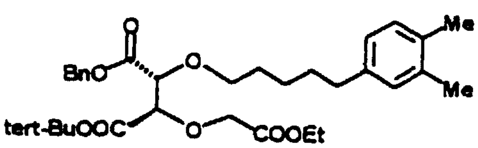

The 2-hydroxy compound (1.07 g) obtained in Example 31-(3) was treated in the same manner as in Example 38-(1), whereby the title compound (0.82 g) was obtained as a colorless oil.

MS (FAB+) m/Z: 579$(M+Na)^+$.

$^1$H-NMR($CDCl_3$)δ: 1.24(3H,t,J=7.1Hz), 1.31-1.38(2H,m), 1.47(9H,s), 1.53-1.65(4H,m), 2.21(3H,s), 2.22(3H,s), 2.49(2H,t,J=7.8Hz), 3.28-3.34(1H,m), 3.70(1H,dt,J=8.8,6.8Hz), 4.11-4.19(1H,m), 4.37(1H,d,J=2.9Hz), 4.48(1H,d,J=2.9Hz), 5.24(1H,d,J=12.2Hz), 5.27(1H,d,J=12.2Hz), 6.87(1H,d,J=7.8Hz), 6.92(1H,s), 7.01(1H,d,J=7.8Hz), 7.31-7.36(3H,m), 7.40-7.51(2H,m).

(2) (2R,3R)-3-tert-Butoxycarbonyl-2-[5-(3,4-dimethylphenyl)pentyloxy]-3-ethoxycarbonylmethoxypropionic acid

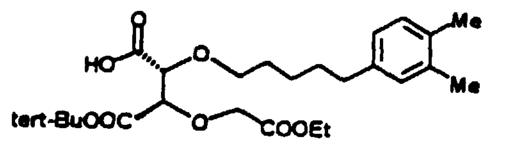

In the same manner as in Example 31-(5), the title compound (0.68 g) as a colorless oil was prepared from the triester compound (0.80 g) obtained in (1).

MS (FAB+) m/Z: 489(M+Na)$^+$.
$^1$H-NMR($CDCl_3$)δ: 1.26(3H,t,J=7.1Hz), 1.32-1.39(2H,m), 1.50(9H,s), 1.54-1.65(4H,m), 2.21(3H,s), 2.23(3H,s), 2.51(2H,t,J=7.6Hz), 3.44(1H,dt,J=8.8,6.8Hz), 3.66(1H,dt,J=8.8,6.8Hz), 4.15(1H,d,J=16.6Hz), 4.19(2H,q,J=6.8Hz), 4.37(1H,d,J=2.9Hz), 4.40(1H,d,J=2.9Hz), 4.41(1H,d,J=16.6Hz), 6.87(1H,d,J=7.8Hz), 6.92(1H,s), 7.02(1H,d,J=7.8Hz).

(3) tert-Butyl (2R,3R)-3-[5-(3,4-dimethylphenyl)pentyloxy]-2-ethoxycarbonylmethoxy-3-[N-(5-hydroxypentyl)-N-methylcarbamoyl]propionate

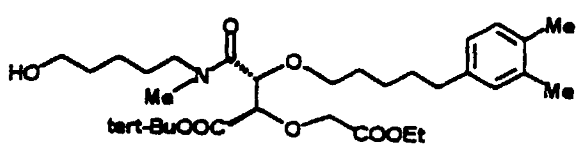

In the same manner as in Example 41-(1) except for the use of the (5-hydroxypentyl)methylamine (0.37 g) of Referential Example 15 instead of 5-hydroxypentylamine, the title compound (1.19 g) was prepared from the carboxylic acid compound (1.05 g) obtained in (2).

MS (FAB+) m/Z: 566(M+H)$^+$.
$^1$H-NMR($CDCl_3$, rotamer mixture)δ: 1.26(3H,t,J=7.1Hz), 1.33-1.63(12H,m), 1.47(9H,s), 2.22(3H,s), 2.23(3H,s), 2.51(2H,t,J=7.8Hz), 2.91,3.19(total 3H,s each), 3.33-3.48(3H,m), 3.53-3.62(3H,m), 4.07-4.53(6H,m), 6.88(1H,d,J=7.3Hz), 6.93(1H,s), 7.02(1H,d,J=7.3Hz).

(4) tert-Butyl (2R,3R)-3-[N-[5-(3-chloro-4-methylphenylamino)pentyl]-N-methylcarbamoyl]-3-[5-(3,4-dimethylphenyl)pentyloxy]-2-ethoxycarbonylmethoxypropionate

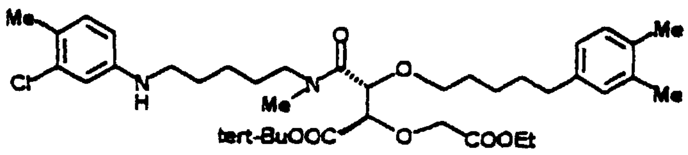

In the same manner as in Example 25, the title compound (0.11 g) as a pale yellow oil was prepared from the alcohol compound (0.23 g) obtained in (3).

$^1$H-NMR($CDCl_3$, rotamer mixture)δ: 1.23-1.67(15H,m), 1.47(9H,s), 2.21(3H,s), 2.22(3H,s), 2.23(3H,s), 2.51(2H,t,J=7.8Hz), 2.91,3.19(total 3H,s each), 3.02-3.08(2H,m), 3.31-3.64(4H,m), 3.64(1H,broad), 4.06-4.53(6H,m), 6.40(1H,dd,J=8.3,2.4Hz), 6.58(1H,d,J=2.4Hz), 6.87(1H,d,J=7.3Hz), 6.92(1H,s), 6.96(1H,d,J=8.3Hz), 7.02(1H,d,J=7.3Hz).

(5) (2R,3R)-3-[N-[5-(3-Chloro-4-methylphenylamino)pentyl]-N-methylcarbamoyl]-3-[5-(3,4-dimethylphenyl)pentyloxy]-2-ethoxycarbonylmethoxypropionic acid

In the same manner as in Example 26, the title compound (41 mg) as a pale yellow viscous oil was prepared from the diester compound (60 mg) obtained in (4).

MS (FAB+) m/Z: 633$(M+H)^+$.
$^1$H-NMR($CDCl_3$, rotamer mixture)δ: 1.21-1.72(15H,m), 2.22(3H,s), 2.23(3H,s), 2.33(3H,s), 2.49(2H,t,J=7.6Hz), 2.95,3.13(total 3H,s each), 3.08-3.75(6H,m), 4.18-4.64(6H,m), 6.88(1H,d,J=7.5Hz), 6.93(1H,s), 7.02(1H,d,J=7.5Hz), 7.12-7.31(3H,m), 8.18(2H,broad).

Example 53

(2R,3R)-2-Carboxymethoxy-3-[N-[5-(3,4-dimethylphenylamino)pentyl]-N-methylcarbamoyl]-3-[5-(3,4-dimethylphenyl)pentyloxy]propionic acid

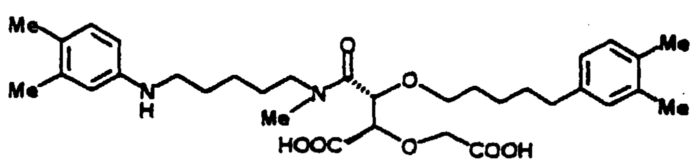

(1) (2R,3R)-3-[N-[5-(3,4-Dimethylphenylamino)pentyl]-N-methylcarbamoyl]-3-[5-(3,4-dimethylphenyl)pentyloxy]-2-ethoxycarbonylmethoxypropionic acid

In the same manner as in Example 52 except for the use of N-(3,4-dimethylphenyl)-2,4-dinitrobenzenesulfonamide instead of N-(3-chloro-4-methylphenyl)-2,4-dinitrobenzenesulfonamide, the title compound was obtained as a colorless viscous oil.

MS (FAB+) m/Z: 613$(M+H)^+$.

$^1$H-NMR($CDCl_3$, rotamer mixture)δ: 0.87-1.55(10H,m), 1.26(3H,t,J=7.1Hz), 1.72(2H,broad), 2.21(3H,s), 2.23(6H,s), 2.24(3H,s), 2.49(2H,t,J=7.6Hz), 3.04(1H,broad), 2.92,3.11(total 3H,s each), 3.26(2H,broad), 3.47(2H,broad), 3.76(1H,broad), 4.18-4.36(2H,m), 4.20(2H,q,J=7.1Hz), 4.41(1H,d,J=6.4Hz), 4.60(1H,d,J=6.4Hz), 6.87(1H,d,J=7.3Hz), 6.92(1H,s), 7.02(1H,d,J=7.8Hz), 7.13(1H,d,J=7.8Hz), 7.18-7.23(2H,m), 10.07(2H,broad).

(2) (2R,3R)-2-Carboxymethoxy-3-[N-[5-(3,4-dimethylphenylamino)pentyl]-N-methylcarbamoyl]-3-[5-(3,4-dimethylphenyl)pentyloxy]propionic acid

The ethyl ester compound (95 mg) obtained in (1) was dissolved in tetrahydrofuran (2 ml). To the resulting solution was added 1N sodium hydroxide (0.46 ml), and the mixture was stirred at room temperature for 1 hour. To the reaction mixture was added 1N hydrochloric acid (0.55 ml) and the resulting mixture was concentrated under reduced pressure. The residue was purified by chromatography on the column of "DIAION® HP-20" (elution with acetonitrile), whereby the title compound (34 mg) was obtained as a pale yellow oil.

MS (FAB+) m/Z: 585$(M+H)^+$.
$^1$H-NMR($CD_3OD$, rotamer mixture)δ: 0.88-1.67(12H,m), 2.18(3H,s), 2.19(3H,s), 2.20(3H,s), 2.22(3H,s), 2.47(2H,t,J=7.6Hz), 2.91,3.15(total 3H,s each), 3.17-3.61(6H,m), 4.17(2H,s), 4.30,4.34(total 1H,d each,J=4.9/4.9Hz), 4.60(1H,d,J=4.9Hz), 6.78-7.09(2H,m), 6.83(1H,d,J=7.8Hz), 6.89(1H,s), 6.96(1H,d,J=7.8Hz), 7.07(1H,d,J=7.8Hz).

Example 54

(2R,3R)-2-Carboxymethoxy-3-[5-(3,4-dimethylphenyl)pentyloxy]-3-[N-[5-(5-indanylamino)pentyl)-N-methylcarbamoyl]propionic acid

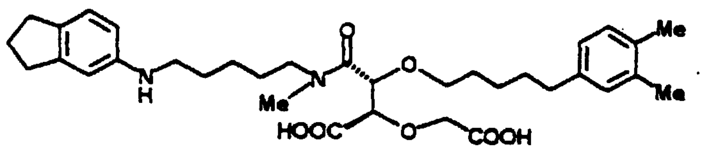

(1) (2R,3R)-3-[5-(3,4-Dimethylphenyl)pentyloxy]-2-ethoxycarbonylmethoxy-3-[N-[5-(5-indanylamino)pentyl)-N-methylcarbamoyl]propionic acid

The reaction was effected in the same manner as in Example 52 except for the use of N-(5-indanyl)-2,4-dinitrobenzenesulfonamide instead of N-(3-chloro-4-methylphenyl)-2,4-dinitrobenzenesulfonamide, whereby the title compound was obtained as a colorless viscous oil.

MS (FAB+) m/Z: 625$(M+H)^+$.
$^1$H-NMR($CDCl_3$, rotamer mixture)δ: 0.86-1.56(10H,m), 1.26(3H,t,J=7.0Hz), 1.73(2H,broad s), 2.08(2H,t,J=7.3Hz), 2.21(3H,s), 2.23(3H,s), 2.48(2H,t,J=7.6Hz), 2.86-2.92(4H,m), 3.05(1H,m), 2.92,3.12(total 3H,s each), 3.27(2H,broad s), 3.48(2H,broad s), 3.77(1H,m), 4.19(2H,q,J=7.0Hz), 4.24-4.36(2H,m), 4.41(1H,broad), 4.60(1H,d,J=5.4Hz), 6.87(1H,d,J=7.3Hz), 6.92(1H,s), 7.01(1H,d,J=7.3Hz), 7.22(2H,m), 7.32(1H,s), 9.02(2H,broad).

(2) (2R,3R)-2-Carboxymethoxy-3-[5-(3,4-dimethylphenyl)pentyloxy]-3-[N-[5-(5-indanylamino)pentyl)-N-methylcarbamoyl]propionic acid

In the same manner as in Example 53-(2), the title compound as a pale yellow oil was prepared from the ethyl ester compound obtained in (1).

MS (FAB+) m/Z: 597$(M+H)^+$.
$^1$H-NMR($CD_3OD$, rotamer mixture)δ: 0.88-1.68(12H,m), 2.04(2H,t,J=7.1Hz), 2.19(3H,s), 2.20(3H,s),

2.47(2H,t,J=7.3Hz), 2.81-2.85(4H,m), 2.92,3.15(total 3H,s each), 3.26-3.60(6H,m), 4.16(2H,s), 4.31(1H,broad), 4.62(1H,broad), 6.82-6.84(2H,m), 6.89(1H,s), 6.96(1H,d,J=6.8Hz), 7.06(1H,d,J=7.8Hz), 7.13(1H,d,J=6.8Hz).

Referential Example 1

tert-Butyl benzyl L-tartrate

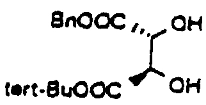

In methylene chloride (50 ml), L-tartaric acid (5.0 g) was suspended, followed by the dropwise addition of O-tert-butyl-N,N'-diisopropylisourea (10.0 g) under cooling with ice water. After stirring at room temperature for 16 hours, the insoluble solid was filtered off and, from the filtrate, the solvent was distilled off under reduced pressure. The residue was dissolved in dimethylformamide (100 ml). To the resulting solution was added triethylamine (8.3 ml) and then, benzyl bromide (5.9 ml) was added dropwise. After stirring at room temperature for 3 hours, the reaction mixture was poured into ice water, followed by extraction with ethyl acetate. The extract was dried over anhydrous sodium sulfate. The solvent was then distilled off under reduced pressure. The residue was purified by chromatography on a silica gel column (elution with 20% ethyl acetate-hexane), followed by crystallization from small amounts of ethyl acetate and hexane, whereby the title compound (1.56 g) was obtained as colorless crystals.

Melting point: 72 to 74°C
$[\alpha]_D^{25}$ +14.3° (c=1.00, acetone)
$^1$H-NMR($CDCl_3$)δ: 1.50(9H,s), 3.07(1H,d,J=7.8Hz), 3.19(1H,d,J=6.3Hz), 4.44(1H,dd,J=6.3,2.0Hz), 4.54(1H,dd,J=7.8,2.0Hz), 5.28(1H,d,J=12.2Hz), 5.29(1H,d,J=12.2Hz), 7.34-7.38(5H,m).

| Elementary analysis for $C_{15}H_{20}O_6$ | | |
|---|---|---|
| Calculated: | C, 60.80; | H, 6.80. |
| Found: | C, 60.95; | H, 6.71. |

Referential Example 2

Synthesis of ethyl benzyl L-tartrate

(1) Sodium ethyl L-tartrate

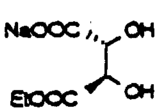

To a solution (100 ml) of diethyl L-(+)-tartrate (25 g) in ethanol was added a 2N aqueous sodium hydroxide solution (47.5 ml) under cooling with ice water, and the mixture was stirred for 2 hours. The crystals so precipitated were collected by filtration, whereby the title compound (15.0 g) was obtained as colorless crystals.

Melting point: 201 to 203°C
$^1$H-NMR($CD_3OD$)δ: 1.25-1.30(3H,m), 3.30(2H,broad s), 3.33(1H,d,J=6.8Hz), 4.15-4.30(2H,m), 4.52(1H,d,J=6.8Hz).

(2) Ethyl benzyl L-tartrate

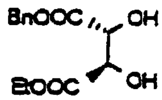

Sodium ethyl L-tartrate (8.15 g) was dissolved in N,N-dimethylformamide (30 ml) and to the resulting solution, triethylamine (7.7 ml) and benzyl bromide (6.6 ml) were added under cooling with ice water. The resulting mixture was stirred overnight. The reaction mixture was diluted with diethyl ether (500 ml), washed successively with water and saturated saline and dried over anhydrous sodium sulfate. The solvent was then distilled off under reduced pressure. The residue was purified by chromatography on a silica gel column (elution with 50% ethyl acetate-hexane), whereby the title compound (6.68 g) was obtained as a colorless oil.

$^1$H-NMR($CDCl_3$)δ: 1.31(3H,t,J=7.3Hz), 3.17(2H,m), 4.30(2H,q,J=7.3Hz), 4.55-4.60(2H,m), 5.27(1H,d,J=12.2Hz), 5.31(1H,d,J=12.2Hz), 7.33-7.39(5H,m).

Referential Example 3

5-(2-Naphthyl)-2-pentenyl iodide

(1) 5-(2-Naphthyl)-2-pentenol

Sodium hydride (60% in oil, 2.0 g) was suspended in tetrahydrofuran (50 ml), followed by the dropwise addition of a solution of ethyl diethylphosphonoacetate (11.4 g) in tetrahydrofuran (50 ml). Thirty minutes after the evolution of gas ceased, a solution of 3-(2-naphthyl)propanal (8.95 g) in tetrahydrofuran (50 ml) was added dropwise. After stirring for 30 minutes at room temperature, the reaction mixture was concentrated under reduced pressure. Water was added to the concentrate and the resulting mixture was extracted with ethyl acetate. The extract was washed with saturated saline and dried over anhydrous sodium sulfate. The solvent was then distilled off under reduced pressure, whereby ethyl 5-(2-naphpthyl)-2-pentenoate (14 g) was obtained as an oil.

$^1$H-NMR($CDCl_3$)δ: 1.27(3H,t,J=7.3Hz), 2.58-2.64(2H,m), 2.93(2H,t,J=7.3Hz), 4.17(2H,q,J=7.3Hz), 5.86(1H,d,J=15.6Hz), 7.03(1H,dt,J=15.6,6.8Hz), 7.31(1H,dd,J=8.3,1.5Hz), 7.40-7.47(2H,m), 7.61(1H,s), 7.76-7.81(3H,m).

The resulting compound was dissolved in tetrahydrofuran (140 ml), followed by the dropwise addition of diisobutylaluminum hydride (1M solution in hexane, 120 ml) over 40 minutes under cooling with ice water. After stirring for 1 hour, the reaction mixture was heated to room temperature and sodium sulfate 10 hydrate (60 g) was added thereto. Stirring was conducted for further one hour. The insoluble matter was filtered off, followed by washing with tetrahydrofuran. The filtrate and washing were combined and the solvent was distilled off under reduced pressure. The residue was purified by chromatography on a silica gel column (30 to 40% ethyl acetate-hexane), whereby the title compound (8.10 g) was obtained as colorless crystals.

Melting point: 51 to 52°C
Mass (EI) m/Z: 212($M^+$).
$^1$H-NMR($CDCl_3$)δ: 2.44-2.49(2H,m), 2.87(2H,t,J=7.3Hz), 4.06-4.09(2H,m), 5.69-5.80(2H,m), 7.32(1H,dd,J=8.3,1.5Hz), 7.39-7.47(2H,m), 7.61(1H,s), 7.76-7.81(3H,m).

| Elementary analysis for $C_{15}H_{16}O$ | | |
|---|---|---|
| Calculated: | C, 84.86; | H, 7.60 |
| Found: | C, 84.65; | H, 7.62 |

(2) 5-(2-Naphthyl)-2-pentenyl iodide

The alcohol compound (12.8 g) obtained in (1) and sodium iodide (10.85 g) were dissolved in acetonitrile (140 ml), followed by the dropwise addition of chlorotrimethylsilane (9.1 ml). After stirring at room temperature for 40 minutes, the reaction mixture was poured into water and extracted with diethyl ether. The extract was washed with aqueous sodium thiosulfate and saturated saline and then dried over anhydrous sodium sulfate. The solvent was distilled off under reduced pressure. The residue was purified by chromatography on a silica gel column (elution with 3% ethyl acetate-hexane), whereby the title compound (18.6 g) was obtained as a pale yellow oil.

$^1$H-NMR($CDCl_3$)δ: 2.41-2.46(2H,m), 2.85(2H,t,J=7.3Hz), 3.81-3.89(2H,m), 5.71-5.81(2H,m), 7.30(1H,dd,J=8.3,1.5Hz), 7.39-7.47(2H,m), 7.60(1H,s), 7.75-7.81(3H,m).

Referential Example 4

tert-Butyl (2R,3S)-4-hydroxy-3-methoxymethoxy-2-[5-(2-naphthyl)pentyloxy]butyrate

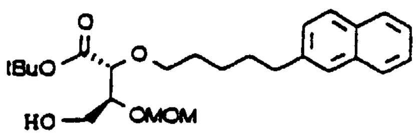

(1) (3R,4S)-4-Hydroxy-3-[5-(2-naphthyl)pentyloxy]-dihydro-2(3H)-furanone

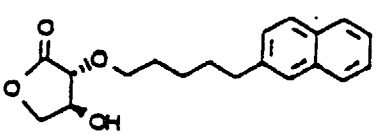

Methyl 3,4-O-isopropylidene-L-threonate (19.4 g) was dissolved in N,N-dimethylformamide (300 ml), followed by the addition of 60% sodium hydride (in oil, 4.10 g) under stirring at room temperature. To the resulting mixture was added dropwise a solution of 5-(2-naphthyl)-2-pentenyl iodide (33.0 g) in N,N-dimethylformamide (50 ml) and the resulting mixture was stirred for 2 hours. The reaction mixture was diluted with diethyl ether and added with water to separate the organic layer. The resulting organic layer was washed with water and saturated saline and dried over anhydrous sodium sulfate. The solvent was then distilled off under reduced pressure. The residue was partially purified by chromatography on a silica gel column (ethyl acetate-hexane 1:2), whereby methyl 3,4-O-isopropylidene-2-O-[5-(2-naphthyl)-2-pentenyl]-L-threonate (17.1 g) was obtained as a colorless oil.

The resulting compound was dissolved in a mixture of methanol (200 ml) and tetrahydrofuran (200 ml). To the resulting solution was added a catalytic amount of 10% palladium-carbon, and the mixture was stirred at room temperature for 6 days under hydrogen atmosphere. The catalyst was filtered off and the filtrate was distilled under reduced pressure, whereby 3,4-O-isopropylidene-2-O-[5-(2-naphthyl)pentyl]-L-threonate was obtained as an oil.

The resulting compound was dissolved in methylene chloride (100 ml). To the resulting solution was added trifluoroacetic acid (5 ml), and the mixture was stirred at room temperature for 19 hours. The reaction mixture was concentrated to dryness under reduced pressure. The residue was purified by chromatography on a silica gel column (ethyl acetate-hexane 2:3), whereby the title compound (5.78 g) was obtained as a colorless oil.

$^1$H-NMR($CDCl_3$)δ: 1.40-1.48(2H,m), 1.64-1.78(4H,m), 2.12-2.17(1H,broad s), 2.78(2H,t,J=7.3Hz), 3.49(1H,s), 3.62(1H,dt,J=9.3,6.4Hz), 4.40-4.44(2H,m), 7.32(1H,dd,J=8.3,1.9Hz), 7.35-7.45(2H,m), 7.60(1H,s), 7.70-7.90(3H,m).

(2) (3R,4S)-4-Methoxymethoxy-3-[5-(2-naphthyl)pentyloxy]-dihydro-2(3H)-furanone

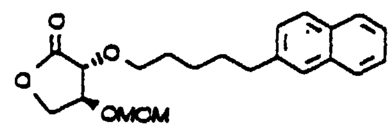

The lactone compound (5.78 g) obtained in (1) was dissolved in methylene chloride (60 ml). To the resulting solution were added diisopropylethylamine (6 ml) and chloromethylmethyl ether (3.5 ml), and the mixture was stirred for 24 hours under cooling with ice water. The reaction mixture was diluted with diethyl ether, washed successively with water and saturated saline and dried over anhydrous sodium sulfate. The solvent was then distilled off under reduced pressure. The residue was purified by chromatography on a silica gel column (ethyl acetate-hexane 1:4), whereby the title compound (4.88 g) was obtained as an oil.

Mass (EI) m/Z: 358($M^+$).
$^1$H-NMR($CDCl_3$)δ: 1.42-1.48(2H,m), 1.65-1.73(4H,m), 2.78(2H,t,J=7.8Hz), 3.35(3H,s), 3.62(1H,dd,J=6.8,2.4Hz), 3.96(1H,dd,J=6.8,2.4Hz), 4.02-4.10(1H,m), 4.07(1H,d,J=6.4Hz), 4.29(1H,dt,J=6.4,6.4Hz), 4.48(1H,dd,J=9.3,6.4Hz), 4.63(1H,d,J=6.8Hz), 4.71(1H,d,J=6.8Hz), 7.32(1H,dd,J=8.3,1.5Hz), 7.40-7.46(2H,m), 7.60(1H,s), 7.75-7.80(3H,m).

(3) tert-Butyl (2R,3S)-4-hydroxy-3-methoxymethoxy-2-[5-(2-naphthyl)pentyloxy]butyrate

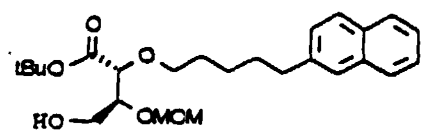

The methoxymethoxy compound (4.88 g) obtained in (2) was dissolved in tetrahydrofuran (8 ml). To the resulting solution was added a 2N aqueous sodium hydroxide solution (8 ml), and the mixture was stirred at room temperature for 3 hours. To the reaction mixture was added 10% hydrochloric acid to make it acidic and the resulting mixture was extracted with diethyl ether. The extract was dried over anhydrous sodium sulfate. The solvent was then distilled off under reduced pressure, whereby (2R,3S)-4-hydroxy-3-methoxymethoxy-2-[5-(2-naphthyl)pentyloxy]butyric acid was obtained as an oil.

The resulting compound was dissolved in tetrahydrofuran (50 ml) and to the resulting solution, O-tert-butyl-N,N'-diisopropylisourea (17 ml) was added dropwise. After stirring at room temperature for 15 hours, the solvent was distilled off under reduced pressure. The residue was purified by chromatography on a silica gel column (ethyl acetate-hexane 1:3), whereby the title compound (3.95 g) was obtained as an oil.

Mass (EI) m/Z: 432($M^+$).
$^1$H-NMR($CDCl_3$)δ: 1.42-1.52(2H,m), 1.47(9H,s), 1.60-1.78(4H,m), 2.77(2H,t,J=7.5Hz), 2.98(1H,dd,J=9.2,3.4Hz),

3.29(1H,dd,J=8.8,6.8Hz), 3.40(3H,s), 3.68-3.78(3H,m), 3.89(1H,s), 3.84-3.96(1H,m), 4.60(1H,d,J=6.8Hz), 4.71(1H,d,J=6.8Hz), 7.32(1H,dd,J=8.3Hz,1.5Hz), 7.38-7.46(2H,m), 7.60(1H,s), 7.74-7.78(3H,m).

Referential Example 5

5-(2-Naphthyl)pentylamine

To a solution (100 ml) of the ethyl 5-(2-naphthyl)-2-pentenoate (1.25 g) obtained in Referential Example 3-(1) in ethanol was added 10% palladium-carbon (125 g), and the mixture was stirred at room temperature for 5.5 hours under hydrogen atmosphere. After removal of the 10% palladium-carbon by filtration, the solvent was distilled off under reduced pressure. The residue was purified by chromatography on a silica gel column (elution with 50% ethyl acetate-hexane), whereby ethyl 5-(2-naphthyl)pentanoate (1.24 g) was obtained. A solution of the resulting compound (1.18 g) in tetrahydrofuran (10 ml) was added dropwise to a suspension of lithium aluminum hydride (190 mg) in tetrahydrofuran (10 ml) under cooling with ice water, and the mixture was stirred for 1.5 hours. To the reaction mixture was added sodium sulfate • 10 hydrate (1.6 g) and the resulting mixture was stirred for 30 minutes. After filtration of the reaction mixture, the filtrate was concentrated under reduced pressure. The residue was purified by chromatography on a silica gel column (elution with 33% ethyl acetate-hexane), whereby 5-(2-naphthyl)pentanol (0.98 g) was obtained as a colorless solid.

Mass (EI) m/Z: 214($M^+$).
$^1$H-NMR($CDCl_3$)δ: 1.23(1H,t,J=5.4Hz), 1.40-1.49(2H,m), 1.58-1.68(2H,m), 1.70-1.80(2H,m), 2.79(2H,t,J=7.6Hz), 3.64(2H,dd,J=12.7,7.3Hz), 7.33(1H,dd,J=8.8,2.0Hz), 7.37-7.47(2H,m), 7.61(1H,s), 7.75-7.85(3H,m).

The resulting alcohol compound (0.96 g), phthalimide (0.99 g) and triphenylphosphine (1.76 g) were dissolved in tetrahydrofuran (100 ml). To the resulting solution was added dropwise diethyl azodicarboxylate (1.06 ml) under cooling with ice water. After stirring at room temperature for 18 hours, the solvent was distilled off under reduced pressure. The residue was purified by chromatography on a silica gel column (elution with 33% ethyl acetate-hexane), whereby 1-[5-(2-naphthyl)pentyl]phthalimide (1.53 g) was obtained as colorless crystals.

Mass (EI) m/Z: 343($M^+$).
$^1$H-NMR($CDCl_3$)δ: 1.37-1.47(2H,m), 1.68-1.80(4H,m), 2.77(2H,t,J=7.3Hz), 3.68(2H,t,J=7.3Hz), 7.28-7.85(12H,m)

To a solution of the resulting phthalimide compound (0.90 g) in ethanol (20 ml) was added hydrazine hydrate (0.26 ml), followed by refluxing under heat for 2.5 hours. After cooling, the crystals so precipitated were filtered and the filtrate was concentrated under reduced pressure. Aqueous sodium hydroxide was added to the residue, followed by extraction with ethyl acetate. The extract was washed with saturated saline and then dried over anhydrous sodium sulfate. The solvent was distilled off, whereby the title compound (0.53 g) was obtained as a pale yellow oil.

Mass (EI+) m/z: 213($M^+$).
$^1$H-NMR($CDCl_3$)δ: 1.34-1.54(6H,m), 1.68-1.76(2H,m), 2.68(2H,t,J=7.1Hz), 2.77(2H,t,J=7.6Hz), 7.32(1H,dd,J=8.0,2.0Hz), 7.38-7.46(2H,m), 7.59(1H,s), 7.71-7.81(3H,m).

To a solution of the resulting compound in ethanol was added concentrated hydrochloric acid, followed by concentration under reduced pressure, whereby the hydrochloride (colorless crystals) of the title compound was obtained.

Melting point: 180 to 181°C

| Elementary analysis for $C_{15}H_{20}ClN$ | | | | |
|---|---|---|---|---|
| Calculated: | C, 72.13; | H, 8.07, | Cl, 14.19; | N, 5.61 |
| Found: | C, 71.77; | H, 8.04; | Cl, 14.12; | N, 5.30 |

In the same manner as in Referential Example 5 except for the use of 5-(1,3-benzodioxol-5-yl)pentanol, 5-(2-benzothiazolyl)pentanol and 5-(2-benzoxazolyl)pentanol instead of 5-(2-naphthyl)pentanol, the compounds of Referential Examples 6 to 8 were obtained, respectively.

Referential Example 6

5-(1,3-Benzodioxol-5-yl)pentylamine

(1) 1-[5-(1,3-Benzodioxol-5-yl)pentyl]phthalimide

Mass (FAB+) m/Z: 337($M^+$).
$^1$H-NMR($CDCl_3$)δ: 1.30-1.40(2H,m), 1.55-1.75(4H,m), 2.52(2H,t,J=7.6Hz), 3.67(2H,t,J=7.3Hz), 5.90(2H,s), 6.59(1H,dd,J=7.8,1.0Hz), 6.45(1H,s), 6.69(1H,d,J=7.8Hz), 7.68-7.87(4H,m).

(2) 5-(1,3-Benzodioxol-5-yl)pentylamine

Mass (EI) m/Z: 207($M^+$).
$^1$H-NMR($CDCl_3$)δ: 1.28-1.62(8H,m), 2.53(2H,t,J=7.8Hz), 2.69(2H,t,J=7.1Hz), 5.91(2H,s), 6.61(1H,dd,J=7.8,1.5Hz), 6.67(1H,d,J=1.5Hz), 6.72(1H,d,J=7.8Hz).

Referential Example 7

5-(2-Benzothiazolyl)pentylamine

(1) 1-[5-(2-Benzothiazolyl)pentyl]phthalimide

Mass (FAB+) m/Z: 351$(M+H)^+$.
$^1$H-NMR($CDCl_3$)δ: 1.33-1.45(2H,m), 1.60-1.70(2H,m), 1.78-1.88(2H,m), 3.05-3.12(2H,m), 3.55-3.62(2H,m), 7.35-8.00(8H,m)

(2) 5-(2-Benzothiazolyl)pentylamine

$^1$H-NMR($CDCl_3$)δ: 1.42-1.60(4H,m), 1.85-2.00(2H,m), 2.68-2.78(2H,m), 3.10-3.20(2H,m), 7.32-7.50(2H,m), 7.84(1H,d,J=8.3Hz), 7.96(1H,d,J=7.8Hz).

Referential Example 8

5-(2-Benzoxazolyl)pentylamine

(1) 5-(2-Benzoxazolyl)pentanol

Monoethyl adipate (2.0 g) was dissolved in benzene (20 ml), followed by the dropwise addition of oxalyl chloride (5 ml) under cooling with ice water. After stirring for one hour, the reaction mixture was concentrated to dryness under reduced pressure. Benzene was added to the residue, followed by azeotropic distillation twice. The residue was dissolved in toluene (30 ml). To the resulting solution was added 2-aminophenol (1.25 g), followed by refluxing under heat for 16 hours. After cooling, the reaction mixture was washed with water and dried over anhydrous sodium sulfate. The solvent was then distilled off under reduced pressure. The residue was purified by chromatography on a silica gel column (elution with 33% ethyl acetate-hexane), whereby ethyl 5-(2-benzoxazolyl)pentanoate (1.11 g) was obtained as a pale yellow solid.

Mass (FAB+) m/Z: 248$(M+H)^+$.
$^1$H-NMR($CDCl_3$)δ: 1.25(3H,t,J=7.3Hz), 1.70-1.83(2H,m), 1.90-2.00(2H,m), 2.38(2H,t,J=7.6Hz), 2.96(2H,t,J=7.6Hz), 4.12(2H,q,J=7.3Hz), 7.27-7.00(4H,m).

The resulting ethyl ester (1.11 g) was dissolved in tetrahydrofuran (50 ml). To the resulting solution was added diisobutyl aluminum hydride (0.95M solution in hexane, 12 ml) at -78°C, and the mixture was stirred for one hour. To the reaction mixture was added sodium sulfate • 10 hydrate and the resulting mixture was heated to room temperature. After the filtration of the insoluble matter, the filtrate was distilled off under reduced pressure. The residue was purified by chromatography on a silica gel column (elution with 50% ethyl acetate-hexane), whereby the title compound (0.62 g) was obtained as a pale yellow solid.

Mass (FAB+) m/Z: 206(M+H)$^+$.
$^1$H-NMR($CDCl_3$)δ: 1.45-1.70(4H,m), 1.85-2.10(3H,m), 2.95(2H,t,J=7.6Hz), 3.67(2H,t,J=6.3Hz), 7.27-7.30(2H,m), 7.45-7.50(1H,m), 7.65-7.70(1H,m).

(2) 1-[5-(2-Benzoxazolyl)pentyl]phthalimide

$^1$H-NMR($CDCl_3$)δ: 1.70-1.80(2H,m), 1.90-2.00(2H,m), 2.93(2H,t,J=7.6Hz), 3.73(2H,t,J=7.1Hz), 7.22-7.88(8H,m).

(3) 5-(2-Benzoxazolyl)pentylamine

Mass (FAB+) m/Z: 335(M+H)$^+$.
$^1$H-NMR($CDCl_3$)δ: 1.40-1.60(4H,m), 1.85-2.06(4H,m), 2.70-2.80(2H,m), 2.90-3.00(2H,M), 7.25-7.70(4H,m).

Referential Example 9

4-tert-Butyldiphenylsilyloxy-1-iodo-2-butene

To a solution of triphenylphosphine (152.4 g) and imidazole (55.4 g) in methylene chloride (800 ml) was added dropwise a solution of iodine powder (148.5 g) in methylene chloride (1200 ml) under cooling with ice water. After stirring for 30 minutes under cooling with ice water, a solution of (Z)-4-tert-butyldiphenylsilyloxy-2-buten-1-ole (148.4 g) in methylene chloride (500 ml) was added dropwise. After stirring for 4 hours, an aqueous solution of sodium thiosulfate was added to separate the organic layer. The organic layer was washed with saturated saline and dried over anhydrous sodium sulfate. The solvent was then distilled off under reduced pressure. The residue was purified by chromatography on a silica gel column (elution with 17% ethyl acetate-hexane), whereby the title compound (164.0 g) was obtained as a yellow oil.

$^1$H-NMR($CDCl_3$)δ: 0.99(9Hs), 3.86(2H,d,J=7.8Hz), 4.11(2H,d,J=4.4Hz), 5.71(1H,dt,J=15.1,4.4Hz), 5.96(1H,dt,J=15.1,7.8Hz), 7.30-7.37(6H,m), 7.59-7.61(4H,m).

Referential Example 10

(3,4-Dimethylphenylmethyl)triphenylphosphonium chloride

A mixture of 3,4-dimethylphenylmethyl chloride (15.5 g), triphenylphosphine (26.2 g) and o-xylene (200 ml) was heated at reflux for 16 hours. After cooling, the insoluble solid was collected by filtration, washed with diethyl ether, dried at 60°C for 7 hours under reduced pressure, and provided for use.

In a similar manner except for the use of, instead of 3,4-dimethylphenylmethyl chloride, corresponding chloride or bromide, (2-chlorophenylmethyl)triphenylphosphonium chloride, (3,4-dichlorophenylmethyl)triphenylphosphonium bromide, (4-trifluorphenylmethyl)triphenylphosphonium bromide, [(1,3-benzodioxol-5-yl)methyl]triphenylphosphonium bromide, (2-benzoxazolylmethyl)triphenyiphoshonium bromide, (2-benzothiazolylmethyl)triphenylphosphonium bromide and (3-chloro-4-methylphenylmethyl)triphenylphosphonium chloride were prepared, respectively.

As the triphenylphosphonium salts other than the above, commercially available ones were employed.

Referential Example 11

In methylene chloride (30 ml), 3,5-dimethylaniline (2.0 g) was dissolved, followed by the addition of 2,4-dinitrobenzenesulfonyl chloride (5.3 g) and pyridine (1.6 ml) under cooling with ice water. After stirring at room temperature

for 3 hours, the reaction mixture was washed with water and saturated saline and dried over anhydrous sodium sulfate. The solvent was then distilled off under reduced pressure. The residue was separated and purified by chromatography on a silica gel column (elution with 20% ethyl acetate-hexane), whereby the compound (4.63 g) of Referential Example 11-c was obtained as a yellow powder.

In the same manner as in the above example except for the use of, instead of 3,5-dimethylaniline, the corresponding aniline derivatives, the reaction was effected, whereby the compounds of Referential Examples 11-a, b and d to m which will be described below were obtained.

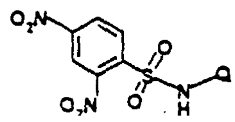

| Ref. Ex. | Q | $^1$H-NMR($CDCl_3$) σ: |
|---|---|---|
| 11-a | 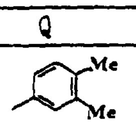 | 2.19(6H, s), 6.89(1H, dd, J=2.0 and 8.3Hz), 6.96(1H, d, J=2.0Hz), 7.02(1H, d, J=8.3Hz), 7.16(1H, s), 8.03(1H, d, J=8.8Hz), 8.37(1H, dd, J=2.4 and 8.8Hz), 8.66(1H, d, J=2.4Hz) |
| 11-b | 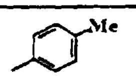 | 2.30(3H, s), 7.06(2H, d, J=8.3Hz), 7.09(2H, d, J=8.3Hz), 8.01(1H, d, J=8.8Hz), 8.37(1H, dd, J=2.4 and 8.8Hz), 8.37(1H, dd, J=2.4 and 8.8Hz), 8.66(1H, d, J=2.4Hz) |
| 11-c | 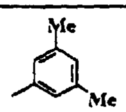 | 2.25(6H, s), 6.81(2H, s), 6.85(1H, s), 8.08(1H, d, J=8.8Hz), 8.40(1H, dd, J=8.8,2.0Hz), 8.67(1H, d, J=2.0Hz) |
| 11-d | 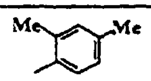 | 2.20(3H, s), 2.28(3H, s), 6.93(1H, d, J=8.3Hz), 7.00(1H, d, J=8.3Hz), 7.01(1H, broad s), 7.06(1H, s), 8.02(1H, d, J=8.3Hz), 8.41(1H, dd, J=1.5 and 8.3Hz), 8.69(1H, d, J=1.5Hz) |
| 11-e | 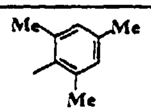 | 2.10(6H, s), 2.27(3H, s), 6.89(2H, s), 6.94(1H, s), 8.14(1H, d, J=8.3Hz), 8.49(1H, dd, J=2.0 and 8.3Hz), 8.73(1H, d, J=2.0Hz) |
| 11-f | 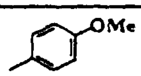 | 3.76(3H, s), 6.79(2H, dd, J=2.0 and 6.8Hz), 7.09(2H, dd, J=2.0 and 6.8Hz), 7.35(1H, s), 7.96(1H, d, J=8.8Hz), 8.37(1H, dd, J=2.0 and 8.8Hz), 8.64(1H, d, J=2.0Hz) |
| 11-g | 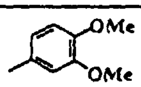 | 3.83(3H, s), 3.85(3H, s), 6.59(1H, dd, J=2.0 and 8.3Hz), 6.69(1H, d, J=8.3Hz), 6.84(1H, d, J=2.4Hz), 7.16(1H, broad s), 8.00(1H, d, J=8.8Hz), 8.38(1H, dd, J=2.0 and 8.8Hz), 8.67(1H, d, J=2.0Hz) |
| 11-h | 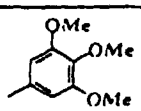 | 3.79(9H, s, 6.43(2H, s), 8.11(1H, d,, 8.3Hz), 8.43(1H, dd, J=2.0 and 8.3Hz), 8.67(1H, d, J=2.0Hz) |

| | | |
|---|---|---|
| 11-i | 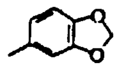 | 5.30(1H, s), 5.98(2H, s), 6.57(1H, d, J=7.8Hz), 6.67(1H, d, J=7.8Hz), 6.77(1H, s), 8.04(1H, d, J=7.8Hz), 8.41(1H, d, J=7.8Hz), 8.67(1H, s) |
| 11-j | 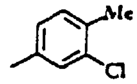 | 2.30(3H, s), 7.00(1H, dd, J=2.4 and 8.3Hz), 7.15(1H, d, J=8.3Hz), 7.22(1H, d, J=2.4Hz), 8.08(1H, d, J=8.3Hz), 8.42(1H, dd, J=2.0 and 8.3Hz), 8.66(1H, d, 2.0Hz) |
| 11-k | 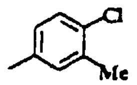 | 2.31(3H, s), 7.00(1H, dd, J=2.0 and 8.3Hz), 7.15(1H, d, J=8.3Hz), 7.23(1H, d, J=2.0z), 8.08(1H, d, J=8.3Hz), 8.42(1H, dd, J=2.4 and 8.3Hz), 8.68(1H, d, J=2.4Hz) |
| 11-l | 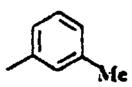 | 2.30(3H, s), 6.99(1H, d, J=8.8Hz), 7.02(1H, s), 7.18(1H, d, J=7.8Hz), 7.16(1H, d, J=7.8Hz), 7.18(1H, d, J=8.8Hz), 8.06(1H, d, J=8.8Hz), 8.37(1H, dd, J=2.4 and 8.8Hz), 8.65(1H, d, J=2.4Hz) |
| 11-m | 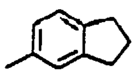 | 2.05(2H, dt, J=7.3 and 7.8Hz), 2.82-2.86(4H, m), 6.88(1H, dd, J=2.9 and 8.3Hz), 7.07(1H, d, J=8.3Hz), 7.22(1H, d, J=2.9Hz), 8.01(1H, d, J=8.3Hz), 8.36(1H, dd, J=2.4 and 8.3Hz), 8.65(1H, d, J=2.4Hz) |

Referential Example 12

6-(2-Naphthyl)hexyl bromide

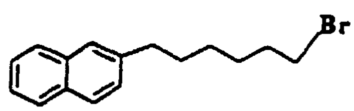

In methylene chloride (100 ml), 2-naphthol (10.0 g) was dissolved, followed by the successive addition of diisopropylethylamine (15 ml) and trifluoromethanesulfonic anhydride (20 g) under cooling with ice water. After stirring at room temperature for 6 hours, the reaction mixture was diluted with diethyl ether and then washed with water. After drying over anhydrous sodium sulfate, the solvent was distilled off under reduced pressure. The residue was purified by chromatography on a silica gel column (elution with 20% ethyl acetate-hexane), whereby 2-naphthyl trifluoromethanesulfonate (19.1 g) was obtained as a colorless oil.

The resulting compound (19.1 g) was dissolved in triethylamine (200 ml). To the resulting solution were added 5-hexyn-1-ol (7.0 g), copper (I) iodide (0.18 g) and bis(triphenylphosphine)palladium dichloride (0.4 g), and the mixture was stirred at 60°C for 10 hours. After dilution with diethyl ether, the insoluble matter was filtered off. The filtrate was concentrated under reduced pressure and the residue was partially purified by chromatography on a silica gel column (elution with 20% ethyl acetate-hexane), whereby crude 6-(2-naphthyl)-5-hexyn-1-ol (14.5 g) was obtained as an oil.

The resulting compound was dissolved in ethanol (100 ml). To the resulting solution was added 10% palladium-carbon (0.5 g), and the mixture was stirred under hydrogen atmosphere for 24 hours. The palladium-carbon was filtered off and the filtrate was concentrated to dryness under reduced pressure. The residue was purified by chromatography on a silica gel column (elution with 17% ethyl acetate-hexane), whereby 6-(2-naphthyl)-1-hexanol (8.0 g) was obtained as an oil.

The resulting compound (6.6 g) was dissolved in methylene chloride (100 ml). To the resulting solution were added dibromotriphenylphosphine (14.6 g) and imidazole (2.0 g), and the mixture was stirred at room temperature for 6 hours. After dilution with diethyl ether, the insoluble matter was filtered off. The filtrate was concentrated under

reduced pressure and the residue was purified by chromatography on a silica gel column (elution with 20% ethyl acetate-hexane), whereby the title compound (7.0 g) was obtained as an oil.

$^1$H-NMR($CDCl_3$)δ: 1.30-1.90(8H,m), 2.78(2H,t,J=7.3Hz), 3.40(2H,t,J=6.8Hz), 7.28-7.50(3H,m), 7.61(1H,s), 7.72-7.85(3H,m).

Referential Example 13

5-(3,4-Dimethylphenyl)pent-2-en-1-yl iodide

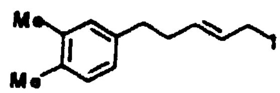

(1) 3-(3,4-Dimethylphenyl)-2-propin-1-ol

To a mixture of 4-iodo-o-xylene (30 g), dichlorobis(triphenylphosphine)palladium (II) (1.81 g), copper (I) iodide (0.49 g) and triethylamine (500 ml) was added 2-propin-1-ol (10.9 g), and the mixture was stirred at room temperature for 21 hours under argon gas atmosphere. The insoluble matter was filtered off by using Celite and washed with hexane. The filtrate and washing were combined, followed by distillation under reduced pressure. The residue was purified by chromatography on a silica gel column (elution with 15 to 20% ethyl acetate-hexane), whereby the title compound (21.2 g) was obtained as a yellow oil.

MS (EI) m/Z: 160($M^+$).
$^1$H-NMR($CDCl_3$)δ: 1.80(1H,t,J=6.4Hz), 2.22(3Hs), 2.24(3H,s), 4.48(2H,d,J=6.4Hz), 7.06(1H,d,J=7.6Hz), 7.17(1H,d,J=7.6Hz), 7.22(1H,s).

(2) 3-(3,4-Dimethylphenyl)-1-propanol

In ethanol (300 ml), 3-(3,4-dimethylphenyl)-2-propin-1-ol (21.1 g) was dissolved. To the resulting solution was added 10% palladium-carbon (0.5 g), and the mixture was stirred at room temperature for 3 days under hydrogen atmosphere. The palladium-carbon was filtered off and the filtrate was concentrated to dryness under reduced pressure. The residue was dissolved in methylene chloride, followed by drying over anhydrous sodium sulfate. The solvent was then distilled off under reduced pressure, whereby the title compound (20.3 g) was obtained as a colorless oil.

MS (EI+) m/Z: 164($M^+$).
$^1$H-NMR($CDCl_3$)δ: 1.83-1.90(2H,m), 2.22(3H,s), 2.23(3H,s), 2.63(2H,t,J=7.8Hz), 3.66(2H,t,J=6.6Hz), 6.93(1H,d,J=7.6Hz), 6.97(1H,s), 7.04(1H,d,J=7.6Hz).

(3) 3-(3,4-Dimethylphenyl)propanal

Oxalyl chloride (12.9 ml) was added to methylene chloride (200 ml), followed by cooling to -78°C and stirring. A solution of dimethylsulfoxide (17.4 ml) in methylene chloride (50 ml) was added dropwise over 55 minutes and the resulting mixture was stirred for further 15 minutes. A solution of 3-(3,4-dimethylphenyl)-1-propanol (20.2 g) in methylene chloride (150 ml) was added dropwise over 1 hour and the resulting mixture was stirred for 50 minutes. Triethylamine (86 ml) was added dropwise and the resulting mixture was stirred for 15 minutes. After stirring for one hour under cooling with ice water, water (500 ml) was added to separate the organic layer. The resulting organic layer was washed successively with dilute hydrochloric acid and saturated saline and dried over anhydrous sodium sulfate. The solvent was then distilled off under reduced pressure. The residue was purified by chromatography on a silica gel column (elution with 5% ethyl acetate-hexane), whereby the title compound (16.9 g) was obtained as a pale yellow oil.

MS (EI) m/Z: 162($M^+$).
$^1$H-NMR($CDCl_3$)δ: 2.22(3H,s), 2.23(3H,s), 2.72-2.76(2H,m), 2.87(2H,t,J=7.6Hz), 6.91-6.93(1H,m), 6.96(1H,s),

7.05(1H,d,J=7.3Hz), 9.81(1H,t,J=1.5Hz).

(4) Ethyl 5-(3,4-dimethylphenyl)pent-2-enoate

In the same manner as in Referential Example 3-(1), synthesis was carried out using the 3-(3,4-dimethylphenyl)propanal obtained in (3), whereby the title compound (22.1 g) was obtained as a pale yellow oil.

MS (EI) m/Z: 232($M^+$).
$^1$H-NMR($CDCl_3$)δ: 1.28(3H,t,J=7.3Hz), 2.22(3H,s), 2.23(3H,s), 2.46-2.52(2H,m), 2.46-2.52(2H,m), 2.70(2H,t,J=7.8Hz), 4.18(2H,q,J=7.3Hz), 5.85(1H,d,J=15.6Hz), 6.91(1H,d,J=7.8Hz), 6.95-7.05(3H,m)

(5) 5-(3,4-Dimethylphenyl)pent-2-en-1-ol

In the same manner as in Referential Example 3-(1), synthesis was carried out using the ethyl 5-(3,4-dimethylphenyl)pent-2-enoate (22.0 g) obtained in (4), whereby the title compound (15.4 g) was obtained as a colorless oil.

MS (EI) m/Z: 190($M^+$).
$^1$H-NMR($CDCl_3$)δ: 2.22(3H,s), 2.23(3H,s), 2.31-2.37(2H,m), 2.63(2H,t,J=7.8Hz), 4.03-4.11(2H,m), 5.66-5.74(2H,m), 6.91(1H,d,J=7.6Hz), 6.95(1H,s), 7.04(1H,d,J=7.6Hz).

(6) 5-(3,4-Dimethylphenyl)pent-2-en-1-yl iodide

In the same manner as in Referential Example 3-(1), synthesis was carried out using the 5-(3,4-dimethylphenyl)pent-2-en-1-ol (15.4 g) obtained in (5), the title compound (23.5 g) was obtained as a pale yellow oil.

MS (EI) m/Z: 300($M^+$).
$^1$H-NMR($CDCl_3$)δ: 2.22(3H,s), 2.23(3H,s), 2.30-2.34(2H,m), 2.61(2H,t,J=7.8Hz), 3.86(2H,dd,J=4.9,2.0Hz), 5.73-5.76(2H,m), 6.90(1H,d,J=7.6Hz), 6.93(1H,s), 7.04(1H,d,J=7.6Hz).

Referential Example 14

Methyl[5-(2-naphthyl)pentyl]amine hydrochloride

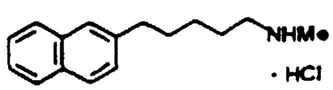

(1) Ethyl N-[5-(2-naphthyl)pentyl]carbamate

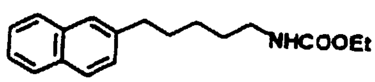

The [5-(2-naphthyl)pentyl]amine hydrochloride (20 g) synthesized in Referential Example 5 was suspended in methylene chloride (200 ml), followed by the addition of triethylamine (20 g) under stirring. To the reaction mixture was added dropwise ethyl chlorocarbonate (10.5 g) over 5 minutes under cooling with ice water, and the resulting mixture was stirred at room temperature for 2 hours. The solvent was then distilled off under reduced pressure. The residue was dissolved in ethyl acetate. The resulting solution was washed with dilute hydrochloric acid, 5% sodium bicarbonate and saturated saline and dried over anhydrous sodium sulfate. The solvent was distilled off under reduced pressure and the

residue was purified by chromatography on a silica gel column (elution with 30% ethyl acetate-hexane), whereby the title compound (19.5 g) was obtained as colorless crystals.

Melting point: 44 to 46°C
MS (EI) m/Z: 285($M^+$).
$^1$H-NMR($CDCl_3$)δ: 1.22(3H,t,J=6.8Hz), 1.35-1.42(2H,m), 1.50-1.58(2H,m), 1.69-1.75(2H,m), 2.77(2H,t,J=7.6Hz), 3.10-3.19(2H,m), 4.09(2H,q,J=6.8Hz), 4.35-4.63(1H,broad), 7.31(1H,d,J=8.3Hz), 7.39-7.46(2H,m), 7.59(1H,s), 7.75-7.80(3H,m)

| Elementary analysis for $C_{18}H_{23}NO_2$ | | | |
|---|---|---|---|
| Calculated: | C, 75.76; | H, 8.12, | N, 4.91. |
| Found: | C, 75.68; | H, 8.12; | N, 4.86. |

(2) Methyl[5-(2-naphthyl)pentyl]amine hydrochloride

Lithium aluminum hydride (7.78 g) was suspended in methylene chloride (200 ml), followed by the dropwise addition of a solution of the carbamate (19.49 g) obtained in (1) in tetrahydrofuran (80 ml) under stirring. After refluxing under heat for 4.5 hours, the reaction mixture was cooled with ice water and stirred. Sodium sulfate • 10 hydrate was added in portions to decompose excess lithium aluminum hydride, followed by stirring for further 30 minutes. The insoluble matter was filtered through Celite and washed with tetrahydrofuran. The filtrate and washing were combined and the mixture was distilled under reduced pressure, whereby the free base of the title compound was obtained.

The resulting free base was dissolved in ethanol (200 ml) and to the resulting solution, concentrated hydrochloric acid (7 ml) was added. The resulting mixture was concentrated under reduced pressure. Ether was added to the concentrate and the solid so precipitated was collected by filtration, whereby the title compound (14.8 g) was obtained as colorless crystals.

Melting point: 150 to 158°C
MS (FAB+) m/Z: 228 (free base + $H)^+$.
$^1$H-NMR($CD_3OD$)δ: 1.40-1.49(2H,m), 1.67-1.82(4H,m), 2.65(3H,s), 2.82(2H,t,J=7.3Hz), 2.95(2H,t,J=7.6Hz), 7.31-7.45(3H,m), 7.63(1H,s), 7.72-7.79(3H,m).

| Elementary analysis for $C_{16}H_{22}ClN$ | | | | |
|---|---|---|---|---|
| Calculated: | C, 72.85; | H, 8.41, | Cl, 13,44; | N, 5.31. |
| Found: | C, 72.71; | H, 8.42; | Cl, 13.38; | N, 5.29. |

Referential Example 15

(5-Hydroxypentyl)methylamine

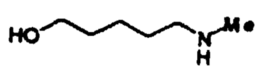

(1) Benzyl N-(5-hydroxypentyl)-N-methylcarbamate

In the same manner as in Referential Example 14-(1), 5-hydroxypentylamine (1.03 g) was treated to obtain ethyl N-(5-hydroxypentyl)carbamate (1.74 g). The resulting compound (0.74 g) was treated in the same manner as in Referential Example 14-(2), whereby slightly crude (5-hydroxypentyl)methylamine (free base, 0.68 g) was obtained. The resulting compound was dissolved in methylene chloride (8 ml). To the resulting solution were added triethylamine (0.6 ml) and benzyl chlorocarbonate (0.6 ml) under cooling with ice water, and the mixture was stirred at room temperature for one day. The reaction mixture was concentrated under reduced pressure and the residue was dissolved in ethyl acetate. The resulting solution was washed successively with 1N hydrochloric acid, 1N sodium hydroxide and saturated saline and dried over anhydrous sodium sulfate. The solvent was then distilled off under reduced pressure. The residue was purified by chromatography on a silica gel column (elution with 33 to 50% ethyl acetate-hexane), whereby the title compound (0.64 g) was obtained as a colorless oil.

$^1$H-NMR($CDCl_3$)δ: 1.37-1.61(6H,m), 2.92(3H,s), 3.30(2H,m), 3.58-3.64(2H,m), 5.12(2H,s), 7.35(5H,s).

(2) (5-Hydroxypentyl)methylamine

The carbamate compound (0.64 g) obtained in (1) was dissolved in methanol (10 ml). To the resulting solution was added 10% palladium-carbon (64 mg), and the mixture was stirred at room temperature for 8 hours under hydrogen atmosphere. The palladium-carbon was filtered off and the filtrate was concentrated to dryness under reduced pressure, whereby the title compound (0.28 g) was obtained as a pale yellow oil.

MS (FAB+) m/Z: 118$(M+H)^+$.
$^1$H-NMR($CDCl_3$)δ: 1.38-1.63(6H,m), 1.79(1H,broad), 2.43(3H,s), 2.59(2H,t,J=7.1Hz), 3.64(2H,m).

Test

The squalene synthetase inhibitory activity and cholesterol synthesis inhibitory activity in rat liver cells, each of the invention compound, were confirmed by the below-described methods.

1. Squalene synthetase inhibitory activity

(1) Preparation of enzyme source

As an enzyme source to measure squalane synthetase activity, we prepared a microsome fraction from a human hepatoma-derived cell line, HepG2 cells, according to the method described by Schechter et al. (Journal of Biological Chemistry, Vol. 267, pp.8628-8635, 1992).

Described specifically, HepG2 cells were first homogenized in the presence of a 10 mM N-2-hydroxyethylpiperazin-N'-2-ethanesulfonate (HEPES) buffer containing 0.3M sucrose, 1 mM dithiothreitol (DTT), 1 mM sodium ethylenediaminetetraacetate (EDT) and various protease inhibitors and adjusted to pH 7.4. The resulting mixture was subjected to centrifugal separation at 2,000 x g for 5 minutes and then 10,000 x g for 15 minutes. The protease inhibitors employed were phenylmethanesulfonyl fluoride (PMSF), leupeptin and aprotinin, which were added to give the final concentration of 1 mM, 10 μM and 5 μg/ml, respectively. The supernatant after centrifugation was centrifuged further at 105,000 xg for 60 minutes. The precipitate thus obtained was suspended in a 20 mM phosphate buffer (pH 7.4) containing 1 mM DTT, 1 mM EDTA and the above-described protease inhibitors and the resulting suspension was washed twice by centrifugation at 105,000 x g for 30 minutes. The precipitate was then suspended in a 20 mM phosphate buffer (pH 7.4) containing 1 mM DTT and 1 mM EDTA and the resulting suspension was similarly centrifuged. The precipitate obtained finally was provided as the microsome fraction for the measurement of the enzyme activity.

(2) Measurement of squalene synthetase inhibitory activity

The measurement of squalene synthetase activity was also carried out in accordance with the method of Schechter et al. reported in "Journal of Biological Chemistry, Vol. 267, pp.8628-8635 (1992)".

Described specifically, a test medicament dissolved in water or dimethylsulfoxide (DMSO) was added to an enzyme reaction mixture (50 μl in total) containing 5 mM nicotinamide adenine dinucleotide phosphate reduced form (NADPH), 5 mM 3-[(3-cholamidopropyl)dimethylammonio]propanesulfonate (CHAPS), 10 mM potassium fluoride, 10 mM magnesium chloride, 10 mM DTT, 1 μg/ml of NB-598 (a squalene epoxidase inhibitor described in the Journal of Biological Chemistry, Vol. 265, pp. 18075-18078, 1990), 50 mM HEPES buffer (pH 7.4), 0.01 to 0.1 μg of the HepG2

cell microsome preparation and 5 µM [$^3$H]farnesyl pyrophosphate, followed by reaction at 37°C for 20 minutes (reactions were initiated by addition of [$^3$H]farnesyl pyrophosphate to the enzyme reaction mixture which was preincubated at 37°C for 10 minutes). The enzyme reaction was terminated by addition of 5 µl of 1M EDTA (pH 9.2). Five µl of a 0.5% squalene-ethanol solution was added to the mixture. A 40 µl portion of the resulting mixture was spotted onto a plastic-backed thin-layer chromatography sheet and developed with 5% toluene-95% hexane. After being dried, the thin-layer chromatography sheet was placed in iodine vapor for color development, whereby the band of squalane was identified. The band was cut out by scissors, put into a vial and added with 10 ml of Aquasol-2 (New England Nuclear Research Product Inc./USA). The radioactivity was measured by a liquid scintillation counter. The squalane synthetase inhibitory activity was expressed by the concentration ($IC_{50}$, molar concentration) inhibiting 50% of the radioactivity incorporated in squalene.

2. Cholesterol synthesis inhibitory activity in rat liver cells

(1) Preparation of rat liver cells

Liver cells were isolated from rat livers by the *in situ* collagenase perfusion method. Described specifically, a 6-week-old male SD rat was intraperitoneally administered with a 50 mg/ml pentobarbital sodium solution and thereby anesthetized. After ventrotomy and cannulation from the portal vein, the rat was sacrificed under exsanguination by the excision of the lower large vein. From the cannula, a Hanks solution (pH 7.2) containing 2% albumin, 0.5 mM ethyleneglycolbis(2-aminoethylether) tetraacetate (EGTA) and 10 mM HEPES and being free from a divalent calcium ion and a divalent magnesium ion was subjected to perfusion (flow rate: 0.5 ml/sec) for 5 minutes. The Hanks solution was then changed to another Hanks solution (pH 7.5) having 0.05% collagenase, 4 mM calcium chloride and 10 mM HEPES added thereto and perfusion was continued for further 15 to 20 minutes. After confirmation of the sufficient digestion by collagenase, the liver tissue was placed on a Petri dish, 30 ml of a Dulbeco's modified Eagle's (DEM) medium was added thereto and cells were dispersed by drawing through suction. A cell filter was employed to remove the undigested tissue. The resulting cell suspension was subjected to centrifugal separation at 600 rpm for 1 minute. A 30 ml of DME medium was added to the sediment to disperse the cells, followed by centrifugal separation at 600 rpm for 1 minute. After these washing procedures by centrifugation were repeated three times in total, the liver cell suspension was suspended in a DME medium containing 10% of lipoprotein deficient serum (LPDS). The resulting suspension was seeded on a commercially-available 6-well plate coated with collagen at a cell density of 1 x $10^6$ cells per well, followed by incubation overnight in a $CO_2$ incubator (5% $CO_2$ gas, 37°C).

(2) Measurement of cholesterol synthesis inhibitory activity

The uptake of [$^{14}$C]-acetic acid radioactivity in cholesterol was measured using the liver cells prepared in the above-described manner and the cholesterol synthesis inhibitory activity of the test medicament was evaluated.

Described specifically, the cell culture medium of the liver cells was replaced by a 25 mM HEPES-10% LPDS-DME medium (pH 7.4) containing the test medicament, followed by incubation for one hour in a $CO_2$ incubator. Then, [$^{14}$C] sodium acetate was added to give the final concentration of 1 µCi/ml. After incubation for further one hour in the $CO_2$ incubator, the cell culture medium was removed. The cells were washed three times with a phosphate buffered saline (pH 7.2) and then dissolved in 1 ml of a 0.1N aqueous sodium hydroxide solution. A portion (10 µl) of the resulting cell lysate was subjected to protein determination in accordance with the Lowry's method. To the remaining portion were added 2 ml of ethanol and 0.5 ml of a 50% aqueous potassium hydroxide solution, followed by saponification at 75°C for 1 hour. After the completion of the saponification, [$^3$H]cholesterol of 50,000 dpm was added to the sample as an internal standard. Under ice cooling, 4.5 ml of petroleum ether was added and the resulting mixture was vigorously stirred, whereby unsaponified lipid was extracted. The resulting petroleum ether layer was transferred to another test tube, evaporated to dryness under a nitrogen gas stream and dissolved in 50 µl of dichloromethane-methanol (2:1) containing 10 mg/ml of cholesterol. The resulting solution was spotted to a plastic backed thin-layer chromatography sheet and developed with toluene-ethyl acetate (3:1). After the completion of the development, the sheet was dried and placed in an iodine vapor for color development, whereby the band of cholesterol was identified. The band was cut out by scissors and put into a scintillation vial. To the vial, 10 ml of Aquasol 2 was added and $^{14}$C and $^3$H radioactivities were counted by a liquid scintillation counter.

The cholesterol synthesis inhibitory activity of the test medicament was calculated in the below-described procedures. First, radioactivity of [$^3$H]cholesterol added as an internal standard was measured and based on the results, sample-to-sample differences in the extraction ratio with petroleum ether were corrected in accordance with the following formula:

$$\text{Uptake (dpm) of } [^{14}C] \text{ acetic acid radioactivity in cholesterol} = \frac{\text{Measuring results of } ^{14}C \text{ radioactivity (dpm) x 50,000 (dpm)}}{\text{Measuring results of } ^{3}H \text{ radioactivity (dpm)}}$$

Based on the above-described value, the uptake (unit: dpm/mg protein) of $^{14}C$ radioactivity per unit protein amount was determined using the protein concentration measuring results of the cell lysate. The cholesterol synthesis inhibitory activity of the test medicament was expressed by the concentration ($IC_{50}$, molar concentration) inhibiting 50% of the radioactivity uptake into cholesterol.

3. Experimental Results

Squalene synthetase inhibitory activity ($IC_{50}$) of the compound of Example 1, determined by the method 1 described above, was 0.3 nM. With regards to the compouonds of examples and those described in two patents, that is, (2S)-2- [N- {(1S,2R)-3-(3,4-dichlorophenyl)-1-methyl-2-(2-naphthoyloxy)propyl} carbamoylmethyl] succinic acid (compound A)(Japanese Patent Application Laid-Open No. Hei 7-138214) and N- [(3R,5S)-7-chloro-5-(2-chlorophenyl)-1-neopentyl-2-oxo-1,2,3,5-tetrahydro-4,1-benzoxazepin-3-acetyl] aminoacetic acid (coumpound B)(EP567026A), the cholesterol synthesis inhibitory action in rat liver cells was compared by method 2 (Table 1).

Table 1

| Cholesterol synthesis inhibitory action in rat liver cells | |
|---|---|
| Compound No. | $IC_{50}$ ($10^{-6}$M) |
| Example 1 | 0.15 |
| Example 20-n | 0.03 |
| Example 48 | 0.019 |
| Compound A | 0.32 |
| Compound B | 0.39 |

As described above, the invention compound has been confirmed to exhibit excellent squalene synthetase inhibitory action and cholesterol synthesis inhibitory action.

**Capability of Exploitation in Industry**

The compound of the present invention exhibits potent squalene synthetase inhibitory action and is therefore useful as a pharmaceutical for the remedy and prevention of hypercholesterolemia, hyperlipemia or arteriosclerosis.

**Claims**

**1.** A substituted propionyl derivative represented by the following formula (1):

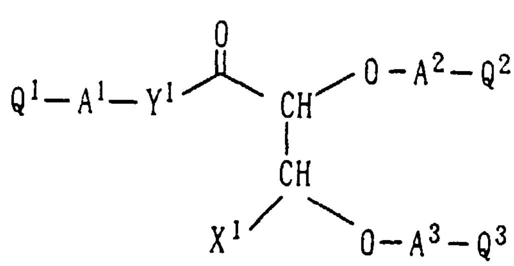

(1)

[wherein, $X^1$ represents a carboxyl, tetrazol-5-yl, phosphonic acid or sulfonic acid group which may be esterified;

$Y^1$ represents a single bond, -O- or -N($R^1$)- {wherein $R^1$ represents a hydrogen atom, a hydroxyl group or a

substituted or unsubstituted hydrocarbon group};
at least one of $A^1$, $A^2$ and $A^3$ represents a group represented by the following formula (2):

$$-R^2-a^1-R^3-a^2 \rightarrow \quad (2)$$

{wherein, $R^2$ represents a substituted or unsubstituted, divalent hydrocarbon group having 2 to 12 carbon atoms, $R^3$ represents a single bond or a substituted or unsubstituted, divalent hydrocarbon groups having 1 to 12 carbon atoms, $a^1$ and $a^2$ individually represent a single bond, -S-, -SO-, - $SO_2$-, -$SO_2$NH-, -O-, -N($R^4$)- {wherein $R^4$ represents a hydrogen atom, a hydroxyl group or a substituted or unsubstituted hydrocarbon group}, -CON($R^5$)- {wherein $R^5$ represents a hydrogen atom, a hydroxyl group or a substituted or unsubstituted hydrocarbon group}, -C(=O)- or -Si($R^6$)($R^7$)- {wherein $R^6$ and $R^7$ individually represent a substituted or unsubstituted hydrocarbon group}, and → means bonding with $Q^1$, $Q^2$ or $Q^3$}, the remaining one or two of $A^1$, $A^2$ and $A^3$ are the same or different and each independently represents a group represented by the following formula (3):

$$-R^8-a^3-R^9-a^4 \rightarrow \quad (3)$$

{wherein, $R^8$ and $R^9$ individually represent a single bond or a substituted or unsubstituted, divalent hydrocarbon group having 1 to 12 carbon atoms, $a^3$ and $a^4$ individually represent a single bond, -S-, -SO-, -$SO_2$-, -$SO_2$NH-, -O-, - N($R^{10}$)- {wherein $R^{10}$ represents a hydrogen atom, a hydroxyl group or a substituted or unsubstituted hydrocarbon group}, -CON($R^{11}$)- {wherein $R^{11}$ represents a hydrogen atom, a hydroxyl group or a substituted or unsubstituted hydrocarbon group], -C(=O)- or -Si($R^{12}$)($R^{13}$)- {wherein $R^{12}$ and $R^{13}$ individually represent a substituted or unsubstituted hydrocarbon group}, and → means bonding with $Q^1$, $Q^2$ or $Q^3$};
at least one of $Q^1$, $Q^2$ and $Q^3$ represents a substituted or unsubstituted cyclic hydrocarbon group or a substituted or unsubstituted heterocyclic group and the remaining one or two of $Q^1$, $Q^2$ and $Q^3$ individually represent a hydrogen atom, a carboxyl group which may be esterified, a substituted or unsubstituted hydrocarbon group or a substituted or unsubstituted heterocyclic group]; or salt thereof.

2. A substituted propionyl derivative according to claim 1, wherein $X^1$ represents a carboxyl group which may be esterified; or salt thereof.

3. A substituted propionyl derivative according to claim 1 or 2, wherein one or two of $A^1$, $A^2$ and $A^3$ are individually represented by the following formula (2a):

$$-R^2-a^1 \rightarrow \quad (2a)$$

{wherein $R^2$, $a^1$ and → have the same meanings as defined above} and the remaining one or two of $A^1$, $A^2$ and $A^3$ are the same or different and are individually represented by the following formula (3a):

$$-R^8-a^3-R^9-a^4 \rightarrow \quad (3a)$$

{wherein $R^8$, $R^9$, $a^3$, $a^4$ and → have the same meanings as defined above}; or salt thereof.

4. A substituted propionyl derivative according to any one of claims 1 to 3, which is represented by the following formula (1A):

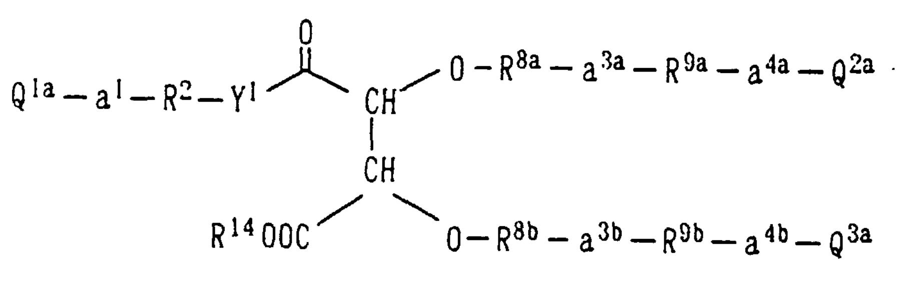

(1A)

[wherein $R^{14}$ represents a hydrogen atom or an ester residue,

$R^{8a}$ and $R^{8b}$ are the same or different and individually have the same meaning as defined in $R^8$,
$a^{3a}$ and $a^{3b}$ are the same or different and individually have the same meaning as defined in $a^3$,
$R^{9a}$ and $R^{9b}$ are the same or different and individually have the same meaning as defined in $R^9$,
$a^{4a}$ and $a^{4b}$ are the same or different and individually have the same meaning as defined in $a^4$,
$Q^{1a}$ represents a substituted or unsubstituted cyclic hydrocarbon group or a substituted or unsubstituted heterocyclic group,
either one of $Q^{2a}$ and $Q^{3a}$ represents a hydrogen atom or a carboxyl group which may be esterified and the other one represents a substituted or unsubstituted cyclic hydrocarbon group or a substituted or unsubstituted heterocyclic group, and
$Y^1$, $a^1$ and $R^2$ have the same meanings as described above]; or salt thereof.

**5.** A substituted propionyl derivative according to claim 4, wherein in the formula (1A), $Q^{1a}$ and $Q^{2a}$ are the same or different and individually represent a substituted or unsubstituted cyclic hydrocarbon group or a substituted or unsubstituted heterocyclic group and $Q^{3a}$ represents a hydrogen atom or a carboxyl group which may be esterified; or salt thereof.

**6.** A substituted propionyl derivative according to claim 4 or 5, wherein in the formula (1A), the group $Y^1$ represents -($NR^1$) [wherein $R^1$ has the same meaning as defined above]; or salt thereof.

**7.** A substituted propionyl derivative or salt thereof according to claim 4 or 5, wherein in the formula (1A), the group $Y^1$ represents -($NR^1$)- [wherein $R^1$ represents a hydrogen atom or an alkyl group]; or salt thereof.

**8.** A substituted propionyl derivative or salt thereof according to any one of claims 4 to 7, wherein in the formula (1A), the group $-R^{8a}-a^{3a}-R^{9a}-$ and $-R^{8b}-a^{3b}-R^{9b}-$ are the same or different and each independently represents a substituted or unsubstituted, divalent $C_{1-12}$ hydrocarbon group; or salt thereof.

**9.** A substituted propionyl derivative according to any one of claims 4 to 8, wherein in the formula (1A), the group $-R^{8b}-a^{3b}-R^{9b}-a^{4b}\rightarrow$ represents a single bond or a $C_{1-3}$ alkylene group; or salt thereof.

**10.** A substituted propionyl derivative according to any one of claims 4 to 8, wherein in the formula (1A), the group $-R^{8b}-a^{3b}-R^{9b}-a^{4b}\rightarrow$ represents a single bond; or salt thereof.

**11.** A substituted propionyl derivative according to any one of claims 4 to 8, wherein in the formula (1A), the group $-R^{8b}-a^{3b}-R^{9b}-a^{4b}\rightarrow$ represents a methylene group; or salt thereof.

**12.** A substituted propionyl derivative according to any one of claims 4 to 11, wherein in the formula (1A), the group $a^1$ represents a single bond or -($NR^4$)- [wherein $R^4$ has the same meaning as defined above]; or salt thereof.

**13.** A substituted propionyl derivative according to any one of claims 4 to 11, wherein in the formula (1A), the group $a^1$ represents a single bond; or salt thereof.

**14.** A substituted propionyl derivative according to any one of claims 4 to 11, wherein in the formula (1A), the group $a^1$ represents -($NR^4$)- [wherein $R^4$ has the same meaning as defined above]; or salt thereof.

**15.** A substituted propionyl derivative according to any one of claims 4 to 11, wherein in the formula (1A), the group $a^1$ represents -($NR^4$)- [wherein $R^4$ represents a hydrogen atom]; or salt thereof.

**16.** A substituted propionyl derivative according to any one of claims 4 to 15, wherein in the formula (1A), the group $a^{4a}$ represents a single bond or -($NR^{10}$)- [wherein $R^{10}$ has the same meaning as defined above]; or salt thereof.

**17.** A substituted propionyl derivative according to any one of claims 4 to 15, wherein in the formula (1A), the group $a^{4a}$ represents a single bond; or salt thereof.

**18.** A substituted propionyl derivative according to any one of claims 4 to 15, wherein in the formula (1A), the group $a^{4a}$ represents -($NR^{10}$)- [wherein $R^{10}$ has the same meaning as defined above]; or salt thereof.

**19.** A substituted propionyl derivative according to any one of claims 4 to 15, wherein in the formula (1A), the group $a^{4a}$ represents -($NR^{10}$)- [wherein $R^{10}$ represents a hydrogen atom]; or salt thereof.

**20.** A substituted propionyl derivative according to claim 4, wherein in the formula (1A),

$R^{14}$ represents a hydrogen atom or an ester residue,
$Q^{1a}$ and $Q^{2a}$ are the same or different and individually represent a substituted or unsubstituted cyclic hydrocarbon group or a substituted or unsubstituted heterocyclic group,
$Q^{3a}$ represents a hydrogen atom or a carboxyl group which may be esterified,
$a^1$ represents a single bond or $-(NR^4)-$ [wherein $R^4$ has the same meaning as defined above],
$R^2$ represents a substituted or unsubstituted divalent hydrocarbon group having 1 to 12 carbon atoms,
$Y^1$ represents $-(NR^1)-$ [wherein $R^1$ has the same meaning as defined above],
the group $-R^{8a}-a^{3a}-R^{9a}-$ represents a substituted or unsubstituted, divalent hydrocarbon group having 1 to 12 carbon atoms,
$a^{4a}$ represents a single bond or $-(NR^{10})-$ [wherein $R^{10}$ has the same meaning as defined above],
$R^{8b}$ has the same meaning as defined in $R^6$,
$a^{3b}$ has the same meaning as defined in $a^3$,
$R^{9b}$ has the same meaning as defined in $R^9$ and
$a^{4b}$ has the same meaning as defined in $a^4$; or salt thereof.

**21.** A substituted propionyl derivative according to claim 4, wherein in the formula (1A), $R^{14}$ represents a hydrogen atom or an ester residue,

$Q^{1a}$ and $Q^{2a}$ are the same or different and individually represent a substituted or unsubstituted cyclic hydrocarbon group or a substituted or unsubstituted heterocyclic group,
$Q^{3a}$ represents a hydrogen atom or a carboxyl group which may be esterified,
$a^1$ represents a single bond or $-(NR^4)-$ [wherein $R^4$ has the same meaning as defined above],
$R^2$ represents a substituted or unsubstituted divalent hydrocarbon group having 1 to 12 carbon atoms,
$Y^1$ represents $-(NR^1)-$ [wherein $R^1$ has the same meaning as defined above],
the group $-R^{8a}-a^{3a}-R^{9a}-$ represents a substituted or unsubstituted, divalent hydrocarbon group having 1 to 12 carbon atoms,
$a^{4a}$ represents a single bond or $-(NR^{10})-$ [wherein $R^{10}$ has the same meaning as defined above], and
$-R^{8b}-a^{3b}-R^{9b}-a^{4b}-$ represents a single bond or a substituted or unsubstituted, divalent hydrocarbon group having 1 to 12 carbon atoms; or salt thereof.

**22.** A substituted propionyl derivative according to any one of claims 1 to 3, which is represented by the following formula (1B):

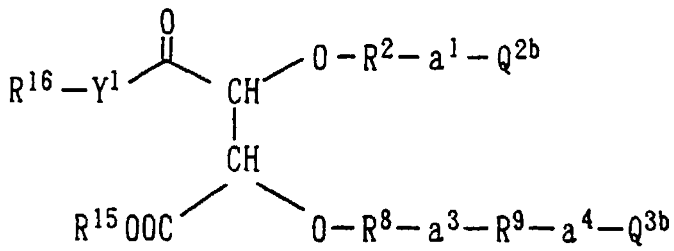

(1 B)

[wherein $R^{15}$ represents a hydrogen atom or an ester residue, $R^{16}$ represents a hydrogen atom or a substituted or unsubstituted hydrocarbon group,

$Q^{2b}$ and $Q^{3b}$ are the same or different and individually represent a substituted or unsubstituted cyclic hydrocarbon group or a substituted or unsubstituted heterocyclic group, and
$Y^1$, $R^2$, $R^8$, $R^9$, $a^1$, $a^3$ and $a^4$ have the same meanings as defined above]; or salt thereof.

**23.** A pharmaceutical which comprises as an effective ingredient a substituted propionyl derivative or salt thereof according to any one of claims 1 to 22.

**24.** A pharmaceutical according to claim 23, which is a remedy • preventive for hypercholesterolemia, hyperlipemia or arteriosclerosis.

**25.** A pharmaceutical composition which comprises a substituted propionyl derivative or salt thereof according to any one of claims 1 to 22 and a pharmaceutically acceptable carrier.

**26.** Use of a substituted propionyl derivative or salt thereof according to any one of claims 1 to 22 as a pharmaceutical.

**27.** Use according to claim 26, wherein the pharmaceutical is a remedy • preventive for hypercholesterolemia, hyperlipemia or arteriosclerosis.

**28.** A method for treating hypercholesterolemia, hyperlipemia or arteriosclerosis, which comprises administering a substituted propionyl derivative or salt thereof according to any one of claims 1 to 22.

# INTERNATIONAL SEARCH REPORT

International application No.
PCT/JP97/04879

A. CLASSIFICATION OF SUBJECT MATTER

Int.Cl[6] C07C62/38, C07C69/716, C07C235/06, C07C235/08, C07C235/10, C07C309/17, C07C311/23, C07C317/18, C07C323/11, C07D319/12,

According to International Patent Classification (IPC) or to both national classification and IPC

B. FIELDS SEARCHED

Minimum documentation searched (classification system followed by classification symbols)

Int.Cl[6] C07C62/38, C07C69/716, C07C235/06, C07C235/08, C07C235/10, C07C309/17, C07C311/23, C07C317/18, C07C323/11, C07D319/12,

Documentation searched other than minimum documentation to the extent that such documents are included in the fields searched

Electronic data base consulted during the international search (name of data base and, where practicable, search terms used)

CA (STN), REGISTRY (STN), WPI (DIALOG)

C. DOCUMENTS CONSIDERED TO BE RELEVANT

| Category* | Citation of document, with indication, where appropriate, of the relevant passages | Relevant to claim No. |
|---|---|---|
| X | CHISATO MUKAI et al., "Highly stereocontrolled total synthesis of (+)-bengamide E", J. Chem. Soc., Perkin Trans. 1 (1995), No. 22, pages 2849-2854 | 1-3 |
| X | W.KEN BUSFIELD et al., "FREE RADICAL INITIATION MECHANISMS IN THE POLYMERISATION OF DIETHYL FUMARATE AND DIETHYL MALEATE STUDIED BY THE NITROXIDE TRAPPING TECHNIQUE", Eur. Polym. J. (1994), Vol. 33, No. 11, pages 1259-1267 | 1-3 |
| X | JP, 3-504500, A (Massachusetts Institute of Technology), October 3, 1991 (03. 10. 91) & US, 5321143, A | 1-3 |
| X | GIUSEPPE CARICATO et al., "Synthesis of $C_2$-Symmetric 1,4-Diketones from Tartaric Acid. A Preliminary Study", Synlett (1994), No. 12, pages 1015-1016 | 1-3 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| March 19, 1998 (19. 03. 98) | March 31, 1998 (31. 03. 98) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP97/04879

C (Continuation). DOCUMENTS CONSIDERED TO BE RELEVANT

| Category* | Citation of document, with indication, where appropriate, of the relevant passages | Relevant to claim No. |
|---|---|---|
| X | OLIVER P. KLEIDERNIGG et al., "The Chemical and Thermal Stability of the Acetamido Group of (R)- and (S)-Atenolol:Synthetic and Chromatographic Studies", Chirality (1994), Vol. 6, No. 5, pages 411-419 | 1-3 |
| X | KEN TAKEBUCHI et al., "Synthesis of the $C_{13-19}$ Unit in the Spiroketal Fragment of Calyculins", Tetrahedron Lett. (1994), Vol. 35, No. 29, pages 5239-5242 | 1-3 |
| X | A. AKELAH et al., "AGRICULTURAL POLYMERS WITH HERBICIDE/FERTILIZER FUNCTION-III. POLYUREAS AND POLY(SCHIFF BASE)S BASED SYSTEMS", Eur. Polym. J. (1993), Vol. 29, No. 8, pages 1041-1045 | 1-3 |
| X | THADDEE GULIK-KRZYWICKI et al., "Electron microscopic study of supramolecular liquid crystalline polymers formed by molecular-recognition-directed self-assembly from complementary chiral components", Proc. Natl. Acad. Sci. U. S. A. (1993), Vol. 90, No. 1, pages 163-167 | 1-3 |
| X | JACEK GAWRONSKI et al., "CONFORMATIONAL DISPARITY OF (R,R)-TARTARIC ACID ESTERS AND AMIDES", Tetrahedron Lett. (1989), Vol. 30, No. 44, pages 6071-6074 | 1-3 |
| X | KEVIN H. BELL, "Selective Aminolysis of Benzoates and Acetates of $\alpha$-Hydroxy Acids and Phenols with Benzylamine and Butan-1-amine", Aust. J. Chem. (1987), Vol. 40, No. 10, pages 1723-1735 | 1-3 |
| X | JP, 3-134092, A (Mitsubishi Petrochemical Co., Ltd.), June 7, 1991 (07. 06. 91) (Family: none) | 22 |
| X | JP, 3-266819, A (Fuji Electric Co., Ltd.), November 27, 1991 (27. 11. 91) (Family: none) | 22 |
| A | EP, 611749, A1 (BANYU PHARMACEUTICAL CO., LTD), February 10, 1994 (10. 02. 94) (Family: none) | 1-25 |

Form PCT/ISA/210 (continuation of second sheet) (July 1992)

INTERNATIONAL SEARCH REPORT

International application No.
PCT/JP97/04879

A. (Continuation) CLASSIFICATION OF SUBJECT MATTER

C07D317/58, C07D317/54, C07D295/20, C07D257/04, C07D263/56, C07D277/62, C07D277/68, C07D207/16, C07F9/38, A61K31/335, A61K31/495, A61K31/19, A61K31/215, A61K31/275, A61K31/18, A61K31/42, A61K31/425, A61K31/40

B. (Continuation) FIELDS SEARCHED

C07D317/58, C07D317/54, C07D295/20, C07D257/04, C07D263/56, C07D277/62, C07D277/68, C07D207/16

Form PCT/ISA/210 (extra sheet) (July 1992)

## INTERNATIONAL SEARCH REPORT

International application No.
PCT/JP97/04879

**Box I Observations where certain claims were found unsearchable (Continuation of item 1 of first sheet)**

This international search report has not been established in respect of certain claims under Article 17(2)(a) for the following reasons:

1. [x] Claims Nos.: 26-28
because they relate to subject matter not required to be searched by this Authority, namely:
Claims 26 to 28 pertain to methods for treatment of the human or animal body by therapy, as well as diagnostic methods, and thus relate to a subject matter which this International Searching Authority is not required, under the provisions of Article 17(2)(a)(i) of the PCT

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.

[ ] No protest accompanied the payment of additional search fees.

Form PCT/ISA/210 (continuation of first sheet (1)) (July 1992)

INTERNATIONAL SEARCH REPORT

International application No.
PCT/JP97/04879

Continuation of Box No. I of continuation of first sheet (1)

and Rule 39.1(iv) of the Regulations under the PCT, to search.

Form PCT/ISA/210 (extra sheet) (July 1992)